(19) Europäisches Patentamt
European Patent Office
Office européen des brevets

(11) **EP 3 029 149 B1**

(12) **EUROPEAN PATENT SPECIFICATION**

(45) Date of publication and mention
of the grant of the patent:
**12.02.2020 Bulletin 2020/07**

(51) Int Cl.:
*C12Q 1/6886* (2018.01)     *G16B 5/00* (2019.01)

(21) Application number: **14196170.6**

(22) Date of filing: **03.12.2014**

(54) **Method of the identification of targets for alternative splicing**

Verfahren zur Identifizierung von Zielmolekülen für alternatives Splicing

Procédé d'identification de cibles pour l'épissage alternatif

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**08.06.2016 Bulletin 2016/23**

(73) Proprietors:
- **Fundació Centre de Regulació Genòmica**
  **08003 Barcelona (ES)**
- **Institució Catalana de Recerca i Estudis Avançats (ICREA)**
  **08010 Barcelona (ES)**

(72) Inventors:
- **Papasaikas, Panagiotis**
  **08003 Barcelona (ES)**
- **Tejedor Vaquero, Juan Ramón**
  **08003 Barcelona (ES)**
- **Valcárcel Juárez, Juan Alberto**
  **08003 Barcelona (ES)**

(74) Representative: **Elzaburu S.L.P.**
**Miguel Angel 21, 2nd floor**
**28010 Madrid (ES)**

(56) References cited:
- **MOORE MICHAEL J ET AL: "An Alternative Splicing Network Links Cell-Cycle Control to Apoptosis", CELL, vol. 142, no. 4, 20 August 2010 (2010-08-20), pages 625-636, XP028931170, ISSN: 0092-8674, DOI: 10.1016/J.CELL.2010.07.019**

- **Panagiotis Papasaikas: "COMPUTATIONAL AND FUNCTIONAL ANALYSES OF SPLICING REGULATION" In: "PhD-Thesis", 1 January 2010 (2010-01-01), Dept. of Biol. Sci.; Carnegie Mellon University, Pittsburgh 2010, XP055189506, pages 1-143, * page 106 "Graphical lasso"; page 107 "Modularity"; page 89 "Reconstruction of a ..."; Fig. 4.2-4.4 ***
- **SOPHIE BONNAL ET AL: "The spliceosome as a target of novel antitumour drugs", NATURE REVIEWS DRUG DISCOVERY, vol. 11, no. 11, 5 November 2012 (2012-11-05), pages 847-859, XP055189513, ISSN: 1474-1776, DOI: 10.1038/nrd3823**
- **MARIA PAOLA PARONETTO ET AL: "Regulation of FAS Exon Definition and Apoptosis by the Ewing Sarcoma Protein", CELL REPORTS, vol. 7, no. 4, 1 May 2014 (2014-05-01), pages 1211-1226, XP055189515, ISSN: 2211-1247, DOI: 10.1016/j.celrep.2014.03.077**
- **MARIAPAOLA PARONETTO ET AL: "The Ewing Sarcoma Protein Regulates DNA Damage-Induced Alternative Splicing", MOLECULAR CELL, CELL PRESS, CAMBRIDGE, MA, US, vol. 43, no. 3, 25 May 2011 (2011-05-25), pages 353-368, XP028276490, ISSN: 1097-2765, DOI: 10.1016/J.MOLCEL.2011.05.035 [retrieved on 2011-06-29]**
- **WOLFGANG J. KÖSTLER ET AL: "Epidermal Growth-Factor - Induced Transcript Isoform Variation Drives Mammary Cell Migration", PLOS ONE, vol. 8, no. 12, 6 December 2013 (2013-12-06), page e80566, XP055189571, DOI: 10.1371/journal.pone.0080566**

- TAZI J ET AL: "Alternative splicing and disease", BIOCHIMICA ET BIOPHYSICA ACTA. MOLECULAR BASIS OF DISEASE, AMSTERDAM, NL, vol. 1792, no. 1, 1 January 2009 (2009-01-01), pages 14-26, XP025838034, ISSN: 0925-4439, DOI: 10.1016/J.BBADIS.2008.09.017 [retrieved on 2008-10-17]

SEQUENCE LISTING

<110> Fundació Centre de Regulació Genòmica (CRG)
Institució Catalana de Recerca i Estudis Avançats (ICREA)

<120> METHOD OF THE IDENTIFICATION OF GENE REGULATION TARGETS

<130> ep1340_i

<160> 46

<170> PatentIn version 3.5

<210> 1
<211> 63
<212> DNA
<213> Homo sapiens

<220>
<221> exon
<222> (1)..(63)
<223> FAS gene

<400> 1

```
gat cca gat cta act tgg ggt ggc ttt gtc ttc ttc ttt tgc caa ttc    48
Asp Pro Asp Leu Thr Trp Gly Gly Phe Val Phe Phe Phe Cys Gln Phe
1               5                   10                  15

cac taa ttg ttt ggg                                                63
His     Leu Phe Gly
            20
```

<210> 2
<211> 116
<212> DNA
<213> Homo sapiens

<220>
<221> exon
<222> (1)..(116)
<223> OLR1 gene

<400> 2

```
gac ttc atc cag caa gca att tcc tat tcc agt ttt cca ttc tgg atg    48
Asp Phe Ile Gln Gln Ala Ile Ser Tyr Ser Ser Phe Pro Phe Trp Met
1               5                   10                  15

ggg ctg tct cgg agg aac ccc agc tac cca tgg ctc tgg gag gac ggt    96
Gly Leu Ser Arg Arg Asn Pro Ser Tyr Pro Trp Leu Trp Glu Asp Gly
            20                  25                  30

tct cct ttg atg ccc cac tt                                         116
Ser Pro Leu Met Pro His
            35
```

<210> 3
<211> 42
<212> DNA
<213> Homo sapiens

<220>
<221> exon
<222> (1)..(42)

<223> PAX6 gene

<400> 3

| acc | cat | gca | gat | gca | aaa | gtc | caa | gtg | ctg | gac | aat | caa | aac | 42 |
|-----|-----|-----|-----|-----|-----|-----|-----|-----|-----|-----|-----|-----|-----|-----|
| Thr | His | Ala | Asp | Ala | Lys | Val | Gln | Val | Leu | Asp | Asn | Gln | Asn | |
| 1 | | | | 5 | | | | | 10 | | | | | |

<210> 4
<211> 62
<212> DNA
<213> Homo sapiens

<220>
<221> exon
<222> (1)..(62)
<223> CHEK2

<400> 4

| cct | tgc | atc | atc | aag | att | aaa | aac | ttt | ttt | gat | gca | gaa | gat | tat | tat | 48 |
|-----|-----|-----|-----|-----|-----|-----|-----|-----|-----|-----|-----|-----|-----|-----|-----|-----|
| Pro | Cys | Ile | Ile | Lys | Ile | Lys | Asn | Phe | Phe | Asp | Ala | Glu | Asp | Tyr | Tyr | |
| 1 | | | | 5 | | | | | 10 | | | | | 15 | | |

| att | gtt | ttg | gaa | tt | 62 |
|-----|-----|-----|-----|-----|-----|
| Ile | Val | Leu | Glu | | |
| | | | 20 | | |

<210> 5
<211> 273
<212> DNA
<213> Homo sapiens

<220>
<221> exon
<222> (1)..(273)
<223> FN1EDB gene

<400> 5

| agg | tgc | ccc | aac | tca | ctg | acc | taa | gct | ttg | ttg | ata | taa | ccg | att | caa | 48 |
|-----|-----|-----|-----|-----|-----|-----|-----|-----|-----|-----|-----|-----|-----|-----|-----|-----|
| Arg | Cys | Pro | Asn | Ser | Leu | Thr | | Ala | Leu | Leu | Ile | | Pro | Ile | Gln | |
| 1 | | | | 5 | | | | | | 10 | | | | | | |

| gca | tcg | gcc | tga | ggt | gga | ccc | cgc | taa | act | ctt | cca | cca | tta | ttg | ggt | 96 |
|-----|-----|-----|-----|-----|-----|-----|-----|-----|-----|-----|-----|-----|-----|-----|-----|-----|
| Ala | Ser | Ala | | Gly | Gly | Pro | Arg | | Thr | Leu | Pro | Pro | Leu | Leu | Gly | |
| 15 | | | | | | 20 | | | | | 25 | | | | | |

| acc | gca | tca | cag | tag | ttg | cgg | cag | gag | aag | gta | tcc | cta | ttt | ttg | aag | 144 |
|-----|-----|-----|-----|-----|-----|-----|-----|-----|-----|-----|-----|-----|-----|-----|-----|-----|
| Thr | Ala | Ser | Gln | | Leu | Arg | Gln | Glu | Lys | Val | Ser | Leu | Phe | Leu | Lys | |
| | 30 | | | | | 35 | | | | | 40 | | | | | |

| att | ttg | tgg | act | cct | cag | tag | gat | act | aca | cag | tca | cag | ggc | tgg | agc | 192 |
|-----|-----|-----|-----|-----|-----|-----|-----|-----|-----|-----|-----|-----|-----|-----|-----|-----|
| Ile | Leu | Trp | Thr | Pro | Gln | | Asp | Thr | Thr | Gln | Ser | Gln | Gly | Trp | Ser | |
| | 45 | | | | | | 50 | | | | | 55 | | | | |

| cgg | gca | ttg | act | atg | ata | tca | gcg | tta | tca | ctc | tca | tta | atg | gcg | gcg | 240 |
|-----|-----|-----|-----|-----|-----|-----|-----|-----|-----|-----|-----|-----|-----|-----|-----|-----|

```
Arg Ala Leu Thr Met Ile Ser Ala Leu Ser Leu Ser Leu Met Ala Ala
    60                  65              70


aga gtg ccc cta cta cac tga cac aac aaa cgg                          273
Arg Val Pro Leu Leu His     His Asn Lys Arg
75                  80
```

<210> 6
<211> 270
<212> DNA
<213> Homo sapiens

<220>
<221> exon
<222> (1)..(270)
<223> FN1EDA gene

<400> 6

```
aca ttg atc gcc cta aag gac tgg cat tca ctg atg tgg atg tcg att      48
Thr Leu Ile Ala Leu Lys Asp Trp His Ser Leu Met Trp Met Ser Ile
1               5               10              15


cca tca aaa ttg ctt ggg aaa gcc cac agg ggc aag ttt cca ggt aca      96
Pro Ser Lys Leu Leu Gly Lys Ala His Arg Gly Lys Phe Pro Gly Thr
            20              25              30


ggg tga cct act cga gcc ctg agg atg gaa tcc atg agc tat tcc ctg     144
Gly     Pro Thr Arg Ala Leu Arg Met Glu Ser Met Ser Tyr Ser Leu
            35              40              45


cac ctg atg gtg aag aag aca ctg cag agc tgc aag gcc tca gac cgg     192
His Leu Met Val Lys Lys Thr Leu Gln Ser Cys Lys Ala Ser Asp Arg
        50              55              60


gtt ctg agt aca cag tca gtg tgg ttg cct tgc acg atg ata tgg aga     240
Val Leu Ser Thr Gln Ser Val Trp Leu Pro Cys Thr Met Ile Trp Arg
        65              70              75


gcc agc ccc tga ttg gaa ccc agt cca cag                             270
Ala Ser Pro     Leu Glu Pro Ser Pro Gln
80                  85
```

<210> 7
<211> 144
<212> DNA
<213> Homo sapiens

<220>
<221> exon
<222> (1)..(144)
<223> NUMB gene

<400> 7

```
cta atg gca ctg act cag cct tcc atg tgc ttg cta agc cag ccc ata      48
Leu Met Ala Leu Thr Gln Pro Ser Met Cys Leu Leu Ser Gln Pro Ile
1               5               10                  15

ctg ctc tag cac ccg tag caa tgc ctg tgc gtg aaa cca acc ctt ggg      96
Leu Leu     His Pro     Gln Cys Leu Cys Val Lys Pro Thr Leu Gly

        20                      25                      30

ccc atg ccc ctg atg ctg cta aca agg aaa ttg cag cca cat gtt cgg     144
Pro Met Pro Leu Met Leu Leu Thr Arg Lys Leu Gln Pro His Val Arg
            35                  40                  45
```

<210> 8
<211> 189
<212> DNA
<213> Homo sapiens

<220>
<221> exon
<222> (1)..(189)
<223> BCL2L1 gene

<400> 8

```
gta gtg aat gaa ctc ttc cgg gat ggg gta aac tgg ggt cgc att gtg      48
Val Val Asn Glu Leu Phe Arg Asp Gly Val Asn Trp Gly Arg Ile Val
1               5               10                  15

gcc ttt ttc tcc ttc ggc ggg gca ctg tgc gtg gaa agc gta gac aag      96
Ala Phe Phe Ser Phe Gly Gly Ala Leu Cys Val Glu Ser Val Asp Lys
        20                      25                      30

gag atg cag gta ttg gtg agt cgg atc gca gct tgg atg gcc act tac     144
Glu Met Gln Val Leu Val Ser Arg Ile Ala Ala Trp Met Ala Thr Tyr
        35                  40                  45

ctg aat gac cac cta gag cct tgg atc cag gag aac ggc ggc tgg         189
Leu Asn Asp His Leu Glu Pro Trp Ile Gln Glu Asn Gly Gly Trp
        50                  55                  60
```

<210> 9
<211> 57
<212> DNA
<213> Homo sapiens

<220>
<221> exon
<222> (1)..(57)
<223> RAC1

<400> 9

```
gtt gga gaa acg tac ggt aag gat ata acc tcc cgg ggc aaa gac aag        48
Val Gly Glu Thr Tyr Gly Lys Asp Ile Thr Ser Arg Gly Lys Asp Lys
1               5                   10                  15

ccg att gcc                                                            57
Pro Ile Ala
```

<210> 10
<211> 147
<212> DNA
<213> Homo sapiens

<220>
<221> exon
<222> (1)..(147)
<223> MST1R gene

<400> 10

```
tat att ggg ctg ggc gct gtg gct gac tgt gtg ggt atc aac gtg acc        48
Tyr Ile Gly Leu Gly Ala Val Ala Asp Cys Val Gly Ile Asn Val Thr
1               5                   10                  15

gtg ggt ggt gag agc tgc cag cac gag ttc cgg ggg gac atg gtt gtc        96
Val Gly Gly Glu Ser Cys Gln His Glu Phe Arg Gly Asp Met Val Val
            20                  25                  30

tgc ccc ctg ccc cca tcc ctg cag ctt ggc cag gat ggt gcc cca ttg       144
Cys Pro Leu Pro Pro Ser Leu Gln Leu Gly Gln Asp Gly Ala Pro Leu
            35                  40                  45

cag                                                                   147
Gln
```

<210> 11
<211> 60
<212> DNA
<213> Homo sapiens

<220>
<221> exon
<222> (1)..(60)
<223> MADD gene

<400> 11

```
tat ttg ggc taa ata ctc taa tgg aga ttg tta ctg aag ccg gcc ccg        48
Tyr Leu Gly     Ile Leu     Trp Arg Leu Leu Leu Lys Pro Ala Pro
1               5                   10

gga gtg gtg aag                                                        60
Gly Val Val Lys
15
```

<210> 12
<211> 61
<212> DNA
<213> Homo sapiens

<220>
<221> exon
<222> (1)..(61)
<223> CASP2 gene

<400> 12

```
gtg cta ttg gat ccc ttg ggc acc tcc ttc tgt tca ctg ctg cca ccg    48
Val Leu Leu Asp Pro Leu Gly Thr Ser Phe Cys Ser Leu Leu Pro Pro
1               5                   10                  15

cct ctc ttg ctc t                                                   61
Pro Leu Leu Leu
            20
```

<210> 13
<211> 129
<212> DNA
<213> Homo sapiens

<220>
<221> exon
<222> (1)..(129)
<223> APAF1 gene

<400> 13

```
ctt ttt gac att cat act agt ggc cta ttg gga gaa atc cac acg ggc    48
Leu Phe Asp Ile His Thr Ser Gly Leu Leu Gly Glu Ile His Thr Gly
1               5                   10                  15

cat cac agc acc atc cag tac tgt gac ttc tcc cca caa aac cat ttg    96
His His Ser Thr Ile Gln Tyr Cys Asp Phe Ser Pro Gln Asn His Leu
            20                  25                  30

gca gtg gtt gct ttg tcc cag tac tgt gta gag                        129
Ala Val Val Ala Leu Ser Gln Tyr Cys Val Glu
        35                  40
```

<210> 14
<211> 111
<212> DNA
<213> Homo sapiens

<220>
<221> exon
<222> (1)..(111)
<223> MINK1 gene

<400> 14

```
acc tcg cag caa ctc cgc ctg gca aat cta tct gca aag gcg ggc aga        48
Thr Ser Gln Gln Leu Arg Leu Ala Asn Leu Ser Ala Lys Ala Gly Arg
1               5               10              15

gcg ggg cac ccc aaa gcc tcc agg gcc ccc tgc tca gcc ccc tgg ccc        96
Ala Gly His Pro Lys Ala Ser Arg Ala Pro Cys Ser Ala Pro Trp Pro
                20              25              30

gcc caa cgc ctc tag                                                    111
Ala Gln Arg Leu
            35
```

<210> 15
<211> 81
<212> DNA
<213> Homo sapiens

<220>
<221> exon
<222> (1)..(81)
<223> MAP3K7 gene

<400> 15

```
cct att cca agc cta aac ggg gcc acc gta aaa ctg ctt cat ttg gca        48
Pro Ile Pro Ser Leu Asn Gly Ala Thr Val Lys Leu Leu His Leu Ala
1               5               10              15

aca ttc tgg atg tcc ctg aga tcg tca tat cag                            81
Thr Phe Trp Met Ser Leu Arg Ser Ser Tyr Gln
            20              25
```

<210> 16
<211> 63
<212> DNA
<213> Homo sapiens

<220>
<221> exon
<222> (1)..(63)
<223> MAP3K7 gene

<400> 16

```
ccc gac agt gat ggt ttt ttg gac agt tca gaa gaa ata tac tac act        48
Pro Asp Ser Asp Gly Phe Leu Asp Ser Ser Glu Glu Ile Tyr Tyr Thr
1               5               10              15

gca aga tct aat ctg                                                    63
Ala Arg Ser Asn Leu
            20
```

<210> 17
<211> 156
<212> DNA
<213> Homo sapiens

<220>

<221> exon
<222> (1)..(156)
<223> NOTCH3 gene

<400> 17

| gcc | cac | gat | gcc | agc | agg | atg | tgg | acg | agt | gtg | ctg | gcc | ccg | cac | cct | 48 |
| Ala | His | Asp | Ala | Ser | Arg | Met | Trp | Thr | Ser | Val | Leu | Ala | Pro | His | Pro | |
| 1 | | | | 5 | | | | | 10 | | | | | 15 | | |

| gtg | gcc | ctc | atg | gta | tct | gca | cca | acc | tgg | cag | gga | gtt | tca | gct | gca | 96 |
| Val | Ala | Leu | Met | Val | Ser | Ala | Pro | Thr | Trp | Gln | Gly | Val | Ser | Ala | Ala | |
| | | | 20 | | | | | 25 | | | | | 30 | | | |

| cct | gcc | atg | gag | ggt | aca | ctg | gcc | ctt | cct | gcg | atc | agg | aca | tca | atg | 144 |
| Pro | Ala | Met | Glu | Gly | Thr | Leu | Ala | Leu | Pro | Ala | Ile | Arg | Thr | Ser | Met | |
| | | 35 | | | | | 40 | | | | | 45 | | | | |

| act | gtg | acc | cca | 156 |
| Thr | Val | Thr | Pro | |
| | 50 | | | |

<210> 18
<211> 132
<212> DNA
<213> Homo sapiens

<220>
<221> exon
<222> (1)..(132)
<223> MAP4K2 gene

<400> 18

| gat | ggc | tcc | cga | ggt | ggc | tgc | tgt | gga | gcg | caa | agg | tgg | cta | caa | tga | 48 |
| Asp | Gly | Ser | Arg | Gly | Gly | Cys | Cys | Gly | Ala | Gln | Arg | Trp | Leu | Gln | | |
| 1 | | | | 5 | | | | | 10 | | | | | 15 | | |

| gct | atg | tga | cgt | ctg | ggc | cct | ggg | cat | cac | tgc | cat | tga | gct | ggg | cga | 96 |
| Ala | Met | | Arg | Leu | Gly | Pro | Gly | His | His | Cys | His | | Ala | Gly | Arg | |
| | | | | | 20 | | | | | 25 | | | | | | |

| gct | gca | gcc | ccc | tct | gtt | cca | cct | gca | ccc | cat | gag | 132 |
| Ala | Ala | Ala | Pro | Ser | Val | Pro | Pro | Ala | Pro | His | Glu | |
| 30 | | | | | 35 | | | | | 40 | | |

<210> 19
<211> 167
<212> DNA
<213> Homo sapiens

<220>
<221> exon
<222> (1)..(167)
<223> PKM2 gene, exon 9

<400> 19

```
ata gct cgt gag gct gag gca gcc atg ttc cac cgc aag ctg ttt gaa        48
Ile Ala Arg Glu Ala Glu Ala Ala Met Phe His Arg Lys Leu Phe Glu
1               5               10              15

gaa ctt gtg cga gcc tca agt cac tcc aca gac ctc atg gaa gcc atg        96
Glu Leu Val Arg Ala Ser Ser His Ser Thr Asp Leu Met Glu Ala Met
            20              25              30

gcc atg ggc agc gtg gag gct tct tat aag tgt tta gca gca gct ttg       144
Ala Met Gly Ser Val Glu Ala Ser Tyr Lys Cys Leu Ala Ala Ala Leu
        35              40              45

ata gtt ctg acg gag tct ggc ag                                        167
Ile Val Leu Thr Glu Ser Gly
    50              55
```

<210> 20
<211> 167
<212> DNA
<213> Homo sapiens

<220>
<221> exon
<222> (1)..(167)
<223> PKM2 gene, exon 10

<400> 20

```
att gcc cgt gag gca gag gct gcc atc tac cac ttg caa tta ttt gag        48
Ile Ala Arg Glu Ala Glu Ala Ala Ile Tyr His Leu Gln Leu Phe Glu
1               5               10              15

gaa ctc cgc cgc ctg gcg ccc att acc agc gac ccc aca gaa gcc acc        96
Glu Leu Arg Arg Leu Ala Pro Ile Thr Ser Asp Pro Thr Glu Ala Thr
            20              25              30

gcc gtg ggt gcc gtg gag gcc tcc ttc aag tgc tgc agt ggg gcc ata       144
Ala Val Gly Ala Val Glu Ala Ser Phe Lys Cys Cys Ser Gly Ala Ile
        35              40              45

atc gtc ctc acc aag tct ggc ag                                        167
Ile Val Leu Thr Lys Ser Gly
    50              55
```

<210> 21
<211> 66
<212> DNA
<213> Homo sapiens

<220>
<221> exon
<222> (1)..(66)
<223> VEGFA gene

<400> 21

```
atg tga caa gcc gag gcg gtg agc cgg gca gga gga agg agc ctc cct     48
Met     Gln Ala Glu Ala Val Ser Arg Ala Gly Gly Arg Ser Leu Pro
1                   5                   10                  15

cag ggt ttc ggg aac cag                                             66
Gln Gly Phe Gly Asn Gln
                20
```

<210> 22
<211> 248
<212> DNA
<213> Homo sapiens

<220>
<221> exon
<222> (1)..(248)
<223> MCL1 gene

<400> 22

```
gca tgc ttc gga aac tgg aca tca aaa acg aag acg atg tga aat cgt     48
Ala Cys Phe Gly Asn Trp Thr Ser Lys Thr Lys Thr Met     Asn Arg
1               5                   10                      15

tgt ctc gag tga tga tcc atg ttt tca gcg acg gcg taa caa act ggg     96
Cys Leu Glu         Ser Met Phe Ser Ala Thr Ala     Gln Thr Gly
                        20                  25

gca gga ttg tga ctc tca ttt ctt ttg gtg cct ttg tgg cta aac act    144
Ala Gly Leu     Leu Ser Phe Leu Leu Val Pro Leu Trp Leu Asn Thr
    30          35                          40

tga aga cca taa acc aag aaa gct gca tcg aac cat tag cag aaa gta    192
    Arg Pro     Thr Lys Lys Ala Ala Ser Asn His     Gln Lys Val
        45              50                          55

tca cag acg ttc tcg taa gga caa aac ggg act ggc tag tta aac aaa    240
Ser Gln Thr Phe Ser     Gly Gln Asn Gly Thr Gly     Leu Asn Lys
            60                      65                      70

gag gct gg                                                          248
Glu Ala
```

<210> 23
<211> 229
<212> DNA
<213> Homo sapiens

<220>
<221> exon
<222> (1)..(229)
<223> CFLAR gene

<400> 23

```
ctt aat cat tct gaa tga tta aat cgt ttc att ttc taa atg tgt tat          48
Leu Asn His Ser Glu     Leu Asn Arg Phe Ile Phe     Met Cys Tyr
1               5                       10

aat gtg ttt agc cct ttc ttg ttg ctg tat gta tga taa cac cct atg          96
Asn Val Phe Ser Pro Phe Leu Leu Leu Tyr Val         His Pro Met
15              20                  25

ccc att gtc ctg atc tga aaa ttc ttg gaa att gtt cca tgt gat taa          144
Pro Ile Val Leu Ile     Lys Phe Leu Glu Ile Val Pro Cys Asp
        30              35                  40

cat gga act gcc tct att aga tgc ttt cca atc ttt tgt tac tac taa          192
His Gly Thr Ala Ser Ile Arg Cys Phe Pro Ile Phe Cys Tyr Tyr
            45              50                  55

taa tgc tat aaa ata aat atc ctt gta ctt ctt tgt g                        229
    Cys Tyr Lys Ile Asn Ile Leu Val Leu Leu Cys
            60              65
```

<210> 24
<211> 35
<212> DNA
<213> Homo sapiens

<220>
<221> exon
<222> (1)..(35)
<223> CASP9 gene, exon 3

<400> 24

```
gtg ctc ttg aga gtt tga ggg gaa atg cag att tg                           35
Val Leu Leu Arg Val     Gly Glu Met Gln Ile
1               5                   10
```

<210> 25
<211> 177
<212> DNA
<213> Homo sapiens

<220>
<221> exon
<222> (1)..(177)
<223> CASP9 gene, exon 4

<400> 25

```
gct tac atc ctg agc atg gag ccc tgt ggc cac tgc ctc att atc aac          48
Ala Tyr Ile Leu Ser Met Glu Pro Cys Gly His Cys Leu Ile Ile Asn
1               5               10                  15

aat gtg aac ttc tgc cgt gag tcc ggg ctc cgc acc cgc act ggc tcc          96
Asn Val Asn Phe Cys Arg Glu Ser Gly Leu Arg Thr Arg Thr Gly Ser
            20              25                  30

aac atc gac tgt gag aag ttg cgg cgt cgc ttc tcc tcg ctg cat ttc         144
Asn Ile Asp Cys Glu Lys Leu Arg Arg Arg Phe Ser Ser Leu His Phe
        35              40                  45

atg gtg gag gtg aag ggc gac ctg act gcc aag                             177
Met Val Glu Val Lys Gly Asp Leu Thr Ala Lys
        50              55
```

<210> 26
<211> 90
<212> DNA
<213> Homo sapiens

<220>
<221> exon
<222> (1)..(90)
<223> CASP9 gene, exon 5

<400> 26

```
aaa atg gtg ctg gct ttg ctg gag ctg gcg cag cag gac cac ggt gct          48
Lys Met Val Leu Ala Leu Leu Glu Leu Ala Gln Gln Asp His Gly Ala
1               5               10                  15

ctg gac tgc tgc gtg gtg gtc att ctc tct cac ggc tgt cag                  90
Leu Asp Cys Cys Val Val Val Ile Leu Ser His Gly Cys Gln
            20              25                  30
```

<210> 27
<211> 148
<212> DNA
<213> Homo sapiens

<220>
<221> exon
<222> (1)..(148)
<223> CASP9 gene, exon 6

<400> 27

```
gcc agc cac ctg cag ttc cca ggg gct gtc tac ggc aca gat gga tgc          48
Ala Ser His Leu Gln Phe Pro Gly Ala Val Tyr Gly Thr Asp Gly Cys
```

```
         1                5                    10                   15

         cct gtg tcg gtc gag aag att gtg aac atc ttc aat ggg acc agc tgc      96
         Pro Val Ser Val Glu Lys Ile Val Asn Ile Phe Asn Gly Thr Ser Cys
                     20                  25                   30

         ccc agc ctg gga ggg aag ccc aag ctc ttt ttc atc cag gcc tgt ggt     144
         Pro Ser Leu Gly Gly Lys Pro Lys Leu Phe Phe Ile Gln Ala Cys Gly
                     35                  40                   45

         ggg g                                                               148
         Gly
```

<210> 28
<211> 69
<212> DNA
<213> Homo sapiens

<220>
<221> exon
<222> (1)..(69)
<223> SYK gene

<400> 28

```
         act tgg tca gcg ggt gga ata atc tca aga atc aaa tca tac tcc ttc      48
         Thr Trp Ser Ala Gly Gly Ile Ile Ser Arg Ile Lys Ser Tyr Ser Phe
         1                5                    10                   15

         cca aag cct ggc cac aga aag                                          69
         Pro Lys Pro Gly His Arg Lys
                     20
```

<210> 29
<211> 132
<212> DNA
<213> Homo sapiens

<220>
<221> exon
<222> (1)..(132)
<223> DIABLO gene

<400> 29

```
         aaa tca gag cct cat tcc ctt agt agt gaa gca ttg atg agg aga gca      48
         Lys Ser Glu Pro His Ser Leu Ser Ser Glu Ala Leu Met Arg Arg Ala
         1                5                    10                   15

         gtg tct ttg gta aca gat agc acc tct acc ttt ctc tct cag acc aca      96
         Val Ser Leu Val Thr Asp Ser Thr Ser Thr Phe Leu Ser Gln Thr Thr
                     20                  25                   30

         tat gcg ttg att gaa gct att act gaa tat act aag                     132
         Tyr Ala Leu Ile Glu Ala Ile Thr Glu Tyr Thr Lys
                     35                  40
```

<210> 30

<211> 98
<212> DNA
<213> Homo sapiens

<220>
<221> exon
<222> (1)..(98)
<223> BMF2 gene

<400> 30

| gca | atg | ctg | gct | atc | ggc | ttc | ctc | tcc | ctg | cca | gtt | tcc | cag | cag | tct | 48 |
| Ala | Met | Leu | Ala | Ile | Gly | Phe | Leu | Ser | Leu | Pro | Val | Ser | Gln | Gln | Ser | |
| 1 | | | | 5 | | | | | 10 | | | | | 15 | | |

| tgc | cca | ttg | ggg | agc | agc | ccc | ccg | aag | ggc | agt | ggc | aac | atc | aag | cag | 96 |
| Cys | Pro | Leu | Gly | Ser | Ser | Pro | Pro | Lys | Gly | Ser | Gly | Asn | Ile | Lys | Gln | |
| | | | 20 | | | | 25 | | | | | 30 | | | | |

| ag | 98 |

<210> 31
<211> 63
<212> DNA
<213> Homo sapiens

<220>
<221> exon
<222> (1)..(63)
<223> BMF3 gene

<400> 31

| gta | cag | att | gcc | cga | aag | ctt | cag | tgc | att | gca | gac | cag | ttc | cac | cgg | 48 |
| Val | Gln | Ile | Ala | Arg | Lys | Leu | Gln | Cys | Ile | Ala | Asp | Gln | Phe | His | Arg | |
| 1 | | | | 5 | | | | | 10 | | | | | 15 | | |

| ctt | cat | gtg | cag | caa | 63 |
| Leu | His | Val | Gln | Gln | |
| | | | 20 | | |

<210> 32
<211> 135
<212> DNA
<213> Homo sapiens

<220>
<221> exon
<222> (1)..(135)
<223> CCNE1 gene

<400> 32

```
gcc ctt aag tgg cgt tta agt ccc ctg act att gtg tcc tgg ctg aat        48
Ala Leu Lys Trp Arg Leu Ser Pro Leu Thr Ile Val Ser Trp Leu Asn
1               5                   10                  15

gta tac atg cag gtt gca tat cta aat gac tta cat gaa gtg cta ctg        96
Val Tyr Met Gln Val Ala Tyr Leu Asn Asp Leu His Glu Val Leu Leu
                20                  25                  30

ccg cag tat ccc cag caa atc ttt ata cag att gca gag               135

                    Pro Gln Tyr Pro Gln Gln Ile Phe Ile Gln Ile Ala Glu
                        35                  40                  45
```

<210> 33
<211> 2974
<212> DNA
<213> Homo sapiens

<220>
<221> exon
<222> (1)..(2974)
<223> CCND1 gene

<400> 33

```
gta  agt  gag  ggt  gat  gtc  cca  ggc  agc  ctt  gcc  ggg  gct  tac  agg  ggg       48
Val  Ser  Glu  Gly  Asp  Val  Pro  Gly  Ser  Leu  Ala  Gly  Ala  Tyr  Arg  Gly
1              5                        10                       15

aga  cac  cta  gtg  cca  cgg  aaa  tgc  cga  ggc  tgg  tgc  caa  ggc  ccc  caa       96
Arg  His  Leu  Val  Pro  Arg  Lys  Cys  Arg  Gly  Trp  Cys  Gln  Gly  Pro  Gln
              20                       25                       30

ggg  tga  caa  ggt  tgg  ggc  tgg  ggc  tgg  gcc  cct  cgg  acc  cca  ggc  cac      144
Gly       Gln  Gly  Trp  Gly  Trp  Gly  Trp  Ala  Pro  Arg  Thr  Pro  Gly  His
                    35                       40                       45

aga  ctg  aca  ggg  cac  cgg  ctt  ctt  cca  ctg  ctc  cta  gaa  ctt  act  gac      192
Arg  Leu  Thr  Gly  His  Arg  Leu  Leu  Pro  Leu  Leu  Leu  Glu  Leu  Thr  Asp
              50                       55                       60

tgg  ctg  gga  ggt  cct  cac  agc  ctt  ctc  acg  tcc  cct  ggg  gct  tcc  agg      240
Trp  Leu  Gly  Gly  Pro  His  Ser  Leu  Leu  Thr  Ser  Pro  Gly  Ala  Ser  Arg
          65                       70                       75

agc  cgt  aga  gtt  tct  ggg  cga  agc  gtc  cgg  gac  gga  ggc  ccc  agg  cgg      288
Ser  Arg  Arg  Val  Ser  Gly  Arg  Ser  Val  Arg  Asp  Gly  Gly  Pro  Arg  Arg
80                       85                       90                       95

ccc  cag  cca  atg  gtc  tgt  gtg  gtg  atg  gtg  tgt  ggg  gtt  agg  ccc  agg      336
Pro  Gln  Pro  Met  Val  Cys  Val  Val  Met  Val  Cys  Gly  Val  Arg  Pro  Arg
               100                      105                      110

cga  gct  ttg  ttt  ggg  cca  caa  tgt  gcg  tgg  cca  ata  aat  aga  tgc  ttg      384
Arg  Ala  Leu  Phe  Gly  Pro  Gln  Cys  Ala  Trp  Pro  Ile  Asn  Arg  Cys  Leu
               115                      120                      125

aaa  agg  gct  cct  gtg  agg  tcc  gag  aca  ccg  gac  aac  ggg  cgg  ata  gag      432
Lys  Arg  Ala  Pro  Val  Arg  Ser  Glu  Thr  Pro  Asp  Asn  Gly  Arg  Ile  Glu
               130                      135                      140

aca  gcc  ttg  ttg  ttt  acg  gcc  tct  ttg  aga  ggc  tgc  tgc  tgt  taa  acc      480
Thr  Ala  Leu  Leu  Phe  Thr  Ala  Ser  Leu  Arg  Gly  Cys  Cys  Cys       Thr
               145                      150                      155

ctg  gga  tga  ctg  tgt  ctt  tct  tct  taa  aaa  tgc  cat  tgt  ttt  att  ccc      528
Leu  Gly       Leu  Cys  Leu  Ser  Ser       Lys  Cys  His  Cys  Phe  Ile  Pro
          160                      165                                170

gag  tct  ttt  ctt  aaa  gaa  aga  att  aaa  atg  aca  atc  aaa  agg  gtt  tgt      576
Glu  Ser  Phe  Leu  Lys  Glu  Arg  Ile  Lys  Met  Thr  Ile  Lys  Arg  Val  Cys
               175                      180                      185
```

```
ggc att tac caa att aga cca gag agg tgg ccg ggt cag ccg ccg gcc          624
Gly Ile Tyr Gln Ile Arg Pro Glu Arg Trp Pro Gly Gln Pro Pro Ala
    190             195             200

ccg cgg tgt gtg agg gag tga ccg cct gac ccc agc ttg ggg ctg ggt          672
Pro Arg Cys Val Arg Glu     Pro Pro Asp Pro Ser Leu Gly Leu Gly
    205             210             215

ggg cct gca aga ccc gtt ttg gct ctg gcc tgg gcc gcc tct tgg tgg          720
Gly Pro Ala Arg Pro Val Leu Ala Leu Ala Trp Ala Ala Ser Trp Trp
    220             225             230             235

tct gcc ctc gag cct ccc ggg gac tcc gca cgg gtc tca gca gat gct          768
Ser Ala Leu Glu Pro Pro Gly Asp Ser Ala Arg Val Ser Ala Asp Ala
                240             245             250

atc tag ggt cca cct gcc tgt ccc ctg cct agt ggt gcc tct gtc ccg          816
Ile     Gly Pro Pro Ala Cys Pro Leu Pro Ser Gly Ala Ser Val Pro
            255             260             265

ggg aca ctg gga gta gcg gct gcc cag ccc atg tgt gtc tcg gaa gag          864
Gly Thr Leu Gly Val Ala Ala Ala Gln Pro Met Cys Val Ser Glu Glu
        270             275             280

gaa gaa gct ttt ttg ccg tgg gac acc gaa gtt ggc agg ggc ctc cct          912
Glu Glu Ala Phe Leu Pro Trp Asp Thr Glu Val Gly Arg Gly Leu Pro
        285             290             295

tct gtg ttc tcg gcc atg gcc tcc ctt gca ccc tgc ccc gtg tta tcc          960
Ser Val Phe Ser Ala Met Ala Ser Leu Ala Pro Cys Pro Val Leu Ser
    300             305             310

ttt ggg ggt ggt gag gtg tcc tca ccc gct gta ggg tgg agg cca gca         1008
Phe Gly Gly Gly Glu Val Ser Ser Pro Ala Val Gly Trp Arg Pro Ala
315             320             325             330

gcc cgc agc tct ctc agg aaa atg gct cag aaa cac cat cga ggc ctc         1056
Ala Arg Ser Ser Leu Arg Lys Met Ala Gln Lys His His Arg Gly Leu
            335             340             345

cag aag ccc agc aaa gag aaa gcc cct cca tca aaa tga aac tcg cgt         1104
Gln Lys Pro Ser Lys Glu Lys Ala Pro Pro Ser Lys     Asn Ser Arg
            350             355             360

ctg cac ttt tca ttt cga act cca cgc cct gag tga aaa ccg ctt ccc         1152
Leu His Phe Ser Phe Arg Thr Pro Arg Pro Glu     Lys Pro Leu Pro
            365             370             375

cgc cag ggg tga ctg ccc tgg gat gtt gct gtc ttc ggg cag ttg tgg         1200
Arg Gln Gly     Leu Pro Trp Asp Val Ala Val Phe Gly Gln Leu Trp
                380             385             390

gaa gtt ggg cgc tgg ccc tta ttt gag tag aga cca tct taa cta gat         1248
Glu Val Gly Arg Trp Pro Leu Phe Glu     Arg Pro Ser     Leu Asp
            395             400                         405

tgg agg cac acg tct cac agc tga cag aca cac ggg gtg aag tta ccc         1296
Trp Arg His Thr Ser His Ser     Gln Thr His Gly Val Lys Leu Pro
            410             415             420

gag gcg gag tcc act ctg cct gat cag cta gtg acc aac gta gct gag         1344
Glu Ala Glu Ser Thr Leu Pro Asp Gln Leu Val Thr Asn Val Ala Glu
```

```
                     425                      430                      435

ccc aga ctc aga aaa acc gtc cac agc aga ggc ccc tgc att ttc tag      1392
Pro Arg Leu Arg Lys Thr Val His Ser Arg Gly Pro Cys Ile Phe
            440                      445                      450

ggc gtg ttc tag aat ttt ctt tgg tgg gtg gaa tgt cca tct gtg caa      1440
Gly Val Phe     Asn Phe Leu Trp Trp Val Glu Cys Pro Ser Val Gln
                455                      460                      465

atc ggg tgc gca gtg cca cac acc agt gac ttt tcg cgg agg agc gtg      1488
Ile Gly Cys Ala Val Pro His Thr Ser Asp Phe Ser Arg Arg Ser Val
            470                      475                      480

ctg cct ttt tgg agc ttc tgg ctg tgg gag aac agc ttt gtc cac cgg      1536
Leu Pro Phe Trp Ser Phe Trp Leu Trp Glu Asn Ser Phe Val His Arg
            485                      490                      495

ggt agc ctt gca ggc agc tgt ggg gcc aga gga atg aag gaa ggt cct      1584
Gly Ser Leu Ala Gly Ser Cys Gly Ala Arg Gly Met Lys Glu Gly Pro
            500                      505                      510

gga gtc tag ctg cat gtg tga ccc tgg agt ggg tca tgg gcg agg gac      1632
Gly Val     Leu His Val     Pro Trp Ser Gly Ser Trp Ala Arg Asp
515                          520                      525

ggg ccg cag gtg aag aat ccc tgg atg gag ctg cca ggc ccc tgg ggc      1680
Gly Pro Gln Val Lys Asn Pro Trp Met Glu Leu Pro Gly Pro Trp Gly
            530                      535                      540

tga gaa ttg aag ctg gct ggt gtt tta ggt tga acg tca gga gtc ttg      1728
    Glu Leu Lys Leu Ala Gly Val Leu Gly     Thr Ser Gly Val Leu
    545                      550                      555

tat ctc acc cca ggc ctc tgg cct cag ttt ccc cat ctg tac agt ggg      1776
Tyr Leu Thr Pro Gly Leu Trp Pro Gln Phe Pro His Leu Tyr Ser Gly
    560                      565                      570

act gtt tgt gca gcc agc ccg gcc agc ttc att tgc cat gat gag aat      1824
Thr Val Cys Ala Ala Ser Pro Ala Ser Phe Ile Cys His Asp Glu Asn
575                      580                      585                  590

tta tct gag ggg cgg gag agg aaa gcc ctc cct ata aag gta cag gcg      1872
Leu Ser Glu Gly Arg Glu Arg Lys Ala Leu Pro Ile Lys Val Gln Ala
                595                      600                      605

cta aaa tgt cgt gac ctc agt ggt cca cct aaa agt cgt tct ggc ctg      1920
Leu Lys Cys Arg Asp Leu Ser Gly Pro Pro Lys Ser Arg Ser Gly Leu
            610                      615                      620

ggt cat cgc ctg tcg tgc tat gcc ttt gtc cag ccc ctt ctg gtt ggg      1968
Gly His Arg Leu Ser Cys Tyr Ala Phe Val Gln Pro Leu Leu Val Gly
            625                      630                      635

agt taa gtg gca cct gtg cgg cac gtg gtg ggg ctg tgg ccc agc cct      2016
Ser     Val Ala Pro Val Arg His Val Val Gly Leu Trp Pro Ser Pro
        640                      645                      650

gct cct tgt gga agg tct gtt tcc tgg gct gcc tag aga ctt ggc ttg      2064
Ala Pro Cys Gly Arg Ser Val Ser Trp Ala Ala     Arg Leu Gly Leu
        655                      660                      665

aag ccc tag cgt ggc ttc ctg gca gtt ggg aca cac aca gcc cca aca      2112
```

```
Lys Pro     Arg Gly Phe Leu Ala Val Gly Thr His Thr Ala Pro Thr
    670             675             680

cat gga gcc ggt tct cca tcc aga agc ccc cgg gca gta agc agc cac    2160
His Gly Ala Gly Ser Pro Ser Arg Ser Pro Arg Ala Val Ser Ser His
    685             690             695

ttc agg ctg cgt ggg act tgc ccg tgg tgg agc cta gga gag gcc cct    2208
Phe Arg Leu Arg Gly Thr Cys Pro Trp Trp Ser Leu Gly Glu Ala Pro
700             705             710             715

ggc tgg gcg tgg cgt tcc aga ttt cac ggc tgc tct ttc cca ctg aca    2256
Gly Trp Ala Trp Arg Ser Arg Phe His Gly Cys Ser Phe Pro Leu Thr
                720             725             730

gtg tgg tgt gga cgc tgc caa ggg agt ctg gag ccc cag agg gtg gag    2304
Val Trp Cys Gly Arg Cys Gln Gly Ser Leu Glu Pro Gln Arg Val Glu
            735             740             745

gtg cag gac ttc cag gag cgt ccg tcg cac tcc acc cga ggg cga gca    2352
Val Gln Asp Phe Gln Glu Arg Pro Ser His Ser Thr Arg Gly Arg Ala
        750             755             760

cct cag tgg ccg cag tgg gtg gat gca tgc tgt gcc agg ctg atg gct    2400
Pro Gln Trp Pro Gln Trp Val Asp Ala Cys Cys Ala Arg Leu Met Ala
        765             770             775

ggc ccc ggg gca cag gcc tga gcg gga gag gat gga ggg gag gga tca    2448
Gly Pro Gly Ala Gln Ala     Ala Gly Glu Asp Gly Gly Glu Gly Ser
780             785             790

atg gtc cag gtc ccc ctg gcc acc cag cat tca tcc tca gtc atg cac    2496
Met Val Gln Val Pro Leu Ala Thr Gln His Ser Ser Ser Val Met His
795             800             805             810

ggc cca agg ctt cga cag cca ttg atc atg gaa ggc cag gtt cac ctc    2544
Gly Pro Arg Leu Arg Gln Pro Leu Ile Met Glu Gly Gln Val His Leu
            815             820             825

aag ggc tgc cac atg gag agg tta agt ctg aaa agg ctg aaa agg cag    2592
Lys Gly Cys His Met Glu Arg Leu Ser Leu Lys Arg Leu Lys Arg Gln
            830             835             840

ggt tca aag ggc ctc ctg tcc aga tca gat ggc act gaa ttc ccc agg    2640
Gly Ser Lys Gly Leu Leu Ser Arg Ser Asp Gly Thr Glu Phe Pro Arg
            845             850             855

gag ctg gca cgg cca gtg gga aca ggc ggt gaa ggc gct gtt gga cat    2688
Glu Leu Ala Arg Pro Val Gly Thr Gly Gly Glu Gly Ala Val Gly His
        860             865             870

ggg gac ggg cag ggg gtg tgc agg gtg ggc ggg caa gca tct ggt gtc    2736
Gly Asp Gly Gln Gly Val Cys Arg Val Gly Gly Gln Ala Ser Gly Val
875             880             885             890

ttg tgg ctc cag aga cca ggt ggg agg tgg agg cat ttg gtc ctg agt    2784
Leu Trp Leu Gln Arg Pro Gly Gly Arg Trp Arg His Leu Val Leu Ser
            895             900             905

gtc ctg aca ggt gat ggc agc tcc cac atc tcg ctc agg ttc aga gga    2832
Val Leu Thr Gly Asp Gly Ser Ser His Ile Ser Leu Arg Phe Arg Gly
        910             915             920
```

90

| ggc | agc | atg | ggc | cga | ggg | aca | gtt | ttg | gct | tag | tct | tgc | tct | tat | aaa | 2880 |
| Gly | Ser | Met | Gly | Arg | Gly | Thr | Val | Leu | Ala | | Ser | Cys | Ser | Tyr | Lys | |
| | | 925 | | | | | 930 | | | | | | 935 | | | |

| ggc | ttc | cgg | gtc | atg | gca | cct | ggg | aag | ggg | ccc | tcg | ctg | cag | gcc | cct | 2928 |
| Gly | Phe | Arg | Val | Met | Ala | Pro | Gly | Lys | Gly | Pro | Ser | Leu | Gln | Ala | Pro | |
| | | 940 | | | | | 945 | | | | | 950 | | | | |

| tct | aag | gac | ccc | ctc | ttc | cca | cct | ctc | ccc | acc | ctc | tct | ctc | tca | g | 2974 |
| Ser | Lys | Asp | Pro | Leu | Phe | Pro | Pro | Leu | Pro | Thr | Leu | Ser | Leu | Ser | | |
| | 955 | | | | | 960 | | | | | 965 | | | | | |

<210> 34
<211> 100
<212> DNA
<213> Homo sapiens

<220>
<221> exon
<222> (1)..(100)
<223> H2AFYgene, exon 6

<400> 34

| ctg | aac | ctt | att | cac | agt | gaa | atc | agt | aat | tta | gcc | ggc | ttt | gag | gtg | 48 |
| Leu | Asn | Leu | Ile | His | Ser | Glu | Ile | Ser | Asn | Leu | Ala | Gly | Phe | Glu | Val | |
| 1 | | | | 5 | | | | | 10 | | | | | 15 | | |

| gag | gcc | ata | atc | aat | cct | acc | aat | gct | gac | att | gac | ctt | aaa | gat | gac | 96 |
| Glu | Ala | Ile | Ile | Asn | Pro | Thr | Asn | Ala | Asp | Ile | Asp | Leu | Lys | Asp | Asp | |
| | | | 20 | | | | | 25 | | | | | 30 | | | |

| cta | g | | | | | | | | | | | | | | | 100 |
| Leu | | | | | | | | | | | | | | | | |

<210> 35
<211> 91
<212> DNA
<213> Homo sapiens

<220>
<221> exon
<222> (1)..(91)
<223> H2AFY gene, exon 6'

<400> 35

| ttg | caa | gtt | gta | cag | gct | gac | att | gcc | tcg | atc | gac | agt | gat | gct | gtc | 48 |
| Leu | Gln | Val | Val | Gln | Ala | Asp | Ile | Ala | Ser | Ile | Asp | Ser | Asp | Ala | Val | |
| 1 | | | | 5 | | | | | 10 | | | | | 15 | | |

| gtt | cac | ccg | aca | aac | act | gac | ttc | tac | atc | ggt | ggt | gaa | gta | g | | 91 |
| Val | His | Pro | Thr | Asn | Thr | Asp | Phe | Tyr | Ile | Gly | Gly | Glu | Val | | | |
| | | 20 | | | | | 25 | | | | | 30 | | | | |

<210> 36
<211> 50
<212> DNA

<213> Homo sapiens

<220>
<221> exon
<222> (1)..(50)
<223> STAT3 gene

<400> 36

```
aac gac ctg cag caa tac cat tga cct gcc gat gtc ccc ccg cac ttt    48
Asn Asp Leu Gln Gln Tyr His     Pro Ala Asp Val Pro Pro His Phe
1               5                    10                      15

ag                                                                  50
```

<210> 37
<211> 102
<212> DNA
<213> Homo sapiens

<220>
<221> exon
<222> (1)..(102)
<223> GADD45A, exon 2

<400> 37

```
gat gga taa ggt ggg gga tgc cct gga gga agt gct cag caa agc cct    48
Asp Gly     Gly Gly Gly Cys Pro Gly Gly Ser Ala Gln Gln Ser Pro
1               5                    10                      15

gag tca gcg cac gat cac tgt cgg ggt gta cga agc ggc caa gct gct    96
Glu Ser Ala His Asp His Cys Arg Gly Val Arg Ser Gly Gln Ala Ala
                20              25                  30

caa cgt                                                            102
Gln Arg
```

<210> 38
<211> 238
<212> DNA
<213> Homo sapiens

<220>
<221> exon
<222> (1)..(238)
<223> GADD45A gene, exon 3

<400> 38

```
cga ccc cga taa cgt ggt gtt gtg cct gct ggc ggc gga cga gga cga        48
Arg Pro Arg     Arg Gly Val Val Pro Ala Gly Gly Gly Arg Gly Arg
1               5               10                  15

cga cag aga tgt ggc tct gca gat cca ctt cac cct gat cca ggc gtt        96
Arg Gln Arg Cys Gly Ser Ala Asp Pro Leu His Pro Asp Pro Gly Val
                20                  25                  30

ttg ctg cga gaa cga cat caa cat cct gcg cgt cag caa ccc ggg ccg       144
Leu Leu Arg Glu Arg His Gln His Pro Ala Arg Gln Gln Pro Gly Pro
            35                  40                  45

gct ggc gga gct cct gct ctt gga gac cga cgc tgg ccc cgc ggc gag       192
Ala Gly Gly Ala Pro Ala Leu Gly Asp Arg Arg Trp Pro Arg Gly Glu
            50                  55                  60

cga ggg cgc cga gca gcc ccc gga cct gca ctg cgt gct ggt gac g         238
Arg Gly Arg Arg Ala Ala Pro Gly Pro Ala Leu Arg Ala Gly Asp
        65                  70                  75
```

<210> 39
<211> 180
<212> DNA
<213> Homo sapiens

<220>
<221> Intron
<222> (1)..(180)
<223> BCL2L11 gene, intron

<400> 39

```
gtaatcctga aggcaatcac ggaggtgaag gggacagctg cccccacggc agccctcagg        60

gcccgctggc cccacctgcc agccctggcc cttttgctac cagatccccg cttttcatct       120

ttatgagaag atcctccctg ctgtctcgat cctccagtgg gtatttctct tttgacacag       180
```

<210> 40
<211> 90
<212> DNA
<213> Homo sapiens

<220>
<221> exon
<222> (1)..(90)
<223> BCL2L11 gene, exon 4

<400> 40

```
aca gga gcc cag cac cca tga gtt gtg aca aat caa cac aaa ccc caa        48
Thr Gly Ala Gln His Pro     Val Val Thr Asn Gln His Lys Pro Gln
1               5               10                  15

gtc ctc ctt gcc agg cct tca acc act atc tca gtg caa tgg                90
Val Leu Leu Ala Arg Pro Ser Thr Thr Ile Ser Val Gln Trp
            20                  25
```

<210> 41
<211> 104
<212> DNA
<213> Homo sapiens

<220>
<221> exon
<222> (1)..(104)
<223> BCL2L11 gene, exon 5

<400> 41

```
ctt cca tga ggc agg ctg aac ctg cag ata tgc gcc cag aga tat gga        48

Leu Pro     Gly Arg Leu Asn Leu Gln Ile Cys Ala Gln Arg Tyr Gly
1                   5               10                  15

tcg ccc aag agt tgc ggc gta ttg gag acg agt tta acg ctt act atg        96
Ser Pro Lys Ser Cys Gly Val Leu Glu Thr Ser Leu Thr Leu Thr Met
                20                  25                  30

caa gga gg                                                             104
Gln Gly
```

<210> 42
<211> 69
<212> DNA
<213> Homo sapiens

<220>
<221> exon
<222> (1)..(69)
<223> BIRC5 gene, exon 2b

<400> 42

```
tgg gcc ggg cac ggt ggc tta cgc ctg taa tac cag cac ttt ggg agg        48
Trp Ala Gly His Gly Gly Leu Arg Leu     Tyr Gln His Phe Gly Arg
1                   5                   10                  15

ccg agg cgg gcg gat cac gag                                            69
Pro Arg Arg Ala Asp His Glu
                20
```

<210> 43
<211> 118
<212> DNA
<213> Homo sapiens

<220>
<221> exon
<222> (1)..(118)
<223> BIRC5 gene, exon 3

<400> 43

| aga | gga | aca | taa | aaa | gca | ttc | gtc | cgg | ttg | cgc | ttt | cct | ttc | tgt | caa | 48 |
| Arg | Gly | Thr | | Lys | Ala | Phe | Val | Arg | Leu | Arg | Phe | Pro | Phe | Cys | Gln | |
| 1 | | | | 5 | | | | | 10 | | | | | | 15 | |

| gaa | gca | gtt | tga | aga | att | aac | cct | tgg | tga | att | ttt | gaa | act | gga | cag | 96 |
| Glu | Ala | Val | | Arg | Ile | Asn | Pro | Trp | | Ile | Phe | Glu | Thr | Gly | Gln | |
| | | | | | 20 | | | | | 25 | | | | | | |

| aga | aag | agc | caa | gaa | caa | aat | t | | | | | | | | | 118 |
| Arg | Lys | Ser | Gln | Glu | Gln | Asn | | | | | | | | | | |
| 30 | | | | | 35 | | | | | | | | | | | |

<210> 44
<211> 165
<212> DNA
<213> Homo sapiens

<220>
<221> exon
<222> (1)..(165)
<223> BIRC5 gene, exon 3b

<400> 44

| gag | aga | gct | ctg | tta | gca | gaa | tga | aaa | aat | tgg | aag | cca | gat | tca | ggg | 48 |
| Glu | Arg | Ala | Leu | Leu | Ala | Glu | | Lys | Asn | Trp | Lys | Pro | Asp | Ser | Gly | |
| 1 | | | | 5 | | | | | 10 | | | | | | 15 | |

| agg | gac | tgg | aag | caa | aag | aat | ttc | tgt | tcg | agg | aag | agc | ctg | atg | ttt | 96 |
| Arg | Asp | Trp | Lys | Gln | Lys | Asn | Phe | Cys | Ser | Arg | Lys | Ser | Leu | Met | Phe | |
| | | | | 20 | | | | | 25 | | | | | 30 | | |

| gcc | agg | gtc | tgt | tta | act | gga | cat | gaa | gag | gaa | ggc | tct | gga | ctt | tcc | 144 |
| Ala | Arg | Val | Cys | Leu | Thr | Gly | His | Glu | Glu | Glu | Gly | Ser | Gly | Leu | Ser | |
| | | | 35 | | | | | 40 | | | | | 45 | | | |

| tcc | agg | agt | ttc | agg | aga | aag | | | | | | | | | | 165 |
| Ser | Arg | Ser | Phe | Arg | Arg | Lys | | | | | | | | | | |
| | | | 50 | | | | | | | | | | | | | |

<210> 45
<211> 54
<212> DNA
<213> Homo sapiens

<220>
<221> exon
<222> (1)..(54)
<223> SMN1 gene

<400> 45

| ggt | ttc | aga | caa | aat | caa | aaa | gaa | gga | agg | tgc | tca | cat | tcc | tta | aat | 48 |
| Gly | Phe | Arg | Gln | Asn | Gln | Lys | Glu | Gly | Arg | Cys | Ser | His | Ser | Leu | Asn | |
| 1 | | | | 5 | | | | | 10 | | | | | | 15 | |

| taa | gga | | | | | | | | | | | | | | | 54 |
| | Gly | | | | | | | | | | | | | | | |

<210> 46
<211> 54
<212> DNA
<213> Homo sapiens

<220>
<221> exon
<222> (1)..(54)
<223> SMN2

<400> 46

```
ggt ttt aga caa aat caa aaa gaa gga agg tgc tca cat tcc tta aat       48
Gly Phe Arg Gln Asn Gln Lys Glu Gly Arg Cys Ser His Ser Leu Asn
1               5                   10                  15

taa gga                                                               54
    Gly
```

**Description**

**FIELD OF THE INVENTION**

The present invention relates to a method for identifying functional associations between regulators of alternative splicing (AS), or between a particular splicing regulator involved in a disease and a compound that will become a candidate to drug against said disease, from a network of functional interactions among factors involved in intron removal itself and/or in the regulation of either the activity of the splicing machinery or the selection among alternative patterns of intron removal (alternative splicing), which method is based on the previous construction of the network by applying a combination of algorithms to a collection of data related to alternative splicing.

**BACKGROUND OF THE INVENTION**

Removal of introns from mRNA precursors (pre-mRNA splicing) is an essential step for the generation of functional mRNAs in eukaryotes. Alternative patterns of intron removal (alternative splicing, AS) expand the coding potential of the genome and allow versatile regulation of gene expression in multicellular organisms. Most human transcripts undergo AS, thus representing an important mechanism for the generation of proteome diversity (Nilsen and Graveley, 2010). The process is tightly regulated to set up specific AS programs across different tissues or during development (Barbosa-Morais et al., 2012; Merkin et al., 2012).

Pre-mRNA splicing is carried out by the spliceosome, one of the most complex molecular machineries of the cell, composed of 5 small nuclear ribonucleoprotein particles (U1, U2, U4/5/6 snRNP) and about 150 additional polypeptides (reviewed by Wahl et al., 2009). Detailed biochemical studies using a small number of model introns have delineated a sequential pathway for the assembly of spliceosomal subcomplexes. For example, U1 snRNP recognizes the 5' splice site, and U2AF -a heterodimer of 35 and 65KDa subunits- recognizes sequences at the 3' end of introns. U2AF binding helps to recruit U2 snRNP to the upstream branch point sequence, forming complex A. U2 snRNP binding involves interactions of pre-mRNA sequences with U2 snRNA as well as with U2 proteins (e.g. SF3B1). Subsequent binding of pre-assembled U4/5/6 tri-snRNP forms complex B, which after a series of conformational changes forms complexes Bact and C, concomitant with the activation of the two catalytic steps that generate splicing intermediates and products. Transition between spliceosomal subcomplexes involves profound dynamic changes in protein composition as well as extensive rearrangements of base pairing interactions between snRNAs and between snRNAs and splice site sequences (Wahl et al., 2009). RNA structures contributed by base pairing interactions between U2 and U6 snRNAs serve to coordinate metal ions critical for splicing catalysis (Fica et al., 2013), implying that the spliceosome is an RNA enzyme whose catalytic center is only established upon assembly of its individual components.

Then, it can be considered that the major splicing apparatus (spliceosome), as summarized by Cooper et al. (Cooper et al., 2009), is composed of a core with 5 small nuclear ribonucleinprotein complexes (snRNPs), each containing one or two snRNAs (U1, U2, U4/U6, and U5), and numerous protein factors. These splicing factors include RNA-binding proteins (e.g., U2AF, SF1 and SRF) and different enzymes (helicases/RNPases, kinases, phosphastases, etc.). These modulate the structure and the orderly stepwise associations, dissociations and conformational transitions of the pre-mRNA, snRNAs and protein complexes to facilitate the splicing reaction, proofreading and substrate release. Each snRNP, except U6 and U6atac, have in common a stable seven-membered ring of Sm proteins (the Sm core), as well as several snRNA-specific proteins.

The pre-mRNAs elements that define the splicing junctions are bound by RNA-binding proteins, which are some of the factors involved in the splicing process which are not part of the spliceosome itself. RNA-binding proteins have either positive (primarily SR proteins: serinearginine-rich domain containing proteins) or negative (primarily hnRNP proteins) effects on spliceosome assembly in their vicinity to maintain appropriate constitutive splicing or to regulate alternative splicing. The repertoire of mRNA binding proteins is unique to each cell type and thus the expression and activity of them is critical for normal alternative splicing and polyadenylation.

Upon splicing, additional proteins are acquired at the splice junctions (the exon junction complex, EJC), numerous proteins are removed, but other ones remain bound to the mRNA and shuttle with it to the cytoplasm, where they also have roles in mRNA translation, stability and localization in the cytoplasm, together with other proteins and molecules, such as microRNAs. The large number of proteins and regulatory RNAs in post-transcriptional RNA processing and the enormously intricate network of interactions among them provide cells with exquisite capacity to fine tune their transcriptome and rapidly adjust their proteome in response to stimuli, but it also increases exposure to mutations and extends their vulnerability to mis-regulation that causes numerous diseases.

Differential selection of alternative splice sites in a pre-mRNA (alternative splicing, AS) is a prevalent mode of gene regulation in multicellular organisms, often subject to developmental regulation (Nilsen and Graveley, 2010) and frequently altered in disease (Cooper et al., 2009; Bonnal et al., 2012). Substantial efforts made to dissect mechanisms of AS regulation on a relatively small number of pre-mRNAs have provided a consensus picture in which protein factors recognizing cognate auxiliary sequences in the pre-mRNA promote or inhibit early events in spliceosome assembly (Fu and Manley, 2014). As commented above, these regulatory factors include members of the hnRNP and SR protein families, which often display cooperative or antagonistic functions depending on the position of their binding sites relative to the regulated splice sites. Despite important progress (Barash et al., 2010; Zhang et al., 2010), the combinatorial nature of these contributions complicates the formulation of integrative models for AS regulation and its relation with physiological regulation, with the activity of other cellular pathways and, particularly, with the etiology and development of some diseases.

As reviewed by Cooper et al. (Cooper et al., 2009), given that splicing depends on a complex core, numerous RNA-binding proteins and an enormously intricate network of interactions among them, there is a high probability of mutations that disrupt any of the components of RNPs, either RNAs or proteins, or the factors required for their assembly and that can be deleterious to cells and cause disease. The complexity increases when one considers not only the number of splicing events, sometimes large, that are required to produce an mRNA, but also the number of splicing options, sometimes vast, exhibited by some pre-mRNA, that allow to obtain many different alternative splicing variants from certain pre-mRNA, each of them often exhibiting different tissue-expression pattern, functioning and/or regulation. With this enormous increase in complexity comes an increased susceptibility to malfunction. Indeed, a large number of human diseases result from mutations or mis-regulation of the splicing process, particularly neuromuscular and neurodegenerative diseases and cancer.

Thus, defects on pre-mRNA splicing have emerged as a common disease-causing mechanism underlying many human genetic ailments. Some diseases result from mutations that affect the splicing of the transcript produced from the same gene (in *cis*), while many others result from mutations in the splicing machinery and the regulatory proteins that affect splicing of other transcripts (in *trans*).

For instance, it is now known that genes encoding splicing factors, including the drug target splicing factor 3B subunit 1 (SF3B1), are among the most highly mutated in various haematological malignancies such as chronic lymphocytic leukaemia and myelodysplastic syndromes, highlighting the role of splicing factors in cancers. Consequently, alteration of the alternative splicing pattern of different pre-mRNAs has been often associated with different cancer diseases and seems to be even an important factor in the stimulation of tumor growth. This is the case, for example, of Fas/CD95 receptor, which exhibits different isoforms resulting from alternative splicing, which isoforms have different roles in apoptosis: Fas/CD95 exon 6 encodes a trans-membrane domain, and skipping of this exon leads to an mRNA encoding a soluble form of the receptor that can act as a decoy and prevent cell death (Cheng et al., 1994; Cascino et al., 1995; Liu et al., 1995; Papoff et al., 1996:). A switch in isoforms from soluble to membrane-bound occurs during T lymphocyte activation and miss-regulation of this switch is associated with Autoimmune Lymphoproliferative syndrome (Roesler et al., 2005).

The discovery of disease-causing mutations in RNAs is yielding a range of therapeutic targets. But developing therapeutics requires a deeper understanding of RNA biology and chemistry and, particularly, a better understanding of the factors involved in the regulation of splicing and their contribution to alternative splicing, as well as the development of appropriate tools for handling the intricate network of interactions among them.

Genome-wide and high-throughput content screenings have emerged as powerful tools for the identification of novel regulators of a variety of cellular processes, including cell cycle progression or cell death. Few genome-scale screens for the identification of novel splicing regulators in mammalian cells have been reported. Using a luciferase-based reporter minigene and a cDNA expression screen, Warzecha et al. (2009) identified ESRP1 and ESRP2 as epithelial-specific splicing regulators governing the splicing of the FGFR2 receptor. Using dual fluorescent reporters, a gain-of-function cDNA screen identified novel regulators of post-synaptic density protein 95 (Psd-95) AS (Zheng et al., 2013).

Moore et al. (2010) employed fluorescent reporters to carry out a genome-scale siRNA screen to identify novel regulators of AS of two apoptotic genes, BCL-X and MCL-1. They proposed a network of regulatorsthat modulate the mentioned two splicing events based upon previous knowledge of physical interactions involving these proteins, which allowed the authors to propose mutual influences between the processes of splicing and cell cycle control. The mentioned network does not allow deriving from it any statistically meaningful functional relationships between a high number of factors such as the complete set of components of the spliceosome.

Despite their proved value, artificial reporter systems may fail to capture aspects of physiological regulation of AS, including those linked to the complex interplay between chromatin and splicing at endogenous gene loci or those affected by overexpression of fusion transcripts. Large content genome-wide screens based on endogenous transcript measurements have been technically challenging and only one high-content mutational screen in yeast identified novel splicing regulators for endogenous target genes (Albulescu et al., 2012).

The use of software programs and methodologies based on algorithms for handling large amounts of biological data, including databases, comparing the obtained data or register in databases, establishing differences and similarities with data introduced by the user and, even, deducing common patterns or relations between different molecules, structural motifs or metabolic pathways, is becoming increasingly common in Biotech research. The platforms and applications based on such algorithms and implementing their practical applications are becoming indispensable tools for deciding assay reagents or adjuvants, such as primers, probes o siRNAs, or for preselecting possible target assays, being complementary to or even replacing some traditional research reagents or methodologies. The use of tool resource web pages such as that of NCBI (http://blast.ncbi.nlm.nih.gov/Blast.cgi), where programs and tools based on the implementation of the BLAST algorithm can be accessed freely for establishing or ruling out nucleotide sequences homologies, has opened the door to many different applications that are not always of free access but that innovative industries do not hesitate to pay for, due to their valuable applicability as predictive, analytical or comparative research tools: Ingenuity, for gene ontology/pathway analysis (http://www.ingenuity.com); CLC Genomics Workbench, for analyzing RNA sequence data (http://www.clcbio.com/blog/clc-genomics-workbench-7-0/); FoldX, for ab initio prediction of protein folding (http://foldx.crg.es); Dnastar Lasergene, for protein sequence analysis visualization and structure prediction (http://www.dnastar.com/); Geneious, for High Throughput Sequencing Analysis (http://www.geneious.com/), or SYBYL®-X Suite, for drug design and molecular modeling (http://www.certara.com/products/molmod/svbvl-x).

The PhD Thesis of Panagiotis Papasaikas, "Computational and Functional Analysis of Splicing Regulations" (Dept. of Biol. Sic.; Carnegie Mellon University, Pittsburgh 2010, pages 1-143) is an example of the use of software tools available in Internet that enables the application of algorithms for facilitating the handling and presentation of large amounts of data and inferring functional associations. It details the application of network analysis methods for the reconstruction of a network of exons based on their co-regulation during Drosophila development. In said work, splicing regulators (specifically, RNA binding proteins) are associated to specific exons or exon modules (groups of co-regulated exons) according to similarity between their expression patterns and the pattern of splicing regulation. The data used are transcriptome quantifications from high-throughput experiments (e.g. whole genome tiling-arrays) at different timepoints during development. Said work established a method to infer relationships through the combination of robust correlation measurements and regularization-based algorithms for graphical model selection, modularity and network reconstruction such as Graphical lasso or Pearson's correlation. But it did not attempt to identify or model functional interactions among splicing regulators; it does not even allow such type or analysis, because the input data are not suitable for it, since there is no information, for instance, on the effects of perturbing the concentration of the splicing regulators, or about how to modify the method of the mentioned work for a different purpose.

Taking into account the large amount of factors that seem to be involved in alternative splicing regulation and the associated difficulties for establishing and, particularly, handling their network of interactions and inferring associations with research interest and/or practical application such as possible targets for disease therapy or candidates to drugs that interact with said targets, it would be very useful to develop a methodology, based on algorithms and their computer implementation, that would facilitate the identification of associations between factors involved in general or specific AS regulation, in the connection with particular metabolic pathways, in the response to internal or external perturbation such as the administration of drugs or other compounds and, from that information, selecting targets for drugs intended to ameliorate diseases associated to alternative splicing or selecting compounds having such factors as targets that could be considered candidates to drugs for the treatment of such disease or for ameliorating some of their symptoms or even for identifying common features among the factors associated to a particular one that might be used as target for compounds trying to modulate such feature.

However, such approach has not ever been reported for analysing alternative splicing regulation, nor has ever been suggested for inferring data such as interactions among factors previously not known or possible new targets of drugs having an influence on alternative splicing. And, particularly, it had not been suggested the basic criteria upon which basing the analysis of splicing events and the comparison of factors involved in their regulation for developing such a methodology.

The present invention provides a solution to such problems.

## SUMMARY OF THE INVENTION

The present invention refers to a method for identifying targets for the modulation of alternative splicing, which comprises the steps of:

a) generating a network of functional interactions among factors involved in splicing regulation by a method that comprises the steps of:

i) perturbing the alternative splicing conditions in a group of cells by submitting each cell to at least one perturbing stimulus which consists of inhibiting the expression of a multiplicity of genes whose products are involved in splicing regulation, each gene having been selected from one of the following groups:

a. components of the spliceosome core,
b. auxiliary factors which are part of the splicing machinery and that are not components of the spliceosome core,

c. factors involved in mRNA processing that are not part of the splicing machinery,

d. genes involved in modulating chromatin structure,

and/or any other change of the condition of the cell;

ii) selecting a multiplicity of alternative splicing events and assessing for each event the impact of inhibiting each one of the multiplicity of inhibited genes, or any other perturbation of the cell condition, on the use of alternative splicing sites, by calculating said impact as the inclusion percentage of the exon that can be excluded or included upon inhibition of the gene or actuation of the perturbing stimulus;

iii) generating a perturbation profile of alternative splicing events for each particular inhibited gene or perturbing stimulus;

iv) deriving a network that models functional interconnections of splicing perturbations based on the effect of each perturbing stimulus on alternative splicing events by performing the steps of:

a. preprocessing and reducing the obtained data of the impact of each perturbation in each selected alternative splicing event by a process which involves:

- removing sparse variables and imputing missing values,
- removing uninformative events,
- scaling data by standardization,

b. carrying out a robust estimation of covariance of perturbation profiles of alternative splicing events for each possible pair of perturbing stimulus,

c. constructing the network basing it on the graphical lasso model selection algorithm;

b) identifying as a target for alternative splicing regulation any perturbing stimulus that corresponds to a node of the network that is connected with another node that corresponds to a factor that is known to have an effect on the event or events of alternative splicing whose modulation is sought;

wherein the impact on the use of alternative splicing sites resulting from inhibiting each one of the multiplicity of inhibited genes, or from any other perturbation of the cell condition, is assessed for at least 35 alternative splicing events, and wherein

for carrying out step iii), the inclusion percentage of each exon is transformed in its corresponding Z-score before generating the perturbation profile of alternative splicing events for each particular inhibited gene or perturbing stimulus;

in step iv)a., data are preprocessed and reduced before deriving the network by:

- removing the data corresponding to inhibited genes or any other perturbing stimulus for which it has not been obtained a result for the impact of knocking them down for more than a half of the set of the analyzed alternative splicing events, for considering the knockdown of said gene a sparse variable,
- filling-in the remaining missing values by applying k-nearest-neighbour based imputation,
- removing the data corresponding to alternative splicing events not affected to a significant degree by knocking down genes or submitting cells to any other perturbing stimulus selecting them as those where the median absolute of the PSI changes with regard to the corresponding control is less than 1 ;
- scaling data by standardization;

in step iv)b., the estimation of covariance of perturbation profiles of alternative splicing events is carried out by quantifying the correlation between each pair of perturbation profile basing it on an iterative weighting algorithm, so that the estimated correlation for two pairs of genes or perturbing stimuli Xa and Xb is the weighted Pearson's correlation

$$p(X_a, X_b; w)$$

wherein w is a vector whose length is equal to the number n of alternative splicing events that is processed after data preprocessing, which vector contains the reliability values $R^u_{a,b}$ corresponding to each Z-score for the estimation of the correlation between two perturbation profiles, wherein the values $R^u_{a,b}$ are calculated with the formula

$$R^u_{a,b} = 1 / \frac{\sum_{i=1}^{p} D_u(X_a, X_i)\mathbb{I}(a, i) + \sum_{i=1}^{p} D_u(X_b, X_i)\mathbb{I}(b, i)}{\sum_{i=1}^{p} \mathbb{I}(a, i) + \sum_{i=1}^{p} \mathbb{I}(b, i)}$$

where II($a,i$) is the indicator function:

$$\mathbb{I}(a, i) := \begin{cases} 1 \text{ if } i \neq a \text{ AND } |p(X_a, X_i)| > T \\ 0 \text{ otherwise} \end{cases}$$

$T$ being a minimum correlation threshold for considering a pair of variables, and $D_u(X_a, X_b)$ is the deleted residual distance

$$D_u(X_a, X_b) = |p(X_a, X_b) - p_{-u}(X_a, X_b)|$$

wherein $p(X_a, X_b)$ and $p_{-u}(X_a, X_b)$ are the Pearson's correlation estimates before or after removing the observations $M_{au}$, $M_{bu}$ coming from an splicing event $u$, and using iteratively said algorithm until estimations converge; and

in step iv)c., a network is constructed from the correlation matrix obtained in iv)b., basing it on the gLasso algorithm by optimizing the log-likelihood function:

$$\log \det \Theta - tr(S\Theta) - r\|\Theta\|_1$$

where $\Theta$ is an estimate for the inverse covariance matrix $\Sigma^{-1}$, $S$ is the empirical covariance matrix of the data, $\|\Theta\|_1$ is the $L1$ norm i.e. the sum of the absolute values of all elements in $\Theta^{-1}$, and $r$ is a regularization parameter which is selected based on estimates of the rate of discovery of false data or FDR by:

- constructing random networks after permuting the sample data for every gene,
- repeating the process for 100 permutations and for random subsamples of the ASEs of size $S=\{3..35\}$ to obtain an estimate of the number of random network edges $|E|^S_R$ recovered for each sample size S at a fixed glasso regularization parameter,
- estimating the number of actual edges $|E|^S_A$ for random ASEs subsamples of the same sizes using the observed (non-permuted) data, and
- estimating the FDR for the network edges corresponding to each subsample size $S$ as the ratio of the mean number of edges recovered in the random graphs over the mean number of edges recovered in the actual graphs for all the different permutations and random subsamples of that size

$$FDR_S = \frac{mean(|E|^S_R)}{mean(|E|^S_A)} \ ,$$

and

complementing step iv)c., by defining modules in the network following the steps of:

- using the algorithm of network modularity $M(p)$ which is defined as:

$$M(p) = \sum_{k=1}^{m} \left[ \frac{l_k}{L} - \left(\frac{d_k}{2L}\right)^2 \right]$$

where $m$ is the number of modules in $p$, $I_k$ is the number of connections within module $k$, $L$ is the total number of network connections and $d_k$ is the sum of the degrees of the nodes in module $k$, and

- identifying modules of genes or perturbation stimuli that exhibit similar perturbation profiles among the assayed events by maximizing the network's modularity using the Clauset's greedy community detection algorithm, and displaying the network by clustering genes or perturbing stimuli in said modules.

A possible of embodiment of the method of the invention of particular interest is that one wherein an additional step for identifying general and secondary functional interactions among splicing factors is carried out, which step comprises the substeps of:

i. repeating substeps i) to iv) of step a) of the method with different subsamples of data, each subsample consisting of different combinations of at least one half of the alternative splicing events initially analyzed, wherein the number of subsamples is preferably at least one order of magnitude higher than the number of inhibited genes or the total number of perturbing stimuli applied,

ii. identifying as functional interactions among splicing regulatory factors that are general and essential for the splicing process as those corresponding to connections that are present in at least 90% of the reconstructed subnetworks,

iii. Identifying as functional interactions which are specific of particular events of groups of events of alternative splicing as those corresponding to connections with an absolute correlation value higher than 0.5 and which are present in more than 20% of the reconstructed subnetworks and that, additionally, are absent of more than 20% of the remaining reconstructed subnetworks and with an absolute correlation value lower than 0.2.

An additional possible embodiment of the present invention, also with particular interest, is that one wherein, once applied steps a) and b) of the method, those nodes that appear clustered in a same region of a network with connections among them are identified as corresponding to splicing factors forming part of a same splicing subcomplex.

Particularly important, and also compatible with the previous ones, are those embodiments of the method of the invention wherein at least one of the splicing perturbing stimuli whose effect on the set of alternative splicing events is analyzed consists of submitting the corresponding cell or cells to the action of an added compound. The added compound can be a drug known to have a therapeutic effect on a disease associated with altered alternative splicing, so that any splicing regulatory factor whose corresponding network node has a direct connection with the node corresponding to the drug is identified as a target for directing to it other compounds intended to be candidates to alternative drugs against said disease. A particular example of such embodiments can be those wherein the drug is Spliceostatin A or Meayamycin and any direct connection with it is identified as a target for directing to it other compounds intended to be candidates to alternative drugs against cancer.

Another possible embodiment, closely related to the previous one, is that wherein an added compound is identified as a candidate to drug for the treatment of a disease associated with altered alternative splicing when it has at least a direct connection in common with a second compound, also having added as a perturbing stimulus for carrying out the method or whose functional associations have been previously identified by the method of claim 1 or by any other means, which second compound is a drug known to have an therapeutic effect on a disease associated with altered alternative splicing.

In other possible groups of embodiments, the added compound is known to modulate a physiological process in the cell and any splicing regulatory factor whose corresponding network node has a direct connection with the node corresponding to the drug is identified as a target for directing to it other compounds intended to be candidates to the modulation of the same process.

Additional embodiments derive from carrying out the method both with cells removed from a patient suffering from a disease or malfunction and with a different kind of cells deriving from and individual not suffering that disease or malfunction and those factors showing different connection in the two generated networks could be identified as targets for directing to them compounds intended to be candidates to drugs against said disease or malfunction.

The approaches related to identification of drug targets and candidate to drugs can also be set forth as a method for identifying candidates to drugs against a disease which comprises the steps of carrying out the above discussed method and identifying the compound as candidate to drug against such disease when it shows at least a direct connection in common with another compound previously known to have a therapeutic effect on that disease or with a splicing regulatory factor that is previously known to be a target of at least a drug known to have a therapeutic effect on that disease. This can be considered also an aspect of the present invention.

**BRIEF DESCRIPTION OF THE DRAWINGS**

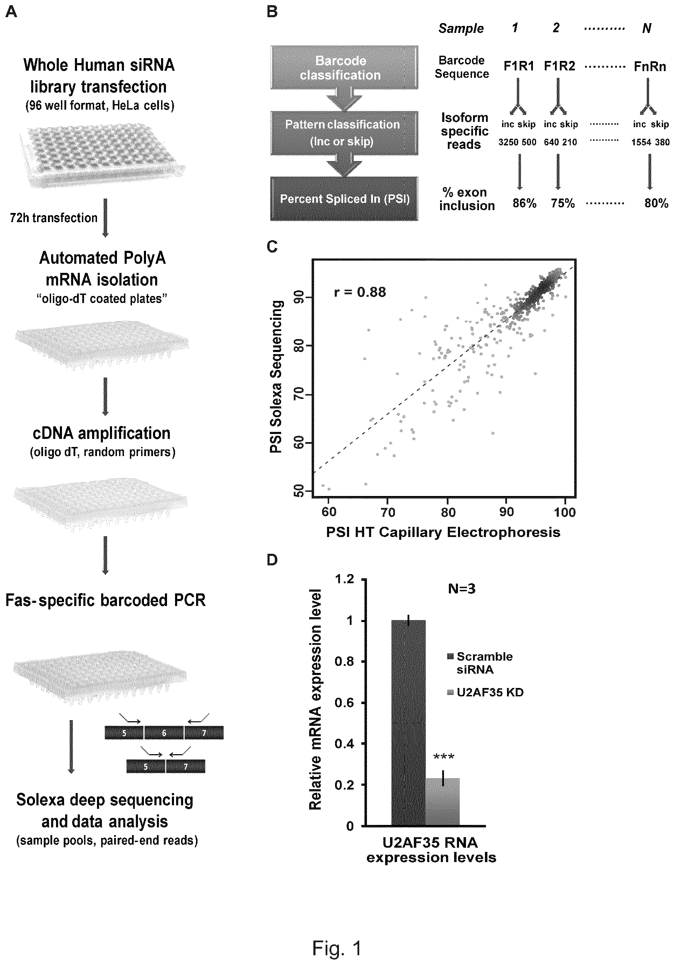

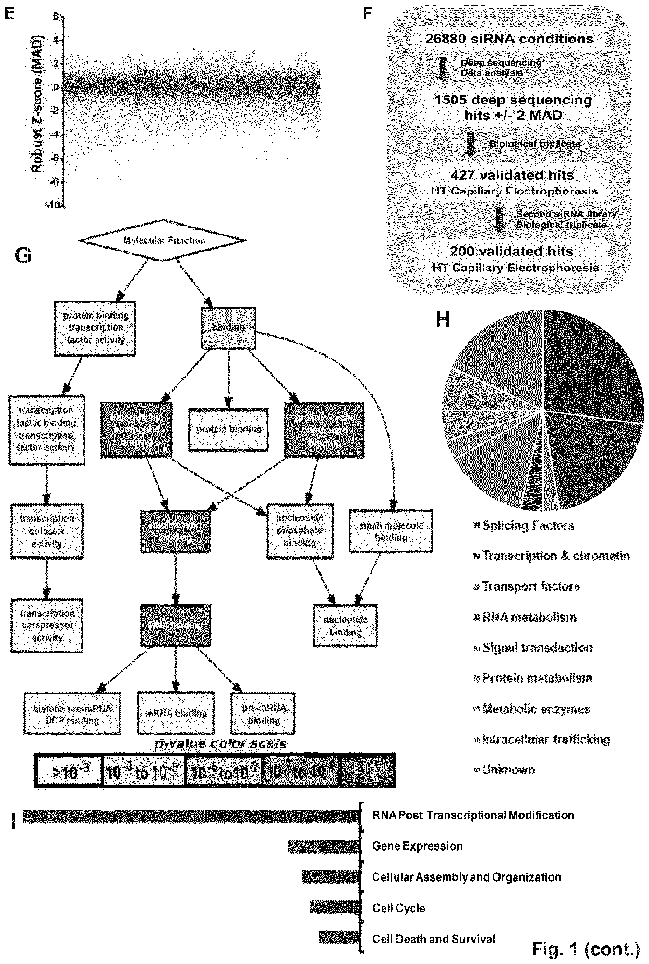

**Fig. 1. Genome-wide screening for regulators of Fas/CD5 receptor AS: Methodology and result analysis.**

- Panel A: Scheme of the screening procedure. In short, an automatized method for siRNA transfection, RNA isolation and reverse transcription that generates cDNA transcriptomes corresponding to the individual knock down of every human gene. This collection is interrogated, in this specific example, by PCR to amplify a region of Fas gene comprising exons 5 and 7, which is alternatively spliced to generate products that include or skip exon 6. cDNAs from each knock down condition are amplified using a particular combination of barcoded primers (see below) and the collection of amplification products from every knock down condition analyzed by paired-ended Solexa sequencing.
- Panel B: Barcode classification and isoform quantification pipeline. Deep sequencing reads were filtered based upon the barcode sequence combination (each knock down condition is characterized by a particular combination of forward (F1-Fn) and reverse (R1-Rn) barcoded primers) and the presence or absence of Fas/CD95 exon 6 sequences. The percentage of exon inclusion isoforms was calculated for each knockdown condition by simply counting the number of exon 6-containing and exon 6-lacking deep sequencing reads corresponding to molecules containing the particular combination of forward and reverse primers characteristic of that condition.
- Panel C. Correlation between Percentage Spliced In (PSI) values obtained by deep sequencing data and by HTCE (High-Throughput Capillary Electrophoresis) for 500 control knockdown conditions. The results validate the deep sequencing approach to quantify alternative splicing changes.
- Panel D. Example of mRNA knockdown validation: RNAs isolated from HeLa cells transfected with scrambled siRNAs or smartpool siRNA library siRNAs against U2AF35. Values represent the mean and s.d. from 3 biological replicas.
- Panel E. Distribution of robust Z-scores across the 26880 siRNA conditions analyzed.
- Panel F. Flowchart of hit validation along the screening procedure. MAD indicates scaled median absolute deviation. The 1505 hits from the initial screen were tested in triplicate by High-Throughput Capillary Electrophoresis (HTCE), and the validated 427 hits were tested using a second siRNA library in biological triplicates by HTCE, generating a final list of 200 hits validated by two siRNA libraries and two different technologies (deep sequencing and HTCE).
- Panel G. Gene ontology enrichment analysis of molecular functions, calculated using Gorilla software. Dark filled boxes correspond to function enrichment with p-value < $10^{-9}$; light grey filled boxes ("binding") correspond to function enrichment with p-value from $10^{-5}$ to $10^{-9}$; the remaining boxes correspond to a function enrichment with p-value from $10^{-3}$ to $10^{-5}$.
- Panel H. Pie chart showing the functional classification of genome-wide screen hits. Classes (functions) are indicated below the chart, beginning with that the highest percentage of hits ("Splicing factors", 28%, occupying the top right-handed quarter and a little portion of the bottom right-handed quarter), and indicating the function of the remaining portions clockwise.
- Panel I. Horizontal bar chart representing the statistical significance (p-*value*) of the enrichment in functional gene ontology terms for the fully validated 200 hits.

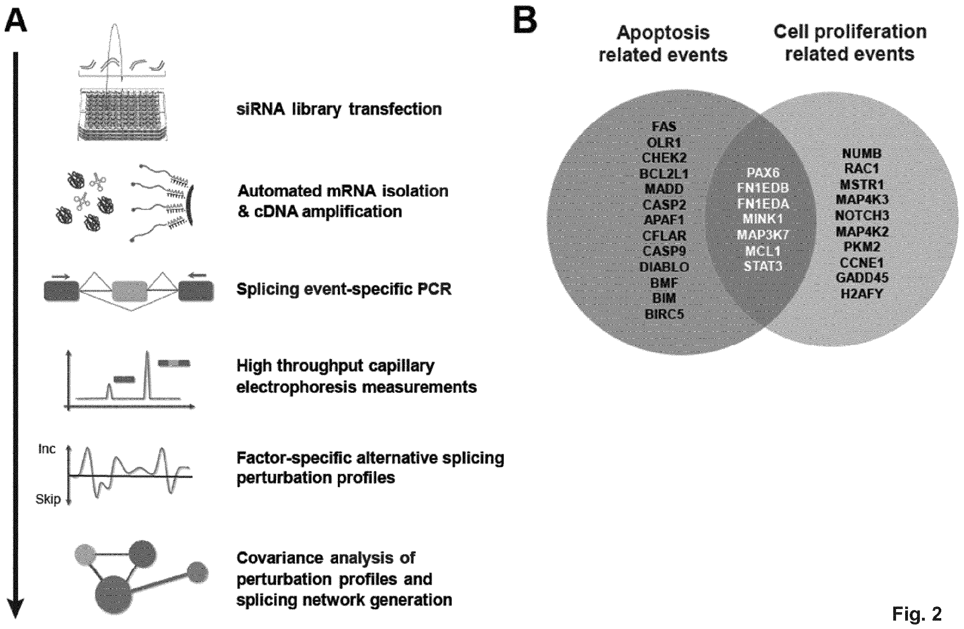

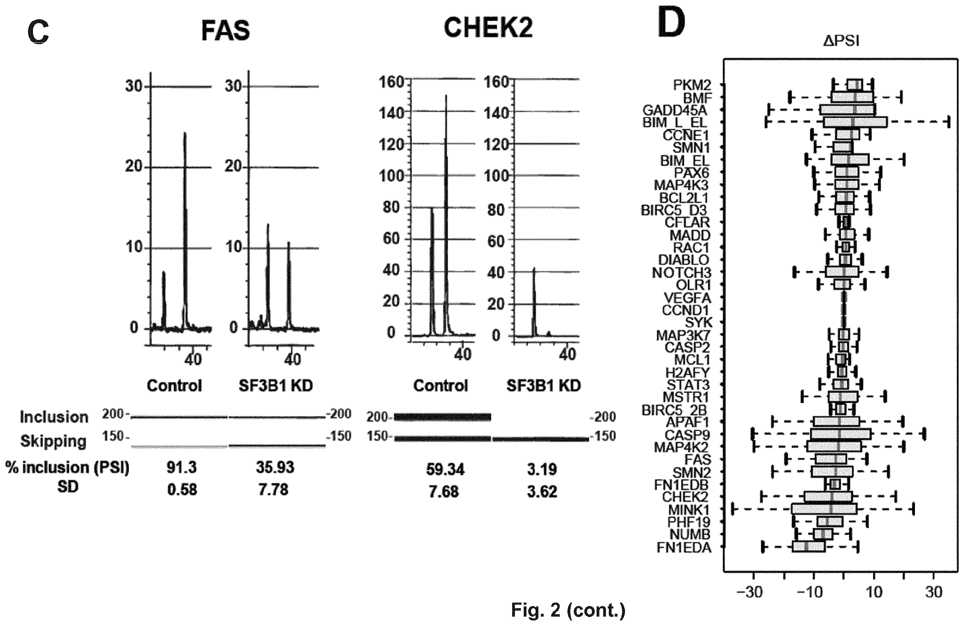

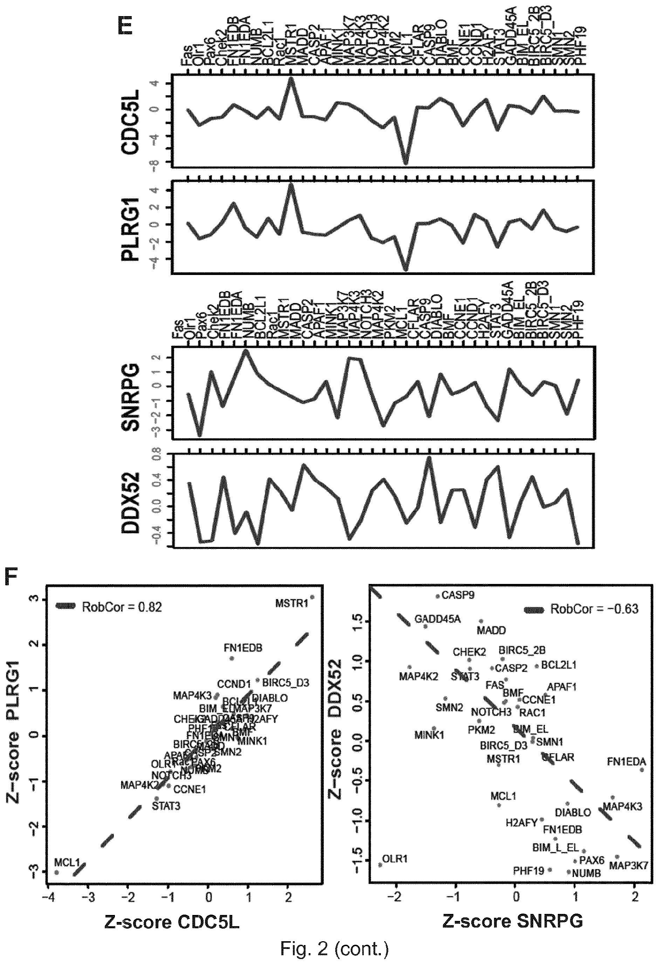

**Fig. 2. Comprehensive mapping of functional interactions between SFs (splicing factors) in ASEs (alternative splicing events) implicated in cell proliferation and apoptosis.**

- Panel A. Flow-chart of the pipeline for network generation. Changes in 36 alternative splicing events are measured upon knock down of each of the individual genes encoding components of the splicing machinery as well as chromatin remodeling factors. An automatized procedure for siRNA transfection, mRNA isolation and splicing event-specific PCRs, analyzed by High Throughput Capillary electrophoresis generates splicing perturbation profiles that represent changes in alternative splicing induced by knock down of a particular factor. Comparison between perturbation profiles of pairs of factors us used to build a functional splicing network.
- Panel B. Genes with ASEs relevant for the regulation of cell proliferation and/or apoptosis used in the network generation assays; the area common to both circles corresponds to the genes involved in both kinds of events.
- Panel C. Examples of HTCE (High Throughout Capillary Electrophoresis) profiles for FAS/CD95 and CHEK2 ASEs under conditions of knockdown of the core splicing factor SF3B1. Upper panels show the HTCE profiles, lower panels represent the relative intensities of the inclusion and skipping isoforms. PSI (percentage of inclusion) values indicate the median and standard deviation of three independent experiments.
- Panel D. Spread of PSI changes observed for each of the events analyzed in this study upon the different knockdowns.
- Panel E. Examples of splicing perturbation profiles for different knockdowns. The profiles represent change towards inclusion (>0) or skipping (<0) for each ASE upon knockdown of the indicated factors, quantified as a robust Z-score. Robust correlation estimates of the perturbation profiles of strongly correlated (PLRG1 versus CDC5L, upper panel) or anti-correlated (DDX52 versus SNRPG, lower panel) factors.
- Panel F. Robust correlation regression of the perturbation profiles of strongly correlated (PLRG1 versus CDC5L, upper panel) or anti-correlated (DDX52 versus SNRPG, lower panel) factors.

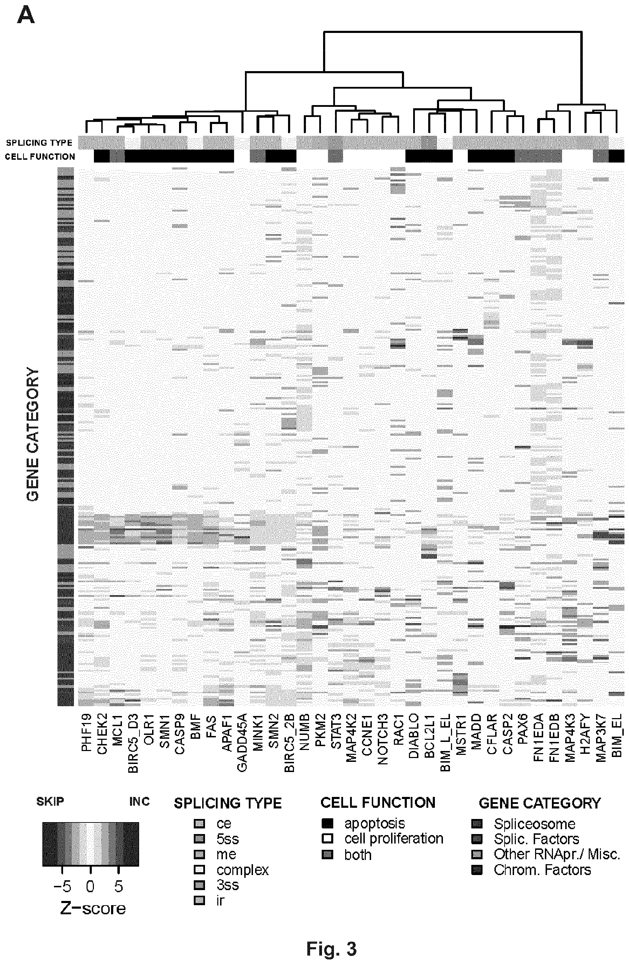

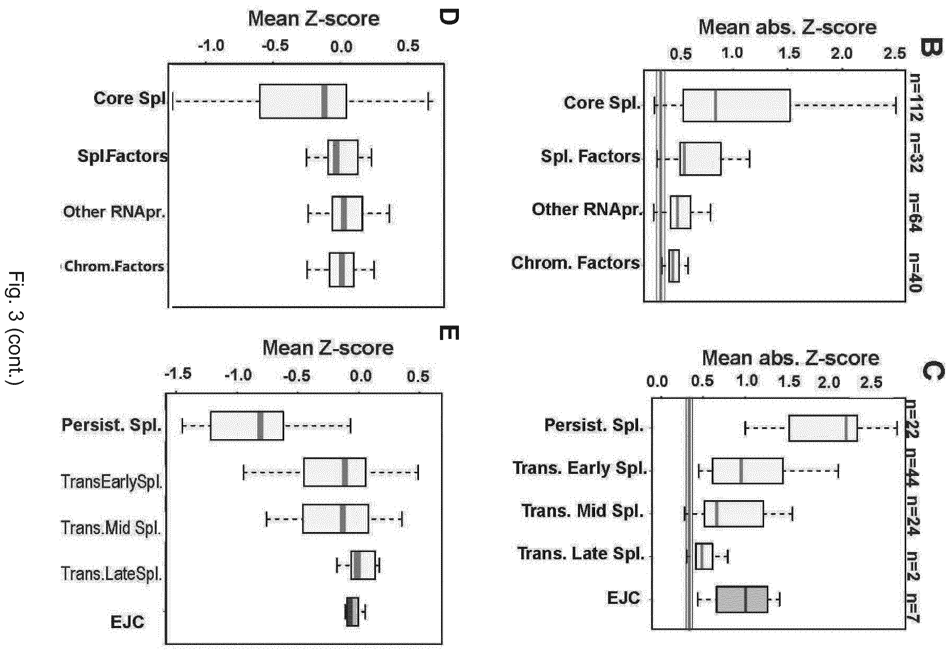

**Fig. 3. Coordinated regulation of splicing events by coherent subsets of SFs.**

- Panel A. Heatmap representation of the results of the screening of alternative splicing events upon knocking down splicing regulation factors. ASEs used in this study are on the X axis, while knockdown conditions on the Y axis. Data are clustered on both dimensions (ward linkage, similarity measure based on Pearson correlation). Information about ASEs, a) type (ce - cassette exon; 5ss - alternative 5' splice site; me - mutually exclusive exon; complex - multi-exonic rearrangement; 3ss - alternative 3' splice site ; ir - intron retention) and b) involvement in apoptosis (dark-coloured boxes) or cell proliferation (non-coloured boxes) or in both process (grey coloured boxes) regulation is indicated in the second level of boxed located over each vertical lane of the heatmap. The tone of each short bar located before each horizontal lane of the heatmap indicates the category of the genes knocked down, the palest tones corresponding to other RNA processing factors or miscellaneous factors (abbreviated "Other RNApr./Misc.") and the darkest ones to chromatin remodeling factors ("Chrom.Factors").
- Panel B. Box-plot representation of the mean absolute z-score of AS changes induced by the knockdown of particular classes of factors, including core and non-core splicing (abbreviated "Spl." in the figure) factors, other RNA processing (abbreviated "pr." In the Figure) factors and chromatin (abbreviated "Chrom." in the Figure) remodeling factors. Median and spread (inter-quartile range) of AS changes of mock siRNA conditions are represented by the thin and wide lines, respectively, located near the bottom of the representation.
- Panel C. Box-plot representation as in panel B for Spliceosomal factors that assemble early and stay as detectable components through the spliceosome cycle (abbreviated "Persist. Spl."), factors that are present only transiently ("Trans.") at early, mid or late stages of assembly, and components of the exon junction complex (EJC).
- Panel D. Box-plot representation of the mean z-score of AS changes induced by the knockdown of particular classes of factors, classified as in panel B.
- Panel E: Box-plot representation of the mean z-score of AS changes induced by the knockdown of particular classes of factors, classified as in panel C.

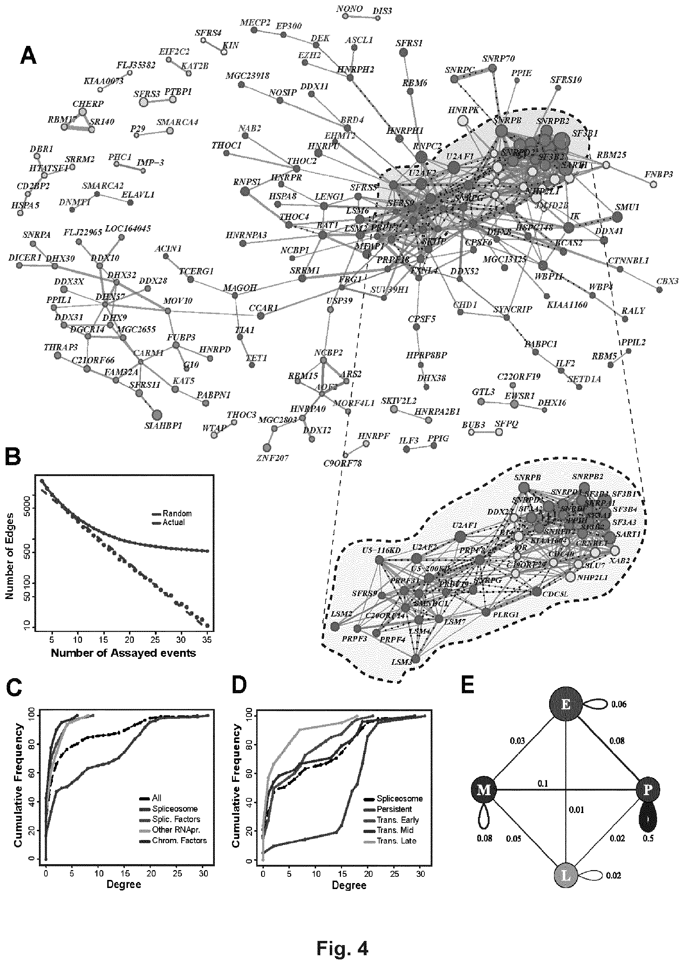

**Fig. 4. Functional splicing regulatory network.**

- Panel A. Graphical representation of the reconstructed splicing network. Nodes (circles) correspond to individual factors and edges (lines) to inferred functional associations. Positive or negative functional correlations are represented in the original by green or red edges, respectively. Edge thickness signifies the strength of the functional interaction, while node size is proportional to the overall impact (median Z-score) of a given knockdown in the regulation of AS. Node coloring depicts the network's natural separation in coherent modules. Known physical interactions as reported in the STRING database (Franceschini et al 2013) are represented in black dotted lines between factors. The inset is an expanded view of factors in the U2 and U4/5/6 snRNP complexes.
- Panel B. Number of recovered network edges using actual (point connected by a continuous line, which is the upper curve in the graph) or randomized (points connected by a discontinuous line, red in the original) datasets as a function of the number of ASEs used. Lines represent the best fit curves for the points.
- Panels C and D. Network degree distribution. The plots represent the cumulative frequency of the number of connections (degree) for different classes of factors, classified as in Figs. 3B and 3C, respectively.
- Panel E. Functional cross-talk between different categories of spliceosome components. Values represent the fraction of observed functional connections out of the total possible connections among factors that belong to the different categories: Persistent - P, transient early - E, transient mid - M or transient late - L factors.

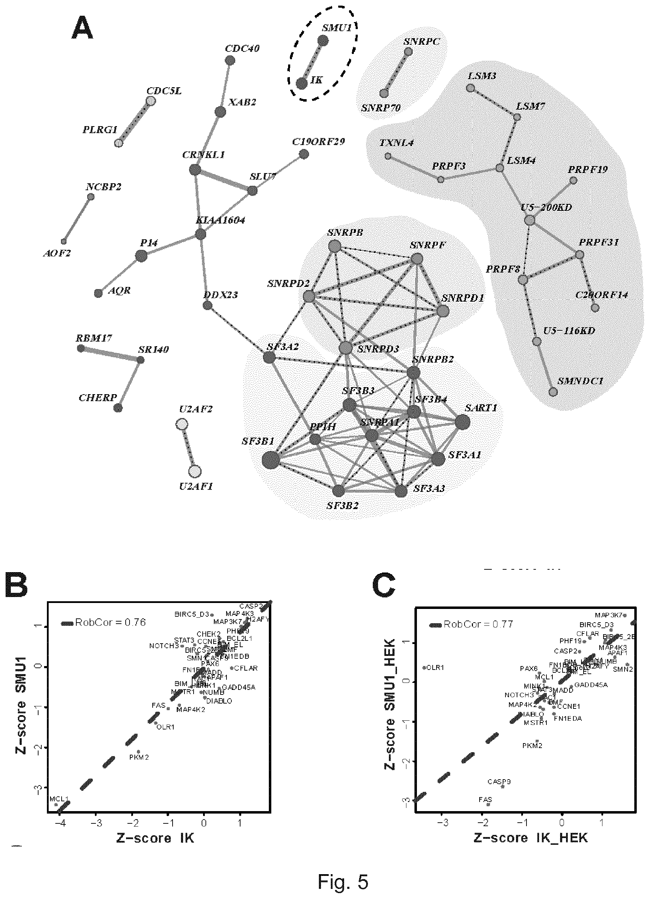

**Fig. 5. Core network involved in AS regulation.**

- Panel A. Graphical representation of the functional connections present in at least 90% of 10.000 networks generated by iterative selection of subsets of 17 out of the 35 ASEs used to generate the complete network. Known spliceosome complexes are highlighted by shadowed areas (U1 snRNP - blue; U2 snRNP - yellow; SM proteins - green; tri-snRNP proteins - red).
- Panels B and C. Consistency of inferred functional interactions in different cell lines. Knockdown of IK or SMU1 was carried out in parallel in HeLa (B) or HEK293 cells (C), and changes for the 35 ASEs were analyzed by RT-PCR and HTCE. Robust correlation estimates and regression for the AS changes observed in IK versus SMU1 knockdowns are shown for each of the cell lines.

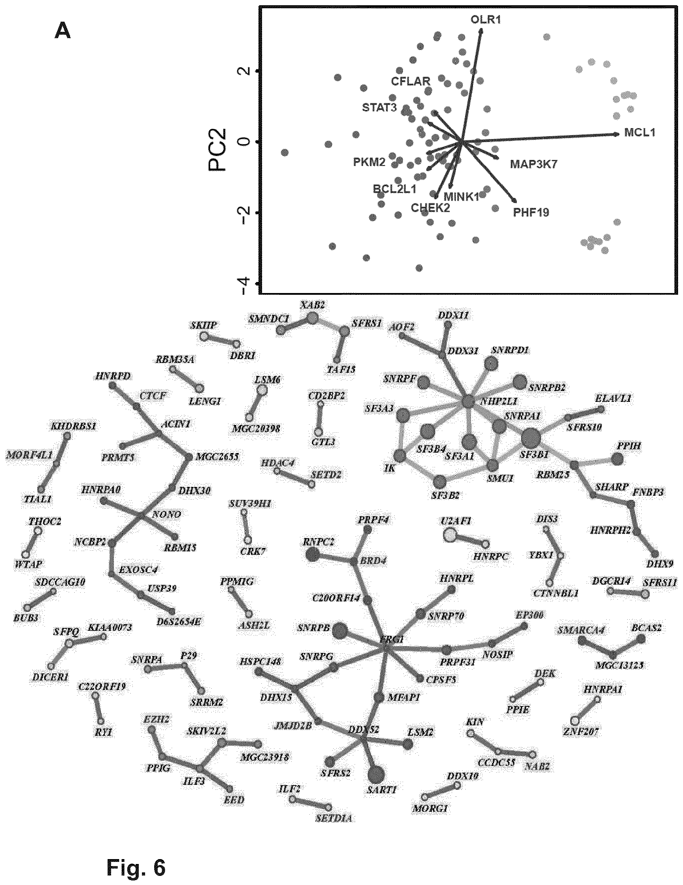

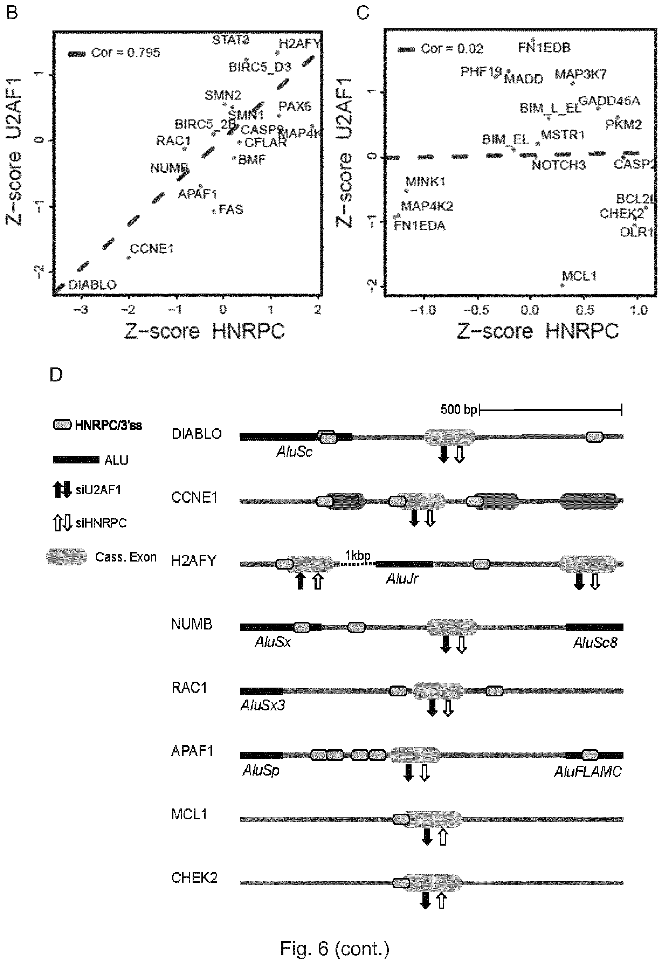

**Fig. 6. Variable functional interactions extracted from networks generated from subsets of ASEs .**

- Panel A. Network of ancillary functional connections characteristic of subsets of ASEs. Lines link SFs that display functional similarities in their effects on subsets of ASEs. Line colors indicate the positions of the interactions in the Principal Component Analysis shown in the inset. The inset captures the different relative contributions of the ASEs in defining these connections. For clarity, only the top ten discriminatory ASEs are shown.
- Panels B and C. Correlation values and regression for the effects of U2AF1 versus HNRPC knockdowns for ASEs in the presence (B) or absence (C) of upstream composite HNRPC binding/3' splice site-like elements.
- Panel D. Schematic representation of the distribution of composite HNRPC binding sites/3' splice site-like sequences (small boxes with blunt corners and a surrounding line) in representative examples of the splicing events analyzed. The position of the exon cassettes that can be skipped/included is represented with larger boxes with blunt corners but without a surrounding line. The relative position of Alu elements in these regions is also shown as broader line portions. Genomic distances are drawn to scale. The direction of the arrows indicates up or down regulation of cassette exons upon knockdown of U2AF1 (black arrows) or HNRPC (white arrows).

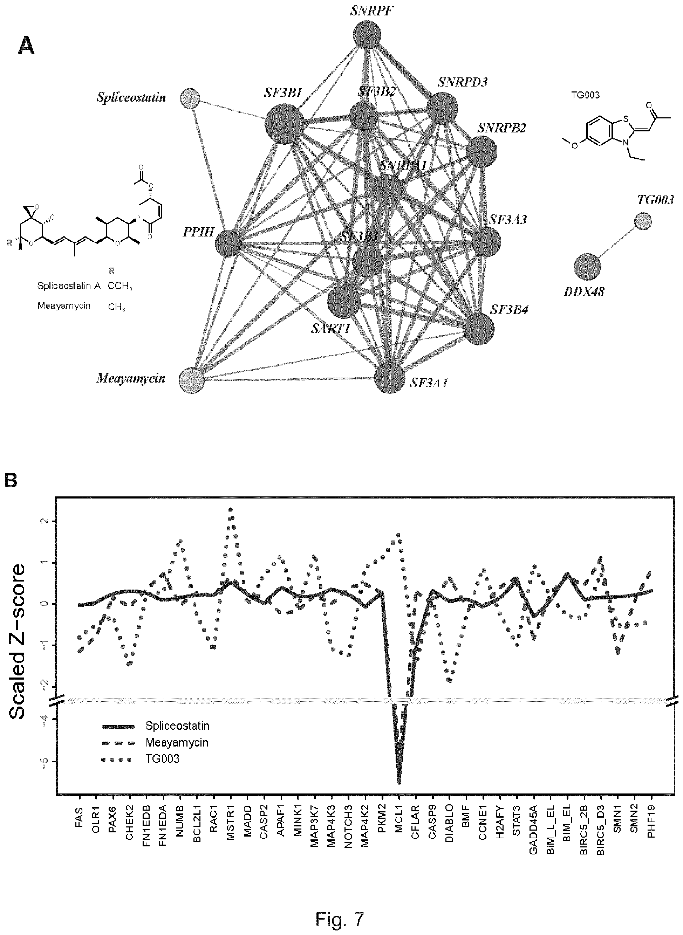

**Fig. 7. Mapping the effects of pharmacological treatments to the splicing network.**

- Panel A. Functional connections of splicing inhibitory drugs Spliceostatin, Meayamycin and TG003 with the splicing machinery. The links were established by comparing the perturbation profiles of alternative splicing changes induced by treatment of cells with the indicated compounds and the perturbation profiles of splicing factor knock downs used to generate the functional network. The chemical formulae of all three drugs are also depicted.
- Panel B. Splicing perturbation profiles for Spliceostatin A (continuous line), Meayamycin (discontinuous line) or TG003 (dotted line) treatments across the 35 ASEs analyzed.

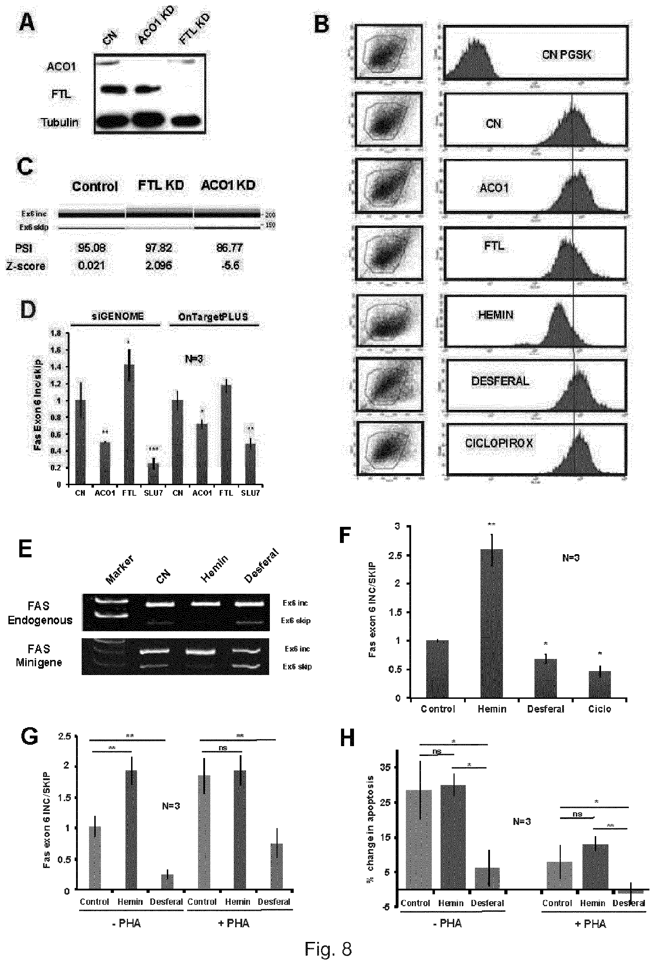

**Fig. 8. Iron homeostasis modulates Fas/CD95 alternative splicing.**

- Panel A. ACO1 and FTL protein levels after depletion of said proteins by siRNA-mediated knockdown (KD) using SiGENOME library. Proteins were detected by western blot analysis under the indicated conditions. CN indicates "control", mock siRNA transfected cells.
- Panel B. Analysis of intracellular iron levels by Phen-green SK fluorescence flow cytometry of facs-sorted HeLa cells (left panels) transfected with siRNAs against ACO1 or FTL, or treated with Hemin, Desferal or cyclopirox olamine, as indicated in the right panels, where the geometric median of Phen-green SK fluorescence intensity for a representative experiment is shown. The X axis represents fluorescence intensity and the Y axis represents cell numbers. CN PGSK indicates autofluorescence in the absence of Phen-green SK treatment. CN corresponds to control cells transfected with scrambled siRNAs and incubated with DMSO instead of Hemin or Desferal. ACO1 and FTL knockdown treatments were also incubated with DMSO prior to experimental measurements.
- Panel C. RT-PCR analysis of Fas/CD95 exon 6 AS upon FTL or ACO1 knockdown, analyzed by capillary electrophoresis. PSI index and Z-score values are indicated.
- Panel D. Real-time qPCR analysis of Fas/CD95 exon 6 AS upon FTL, ACO1 or SLU7 knockdown in HeLa cells using two independent siRNA libraries, and indicated in the upper part of the graph.
- Panel E. RT-PCR analysis of Fas/CD95 AS upon treatment of HeLa cells with 100 $\mu$M Hemin or 100 $\mu$M Desferal, which cause iron overload and depletion, respectively. Both endogenous transcripts (upper panel) or transcripts derived from an expression vector containing Fas/CD95 genomic sequences from exons 5 to 7 (lower panel, analyzed using vector-specific primers) were analyzed. The results are consistent with those of previous panels and show that iron overload enhances exon 6 inclusion while iron depletion has the opposite effect.
- Panel F. Real-time qPCR analysis of Fas/CD95 AS in HeLa cells treated with 100 $\mu$M Hemin, Desferal or cyclopirox olamine.
- Panel G. Real-time qPCR analysis of Fas/CD95 exon 6 AS in RNAs from human peripheral blood mononuclear cells (PBMCs) untreated or treated with phyto-haemagglutinin (PHA, 2 $\mu$M) for 48h, upon changes in iron levels induced by treatment with 100 $\mu$M Hemin, 100 $\mu$M Desferal or DMSO for 12h. Values were normalized to the control -PHA condition. The results confirmed the effects of iron level on Fas alternative splicing on blood cells. For panels C-G, values represent the mean and s.d. of 3 biological replicas. p-values obtained from Welch's t-test are indicated: * 0.05 to 0.01, ** 0.01 to 0.001, *** <0.001.

- Panel H. Induction of Fas-mediated apoptosis in human PBMCs upon modulation of iron levels. Data represent the percentage of change in apoptotic cells upon treatment of human PBMCs with Fas antibody (1 $\mu$g) or isotype control IgG for 16h. Cells were previously exposed (or not) to phyto-haemagglutinin (PHA, 2 $\mu$M) for 48h, and to either 100 $\mu$M Hemin, 100 $\mu$M Desferal or DMSO for 12 hours. Cells were FACS sorted with anti-CD3 antibodies to select T lymphocyte populations and apoptosis induction was normalized to control - PHA condition. Values represent the mean and s.d. of 3 independent biological replicas and p-values from Welch's one-tailed t-test are indicated: * 0.05 to 0.01, ** 0.01 to 0.001, *** <0.001. The results indicate that iron depletion reduces Fas-mediated apoptosis, as expected from its effects on Fas alternative splicing regulation.

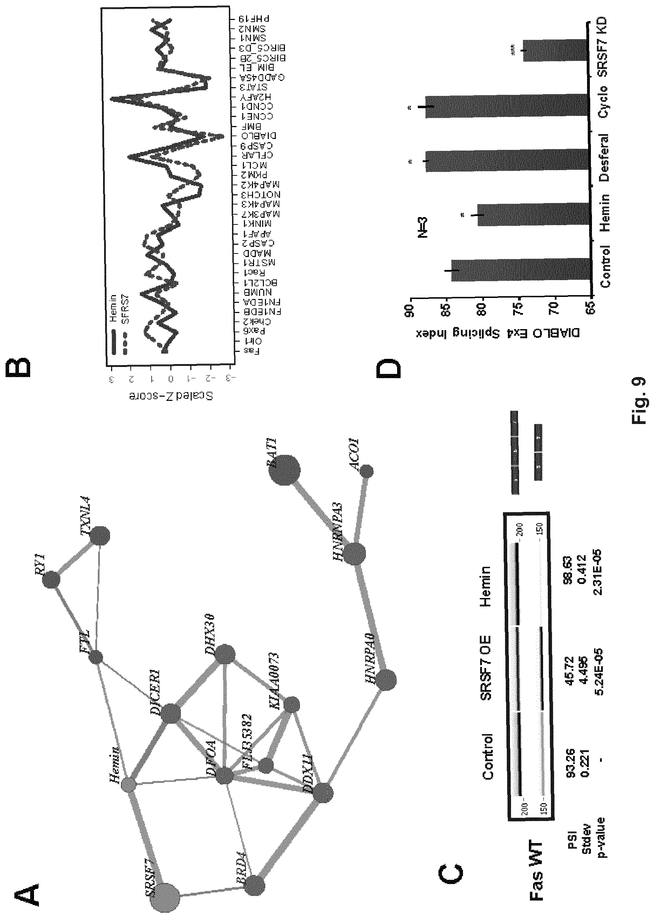

**Fig. 9. Functional links between SRSF7 and iron homeostasis-induced AS.**

- Panel A. Functional links between RNA processing factors and alterations of iron levels, derived from a Splicing network analysis. Lines connecting RNA processing factors and treatments leading to changes in iron levels indicate similar (green in the original) or opposite (red in the original) effects on the profile of AS changes of 36 genes induced by splicing factor or ACO1 / FTL knockdown or by treatment with 100 $\mu$M Hemin or Desferal (DFOA in the Figure). Line widths are proportional to the strength of the correlations observed.
- Panel B. Perturbation profile of AS changes upon SRSF7 knockdown or Hemin treatment across 36 AS events, analyzed by RT-PCR and capillary electrophoresis. Results correspond to scaled Z-score changes from six biological replicas. Positive and negative Z-scores indicate increased exon inclusion or skipping respectively.
- Panel C. SRSF7 overexpression and iron overload have antagonistic effects on Fas/CD95 AS. HeLa cells were transfected with a reporter minigene corresponding to Fas/CD95 exons 5-7 and either an empty vector or a SRSF7 expression vector, and treated either with DMSO (control) or with 100 $\mu$M Hemin; RNAs were isolated and the patterns of Fas/CD95 AS analyzed by capillary electrophoresis. PSI indexes, s.d. and p-values for three independent experiments are shown.
- Panel D. Effects of iron levels perturbation and SRSF7 knockdown on AS of the apoptosis regulatory gene DIABLO. Relative levels of DIABLO exon 4 inclusion were measured in RNAs from HeLa cells upon treatment with 100 $\mu$M Hemin, Desferal or Cyclopirox or mock or SRSF7 knockdown by RT-PCR and capillary electrophoresis. Results correspond to the mean and s.d. of three biological replicates. p-values obtained from Welch's t-test are indicated: * 0.05 to 0.01, *** <0.001.

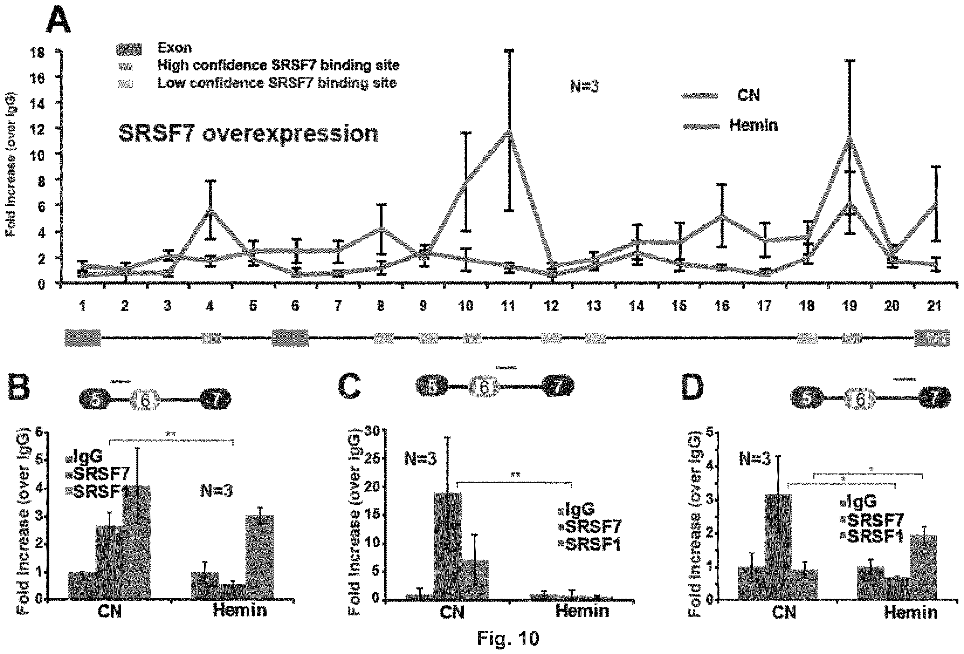

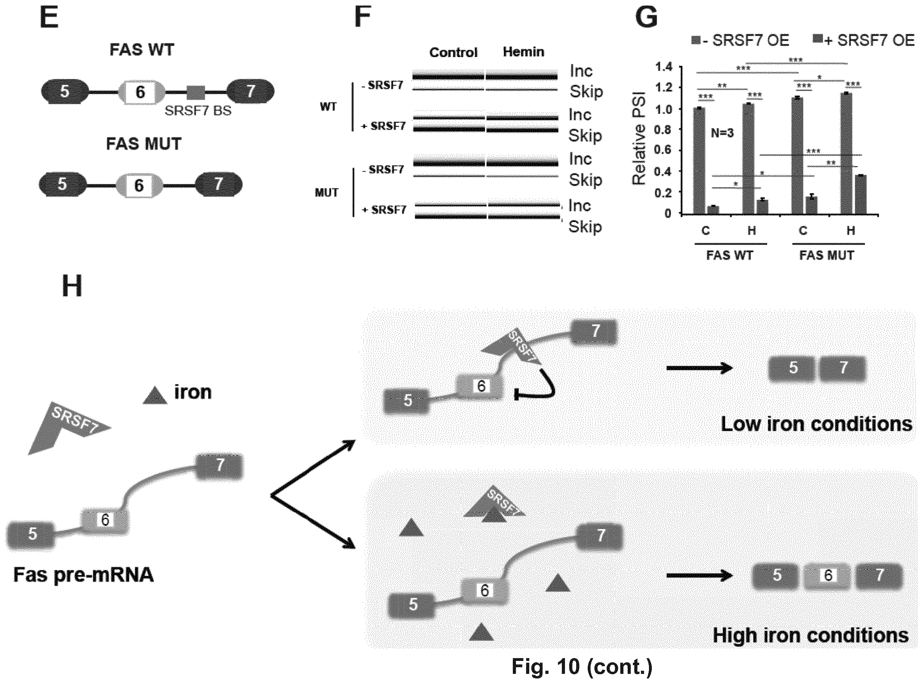

**Fig. 10. Iron regulates SRSF7 RNA binding.**

- Panel A. Identification of SRSF7 binding sites in Fas/CD95 AS region by in vivo crosslinking / immunoprecipitation (CLIP) with control (CN) and hemin-treated HeLa cells. Binding values represent mean and s.d. of fold-increase over control IgG for three independent experiments, after normalization by input levels of each amplicon and condition. The positions of exons and predicted SRSF7 binding sites are indicated.
- Panels B, C and D. Quantification of CLIP signals using antibodies against endogenous SRSF7 (second bar of each group of three) or SRSF1 (third bar of each group of three) in control (CN) or iron overload conditions (Hemin 100 $\mu$M for 24h), normalized to IgG background signals (first bar of each group of three), for the three (high confidence) predicted SRSF7 binding sites indicated over the graphs (5, 6, 7). Values represent mean and s.d. for three independent biological replicates.
- Panel E. Schematic diagram of the deletion mutant lacking the region containing the peak of SRSF7 binding in intron 6.
- Panel F. Effects of SRSF7 overexpression on Fas exon 6 splicing using wild type or mutant minigenes under control conditions or conditions of iron overload (Hemin), analyzed as in Fig. 9C.
- Panel G. Quantification of relative PSI values upon SRSF7 overexpression (OE) under control (C) or iron (H) overload conditions. Values represent mean and s.d. for three biological replicas. p-values obtained from Welch's t-test are indicated: * 0.05 to 0.01, ** 0.01 to 0.001, *** < 0.001.
- Panel H. Model of iron mediated Fas AS regulation. SRSF7 binding to Fas pre-mRNA under low iron conditions promotes exon 6 skipping, while iron overload compromises the Zn knuckle domain-mediated RNA binding of SRSF7, leading to higher levels of exon 6 inclusion

## DETAILED DESCRIPTION OF THE INVENTION

The present invention relates to a method for identifying alternative splicing regulation targets from the identification of functional associations among factors, internal or external, involved in the splicing process and its regulation.

The method is based on the generation of a network of connections among factors involved in performing the splicing process itself and in its regulation, which can be used as a model of functional associations among alternative splicing (AS) factors and/or among AS regulatory factors and stimulae perturbing AS in a cell. The network represents a unique compendium of functional interactions among gene products involved in splicing regulation (splicing factors, SF), a discovery tool for coupling cell-perturbing stimuli to splicing regulation and an expansive view of the splicing regulatory landscape and its organization. The network and the method for applying it to the identification of alternative splicing regulation targets are powerful tools for studying the splicing process and its regulation, identifying targets for modulating specific alternative splicing events and even finding common patterns of behaviour among some splicing factors that can be used as starting points for finding common features among them, broading the knowledge about their functions and/or finding common targets for their regulations or activities previously unknown. The identification of new targets for modulating specific alternative events can be a crucial point for having new targets for developing drugs against malfunctions or diseases associated to alterations of alternative splicings, including drugs or even for identifying the alternative splicing events connected with a disease. When one of the splicing factors (perturbing stimuli) assayed is a drug with a known target, the method of the present invention can be of use for identifying candidates to drugs against the same disease among the compounds with the same target, which compounds can be more effective against the disease to treat. As the network also gives an idea of the intensity of the interaction among two factors, the network can also be of use to assess whether a candidate to drug against a disease has a stronger effect over a target than a previous known drug, giving an initial assessment of the possible activity of the compound. The method can also be of use for identifying the biological targets of compounds with a known activity but with an unknown target or, even, without a previous known connection with alternative splicing, and knowing the target can be of use to find other compounds with similar activities but, for instance, with less collateral effects.

The construction of the network that is a basic pillar for carrying out the method of the present invention is based on two fundamental parts:

- A high-throughput screen to evaluate the effects of knocking down a multiplicity of genes (271 genes in the assays prepared for the construction of the network), which genes were selected trying to have a representative of each individual splicing component or regulator of a plurality of functionally important AS Events (ASEs). The screen is designed to assess the individual effect of knocking down each individual gene of the multiplicity of genes on each one of a plurality of selected alternative splicing events. The particular ASEs studied in the assays shown in the present application have been selected due to being significant in the regulation of cell proliferation and/or apoptosis.
- The careful design of a computerized framework, based on a careful selection, implementation and combination of algorithms addressed for the goal of the present invention, for analyzing the data obtained in the mentioned screen and modeling therefrom a network of interactions among the knocked-down genes which enables working with robust processed data and constructing with them a network with a great potentiality for inferring physical and functional relations among the products of said genes, which network is easy to visualize and analyzed for said purpose.

Such an approach had not been suggested previously for handling with the intricate network of connections of the splicing process and having a tool for modeling it, inferring associations among the factors implied and finding targets for modulating alternative splicing and acting on the physiological processes, altered or not, where such alternative splicing event is involved.

It is possible to find in the scientific literature examples of different approaches where networks are modelled for the representation of natural systems (Chart-Aryamontri et al., 2012; Franceschini et al., 2013; Wong et al., 2012). Said networks have been successfully used in order to decipher relations in different circuits, their modes of regulation and how different physiological alterations affect them (Gerstein et al., 2012; Kim et al., 2013; Watson et al., 2013). Many of said approaches give rise to network models of complex representation and/or visualization, which implies that efforts are to be made to draw any conclusion from them. And, as previously said, none of them has been designed for studying and handling the splicing process and its network of interactions.

Key to the approach of the present invention is the premise that the distinct profiles of splicing changes caused by perturbation of regulatory factors depend on the functional bearings of those factors and can, therefore, be used as proxies for inferring functional relationships. Thus, the perturbation profiles of alternative splicing generated for each perturbation stimulus (the knocking down of a gene or any other perturbation of a cell condition) can be regarded as representing the influence of such stimulus on each one of the analyzed ASE, so that the comparative analysis of each possible pair of profiles can be used to infer functional relations among the corresponding gene or perturbation factor.

Using such approach, the present inventors derived a network that recapitulates the topology of known splicing complexes and provides an extensive map of >500 functional associations among -200 splicing factors (SFs). The network accurately captures known physical and functional associations and identifies new ones, revealing remarkable regulatory potential of core spliceosomal components, related to the order and duration of their recruitment during Spliceosome assembly. Additionally, as can be seen in the assays described in the Examples of the present application, such network can be used to identify general and particular mechanisms of AS regulation and to identify key SFs that mediate the effects on cell perturbations that affect AS, such as those induced by iron or by drugs targeting components of the splicing machinery, such as the anti-cancer drugs Spliceostatin and Meayamycin. Such splicing factors represent targets for finding or improving compounds aimed to modulate the process where they are involved, such as iron metabolism, or conditions, disorders and disease where AS is altered, such as some cancer types.

The basic steps for generating a model network and applying it for identifying targets for modulating alternative splicing events and, therefrom, being able to develop means and compounds for modulating the process where they are involved, are summarized in Fig. 2A.

As can be seen in Fig. 2A, the first step is perturbing the alternative splicing conditions in a cell by submitting it to one or more splicing perturbing stimuli. As used in the present application, a splicing perturbing stimulus is any change in the situation / status / condition of a cell that affect the splicing process in said cell. As such stimulus affects the splicing process and, as such, has an activity on its regulation, the term is analogous, in certain cases, to other terms used throughout the present application such as factor involved in splicing regulation or splicing regulation factor or, simply, splicing factor (SF). The term "factor", as used in the present invention, refers to a molecule; then, a splicing regulation factor, as used in the present application, can be any compound exogenous to the cell, such as a drug or any other compound as to provoke a change in at least a cellular process, or biological compounds naturally involved in the regulation of splicing in cells, such as gene products like proteins or different RNA molecules (included snRNAs and siRNAs) or any other biological compound involved, for instance, in signaling or trafficking that might have an effect in the splicing process. When the term is applied to a gene product, it can be also extended to the gene itself encoding or giving rise to such product. The term "stimulus", in turn, has a broader meaning and embraces both factors and any kind of perturbation in the cell, such as induction of changes in cytoplasmic levels of iron.

A particular case of a splicing perturbing stimulus is the inhibition of the expression of a gene whose product is involved (or suspected to be involved) in splicing regulation. Such group of perturbing stimuli have been the ones used in Example 1 for building the splicing network that has been the basis of the additional assays described in Example 2, the examples where the network is applied for infering practical conclusions. The multiplicity of genes to be inhibited are selected from one or more of the following groups:

> a. components of the spliceosome core, that is, components of the core spliceosome compounds composed of U1, U2, U4/5/6 snRNPs and factors associated with A, B, Bact and C complexes (Wahl et al., 2009).
> b. auxiliary factors which are part of the splicing machinery and that are not components of the spliceosome core,
> c. factors involved in mRNA processing that are not part of the splicing machinery, such as those having roles in mRNA translation, polyadenylation, stability and localization in the cytoplasm, included molecules such as microRNAs
> d. genes involved in modulating chromatin structure and remodeling.

The inclusion of the latter, genes involved in modulating chromatin structure, is not only based on previous reports that indicate that such genes indeed have an effect of splicing regulation, but also in the genome-wide screening disclosed in section 1.1. of Example 1 of the present application, aimed to systemically identify regulators of a particular alternative splicing event, that of exon 6 of Fas/CD95 receptor, where several chromatin remodeling and histone modification factors were identified as Fas AS regulators (see Table 4). Some transcription factors and factors involved in RNA processing additional to splicing are also identified as Fas/CD95 alternative splicing regulators. Another important conclusion of the assays set forth in section 1.1. was the large number of factors involved in the regulation of a single alternative splicing event, that of exon 6 of Fas/CD95 receptor, which illustrate the importance of the method of the present invention as a research tool to evaluate the contribution of different classes of splicing regulators to AS regulation in general, handling with the huge amount of data obtained and creating an appropriate model of the intricate network of alternative splicing regulators acting in a cell for infering practical conclusions from it such as the identification of target modulations not only for a particular AS event but also for a particular physiological condition, physiological alteration or even a clinical condition such as a disease.

For that reason, in the assays described in section 1.2. of Example 1, where the construction of a specific functional network of splicing factors is disclosed, genes belonging to all the above-mentioned four groups of splicing factors were included as inhibited genes. Thus, it is a possible embodiment of the present invention that one wherein the multiplicity of genes whose expression is inhibited for generating the network comprises at least one gene selecting from each one of the above mentioned four groups. For the more general cases of generation of a model splicing network, it is important to include as many genes as possible of the two first categories, that is, factors forming part of the spliceosome, because such factors determine the group of basic interactions for the functioning of the machinery of intron removing. In particular, for the assays described in sections 1.2 and 1.3, the 271 human genes indicated in Table 1 were knocked down by transfecting a siRNA library.

The inhibition of gene expression can be done by transfecting a library of corresponding small interference RNAs (siRNAs), or by any other means known by those skilled in the art. There are different siRNA libraries commercially available suitable for the purpose of the present invention, such as ON TARGET PLUS (Dharmacon), where siRNAs for inhibiting genes of the three first categories can be found, or siGENOME (Dharmacon). Whatever the selected knocking down methodology, it is advisable to validate the obtained data with a second technique or a different library, so that the data are robust and reliable.

Once enough time has passed for the inhibition of the expression to occur, mRNA is isolated and processed. In the assays set forth in Example 1 of the present application which have been the basis for the network modeling, mRNA was purified 72 hours posttransfection. As commented at the end of section 1.2.a of Example 1, mRNA isolation 48 hours posttransfection does not mean any significant change in the results obtained. Moreover, as set forth in section 1.2.a of Example 1, cell viability is not generally compromised after 72 h of knockdown of 13 individual core splicing factors, which indicate that the changes in AS are not due to the induction of cell death upon depletion of essential SF even 72 hours posttransfection, so that the time points of 48 hours and 72 hours posttransfection and any other time point comprised between said points can be considered suitable for mRNA isolation, particularly when the assays are performed with HeLa cells.

For mRNA isolation and/or processing, an automatized system for mRNA isolation and cDNA amplification can be used, as in Example 1 of the present application. Subsequently, it is necessary to determine the impact that knocking down each of the genes has had in the use of any one of the set of alternative splicing sites selected for carrying out the method of the present invention. For determining such impact, the percentage of mRNAs samples where the exon has been excluded must be determined and compared with that of a control sample where no inhibition of the same gene has been induced and/or no perturbation stimulus has been provoked.

For determining the relative level of each one of the possible forms of the mRNA corresponding to a gene (with the alternatively spliced exon included or excluded), different techniques well known for those skilled in the art can be used, such as amplifying by PCR the cDNA obtained by retrotranscribing the isolated total mRNA. Total mRNA can be isolated by different known techniques, such as binding to oligo-dTs, which might be linked to a plate surface or to any material suitable for filling affinity columns. For PCR, the primers can be oligonucleotides complementary to the exon-flanking regions. The levels of each mRNA form can be measured by an appropriate technique, such as high throughput capillary electrophoresis. Other techniques or combinations of techniques can be equally used, such as assessing the rate of mRNA isoforms from cDNA amplification by PCR using barcoded primers and deep sequencing, as in the assays carried out on the specific regulation of Fas/CD95 alternative splicing, which can be carried out with equipments available in the market such as those initially developed by Solexa® and currently commercialised by Illumina. Although different techniques and equipments can be used, it is preferable to select them so that they are compatible with the processing of a high number of samples, as in the two approaches disclosed in Example 1 of the present application.

The alternative splicing events (ASE) analysed in the assays shown in section 1.2 of Example 1 were initially 47, as can be seen in Table 5, and were chosen because they are all events relevant for the regulation of cell proliferation and/or apoptosis. Detailed information about all of them can be found on https://s3.amazonaws.com/SplicingNet/ASEs.zip. Fig. 2B shows a classification of the corresponding genes, sorted out in accordance with the cellular process (apoptosis or cell proliferation) where they are involved. The particular set of ASE analysed in the Examples shown in the present application can be considered cancer-relevant, because the identification of new targets for the developing of anti-cancer drugs has been considered to be one of the important embodiments and practical applications, with clear industrial interest, of the method of the present invention, but the selection of other or additional subsets of ASE might be advisable when further insight in the mechanism of alternative splicing of other processes is desired or when targets for the modulation of other different processes, particularly those involved in disease where AS is altered, are sought.

It has been also taken into account that the assays for the constructions of the network has been carried out in HeLa cells and have been confirmed in HEK-293; the use of other cells or a particular interest in a metabolic process, alteration or disease might make preferable the election of a different set of AS events. Any different embodiments based on the selection of different sets of AS events or different cells will be also encompassed by the method of the present invention, and will be compatible with any other different embodiments related to other different features of the present invention.

As discussed in section 1.2.c, data analysis indicates that network reconstruction, as carried out in Example 1 of the present application, converges near 35 events. Therefore, in the method of the present invention at least the impact of each gene knockdown or any other splicing perturbing stimulus is assessed for at least 35 alternative splicing events.

As commented above, the assays set forth in the Examples of the present application have been carried out in HeLa cells and, in some cases, confirmed in HEK-293 cells. The fact that the network connectivity, as discussed below, recapitulates physical and functional links between factors belonging to well-established spliceosomal subcomplexes suggests that these interactions are likely to hold true in other cell types, so that the particular embodiments of the method of the invention, selected in the Examples of the present application, for modelling the network and the network obtained can be considered applicable as a starting point for some applications. It is advisable, however, to apply all the steps of the method of the present invention for each particular different cell and generating the corresponding network, because particular details of the network will probably vary in different cell types.

Once obtained, the processing of the data and the design of a framework for analysing such data and modelling a network from them is another important pillar of the method of the present invention. In particular, the generation of perturbation profiles and their covariance analysis, as well as the processing of the results of such analysis for modelling the network are key for the invention. Thus, the covariance analysis performed and the careful selection of the statistical-computerized tools applied, as well as the specific parameters and conditions chosen for their implementation for the purposes of the present invention have been relevant for the developing it, as previously commented. Although some of the used computer resources are known, the modification performed on some of them and, mainly, their combination and subsequent application not only have enabled the development of the present invention but also have allowed to obtain, through the application of the method of the present invention, a robust and versatile network, easy to visualize and analyze.

For the generation of the perturbation profile corresponding to each perturbing stimulus and, particularly, to the knockdown of each specific gene (when that is the perturbing stimulus), each data corresponding to the percentage of mRNA isoforms with the exon included (PSI) is transformed in its corresponding Z-score (http://en.wikipedia.org/wiki/Standard score; see below the methodological section of the Examples for details about the calculations applied for the assays of the present application).

In accordance with the method of the invention and the approach followed in the Examples of the present application, the perturbation profiles of alternative splicing events are the lines which connect Z-score values obtained for a particular perturbing stimulus for each one the alternative splicing events analyzed in the study. For each perturbing stimulus (for each inhibited gene in the case of the assays of Example 1), the perturbation profile represents its impact along the alternative splicing events analyzed with regard to its magnitude and change direction. Fig. 2E shows the perturbation profiles obtained in Example 1 upon knocking down four different genes, the first two with similar profiles, whereas the other two have opposed profiles, that is, for most ASEs analyzed, the effect of knocking down one of them is opposed to the effect of knocking down the other one.

The construction of the interaction network is based on quantifying the similarity between two perturbation profiles for each pair of factors (inhibited genes or perturbation stimulus in general) included in the study. That quantification has been referred above as covariance analysis.

But another important step before comparing the data corresponding to different perturbation profiles is preprocessing and reducing the obtained data of the impact of each perturbation in each selected alternative splicing event. It comprises removing sparse variables and imputing missing values, removing uninformative events and scaling data by standardization. The specific approaches carried out for the assays of the Examples of the present application are described in the methodological section located immediately before the Examples, under the subheading "Network construction. Data preprocessing and reduction". The scaling of each knocking down is important, because it has as effect that only the relative magnitude (i.e. the shape, not the scale) of the perturbation profiles is important for inferring associations between different factors.

Once said preprocessing has been carried out, the estimation of correlations of the effects of inhibiting the selected genes and/or submitting cells to a perturbation stimulus is carried out. This measure captures the congruence between the shapes of perturbation profiles, while it does not take into account proportional differences in the magnitude of the fluctuations. Crucially, it discriminates between biological and technical outliers and is resistant to distorting effects of the latter.

As explained in the methodological section placed before the Examples themselves, the estimation of covariance of perturbation profiles of alternative splicing events of the method of the invention, which is the one carried out for the assays of the present Examples, is performed by quantifying the correlation between each pair of perturbation profile basing it on an iterative weighting algorithm, so that the estimated correlation for two pairs of genes or perturbing stimuli $X_a$ and $X_b$ is the weighted Pearson's correlation

$$p(X_a, X_b; w)$$

wherein $w$ is a vector whose length is equal to the number $n$ of alternative splicing events that is processed after data preprocessing, which vector contains the reliability values $R^u_{a,b}$ corresponding to each Z-score for the estimation of the correlation between two perturbation profiles, wherein the values $R^u_{a,b}$ are calculated with the formula

$$R^u_{a,b} = 1 \Big/ \frac{\sum_{i=1}^{p} D_u(X_a, X_i)\mathbb{I}(a, i) + \sum_{i=1}^{p} D_u(X_b, X_i)\mathbb{I}(b, i)}{\sum_{i=1}^{p} \mathbb{I}(a, i) + \sum_{i=1}^{p} \mathbb{I}(b, i)}$$

where II(*a,i*) is the indicator function:

$$\mathbb{I}(a,i) := \begin{cases} 1 \text{ if } i \neq a \ AND \ |p(X_a, X_i)| > T \\ 0 \text{ otherwise} \end{cases}$$

*T* being a minimum correlation threshold for considering a pair of variables, and $D_u(X_a, X_b)$ is the deleted residual distance

$$D_u(X_a, X_b) = |p(X_a, X_b) - p_{-u}(X_a, X_b)|$$

wherein $p(X_a, X_b)$ and $p_{-u}(X_a, X_b)$ are the Pearson's correlation estimates before or after removing the observations $M_{au}$, $M_{bu}$ coming from an splicing event *u*, and using iteratively said algorithm until estimations converge.

This approach for estimation the correlation between all possible pairs of perturbation profiles is the one of the present invention. Other approaches will be also compatible with the method disclosed in the present application and are combinable with any embodiment corresponding to any other feature of the present disclosure. Preferably, any methodology selected for estimating the correlation among profiles should be also based on an algorithm that discriminates among outliers arising from technical reason and those corresponding to reliable measures.

Once obtained the matrix of data resulting from the correlation estimation, graphical lasso (gLasso) algorithm was applied in the assays of Example 1, because it provides an attrative solution to the problem of covariance estimation for undirected models, when graph sparsity is a goal. The specific steps carried out for network reconstruction, partition of the network into modules and graph manipulation and plotting can be found in the methodological subsection placed before the Examples, with said same subheadings ("Network reconstruction: Graphical model selection using Graphical Lasso", "Network reconstruction. Partition of network into modules" and "Graph Manipulation and Plotting"). Any other algorithms for the generation of graphical models and their subsequent partition into modules could have been also applied, provided that they would have been implemented in order to handle the matrix of data arising from the estimations of correlations. It must be also taken into account that, in the approach set forth in Example 1, the present inventors identify modules of genes that exhibit similar perturbation profiles among the assayed events by maximizing the network modularity, specifically using the greedy community detection algorithm, which has been of help to group genes with physical associations, such as those corresponding to the spliceosome, and even recapitulating their functionality and even the moment of association to the spliceosome, an advantage that should preferably try to maintain in case alternative algorithm are selected for carrying out a method like the one of the present disclosure. The method of the present invention is performed with the algorithms and implementations detailed in the Examples section and its methodological subsection.

Additionally to the application of gLasso and the partition into modules, it is also important for reconstructing the network specifying a regularization parameter, which can be seen as a penalty that can be tuned to control the number of inferred connections together with the false discovery rate (FDR) of the final model. For a given regularization parameter, both the number of inferred connections and the FDR are inversely correlated with the number of assayed ASEs. The random subsampling carried out as described in the methodological subsection "Data subsampling for identification of indispensable and ancillary connections" and in section 2.1. of Example 2 indicates that the network reconstruction converges to -500 connections with FDR<5% and approximately 35 ASEs, which is an indication that, at least for the particular network constructed, including additional ASEs in the first steps of the method might have little effect in the accuracy of the network.

Performing the method of the present invention as above explained, and as it is detailed in section 1.2 of Example 1 of the present application, gave rise to the network of functional interactions depicted in Fig. 4A. It comprises 196 nodes, which represent the regulatory factors (knocked down genes maintained after preprocessing and reduction of data) and 541 connections (edges), 518 corresponding to positive functional associations (green connecting lines in the original) and 23 corresponding to negative associations (red connecting lines in the original). Line thickness is proportional to the strength of the functional interaction and node size is proportional to the overall impact (median Z-score) of a given knockdown in regulation of AS. Known physical interactions, as reported in the STRING database, are represented adding a black dotted line over the line connecting two nodes (factors).

The topology of the network recapitulates the topology of the known splicing complexes and provides an extended map of more than 500 functional associations. It can be easily identified a "central" grouping of densely connected factors where the components of the spliceosome core are comprised, where the factors physically associated to U2 snRNP and factors functionally related with its function can be found, as well as an adjacent grouping, corresponding to the factors physically associated to snRNP U5 or U4/U5/U6 snRNP. In the periphery there are factors with a lower connectivity with some connections towards the central core, where the classical splicing regulatory factors can be found, as well as several factors involved in modelling chromatin structure. An intermediate category, with a lower density of connections with regard to the central core, corresponds to RNA dependent DEAD/X box helicases. Persistent factors of the splicesome core are particularly well connected ones to the others.

The results obtained warrant that the comparison of perturbation profiles of alternative splicing can be used as an appropriate approach for finding physical or functional connections among regulatory factors of the splicing process or even functional synergies among them.

The obtained network provides comprehensive information about SFs (and some additional chromatin factors) relevant to understand the regulation of 35 AS events important for cell proliferation and apoptosis. But not all connections correspond to previously known associations, which demonstrates the utility of the network as a tool for getting further insight in the mechanism of alternative splicing, so that the method of the present invention, based on the combined experimental and statistical approach described in detail in the Examples of the present application, provides a powerful toolset for studying the splicing process and its regulation. It can be useful to systematically capture information about other aspects of transcriptional or post-transcriptional gene regulation by analizyng the functional connections of factors known to be involved in such process, also providing new targets for transcriptional or post-transcriptional regulation of particular genes

The network analysis also reveals functional links between SFs, which in some cases are based upon physical interactions. The reconstruction of the known topology of some complexes by the network suggests that the approach captures important aspects of the operation of these particles and therefore, the method of the present invention has the potential to provide novel insights into the composition and intricate workings of multiple Spliceosomal sub-complexes. In fact, the method can be used for identifying splicing factors forming part of a same splicing subcomplex, as those splicing factors that correspond to nodes that appear clustered in a same region of a network and with connections among them. Preferably, said nodes will be highly connected among them to identify them as corresponding to factors belonging to the same splicing subcomplex.

As an example of applications related to broaden the knowledge about the splicing process and the intricate network of connections among the factors involved in it, the method of the invention can be used to identify, for instance, functional connections that persist in all kinds of alternative splicing events and that can be considered to correspond to general interactions among splicing factors, essential for the process being carried out irrespective of the alternative splicing event considered, and those corresponding to associations of factors that are specific for certain ASEs or groups of ASEs. Such possible embodiments of the method of the invention are exemplified in sections 2.1.a and 2.1.b of Example 2 and can even be considered an additional aspect of the present invention, due to the utility to identify the specific splicing regulatory factors implied in certain conditions, such as particular disease conditions.

Additionally, the method of the present invention can serve as a resource for exploring mechanisms of AS regulation induced by perturbations of the system, including genetic manipulations, internal or external stimuli or exposure to drugs. As an example, as it is disclosed in section 2.3., the network can be used to identify a link between AS changes and variations in the cellular condition or status (in the case of section 2.3.a, variations in intracellular iron). Firstly, as explained in section 2.3.a of Example 2, a link between intracellular iron availability and differential regulation of Fas/CD95 alternatively spliced isoforms was revealed. Secondly, it was found that, after treatment with the compound Hemin, which induces iron excess and increased Fas/CD95 exon 6 inclusion, a significant correlation was found between hemin and the function of SRSF7, which can be explained in terms of iron-induced changes in the RNA binding and splicing regulatory activity of this Zinc-knuckle-containing SR protein. Other examples include a) the identification of the extensive regulatory overlap between the interacting proteins IK/RED and SMU1, relevant for the control of programmed cell death; b) evidence for a general role of uridine-rich hnRNP C binding sequences (some associated with transposable elements, Zarnack et al., 2013) in the regulation of nearby alternative exons via antagonism with U2AF; and c) delineation of U2 snRNP components and other factors, including PPIH, that are functionally linked to the effects of anti-tumor drugs on AS.

The assays set forth in the present application reveal changes of AS regulation mediated either by core splicing components or by classical regulatory factors like proteins of the SR and hnRNP families. While the central core of functional links is likely to operate globally, alternative configurations (particularly in the periphery of the network) are likely to emerge if similar approaches are applied to other cell types, genes or biological contexts, what makes advisable, as previously commented, to select the cell types wherein the assays are carried out, the set of inhibited genes and/or additional perturbing stimulus for submitting cells to them, and the set of alternative splicing events specially for purpose which is sought when the method of the present invention is applied.

Regarding the broadening in the knowledge of the spliceosomal machinery itself that the present invention can provide, it is remarkable that the assays set forth in the present application suggest a considerable versatility in the effects of core spliceosome factors on alternative splicing. In contrast with the standard function of classical splicing regulators acting through cognate binding sites specific of certain target RNAs, core factors could both carry out general, essential functions for intron removal and in addition display regulatory potential if their levels become limiting for the function of complexes, leading to differences in the efficiency or kinetics of assembly on alternative splice sites. This could be the case for the knockdown of SmB/B', a component of the Sm complex present in most Spliceosomal snRNPs, which leads to autoregulation of its own pre-mRNA as well as to effects on hundreds of other ASEs, particularly in genes encoding RNA processing factors (Saltzman et al., 2011).

The results of the genome-wide siRNA screen for regulators of Fas AS set forth in section 1.1. of Example 1 of the present application provide unbiased evidence that an extensive number of core SFs have the potential to contribute to the regulation of splice site selection. This is in fact the most populated category of screen hits. Similarly, the results inferred form the network highlight coherent effects on alternative splice site choice of multiple core components, revealing also that the extent of their effects on alternative splice site selection can be largely attributed to the duration and order of their recruitment in the splicing reaction.

Remarkably, effects on splice site selection are associated with depletion not only of complexes involved in early splice site recognition, but also of factors involved in complex B formation or even in catalytic activation of fully-assembled spliceosomes. While particular examples of AS regulation at the time of the transition from complex A to B and even at later steps had been reported, the results disclosed in the present application indicate that the implication of late factors is quite general. Such links, e.g. those involving PRP8 and other interacting factors closely positioned near the reacting chemical groups at the time of catalysis, like PRP31 or U5 200KD, are actually part of the persistent functional interactions that emerge from the analysis of any subsample of ASEs analyzed, highlighting their general regulatory potential in splice site selection. Regarding how factors involved in late steps of the splicing process can influence splice site choices, it is conceivable that limiting amounts of late spliceosome components would favor splicing of alternative splice site pairs harboring early complexes that can more efficiently recruit late factors, or influence the kinetics of conformational changes required for catalysis. In this context, the regulatory plasticity revealed by the present network analysis may be contributed, at least in part, by the emerging realization that a substantial number of SFs contain disordered regions when analyzed in isolation. Such regions may be flexible to adopt different conformations in the presence of other spliceosomal components, allowing alternative routes for spliceosome assembly on different introns, with some pathways being more sensitive than others to the depletion of a general factor.

Kinetic effects on the assembly and /or engagement of splice sites to undergo catalysis would be particularly effective if spliceosome assembly is not an irreversible process. Results of single molecule analysis are indeed compatible with this concept (Tseng and Cheng, 2008; Abelson et al., 2010; Hoskins et al., 2011; Hoskins and Moore, 2012; Shcherbakova et al., 2013), thus opening the extraordinary complexity of conformational transitions and dynamic compositional changes of the spliceosome as possible targets for regulation. Interestingly, while depletion of early factors tends to favor exon skipping, depletion of late factors causes a similar number of exon inclusion and skipping effects, suggesting high plasticity in splice site choice at this stage of the process.

Given this potential, it is conceivable that modulation of the relative concentration of core components can function as a physiological mechanism for splicing regulation, for example during development and cell differentiation. Indeed, variations in the relative levels of core spliceosomal components have been reported (Wong et al., 2013). Furthermore, recent reports revealed the high incidence of mutations in core SFs in cancer. For example, the gene encoding SF3B1, a component of U2 snRNP involved in branch point recognition, has been described as among the most highly mutated in myelodysplastic syndromes (Yoshida et al., 2011), chronic lymphocytic leukemia (Quesada et al., 2011) and other cancers (reviewed in Bonnal, 2012), correlating with different disease outcomes. Remarkably, SF3B1 is the physical target of anti-tumor drugs like Spliceostatin A and -likely- Meayamycin, as captured by the assays set forth in Example 2 of the present application. These observations are again consistent with the idea that depletion, mutation or drug-mediated inactivation of core SFs may not simply cause a collapse of the splicing process -at least under conditions of limited physical or functional depletion- but rather lead to changes in splice site selection that can contribute to physiological, pathological or therapeutic outcomes. SF3B1 participates in the stabilization and proofreading of U2 snRNP binding to the branch point region and it is therefore possible that variations in the activity of this protein result in switches between alternative 3' splice sites harboring branch sites with different strengths and/or flanking decoy binding sites. Similar concepts may apply to explain the effects of mutations in other SFs linked to disease, including for example mutations in PRP8 leading to Retinitis Pigmentosa.

All the mentioned results and hypothesis inferred therefrom about the functioning of the spliceosome and its physiological regulation illustrate how the method of the present invention, of generation and analysis of a network of functional associations among factors involved in alternative splicing regulation, means by itself a research tool of great interest (comparable or greater than that of some apparatus, compounds or kits that are purchased for research purposes) for broadening the knowledge of splicing functioning and regulation and reaching conclusion that can be the basis and/or a starting point for envisaging or developing more direct industrial application, such as the identification and developing of compounds capable of modulate certain metabolic processes or even capable of acting on steps of the process that lead to altered alternative splicing and, as a consequence, to misfunctions, clinical conditions or diseases related to altered alternative splicing.

The applicability of the method of the present invention as a piece in the development of applications with more direct industrial interest can be seen more clearly when it is considered the potentiality of the method when the set of perturbing stimuli selected for subjecting cell to them is not only restricted to the inhibition / knocking down of specific genes that are (at least potentially) splicing regulatory factors, but at least a different perturbing stimulus is included, such as submitting the corresponding cell or cells to the action of an added compound.

The drug can be, for example, a drug known to have a therapeutic effect on a disease associated with altered alternative splicing, as it has been done in section 2.2 of Example 2 of the present application, where the known anti-cancer drug Spliceostatin A, and a structurally similar drug, Meayamycin, have been added to cells. Applying the method of the invention including such perturbing stimuli gives rise to finding that both drugs have direct connections with SF3B1, which is a known physical target of Spliceostatin, which confirms said factor as a target of interest for the identification and developing of other anti-cancer drugs. Performing the method of the invention with a different drug would allow to identify possible targets for the development of additional drugs against the disease where such drug has a therapeutic activity, as the splicing regulatory factors whose node in the network has a direct connection with the node corresponding to the added drug. Those compounds having one of such factors as targets could be considered candidates to drugs against the disease of interest. The approach of Example 2 shows a method for the identification of new potential anti-cancer drugs from the previous identification of the targets where they can be directed.

Alternatively, an added compound can be identified as a drug for the treatment of a disease associated with altered alternative splicing when it has at least a direct connection in common with a second compound, which second compound is a drug known to have a therapeutic effect on a disease associated with altered alternative splicing. The second compound can also have been added as a perturbing stimulus for carrying out the method of the invention or their functional associations can have been previously identified, either by the method of the present invention or by any other method.

Thus, the method of the present invention can be of help for finding additional targets for the developing of drugs or even for finding drugs against diseases so important as cancer, Duchenne muscular dystrophy, spinal muscular atrophy, or other neuromuscular or neurological disease that are associated to altered alternative splicing.

Analogously to the embodiments disclosed for drugs, the method of the invention can be also of use for identifying new targets for the development of modulators of physical process where an added compound is known to act as a modulator or new candidates to modulators of said process. Noteworthy, the fact that the network modelled for carrying out the method of the present invention also shows which connections correspond to positive or negative functional associations among the splicing factors represented in the nodes and, even, the width of the connecting lines is proportional to the strength of association, the method of the invention and its network can be used for selecting candidates to compounds having a different modulating direction that the compound whose target is already known. The case of hemin and desferal can illustrate this potentiality: the fact that their connection is negative indicates that they have opposite effects regarding intracellular iron, information which could be of use when the activity of one of them is not known and a compound when the opposed activity is sought.

Finally, the method can even be carried out both with cells removed from a patient suffering from a disease or malfunction and with a different kind of cells deriving from and individual not suffering that disease or malfunction and those factors showing different connection in the two generated networks could be identified as targets for directing to them compounds intended to be candidates to drugs against said disease or malfunction.

In summary, the data and methodological approaches presented in this study provide a rich resource for understanding the function of the spliceosome and the mechanisms of AS regulation, including the identification of targets of physiological, pathological or pharmacological perturbations within the complex splicing machinery.

**EXAMPLES**

The assays described in the following Examples were carried out using the following methodologies and materials:

- Cell lines

HeLa CCL-2 cells and HEK293 cells were purchased from the American Type Culture Collection (ATCC). Cells were cultured in Glutamax Dulbecco's modified Eagle's medium (Gibco, Life Technologies) supplemented with 10% fetal bovine serum (Gibco, Life Technologies) and penicillin/streptomycin antibiotics (penicillin 500 u/ml; streptomycin 0.5 mg/ml, Life Technologies). Cell culture was performed in cell culture dishes in a humidified incubator at 37°C under 5% $CO_2$.

- Cell proliferation assays

HeLa cells were forward-transfected with siRNAs targeting IK, SMU1 or both genes by plating 2500 cells over a mixture containing siRNA-RNAiMAX lipofectamine complexes, as previously described. Indirect estimations of cell proliferation were obtained 24, 48 and 72 hours post transfection by treating the cells for 4h with resazurin (5 $\mu$M,) prior to the fluorescence measurements (544 nm excitation and 590 nm emission wavelengths). Plate values were obtained with an Infinite 200 PRO series multiplate reader (TECAN).

- <u>Cell apoptosis assays: siRNAs against IK, SMU1</u>

Hela cells were transfected with siRNAs against IK, SMU1 or both genes, as described above. Cells were trypsinized and collected for analysis 72 hours post transfection. Pellets were washed once with complete DMEM medium (10% serum plus antibiotics), and three times with PBS 1X. Cells were then resuspended in 200 $\mu$l Binding buffer provided by the Annexin V-FITC Apoptosis Detection Kit (eBiosciences), resulting in a final density of $4 \times 10^5$ cells/ml. 5 $\mu$l of Annexin V-FITC was added to each sample followed by 10 min incubation at room temperature in dark conditions. Cells were washed once with 500 $\mu$l binding buffer and incubated with 10 $\mu$l propidium iodide (20 $\mu$g/ml) for 5 minutes. FACS analysis was performed using a FACSCalibur flow cytometry system (BD Biosciences).

- <u>Cell apoptosis assays: Fas-mediated apoptosis.</u>

PBMCs cells were supplemented over a total period of 48 hours with PHA (10 $\mu$g/ml, Sigma Aldrich) or vehicle. 24 hours after initial stimulation, cells were treated for a period of 12 hours with either mock, hemin or desferal conditions (100 $\mu$M) and cells were washed twice with PBS 1X before apoptosis induction. Fas mediated apoptosis was induced by incubating treated cells with anti-Fas antibody (1 $\mu$g/ml, Human activating clone CH11, Millipore) or isotype control IgG over a period of 16 hours. Cells were collected and pellets were washed two times with PBS 1X, resuspended and incubated in blocking buffer containing human IgG and 1% BSA for 15 min. Cells were stained with 10 $\mu$l human anti-CD3 antibody labeled with PerCP (BD Biosciences) for 30 min and washed one with PBS 1X. Cells were then resuspended in 100 $\mu$l binding buffer provided by the Annexin V-FITC Apoptosis Detection Kit (eBiosciences), resulting in a final density of $6 \times 10^5$ cells/ml. 5 $\mu$l of annexin V-FITC was added at each sample followed by 10 min incubation at room temperature in dark conditions. Cells were washed with 500 $\mu$ binding buffer and incubated with 1:10000 DAPI dilution for 5 minutes. FACS analysis was performed using a LSR II flow cytometry system (BD Biosciences).

- <u>Affymetrix arrays</u>

HeLa cells were transfected with siRNAs against IK, SMU1 or RBM6 in biological triplicate as described above or -for RBM6- as described in Bechara et al., 2013. RNA was isolated using the Rneasy minikit (Qiagen). RNA quality was estimated by Agilent Bionalyzer nano assay and samples were hybridized to GeneChip Human Exon 1.0 ST Array (Affymetrix) by Genosplice Technology (http://www.genosplice.com/services/rna-analysis). Array analysis was performed by Genosplice Technology and the date (gene expression, alternative splicing changes and gene ontologies upon IK, SMU1 knockdown conditions) is available at: https://s3.amazonaws.com/SplicingNet/HJAY arrays.zip and at GEO database with the accession number GSE56605.

- <u>siRNA library Transfections and mRNA isolation</u>

For the genome-wide screening carried out about Fas/CD95 receptor, HeLa cells were forward transfected in triplicate in 96 well plates with a siRNA library against known and predicted protein-coding human genes, comprising >21000 siRNAs (genome wide screen, SMARTpools siGENOME, Thermo-Scientific) or 250 spliceosome related siRNAs (Pilot screen, SMARTpools ON TARGET PLUS, Thermo-Scientific) using an automatized robotic procedure (Sciclone Liquid Handling workstation, Perkin Elmer).

In the assay directed to the generation of the network, HeLa cells were transfected in biological triplicates with siRNAs pools (ON TARGET plus smartpool, Dharmacon, Thermo Scientific) against 270 splicing and chromatin remodeling factors (see Table 1).

In both cases, endogenous cellular mRNAs were purified 72 hours post transfection by an automated producer using oligo dT-coated 96 well plates (mRNA catcher PLUS, Life Technologies) following the manufacturer's instructions. RNA samples corresponding to treatments with splicing arresting drug were isolated with the Maxwell 16 LEV simplyRNA kit (Promega).

## Table 1.- Knocked-down splicing and chromatin remodeling factors

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| 1 | A01 | - | - | No cells | | - | - | / | NA | NA |
| 2 | A02 | - | - | No cells | | - | - | / | NA | NA |
| 3 | A03 | L-005151-01 | J-005151-09 | SRSF4 | SRP75 | 6429 | NM_005626 | SR proteins / SR proteins | Spl. Factors | SR proteins |
| 4 | A04 | L-011929-00 | J-011929-05 | SRSF11 | p54 | 9295 | NM_004768 | SR proteins / SR proteins | Spl. Factors | SR proteins |
| 5 | A05 | L-016067-01 | J-016067-09 | SRSF6 | SRP55 | 6431 | NM_006275 | SR proteins / SR proteins | Spl. Factors | SR proteins |
| 6 | A06 | L-007279-01 | J-007279-09 | SRSF5 | SRP40 | 6430 | NM_006925 | SR proteins / SR proteins | Spl. Factors | SR proteins |
| 7 | A07 | L-018672-01 | J-018672-09 | SRSF1 | SF2 / ASF | 6426 | NM_006924 | SR proteins / SR proteins | Spl. Factors | SR proteins |
| 8 | A08 | L-015909-00 | J-015909-05 | SRSF7 | 9G8 | 6432 | NM_001031684 | SR proteins / SR proteins | Spl. Factors | SR proteins |
| 9 | A09 | L-019711-00 | J-019711-05 | SRSF2 | SC35 | 6427 | NM_003016 | SR proteins / SR proteins | Spl. Factors | SR proteins |
| 10 | A10 | L-019529-01 | J-019529-09 | SRSF9 | SRP30C | 8683 | NM_003769 | SR proteins / SR proteins | Spl. Factors | SR proteins |
| 11 | A11 | L-007278-00 | J-007278-05 | SRSF10 | hTRA2B | 6434 | NM_004593 | SR proteins / SR proteins | Spl. Factors | SR proteins |
| 12 | A12 | L-030081-00 | J-030081-05 | SRSF3 | SRP20 | 6428 | NM_003017 | SR proteins / SR proteins | Spl. Factors | SR proteins |
| 13 | B01 | - | - | Untransfected | | - | - | / | NA | NA |
| 14 | B02 | - | - | Untransfected | | - | - | / | NA | NA |
| 15 | B03 | L-015368-00 | J-015368-07 | SRRM2 | SRM300 | 23524 | NM_016333 | SR related proteins / Non-snRNP spliceosome-assembly proteins | Spl. Factors | NA |
| 16 | B04 | L-021234-01 | J-021234-09 | DDX46 | PRP5 homolog | 9879 | NM_014829 | U2 snRNP related / Non-snRNP spliceosome-assembly proteins | Core Spl. | Trans. Early Spl. |
| 17 | B05 | L-019013-02 | J-019013-17 | CRNKL1 | hCRN | 51340 | NM_016652 | hPrp19 / Cdc5L related / Non-snRNP spliceosome-assembly proteins | Core Spl. | Trans. Mid Spl. |

EP 3 029 149 B1

| siRNA | Well | Pool Catalog Number | Duplex Catalog Number | Gene Symbol | Other names | GENE ID[1] | Gene Accession[2] | Luhrmann classification / Robin Reed classification | Network Classification (1st level)[3,4] | Network Classification (2nd level)[3,4] |
|---|---|---|---|---|---|---|---|---|---|---|
| 18 | B06 | L-011237-00 | J-011237-05 | CDC5L | hCDC5 | 988 | NM_001253 | hPrp19 / Cdc5L complex / Non-snRNP spliceosome-assembly proteins | Core Spl. | Trans. Mid Spl. |
| 19 | B07 | L-019672-00 | J-019672-05 | NCBP1 | CBP80 | 4686 | NM_002486 | Cap binding complexes / Non-snRNP spliceosome-assembly proteins | Other | NA |
| 20 | B08 | L-012662-01 | J-012662-09 | SF1 | ZNF162 | 7536 | NM_201997 | abundant in first or only present in A complex / Non-snRNP spliceosome-assembly proteins | Core Spl. | Trans. Early Spl. |
| 21 | B09 | L-012446-00 | J-012446-05 | SKIIP | SNW1 , PRPF45 | 22938 | NM_012245 | hPrp19 / Cdc5L related / Non-snRNP spliceosome-assembly proteins | Core Spl. | Trans. Mid Spl. |
| 22 | B10 | L-012505-01 | J-012505-09 | SIAHBP1 | PUF60 | 22827 | NM_014281 | U2 snRNP related / Non-snRNP spliceosome-assembly proteins | Core Spl. | Trans. Early Spl. |
| 23 | B11 | L-019593-00 | J-019593-05 | PLRG1 | PRL1, PRPF46 | 5356 | NM_002669 | hPrp19 / Cdc5L complex / Non-snRNP spliceosome-assembly proteins | Core Spl. | Trans. Mid Spl. |
| 24 | B12 | L-004668-00 | J-004668-05 | PRPF19 | PSO4 | 27339 | NM_014502 | hPrp19 / Cdc5L complex / Non-snRNP spliceosome-assembly proteins | Core Spl. | Trans. Mid Spl. |
| 25 | C01 | - | - | Mock SiRNA | | - | - | / | NA | NA |
| 26 | C02 | - | - | Mock SiRNA | | - | - | / | NA | NA |
| 27 | C03 | L-012380-02 | J-012380-18 | U2AF2 | U2AF65 | 11338 | NM_001012478 | U2 snRNP related / Non-snRNP spliceosome-assembly proteins | Core Spl. | Trans. Early Spl. |
| 28 | C04 | L-012325-01 | J-012325-09 | U2AF1 | U2AF35 | 7307 | NM_006758 | U2 snRNP related / Non-snRNP spliceosome-assembly proteins | Core Spl. | Trans. Early Spl. |
| 29 | C05 | L-016257-00 | J-016257-05 | SMNDC1 | SMNR, SPF30 | 10285 | NM_005871 | U2 snRNP related / Non-snRNP spliceosome-assembly proteins | Core Spl. | Trans. Early Spl. |
| 30 | C06 | L-015327-00 | J-015327-05 | NCBP2 | CBP20 | 22916 | NM_007362 | CBP20 / Non-snRNP spliceosome-assembly proteins | Other | NA |
| 31 | C07 | L-027984-01 | J-027984-09 | FNBP3 | PRPF40A | 55660 | XM_938514 | abundant in first or only present in A complex / Non-snRNP spliceosome-assembly proteins | Core Spl. | Trans. Early Spl. |
| 32 | C08 | L-018811-00 | J-018811-05 | SNRP70 | U1AP1, U1-70K | 6625 | NM_003089 | U1 snRNP / U1 snRNP specific proteins | Core Spl. | Trans. Early Spl. |
| 33 | C09 | L-019435-02 | J-019435-17 | SNRPA | U1A, mud1 | 6626 | NM_004596 | U1 snRNP / U1 snRNP specific proteins | Core Spl. | Trans. Early Spl. |
| 34 | C10 | L-019574- | J-019574- | SNRPC | U1C | 6631 | NM_003093 | U1 snRNP / U1 snRNP specific proteins | Core Spl. | Trans. Early Spl. |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | 02 | 17 | | | | | | | |
| 35 | C11 | L-020061-01 | J-020061-13 | SF3B1 | SAP155, SF3b155 | 23451 | NM_001005526 | U2 snRNP / U2 snRNP specific proteins | Core Spl. | Persist. Spl. |
| 36 | C12 | L-026599-01 | J-026599-09 | SF3B2 | SAP145, SF3b145 | 10992 | NM_006842 | U2 snRNP / U2 snRNP specific proteins | Core Spl. | Persist. Spl. |
| 37 | D01 | L-011803-00 | J-011803-05 | PABPN1 | PABP2 | 8106 | NM_004643 | 3´end processing / 3´end processing | Other | NA |
| 38 | D02 | L-006455-00 | J-006455-06 | SFPQ | PSF | 6421 | NM_005066 | other / other | Other | NA |
| 39 | D03 | L-020085-00 | J-020085-05 | SF3B3 | SAP130, SF3b130 | 23450 | NM_012426 | U2 snRNP / U2 snRNP specific proteins | Core Spl. | Persist. Spl. |
| 40 | D04 | L-016051-00 | J-016051-05 | SF3A1 | SF3a120,l SAP114, PRPF21 | 10291 | NM_001005409 | U2 snRNP / U2 snRNP specific proteins | Core Spl. | Persist. Spl. |
| 41 | D05 | L-018282-02 | J-018282-17 | SF3A2 | SAP62, PRPF11, SF3a66 | 8175 | NM_007165 | U2 snRNP / U2 snRNP specific proteins | Core Spl. | Persist. Spl. |
| 42 | D06 | L-019808-00 | J-019808-05 | SF3A3 | SAP61, SF3a60, PRPF9 | 10946 | NM_006802 | U2 snRNP / U2 snRNP specific proteins | Core Spl. | Persist. Spl. |
| 43 | D07 | L-017190-00 | J-017190-05 | SF3B4 | SAP49, SF3b49 | 10262 | NM_005850 | U2 snRNP / U2 snRNP specific proteins | Core Spl. | Persist. Spl. |
| 44 | D08 | L-019577-01 | J-019577-09 | SNRPA1 | U2 snRNP A, Lea1 | 6627 | NM_003090 | U2 snRNP / U2 snRNP specific proteins | Core Spl. | Persist. Spl. |
| 45 | D09 | L-016910-01 | J-016910-09 | SNRPB2 | U2 snRNP B, msl1 | 6629 | NM_198220 | U2 snRNP / U2 snRNP specific proteins | Core Spl. | Persist. Spl. |
| 46 | D10 | L-020260-02 | J-020260-18 | P14 | SF3B14, SAP14 | 51639 | NM_016047 | U2 snRNP / U2 snRNP specific proteins | Core Spl. | Persist. Spl. |
| 47 | D11 | L-012252-00 | J-012252-06 | PRPF8 | 220 kDa U5 | 10594 | NM_006445 | U5 snRNP / U5 snRNP specific proteins | Core Spl. | Persist. Spl. |
| 48 | D12 | L-014161-00 | J-014161-05 | U5-200KD | SNRNP200, 200 kDa | 23020 | NM_014014 | U5 snRNP / U5 snRNP specific proteins | Core Spl. | Persist. Spl. |

| siRNA | Well | Pool Catalog Number | Duplex Catalog Number | Gene Symbol | Other names | GENE ID[1] | Gene Accession[2] | Luhmann classification / Robin Reed classification | Network Classification (1st level)[3,4] | Network Classification (2nd level)[3,4] |
|---|---|---|---|---|---|---|---|---|---|---|
| | | | | | U5, Brr2 | | | | | |
| 49 | E01 | L-007756-01 | J-007756-09 | NONO | P54NRB | 4841 | NM_007363 | other / other | Other | NA |
| 50 | E02 | L-020672-01 | J-020672-09 | RBM35A | ESRP1 | 54845 | NM_017697 | other / other | Other | NA |
| 51 | E03 | L-019851-01 | J-019851-09 | U5-116KD | 116 kDa U5, hSNU114, EFTUD2 | 9343 | NM_004247 | U5 snRNP / U5 snRNP specific proteins | Core Spl. | Persist. Spl. |
| 52 | E04 | L-012821-01 | J-012821-09 | C20ORF14 | PRP6 homolog, 102 kDa U5 | 24148 | NM_012469 | U5 snRNP / U5 snRNP specific proteins | Core Spl. | Trans. Early Spl. |
| 53 | E05 | L-019861-01 | J-019861-09 | DDX23 | PRP28 homolog, 100 kDa U5 | 9416 | NM_004818 | U5 snRNP / U5 snRNP specific proteins | Core Spl. | Trans. Early Spl. |
| 54 | E06 | L-020057-00 | J-020057-05 | CD2BP2 | Snu40, 52 kDa U5 | 10421 | NM_006110 | U5 snRNP / U5 snRNP specific proteins | Core Spl. | Trans. Early Spl. |
| 55 | E07 | L-019860-00 | J-019860-05 | HPRP8BP | SNRNP40, 40 kDa U5 | 9410 | NM_004814 | U5 snRNP / U5 snRNP specific proteins | Core Spl. | Persist. Spl. |
| 56 | E08 | L-008361-01 | J-008361-09 | TXNL4 | 15 kDa U5 | 10907 | NM_006701 | U5 snRNP / U5 snRNP specific proteins | Core Spl. | Trans. Early Spl. |
| 57 | E09 | L-019836-01 | J-019836-09 | PRPF3 | U4/U6 snRNP 90 kDa protein | 9129 | NM_004698 | U4/U6 snRNP / U4/U6 snRNP specific proteins | Core Spl. | Trans. Early Spl. |
| 58 | E10 | L-015638-00 | J-015638-05 | PRPF4 | U4/U6 snRNP 60 kDa protein | 9128 | NM_004697 | U4/U6 snRNP / U4/U6 snRNP specific proteins | Core Spl. | Trans. Early Spl. |
| 59 | E11 | L-020525-00 | J-020525-05 | PRPF31 | U4/U6 snRNP 61 kDa protein, | 26121 | NM_015629 | U4/U6 snRNP / U4/U6 snRNP specific proteins | Core Spl. | Trans. Early Spl. |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | | | | NY-BR-99 | | | | | |
| 60 | E12 | L-008907-01 | J-008907-09 | PPIH | CYP20, CYPH | 10465 | NM_006347 | U4/U6 snRNP / U4/U6 snRNP specific proteins | Core Spl. | Trans. Early Spl. |
| 61 | F01 | L-014523-00 | J-014523-05 | RBM35B | ESRP2 | 80004 | NM_024939 | other / other | Other | NA |
| 62 | F02 | L-014790-01 | J-014790-09 | CCDC55 | NSrp70, NSRP1 | 84081 | NM_032141 | other / other | Other | NA |
| 63 | F03 | L-019900-00 | J-019900-05 | NHP2L1 | hSNU13, U4/U6.U5 tri-snRNP 15.5 kDa protein | 4809 | NM_001003796 | U4/U6 snRNP / U4/U6 snRNP specific proteins | Core Spl. | Trans. Early Spl. |
| 64 | F04 | L-017283-00 | J-017283-05 | SART1 | hSNU66, U4/U6.U5 tri-snRNP-associated 110 kDa protein | 9092 | NM_005146 | U4/U6.U5 snRNP / U4/U6.U5 tri-snRNP specific proteins | Core Spl. | Trans. Early. Spl. |
| 65 | F05 | L-006087-00 | J-006087-05 | USP39 | hSAD1, U4/U6.U5 tri-snRNP-associated 65 kDa protein | 10713 | NM_006590 | U4/U6.U5 snRNP / U4/U6.U5 tri-snRNP specific proteins | Core Spl. | Trans. Early. Spl. |
| 66 | F06 | L-012758-01 | J-012758-09 | RY1 | U4/U6.U5 tri-snRNP-associated 27 kDa protein | 11017 | NM_006857 | U4/U6.U5 snRNP / U4/U6.U5 tri-snRNP specific proteins | Core Spl. | Trans. Early. Spl. |
| 67 | F07 | L-017766-01 | J-017766-09 | SNRPB | Sm protein B/B' | 6628 | NM_198216 | SM proteins / Sm/LSm core proteins | Core Spl. | Persist. Spl. |
| 68 | F08 | L-012353-01 | J-012353-09 | SNRPD1 | snRNP core | 6632 | NM_006938 | SM proteins / Sm/LSm core proteins | Core Spl. | Persist. Spl. |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | | | | protein D1 | | | | | |
| 69 | F09 | L-013617-01 | J-013617-09 | SNRPD2 | snRNP core protein D2 | 6633 | NM_004597 | SM proteins / Sm/LSm core proteins | Core Spl. | Persist. Spl. |
| 70 | F10 | L-019085-02 | J-019085-17 | SNRPD3 | snRNP core protein D3 | 6634 | NM_004175 | SM proteins / Sm/LSm core proteins | Core Spl. | Persist. Spl. |
| 71 | F11 | L-019719-02 | J-019719-17 | SNRPE | Sm protein E | 6635 | NM_003094 | SM proteins / Sm/LSm core proteins | Core Spl. | Persist. Spl. |
| 72 | F12 | L-019575-02 | J-019575-17 | SNRPF | Sm protein F | 6636 | NM_003095 | SM proteins / Sm/LSm core proteins | Core Spl. | Persist. Spl. |
| 73 | G01 | L-004597-00 | J-004597-05 | HMGA1 | HMGIY | 3159 | NM_145903 | other / other | Chrom. Factors | NA |
| 74 | G02 | L-008290-00 | J-008290-06 | DBR1 | lariat debranching enzyme | 51163 | NM_016216 | other / other | Other | NA |
| 75 | G03 | L-016821-02 | J-016821-17 | SNRPG | Sm protein G | 6637 | NM_003096 | SM proteins / Sm/LSm core proteins | Core Spl. | Persist. Spl. |
| 76 | G04 | L-017813-00 | J-017813-05 | LSM2 | U6 snRNA-associated Sm-like protein LSm2 | 57819 | NM_021177 | LSm proteins / Sm/LSm core proteins | Core Spl. | Trans. Early Spl. |
| 77 | G05 | L-020240-01 | J-020240-09 | LSM3 | U6 snRNA-associated Sm-like protein LSm3 | 27258 | NM_014463 | LSm proteins / Sm/LSm core proteins | Core Spl. | Trans. Early Spl. |
| 78 | G06 | L-017025-01 | J-017025-09 | LSM4 | U6 snRNA-associated Sm-like protein LSm4 | 25804 | NM_012321 | LSm proteins / Sm/LSm core proteins | Core Spl. | Trans. Early Spl. |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| 79 | G07 | L-019754-01 | J-019754-09 | LSM6 | U6 snRNA-associated Sm-like protein LSm6 | 11157 | NM_007080 | LSm proteins / Sm/LSm core proteins | Core Spl. | Trans. Early Spl. |
| 80 | G08 | L-021189-01 | J-021189-09 | LSM7 | U6 snRNA-associated Sm-like protein LSm7 | 51690 | NM_016199 | LSm proteins / Sm/LSm core proteins | Core Spl. | Trans. Early Spl. |
| 81 | G09 | L-013428-00 | J-013428-05 | DHX38 | PRP16, DDX38 | 9785 | NM_014003 | Catalytic step II and late acting proteins / Catalytic step II and late acting proteins | Core Spl. | Trans. Late Spl. |
| 82 | G10 | L-010506-00 | J-010506-06 | DHX8 | DDX8,PRP22 | 1659 | NM_004941 | second step factors / Catalytic step II and late acting proteins | Core Spl. | Trans. Late Spl. |
| 83 | G11 | L-011250-01 | J-011250-09 | DHX15 | DBP1, DDX15, PRPF43 | 1665 | NM_001358 | U2 snRNP related / Catalytic step II and late acting proteins | Core Spl. | Trans. Early Spl. |
| 84 | G12 | L-017191-01 | J-017191-09 | SLU7 | hSLU7 | 10569 | NM_006425 | second step factors / Catalytic step II and late acting proteins | Core Spl. | Trans. Late Spl. |
| 85 | H01 | L-015405-01 | J-015405-09 | DIS3 | RRP44, EXOSC11 | 22894 | NM_014953 | RNA degradation / RNA degradation | Other | NA |
| 86 | H02 | L-013760-00 | J-013760-05 | EXOSC4 | RRP41, SKI6 | 54512 | NM_019037 | RNA degradation / RNA degradation | Other | NA |
| 87 | H03 | L-013213-01 | J-013213-09 | CDC40 | PRPF17 | 51362 | NM_015891 | abundant first in Bact complex / Catalytic step II and late acting proteins | Core Spl. | Trans. Mid Spl. |
| 88 | H04 | L-011497-00 | J-011497-05 | PRPF18 | hPrp18 | 8559 | NM_003675 | second step factors / Catalytic step II and late acting proteins | Core Spl. | Trans. Late Spl. |
| 89 | H05 | L-020041-02 | J-020041-17 | SRRM1 | SRM160 | 10250 | NM_005839 | SR related proteins / Spliced mRNP/EJC proteins | Spl. Factors | NA |
| 90 | H06 | L-003805-00 | J-003805-05 | BAT1 | 56 kDa U2AF65-associated protein, | 7919 | NM_080598 | EJC / mRNP / Spliced mRNP/EJC proteins | Other | EJC |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | | | | UAP56, ATP-dependent RNA helicase p47 | | | | | |
| 91 | H07 | L-012298-00 | J-012298-06 | RNPS1 | LDC2 | 10921 | NM_080594 | EJC / mRNP / Spliced mRNP/EJC proteins | Other | EJC |
| 92 | H08 | L-012078-00 | J-012078-05 | THOC4 | ALY, ALY/REF | 10189 | NM_005782 | EJC / mRNP / Spliced mRNP/EJC proteins | Other | EJC |
| 93 | H09 | L-003531-00 | J-003531-09 | RBM8A | Y14 | 9939 | NM_005105 | EJC / mRNP / Spliced mRNP/EJC proteins | Other | EJC |
| 94 | H10 | L-011327-00 | J-011327-05 | MAGOH | mago-nashi | 4116 | NM_002370 | EJC / mRNP / Spliced mRNP/EJC proteins | Other | EJC |
| 95 | H11 | L-012278-00 | J-012278-05 | TCERG1 | CA150, TAF2S | 10915 | NM_006706 | Miscellaneous proteins / Other previously reported splicing factors/SAPs | Other | NA |
| 96 | H12 | L-004074-00 | J-004074-11 | PRPF4B | PRP4, PRP4 kinase | 8899 | NM_176800 | Abundant first in B complex / Other previously reported splicing factors/SAPs | Core Spl. | Trans. Early Spl. |
| 97 | A01 | - | - | No cells | | - | - | / | NA | NA |
| 98 | A02 | - | - | No cells | | - | - | / | NA | NA |
| 99 | A03 | L-003774-00 | J-003774-05 | DDX5 | p68 | 1655 | NM_004396 | other / Other previously reported splicing factors/SAPs | Other | NA |
| 100 | A04 | L-012190-00 | J-012190-05 | IK | RED | 3550 | NM_006083 | Abundant first in B complex / Other previously reported splicing factors/SAPs | Core Spl. | Trans. Early Spl. |
| 101 | A05 | L-005264-00 | J-005264-06 | PPM1G | PP2C-gamma | 5496 | NM_002707 | other / Other previously reported splicing factors/SAPs | Other | NA |
| 102 | A06 | L-019598-00 | J-019598-05 | PABPC1 | poly(A) binding protein, cytoplasmic | 26986 | NM_002568 | 3´end processing / Other previously reported splicing factors/SAPs | Other | NA |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| 103 | A07 | L-005158-01 | J-005158-09 | RBM17 | SPF45 | 84991 | NM_032905 | U2 snRNP related / Other previously reported splicing factors/SAPs | Core Spl. | Trans. Early Spl. |
| 104 | A08 | L-019860-00 | J-019860-05 | HPRP8BP | SNRNP40, 40 kDa U5 | 9410 | NM_004814 | U5 snRNP / Other previously reported splicing factors/SAPs | Core Spl. | Persist. Spl. |
| 105 | A09 | L-020627-01 | J-020627-09 | DNAJC8 | SPF31 | 22826 | NM_014280 | other / Other previously reported splicing factors/SAPs | Other | NA |
| 106 | A10 | L-012708-00 | J-012708-05 | BCAS2 | SPF 27 | 10286 | NM_005872 | hPrp19 / Cdc5L complex / Other previously reported splicing factors/SAPs | Core Spl. | Trans. Mid Spl. |
| 107 | A11 | L-014157-00 | J-014157-05 | ACIN1 | ACINUS | 22985 | NM_014977 | EJC / mRNP / RRM-containing proteins | Other | EJC |
| 108 | A12 | L-013625-00 | J-013625-09 | SHARP | RBM15C, msx2-interacting protein | 23013 | NM_015001 | other / RRM-containing proteins | Other | NA |
| 109 | B01 | - | - | Untransfected | | - | - | / | NA | NA |
| 110 | B02 | - | - | Untransfected | | - | - | / | NA | NA |
| 111 | B03 | L-016645-00 | J-016645-05 | HTATSF1 | TAT-SF1 | 27336 | NM_014500 | U2 snRNP related / RRM-containing proteins | Core Spl. | Trans. Early Spl. |
| 112 | B04 | L-011965-00 | J-011965-05 | RNPC2 | CAPER, HCC1, RBM39 | 9584 | NM_004902 | other / RRM-containing proteins | Other | NA |
| 113 | B05 | L-012334-01 | J-012334-09 | CPSF6 | Cleavage and polyadenylation specificity factor 68 kDa subunit, CFIM | 11052 | NM_007007 | other / RRM-containing proteins | Other | NA |
| 114 | B06 | L-010854-00 | J-010854-05 | RBM15 | OTT1 | 64783 | NM_022768 | other / RRM-containing proteins | Other | NA |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| 115 | B07 | L-021186-01 | J-021186-09 | RBM22 | ZC3H16, Cwc2 | 55696 | NM_018047 | hPrp19 / Cdc5L related / RRM-containing proteins | Core Spl. | Trans. Mid Spl. |
| 116 | B08 | L-003976-00 | J-003976-05 | IMP-3 | U3 small nucleolar ribonucleoprotein, homolog | 10643 | NM_006547 | Other / RRM-containing proteins | Other | NA |
| 117 | B09 | L-020189-00 | J-020189-05 | CIRBP | cold inducible RNA-binding protein | 1153 | NM_001280 | other / RRM-containing proteins | Other | NA |
| 118 | B10 | L-020762-00 | J-020762-05 | DDX48 | EIF4A3 | 9775 | NM_014740 | EJC / mRNP / DExD box proteins | Other | EJC |
| 119 | B11 | L-013450-01 | J-013450-09 | DDX17 | p72 | 10521 | NM_030881 | other / DExD box proteins | Other | NA |
| 120 | B12 | L-011477-00 | J-011477-05 | DHX16 | DBP2 | 8449 | NM_003587 | abundant first in Bact complex / DExD box proteins | Core Spl. | Trans. Mid Spl. |
| 121 | C01 | - | - | **Mock SiRNA** | | - | - | / | NA | NA |
| 122 | C02 | - | - | **Mock SiRNA** | | - | - | / | NA | NA |
| 123 | C03 | L-009950-00 | J-009950-05 | DHX9 | DDX9 | 1660 | NM_030588 | other / DExD box proteins | Other | NA |
| 124 | C04 | L-006874-00 | J-006874-05 | DDX3X | DDX3 | 1654 | NM_024005 | other / DExD box proteins | Other | NA |
| 125 | C05 | L-031902-01 | J-031902-09 | SKIV2L2 | KIAA0052 | 23517 | NM_015360 | Miscellaneous proteins / DExD box proteins | Other | NA |
| 126 | C06 | L-015603-01 | J-015603-09 | DHX57 | DDX57 | 90957 | NM_198963 | other / DExD box proteins | Other | NA |
| 127 | C07 | L-017196-01 | J-017196-09 | DHX30 | DDX30 | 22907 | NM_138614 | other / DExD box proteins | Other | NA |
| 128 | C08 | L-003768-01 | J-003768-09 | DHX32 | DDX32, DHLP1 | 55760 | NM_018180 | other / DExD box proteins | Other | NA |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level)[3,4] | Network Classification (2nd level)[3,4] |
|---|---|---|---|---|---|---|---|---|---|---|
| 129 | C09 | L-017205-01 | J-017205-09 | DHX33 | DDX33 | 56919 | NM_020162 | other / DExD box proteins | Other | NA |
| 130 | C10 | L-011842-01 | J-011842-09 | DDX10 | HRH-J8 | 1662 | NM_004398 | other / DExD box proteins | Other | NA |
| 131 | C11 | L-010397-01 | J-010397-09 | DDX24 | DEAD box protein 24 | 57062 | NM_020414 | other / DExD box proteins | Other | NA |
| 132 | C12 | L-012766-01 | J-012766-09 | DDX52 | ROK1 | 11056 | NM_007010 | other / DExD box proteins | Other | NA |
| 133 | D01 | L-015333-00 | J-015333-05 | DIS3L | KIAA1955 | 115752 | NM_133375 | RNA degradation / RNA degradation | Other | NA |
| 134 | D02 | L-003881-00 | J-003881-06 | DEK | D6S231E | 7913 | NM_003472 | other / other | Other | NA |
| 135 | D03 | L-012931-01 | J-012931-09 | DDX31 | FLJ13633 | 64794 | NM_138620 | other / DExD box proteins | Other | NA |
| 136 | D04 | L-011843-00 | J-011843-05 | DDX11 | CHL1, CHLR1, KRG2 | 1663 | NM_004399 | other / DExD box proteins | Other | NA |
| 137 | D05 | L-017202-01 | J-017202-09 | DDX28 | MDDX28 | 55794 | NM_018380 | other / DExD box proteins | Other | NA |
| 138 | D06 | L-033292-00 | J-033292-01 | DDX12 | CHLR2, DDX12 | 440081 | XM_495908 | other / DExD box proteins | Other | NA |
| 139 | D07 | L-022008-01 | J-022008-09 | TDRD9 | C14orf75 | 122402 | NM_153046 | other / DExD box proteins | Other | NA |
| 140 | D08 | L-021627-01 | J-021627-09 | LOC164045 | SEC3D1, HFM1 | 164045 | NM_001017975 | other / DExD box proteins | Other | NA |
| 141 | D09 | L-014162-00 | J-014162-05 | MOV10 | KIAA1631 | 4343 | NM_020963 | other / DExD box proteins | Other | NA |
| 142 | D10 | L-004914-01 | J-004914-09 | XAB2 | HCRN | 56949 | NM_020196 | hPrp19 / Cdc5L related / Proteins with other known motifs | Core Spl. | Trans. Mid Spl. |
| 143 | D11 | L-019911-00 | J-019911-05 | THOC1 | HPR1, P84 | 9984 | NM_005131 | other / Proteins with other known motifs | Other | NA |
| 144 | D12 | L-015383- | J-015383- | LUC7A | CROP | 51747 | NM_006107 | other / Proteins with other known motifs | Other | NA |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | 00 | 05 | | | | | | | |
| 145 | E01 | L-003486-00 | J-003486-11 | EP300 | CRI2, p300, KAT3B | 2033 | NM_001429 | Histone Acetyl transferases / Histone Acetyl transferases | Chrom. Factors | NA |
| 146 | E02 | L-009722-00 | J-009722-05 | KAT2A | GCN5, PCAF-b | 2648 | NM_021078 | Histone Acetyl transferases / Histone Acetyl transferases | Chrom. Factors | NA |
| 147 | E03 | L-023101-02 | J-023101-17 | KIAA1604 | CWC22 | 57703 | NM_020943 | abundant first in Bact complex / Proteins with other known motifs | Core Spl. | Trans. Mid Spl. |
| 148 | E04 | L-029452-00 | J-029452-05 | FUBP3 | FUSE-binding protein 3 | 8939 | XM_932775 | other / Proteins with other known motifs | Other | NA |
| 149 | E05 | L-018424-01 | J-018424-09 | MGC2655 | WDR58, THOC6 | 79228 | NM_024339 | other / Proteins with other known motifs | Other | NA |
| 150 | E06 | L-004031-00 | J-004031-09 | CRK7 | CDK12 | 51755 | NM_016507 | other / Proteins with other known motifs | Other | NA |
| 151 | E07 | L-019966-02 | J-019966-17 | TFIP11 | TIP39, Septin and tuftelin-interacting protein 1 | 24144 | NM_012143 | Abundant first in B complex / Proteins with other known motifs | Core Spl. | Trans. Early Spl. |
| 152 | E08 | L-012335-01 | J-012335-09 | CPSF5 | NUDT21, CFIM25, CPSF25, CPSF5 | 11051 | NM_007006 | other / Proteins with other known motifs | Other | NA |
| 153 | E09 | L-014879-01 | J-014879-09 | THOC3 | hTREX45 | 84321 | NM_032361 | other / Proteins with other known motifs | Other | NA |
| 154 | E10 | L-031204-01 | J-031204-15 | C19ORF29 | NY-REN-24, cactin | 58509 | NM_021231 | Abundant or found only in C complex / Proteins with other known motifs | Core Spl. | Trans. Late Spl. |
| 155 | E11 | L-018665-01 | J-018665-09 | G10 | BUD31, fSAP17, EDG2 | 8896 | NM_003910 | hPrp19 / Cdc5L related / Proteins with other known motifs | Core Spl. | Trans. Mid Spl. |
| 156 | E12 | L-019557- | J-019557- | ZNF207 | | 7756 | NM_001032293 | other / Proteins with other known motifs | Other | NA |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level)[3,4] | Network Classification (2nd level)[3,4] |
|---|---|---|---|---|---|---|---|---|---|---|
| 157 | F01 | L-006301-00 | J-006301-08 | KAT5 | HTATIP, TIP60 | 10524 | NM_182709 | Histone Acetyl transferases / Histone Acetyl transferases | Chrom. Factors | NA |
| 158 | F02 | L-004130-00 | J-004130-05 | CARM1 | PRMT4 | 10498 | NM_199141 | Arginine methyltransferases / Arginine methyltransferases | Chrom. Factors | NA |
| 159 | F03 | L-019234-01 | J-019234-09 | ARS2 | SRRT | 51593 | NM_015908 | RNA binding proteins / Proteins with no known motif | NA | NA |
| 160 | F04 | L-025006-01 | J-025006-09 | THOC2 | CXorf3 | 57187 | NM_020449 | other / Proteins with no known motif | NA | NA |
| 161 | F05 | L-015317-01 | J-015317-09 | C22ORF19 | THOC5, KIAA0983 | 8563 | NM_003678 | Miscellaneous proteins / Proteins with no known motif | NA | NA |
| 162 | F06 | L-014575-01 | J-014575-09 | NIF3L1BP1 | THOC7, fSAP24 | 80145 | NM_025075 | other / Proteins with no known motif | NA | NA |
| 163 | F07 | L-014974-02 | J-014974-17 | MGC13125 | BUD13 | 84811 | NM_032725 | RES complex / Proteins with no known motif | NA | NA |
| 164 | F08 | L-017323-00 | J-017323-05 | WTAP | WT1-associated protein, KIAA0105 | 9589 | NM_152857 | other / Proteins with no known motif | NA | NA |
| 165 | F09 | L-012984-02 | J-012984-17 | C21ORF66 | GCFC | 94104 | NM_058191 | other / Proteins with no known motif | NA | NA |
| 166 | F10 | L-019278-02 | J-019278-17 | DKFZP434I116 | fSAP121, KIAA1429 | 25962 | NM_183009 | other / Proteins with no known motif | NA | NA |
| 167 | F11 | L-013894-01 | J-013894-09 | KIAA1160 | ISY1 | 57461 | NM_020701 | hPrp19 / Cdc5L related / Proteins with no known motif | Core Spl. | Trans. Mid Spl. |
| 168 | F12 | L-020788-02 | J-020788-17 | HSPC148 | CWC15 | 51503 | NM_016403 | other / Proteins with no known motif | NA | NA |
| 169 | G01 | L-015817-00 | J-015817-06 | PRMT5 | 72 kDa ICln-binding protein, Jak-binding | 10419 | NM_006109 | Arginine methyltransferases / Arginine methyltransferases | Chrom. Factors | NA |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | | | | protein 1 | | | | | |
| 170 | G02 | L-004937-00 | J-004937-06 | BRD4 | HUNK1 | 23476 | NM_014299 | Bromodomain proteins / Bromodomain proteins | Chrom. Factors | NA |
| 171 | G03 | L-012310-01 | J-012310-09 | DGCR14 | DGCR13, DGSH, DGSI, ES2 | 8220 | NM_022719 | other / Proteins with no known motif | NA | NA |
| 172 | G04 | L-020289-02 | J-020289-17 | GTL3 | fSAP23 | 29105 | NM_013242 | other / Proteins with no known motif | NA | NA |
| 173 | G05 | L-014270-02 | J-014270-18 | MGC2803 | C19orf43 | 79002 | NM_024038 | other / Proteins with no known motif | NA | NA |
| 174 | G06 | L-012314-02 | J-012314-17 | HNRPA0 | heterogeneous nuclear ribonucleoprotein A0 | 10949 | NM_006805 | hnRNP / Proteins designated as H complex components | Spl. Factors | hnRNP proteins |
| 175 | G07 | L-008221-00 | J-008221-09 | HNRPA1 | hnRNP core protein A1 | 3178 | NM_002136 | hnRNP / Proteins designated as H complex components | Spl. Factors | hnRNP proteins |
| 176 | G08 | L-011690-01 | J-011690-09 | HNRPA2B1 | heterogeneous nuclear ribonucleoprotein B1 | 3181 | NM_002137 | hnRNP / Proteins designated as H complex components | Spl. Factors | hnRNP proteins |
| 177 | G09 | L-019347-00 | J-019347-05 | HNRNPA3 | heterogeneous nuclear ribonucleoprotein A3 | 220988 | NM_194247 | hnRNP / Proteins designated as H complex components | Spl. Factors | hnRNP proteins |
| 178 | G10 | L-011869-01 | J-011869-09 | HNRPC | heterogeneous nuclear ribonucleoprotein C1/C2 | 3183 | NM_004500 | hnRNP / Proteins designated as H complex components | Spl. Factors | hnRNP proteins |
| 179 | G11 | L-004079-00 | J-004079-05 | HNRPD | heterogeneous nuclear ribonucleop | 3184 | NM_001003810 | hnRNP / Proteins designated as H complex components | Spl. Factors | hnRNP proteins |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | | | | rotein D | | | | | |
| 180 | G12 | L-003528-00 | J-003528-06 | PTBP1 | PTB | 5725 | NM_175847 | hnRNP / Proteins designated as H complex components | Spl. Factors | hnRNP proteins |
| 181 | H01 | L-020165-00 | J-020165-07 | CTCF | CCCTC-binding factor | 10664 | NM_006565 | other / other | Chrom. Factors | NA |
| 182 | H02 | L-008529-00 | J-008529-05 | CHD1 | ATP-dependent helicase CHD1 | 1105 | NM_001270 | Chromodomain proteins / Chromodomain proteins | Chrom. Factors | NA |
| 183 | H03 | L-011692-00 | J-011692-05 | HNRPK | heterogeneous nuclear ribonucleoprotein K | 3190 | NM_031263 | hnRNP / Proteins designated as H complex components | Spl. Factors | hnRNP proteins |
| 184 | H04 | L-011293-01 | J-011293-09 | HNRPL | heterogeneous nuclear ribonucleoprotein L | 3191 | NM_001005335 | hnRNP / Proteins designated as H complex components | Spl. Factors | hnRNP proteins |
| 185 | H05 | L-010905-01 | J-010905-09 | HNRPR | Heterogeneous nuclear ribonucleoprotein R | 10236 | NM_005826 | hnRNP / Proteins designated as H complex components | Spl. Factors | hnRNP proteins |
| 186 | H06 | L-012392-01 | J-012392-09 | RALY | HNRPCL2, P542 | 22913 | NM_007367 | hnRNP / Proteins designated as H complex components | Spl. Factors | hnRNP proteins |
| 187 | H07 | L-009497-00 | J-009497-07 | FUS | TLS, 75 kDa DNA-pairing protein | 2521 | NM_001010850 | hnRNP / Proteins designated as H complex components | Spl. Factors | hnRNP proteins |
| 188 | H08 | L-012442-00 | J-012442-06 | ILF3 | DRBF, MPHOSPH4, NF90 | 3609 | NM_153464 | hnRNP / Proteins designated as H complex components | Spl. Factors | hnRNP proteins |
| 189 | H09 | L-017599- | J-017599- | ILF2 | NF45 | 3608 | NM_004515 | hnRNP / Proteins designated as H complex components | Spl. Factors | hnRNP proteins |

EP 3 029 149 B1

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | 00 | 05 | | | | | | | |
| 190 | H10 | L-016218-00 | J-016218-05 | SYNCRIP | HNRPQ, NSAP1 | 10492 | NM_006372 | hnRNP / Proteins designated as H complex components | Spl. Factors | hnRNP proteins |
| 191 | H11 | L-017382-00 | J-017382-05 | MATR3 | KIAA0723 | 9782 | NM_018834 | Miscellaneous proteins / Proteins designated as H complex components | NA | NA |
| 192 | H12 | L-006826-00 | J-006826-05 | BUB3 | budding uninhibited by benzimidaz oles 3 homolog | 9184 | NM_001007793 | other / Proteins designated as H complex components | NA | NA |
| 193 | A01 | - | - | No cells | | - | - | / | NA | NA |
| 194 | A02 | - | - | No cells | | - | - | / | NA | NA |
| 195 | A03 | L-003773-00 | J-003773-08 | ELAVL1 | HUR | 1994 | NM_001419 | RNA binding proteins / Proteins designated as H complex components | NA | NA |
| 196 | A04 | L-008930-00 | J-008930-06 | TAF15 | RBP56, TAF2N | 8148 | NM_139215 | other / Proteins designated as H complex components | NA | NA |
| 197 | A05 | L-010213-00 | J-010213-06 | YBX1 | NSEP1, YB1 | 4904 | NM_004559 | RNA binding proteins / Proteins designated as H complex components | NA | NA |
| 198 | A06 | L-015793-00 | J-015793-05 | CSDA | DBPA | 8531 | NM_003651 | other / Proteins designated as H complex components | NA | NA |
| 199 | A07 | L-005168-00 | J-005168-06 | HSPA1A | HSPA1 | 3303 | NM_005345 | other / Proteins designated as H complex components | NA | NA |
| 200 | A08 | L-017609-00 | J-017609-06 | HSPA8 | HSC70, HSP73, HSPA10 | 3312 | NM_153201 | other / Proteins designated as H complex components | NA | NA |
| 201 | A09 | L-013449-01 | J-013449-09 | HNRPF | heterogene ous nuclear ribonucleop rotein F | 3185 | NM_004966 | hnRNP / Proteins designated as H complex components | Spl. Factors | hnRNP proteins |
| 202 | A10 | L-012107-00 | J-012107-05 | HNRPH1 | heterogene ous nuclear | 3187 | NM_005520 | hnRNP / Proteins designated as H complex components | Spl. Factors | hnRNP proteins |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | | | | ribonucleoprotein H1 | | | | | |
| 203 | A11 | L-012440-01 | J-012440-09 | HNRPH3 | heterogeneous nuclear ribonucleoprotein H3 | 3189 | NM_021644 | hnRNP / Proteins designated as H complex components | Spl. Factors | hnRNP proteins |
| 204 | A12 | L-013245-02 | J-013245-17 | HNRPH2 | heterogeneous nuclear ribonucleoprotein H2 | 3188 | NM_001032393 | hnRNP / Proteins designated as H complex components | Spl. Factors | hnRNP proteins |
| 205 | B01 | - | - | Untransfected | | - | - | / | NA | NA |
| 206 | B02 | - | - | Untransfected | | - | - | / | NA | NA |
| 207 | B03 | L-013501-00 | J-013501-05 | HNRPU | HNRPU, SAFA, U21.1, P120 | 3192 | NM_004501 | hnRNP / Proteins designated as H complex components | Spl. Factors | hnRNP proteins |
| 208 | B04 | L-008198-00 | J-008198-06 | HSPA5 | GRP78 | 3309 | NM_005347 | other / Proteins designated as H complex components | NA | NA |
| 209 | B05 | L-020019-00 | J-020019-05 | KHDRBS1 | SAM68 | 10657 | NM_006559 | other / Others | Other | NA |
| 210 | B06 | L-004839-00 | J-004839-09 | SRPK2 | SRSF protein kinase 2 | 6733 | NM_182692 | other / Others | Other | NA |
| 211 | B07 | L-009065-01 | J-009065-09 | RBM10 | GPATCH9 | 8241 | NM_152856 | abundant in first or only present in A complex / Others | Core Spl. | Trans. Early Spl. |
| 212 | B08 | L-003483-00 | J-003483-09 | DICER1 | DICER | 23405 | NM_030621 | other / Others | Other | NA |
| 213 | B09 | L-005119-00 | J-005119-11 | EWSR1 | EWS | 2130 | NM_013986 | other / Others | Other | NA |
| 214 | B10 | L-020032-00 | J-020032-05 | RBM6 | DEF3 | 10180 | NM_005777 | other / Others | Other | NA |
| 215 | B11 | L-009220- | J-009220- | RBM5 | H37, | 10181 | NM_005778 | abundant in first or only present in A complex / Others | Core Spl. | Trans. Early Spl. |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | 00 | 07 | | LUCA15 | | | | | |
| 216 | B12 | L-013042-00 | J-013042-05 | TIA1 | p40-TIA-1 | 7072 | NM_022037 | other / Others | Other | NA |
| 217 | C01 | - | - | **Mock SiRNA** | | - | - | / | NA | NA |
| 218 | C02 | - | - | **Mock SiRNA** | | - | - | / | NA | NA |
| 219 | C03 | L-011405-00 | J-011405-05 | TIAL1 | TIA-1-related protein | 7073 | NM_003252 | other / Others | Other | NA |
| 220 | C04 | L-027984-01 | J-027984-09 | FNBP3 | PRPF40A | 55660 | XM_938514 | abundant in first or only present in A complex / Early splicing factors | Core Spl. | Trans. Early Spl. |
| 221 | C05 | L-021976-00 | J-021976-05 | RBM25 | RNPC7, S164, Snu71 | 58517 | NM_021239 | abundant in first or only present in A complex / Early splicing factors | Core Spl. | Trans. Early Spl. |
| 222 | C06 | L-023607-02 | J-023607-18 | SR140 | U2SURP | 23350 | NM_001080415 | U2 snRNP related / Early splicing factors | Core Spl. | Trans. Early Spl. |
| 223 | C07 | L-016176-02 | J-016176-17 | CHERP | DAN26, SCAF6 | 10523 | NM_006387 | U2 snRNP related / Early splicing factors | Core Spl. | Trans. Early Spl. |
| 224 | C08 | L-013828-02 | J-013828-17 | CCAR1 | CARP1, DIS | 55749 | NM_018237 | abundant in first or only present in A complex / Early splicing factors | Core Spl. | Trans. Early Spl. |
| 225 | C09 | L-019907-00 | J-019907-05 | THRAP3 | TRAP150 | 9967 | NM_005119 | abundant in first or only present in A complex / Early splicing factors | Core Spl. | Trans. Early Spl. |
| 226 | C10 | L-012385-02 | J-012385-18 | WBP4 | FBP21, FNBP21 | 11193 | NM_007187 | Abundant first in B complex / B complex stage | Core Spl. | Trans. Early Spl. |
| 227 | C11 | L-020071-02 | J-020071-18 | MFAP1 | microfibrillar-associated protein 1 | 4236 | NM_005926 | Abundant first in B complex / B complex stage | Core Spl. | Trans. Early Spl. |
| 228 | C12 | L-021129-01 | J-021129-09 | SMU1 | fSAP57, smu-1 suppressor of mec-8 and unc-52 | 55234 | NM_018225 | Abundant first in B complex / B complex stage | Core Spl. | Trans. Early Spl. |

EP 3 029 149 B1

EP 3 029 149 B1

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | | | | homolog | | | | | |
| 229 | D01 | L-006379-01 | J-006379-09 | MORF4L1 | MRG15 | 10933 | NM_006791 | Chromodomain proteins / Chromodomain proteins | Chrom. Factors | NA |
| 230 | D02 | L-003493-00 | J-003493-10 | HDAC1 | RPD3L1 | 3065 | NM_004964 | Histone Deacetylases / Histone Deacetylases | Chrom. Factors | NA |
| 231 | D03 | L-017535-00 | J-017535-05 | CTNNBL1 | C20orf33 | 56259 | NM_030877 | hPrp19 / Cdc5L complex / B complex stage | Core Spl. | Trans. Mid Spl. |
| 232 | D04 | L-022214-01 | J-022214-09 | AQR | KIAA0560 | 9716 | NM_014691 | hPrp19 / Cdc5L related / B complex stage | Core Spl. | Trans. Mid Spl. |
| 233 | D05 | L-012716-01 | J-012716-09 | PQBP1 | NPW38 | 10084 | NM_001032385 | hPrp19 / Cdc5L complex / B complex stage | Core Spl. | Trans. Mid Spl. |
| 234 | D06 | L-013343-00 | J-013343-05 | WBP11 | NPWBP, SIPP1, SNP70 | 51729 | NM_016312 | hPrp19 / Cdc5L complex / B complex stage | Core Spl. | Trans. Mid Spl. |
| 235 | D07 | L-012460-00 | J-012460-05 | KIN | BTCD, KIN17 | 22944 | NM_012311 | Miscellaneous proteins / B complex stage | NA | NA |
| 236 | D08 | L-010394-00 | J-010394-05 | DDX41 | ABS | 51428 | NM_016222 | Abundant or found only in C complex / C complex stage | Core Spl. | Trans. Late Spl. |
| 237 | D09 | L-013901-02 | J-013901-17 | FLJ22965 | CXorf56 | 63932 | NM_022101 | Abundant or found only in C complex / C complex stage | Core Spl. | Trans. Late Spl. |
| 238 | D10 | L-020239-01 | J-020239-09 | P29 | SYF2 homolog, CBPIN, GCIPIP | 25949 | NM_207170 | Abundant or found only in C complex / C complex stage | Core Spl. | Trans. Late Spl. |
| 239 | D11 | L-013065-01 | J-013065-09 | DHX35 | C20orf15, DDX35 | 60625 | NM_021931 | Abundant or found only in C complex / C complex stage | Core Spl. | Trans. Late Spl. |
| 240 | D12 | L-031204-01 | J-031204-15 | C19ORF29 | Cactin, NY-REN-24 | 58509 | NM_021231 | Abundant or found only in C complex / C complex stage | Core Spl. | Trans. Late Spl. |
| 241 | E01 | L-003540-00 | J-003540-09 | SIRT1 | SIR2L1 | 23411 | NM_012238 | Histone Deacetylases / Histone Deacetylases | Chrom. Factors | NA |
| 242 | E02 | L-003495-00 | J-003495-06 | HDAC2 | YAF1 | 3066 | NM_001527 | Histone Deacetylases / Histone Deacetylases | Chrom. Factors | NA |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| 243 | E03 | L-016456-02 | J-016456-21 | FLJ35382 | C1orf55 | 163859 | NM_152608 | Abundant or found only in C complex / C complex stage | Core Spl. | Trans. Late Spl. |
| 244 | E04 | L-020231-02 | J-020231-18 | C9ORF78 | HCA59 | 51759 | NM_016520 | Abundant or found only in C complex / C complex stage | Core Spl. | Trans. Late Spl. |
| 245 | E05 | L-007204-00 | J-007204-05 | NOSIP | CGI-25, eNOS interacting protein | 51070 | NM_015953 | Abundant or found only in C complex / C complex stage | Core Spl. | Trans. Late Spl. |
| 246 | E06 | L-014332-00 | J-014332-05 | LENG1 | leukocyte receptor cluster (LRC) member 1 | 79165 | NM_024316 | Abundant or found only in C complex / C complex stage | Core Spl. | Trans. Late Spl. |
| 247 | E07 | L-014860-00 | J-014860-05 | MORG1 | WDR83 | 84292 | NM_032332 | Abundant or found only in C complex / C complex stage | Core Spl. | Trans. Late Spl. |
| 248 | E08 | L-015142-01 | J-015142-09 | MGC20398 | CCDC16, ZNF830 | 91603 | NM_052857 | abundant first in Bact complex / C complex stage | Core Spl. | Trans. Mid Spl. |
| 249 | E09 | L-015455-02 | J-015455-13 | MGC23918 | CCDC12 | 151903 | NM_144716 | abundant first in Bact complex / C complex stage | Core Spl. | Trans. Mid Spl. |
| 250 | E10 | L-011864-00 | J-011864-05 | FRG1 | FSHD region gene 1 | 2483 | NM_004477 | other / C complex stage | Core Spl. | Trans. Late Spl. |
| 251 | E11 | L-003235-00 | J-003235-14 | CDK10 | cyclin-dependent kinase 10 | 8558 | NM_052987 | Abundant or found only in C complex / C complex stage | Core Spl. | Trans. Late Spl. |
| 252 | E12 | L-012678-02 | J-012678-17 | DXS9928E | FAM50A | 9130 | NM_004699 | Abundant or found only in C complex / C complex stage | Core Spl. | Trans. Late Spl. |
| 253 | F01 | L-003497-00 | J-003497-07 | HDAC4 | KIAA0288 | 9759 | NM_006037 | Histone Deacetylases / Histone Deacetylases | Chrom. Factors | NA |
| 254 | F02 | L-003499-00 | J-003499-05 | HDAC6 | KIAA0901 | 10013 | NM_006044 | Histone Deacetylases / Histone Deacetylases | Chrom. Factors | NA |
| 255 | F03 | L-019978-02 | J-019978-17 | D6S2654E | X5L | 26240 | NM_012135 | other / C complex stage | Core Spl. | Trans. Late Spl. |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level)[3,4] | Network Classification (2nd level)[3,4] |
|---|---|---|---|---|---|---|---|---|---|---|
| 256 | F04 | L-020251-02 | J-020251-17 | FAM32A | CGI-144 | 26017 | NM_014077 | other / C complex stage | Core Spl. | Trans. Late Spl. |
| 257 | F05 | L-004742-01 | J-004742-09 | KIAA0073 | PPWD1 | 23398 | NM_015342 | Abundant or found only in C complex / PPIases | Core Spl. | Trans. Late Spl. |
| 258 | F06 | L-009868-01 | J-009868-09 | PPIL3 | Cyclophilin J | 53938 | NM_130906 | abundant first in Bact complex / PPIases | Core Spl. | Trans. Mid Spl. |
| 259 | F07 | L-009466-01 | J-009466-09 | PPIE | CYP33 | 10450 | NM_203456 | hPrp19 / Cdc5L related / PPIases | Core Spl. | Trans. Mid Spl. |
| 260 | F08 | L-008256-00 | J-008256-05 | PPIL1 | CYPL1 | 51645 | NM_016059 | hPrp19 / Cdc5L related / PPIases | Core Spl. | Trans. Mid Spl. |
| 261 | F09 | L-020024-01 | J-020024-09 | SDCCAG10 | CWC27 | 10283 | NM_005869 | abundant first in Bact complex / PPIases | Core Spl. | Trans. Mid Spl. |
| 262 | F10 | L-007205-00 | J-007205-05 | PPIL2 | CYP6 | 23759 | NM_014337 | abundant first in Bact complex / PPIases | Core Spl. | Trans. Mid Spl. |
| 263 | F11 | L-008293-00 | J-008293-06 | PPIG | CASP10 | 9360 | NM_004792 | Abundant or found only in C complex / PPIases | Core Spl. | Trans. Late Spl. |
| 264 | F12 | L-008307-00 | J-008307-05 | ASCL1 | ASH1, BHLHA46, HASH1 | 429 | NM_004316 | Lysine methyltransferases / Lysine methyltransferases | Chrom. Factors | NA |
| 265 | G01 | L-014635-02 | J-014635-19 | TET1 | CXXC6, KIAA1676, LCX | 80312 | NM_030625 | Lysine demethylases / Lysine demethylases | Chrom. Factors | NA |
| 266 | G02 | L-003496-00 | J-003496-09 | HDAC3 | RPD3-2, SMAP45 | 8841 | NM_003883 | Histone Deacetylases / Histone Deacetylases | Chrom. Factors | NA |
| 267 | G03 | L-009604-00 | J-009604-07 | SUV39H1 | KMT1A, SUV39H | 6839 | NM_003173 | Lysine methyltransferases / Lysine methyltransferases | Chrom. Factors | NA |
| 268 | G04 | L-022793-01 | J-022793-09 | SETD1A | KIAA0339, KMT2F, SET1, SET1A | 9739 | NM_014712 | Lysine methyltransferases / Lysine methyltransferases | Chrom. Factors | NA |
| 269 | G05 | L-006937-00 | J-006937-05 | EHMT2 | BAT8, C6orf30, | 10919 | NM_025256 | Lysine methyltransferases / Lysine methyltransferases | Chrom. Factors | NA |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level )[3,4] | Network Classification (2nd level)[3,4] |
|---|---|---|---|---|---|---|---|---|---|---|
| | | | | | G9A, KMT1C, NG36 | | | | | |
| 270 | G06 | L-012448-00 | J-012448-05 | SETD2 | HIF1, HYPB, KIAA1732, KMT3A, SET2 | 29072 | NM_014159 | Lysine methyltransferases / Lysine methyltransferases | Chrom. Factors | NA |
| 271 | G07 | L-013094-00 | J-013094-07 | MECP2 | MRX16 | 4204 | NM_004992 | Methyl-CpG DNA Binding Domain (MDB) Proteins / Methyl-CpG DNA Binding Domain (MDB) Proteins | Chrom. Factors | NA |
| 272 | G08 | L-011555-00 | J-011555-05 | MBD2 | NY-CO-41 | 8932 | NM_015832 | Methyl-CpG DNA Binding Domain (MDB) Proteins / Methyl-CpG DNA Binding Domain (MDB) Proteins | Chrom. Factors | NA |
| 273 | G09 | L-020080-00 | J-020080-05 | NAB2 | MADER | 4665 | NM_005967 | Nucleosome-Remodeling and Histone Deacetylase / Nucleosome-Remodeling and Histone Deacetylase | Chrom. Factors | NA |
| 274 | G10 | L-005230-01 | J-005230-09 | BMI1 | PCGF4, RNF51 | 648 | NM_005180 | Polycomb Group Genes / Polycomb Group Genes | Chrom. Factors | NA |
| 275 | G11 | L-017581-00 | J-017581-06 | EED | WAIT1 | 8726 | NM_003797 | Polycomb Group Genes / Polycomb Group Genes | Chrom. Factors | NA |
| 276 | G12 | L-011850-00 | J-011850-05 | PHC1 | EDR1, PH1 | 1911 | NM_004426 | Polycomb Group Genes / Polycomb Group Genes | Chrom. Factors | NA |
| 277 | H01 | L-009223-00 | J-009223-05 | AOF2 | KDM1A, KDM1, KIAA0601, LSD1 | 23028 | NM_015013 | Lysine demethylases / Lysine demethylases | Chrom. Factors | NA |
| 278 | H02 | L-004290-00 | J-004290-08 | JMJD2B | JHDM3B, KIAA0876, KDM4B | 23030 | NM_015015 | Lysine demethylases / Lysine demethylases | Chrom. Factors | NA |
| 279 | H03 | L-021410-00 | J-021410-05 | PHC2 | EDR2, PH2 | 1912 | NM_004427 | Polycomb Group Genes / Polycomb Group Genes | Chrom. Factors | NA |
| 280 | H04 | L-004218-00 | J-004218-06 | EZH2 | KMT6 | 2146 | NM_152998 | Polycomb Group Genes / Polycomb Group Genes | Chrom. Factors | NA |
| 281 | H05 | L-010431-00 | J-010431-05 | SMARCA4 | BAF190A, BRG1, | 6597 | NM_003072 | SWI/SNF Complex Components / SWI/SNF Complex Components | Chrom. Factors | NA |

| siRNA | Well | Pool Catalog Number | Duplex Catalog Number | Gene Symbol | Other names | GENE ID[1] | Gene Accession[2] | Luhrmann classification / Robin Reed classification | Network Classification (1st level)[3,4] | Network Classification (2nd level)[3,4] |
|---|---|---|---|---|---|---|---|---|---|---|
| 282 | H06 | L-017253-00 | J-017253-05 | SMARCA2 | SNF2B, SNF2L4 BAF190B, BRM, SNF2A, SNF2L2 | 6595 | NM_139045 | SWI/SNF Complex Components / SWI/SNF Complex Components | Chrom. Factors | NA |
| 283 | H07 | L-005055-00 | J-005055-05 | KAT2B | PCAF | 8850 | NM_003884 | Lysine acetyltransferase / Lysine acetyltransferase | Chrom. Factors | NA |
| 284 | H08 | L-004605-00 | J-004605-06 | DNMT1 | AIM, CXXC9, DNMT | 1786 | NM_001379 | others DNA modification / others DNA modification | Chrom. Factors | NA |
| 285 | H09 | L-004639-00 | J-004639-05 | EIF2C2 | AGO2 | 27161 | NM_012154 | others DNA modification / others DNA modification | Chrom. Factors | NA |
| 286 | H10 | L-010033-00 | J-010033-05 | CBX3 | HECH, HP1-GAMMA | 11335 | NM_007276 | Chromobox / Heterochromatin Protein 1 (HP1) / Chromobox / Heterochromatin Protein 1 (HP1) | Chrom. Factors | NA |
| 287 | H11 | L-004665-00 | J-004665-07 | RPS6KA5 | MSK1 | 9252 | NM_182398 | Serine/Threonine Kinases / Serine/Threonine Kinases | Chrom. Factors | NA |
| 288 | H12 | L-019831-00 | J-019831-05 | ASH2L | ASH2L1 | 9070 | NM_004674 | Ubiquitilases / Ubiquitilases | Chrom. Factors | NA |

(1) Gene ID: Identification number of the gene according to HUGO Gene Nomenclature Committee (http://www.genenames.org/)

(2) Gene Accession: Accession number of the gene in GenBank Database (http://www.ncbi.nlm.nih.gov/genbank)

(3) Abbreviations: Spl. Factors: Splicing factors; SR proteins: Splicing-site recognition proteins; Core Spl.: Factors of the spliceosome core; Trans.Early Spl.: Factors joining early the spliceosome; Trans. Mid Spl.: Factors joining the spliceosome in an intermediate stage; Persist. Spl.: Factors persisting in the splicesome along different stages of the splicing process; SM proteins: ; Chrom. Factors: Factors implicated in chromatin structure/regulation;

(4) Network classification:

All genes, as in the case of the genes of the AS events corrrespond to the human genome release GRCh37/hg19and the Ensembl version 77, October 2014, as well as the NCBI Entrez ID of Release 204, October 2014.

- Genome-wide screening. Reverse-transcription (RT-PCR) and Barcoded PCR

Isolated cellular mRNAs were reverse-transcribed in 96 well plates with superscript III retro transcriptase (Invitrogen, Life Technologies) in the presence of oligo-dT (Sigma-Aldrich, Cat No. 0487) and random primers (Life Technologies) following the manufacturer's instructions. Semi-quantitative barcoded PCR primer sets (280 forward, 96 reverse, Sigma-Aldrich, using barcode sequences previously reported by Hamady et al., 2008) were designed to amplify both Fas isoforms corresponding to the inclusion and skipping of Fas exon 6. PCR reactions were performed with a specific combination of forward and reverse barcoded primers and reagents provided in the GoTaq DNA polymerase kit (GoTaq, Promega). RT and PCR protocols were set up for 96 well plates and performed in a Zephyr compact liquid handling workstation (Perkin Elmer).

The list of used barcoded primers is the following one:

**Table 2**

| name | barcode | fas sequence | barcoded primer (5'-->3') |
|---|---|---|---|
| reverse 1 | agacgaca | gaaccttggttttcctttctgtg | agacgacagaaccttggttttcctttctgtg |
| reverse 2 | agagcagt | gaaccttggttttcctttctgtg | agagcagtgaaccttggttttcctttctgtg |
| reverse 3 | gaagtgca | gaaccttggttttcctttctgtg | gaagtgcagaaccttggttttcctttctgtg |
| reverse 4 | gttctgct | gaaccttggttttcctttctgtg | gttctgctgaaccttggttttcctttctgtg |
| reverse 5 | aacgcgtt | gaaccttggttttcctttctgtg | aacgcgttgaaccttggttttcctttctgtg |
| reverse 6 | aagcggta | gaaccttggttttcctttctgtg | aagcggtagaaccttggttttcctttctgtg |
| reverse 7 | cagtgaga | gaaccttggttttcctttctgtg | cagtgagagaaccttggttttcctttctgtg |
| reverse 8 | acacgtca | gaaccttggttttcctttctgtg | acacgtcagaaccttggttttcctttctgtg |
| reverse 9 | gacagtct | gaaccttggttttcctttctgtg | gacagtctgaaccttggttttcctttctgtg |
| reverse 10 | catgtcga | gaaccttggttttcctttctgtg | catgtcgagaaccttggttttcctttctgtg |
| reverse 11 | gttcagca | gaaccttggttttcctttctgtg | gttcagcagaaccttggttttcctttctgtg |
| reverse 12 | atggccat | gaaccttggttttcctttctgtg | atggccatgaaccttggttttcctttctgtg |

(continued)

| name | barcode | fas sequence | barcoded primer (5'-->3') |
|------|---------|--------------|---------------------------|
| reverse 13 | gaacgatg | gaaccttggttttcctttctgtg | gaacgatggaaccttggttttcctttctgtg |
| reverse 14 | gcatgcat | gaaccttggttttcctttctgtg | gcatgcatgaaccttggttttcctttctgtg |
| reverse 15 | gtgtgtgt | gaaccttggttttcctttctgtg | gtgtgtgtgaaccttggttttcctttctgtg |
| reverse 16 | gtgtgact | gaaccttggttttcctttctgtg | gtgtgactgaaccttggttttcctttctgtg |
| reverse 17 | tacggcat | gaaccttggttttcctttctgtg | tacggcatgaaccttggttttcctttctgtg |
| reverse 18 | ataaccgc | gaaccttggttttcctttctgtg | ataaccgcgaaccttggttttcctttctgtg |
| reverse 19 | atcctacc | gaaccttggttttcctttctgtg | atcctaccgaaccttggttttcctttctgtg |
| reverse 20 | ggatcgtt | gaaccttggttttcctttctgtg | ggatcgttgaaccttggttttcctttctgtg |
| reverse 21 | tcagctct | gaaccttggttttcctttctgtg | tcagctctgaaccttggttttcctttctgtg |
| reverse 22 | gtaccaac | gaaccttggttttcctttctgtg | gtaccaacgaaccttggttttcctttctgtg |
| reverse 23 | cacagact | gaaccttggttttcctttctgtg | cacagactgaaccttggttttcctttctgtg |
| reverse 24 | tggtagct | gaaccttggttttcctttctgtg | tggtagctgaaccttggttttcctttctgtg |
| reverse 25 | tctgtgag | gaaccttggttttcctttctgtg | tctgtgaggaaccttggttttcctttctgtg |
| reverse 26 | ctagacca | gaaccttggttttcctttctgtg | ctagaccagaaccttggttttcctttctgtg |
| reverse 27 | ggcctaat | gaaccttggttttcctttctgtg | ggcctaatgaaccttggttttcctttctgtg |
| reverse 28 | aattcggc | gaaccttggttttcctttctgtg | aattcggcgaaccttggttttcctttctgtg |
| reverse 29 | actgtcag | gaaccttggttttcctttctgtg | actgtcaggaaccttggttttcctttctgtg |
| reverse 30 | gcgcaatt | gaaccttggttttcctttctgtg | gcgcaattgaaccttggttttcctttctgtg |
| reverse 31 | ggaagcta | gaaccttggttttcctttctgtg | ggaagctagaaccttggttttcctttctgtg |
| reverse 32 | gtacagga | gaaccttggttttcctttctgtg | gtacaggagaaccttggttttcctttctgtg |
| reverse 33 | ggtaatcc | gaaccttggttttcctttctgtg | ggtaatccgaaccttggttttcctttctgtg |
| reverse 34 | aggttgga | gaaccttggttttcctttctgtg | aggttggagaaccttggttttcctttctgtg |
| reverse 35 | aagcttcg | gaaccttggttttcctttctgtg | aagcttcggaaccttggttttcctttctgtg |
| reverse 36 | atcgcctt | gaaccttggttttcctttctgtg | atcgccttgaaccttggttttcctttctgtg |
| reverse 37 | tatagcgc | gaaccttggttttcctttctgtg | tatagcgcgaaccttggttttcctttctgtg |
| reverse 38 | atggttgc | gaaccttggttttcctttctgtg | atggttgcgaaccttggttttcctttctgtg |
| reverse 39 | gatggaac | gaaccttggttttcctttctgtg | gatggaacgaaccttggttttcctttctgtg |
| reverse 40 | gagtgtga | gaaccttggttttcctttctgtg | gagtgtgagaaccttggttttcctttctgtg |
| reverse 41 | ttgcggat | gaaccttggttttcctttctgtg | ttgcggatgaaccttggttttcctttctgtg |
| reverse 42 | tgagactc | gaaccttggttttcctttctgtg | tgagactcgaaccttggttttcctttctgtg |
| reverse 43 | taatcgcg | gaaccttggttttcctttctgtg | taatcgcggaaccttggttttcctttctgtg |
| reverse 44 | gcggaata | gaaccttggttttcctttctgtg | gcggaatagaaccttggttttcctttctgtg |
| reverse 45 | tggatcca | gaaccttggttttcctttctgtg | tggatccagaaccttggttttcctttctgtg |
| reverse 46 | ggttaagg | gaaccttggttttcctttctgtg | ggttaagggaaccttggttttcctttctgtg |
| reverse 47 | ttaacggc | gaaccttggttttcctttctgtg | ttaacggcgaaccttggttttcctttctgtg |
| reverse 48 | atggttcg | gaaccttggttttcctttctgtg | atggttcggaaccttggttttcctttctgtg |
| reverse 49 | aattgccg | gaaccttggttttcctttctgtg | aattgccggaaccttggttttcctttctgtg |
| reverse 50 | cactgaca | gaaccttggttttcctttctgtg | cactgacagaaccttggttttcctttctgtg |

(continued)

| name | barcode | fas sequence | barcoded primer (5'-->3') |
|---|---|---|---|
| reverse 51 | caagtcgt | gaaccttggttttcctttctgtg | caagtcgtgaaccttggttttcctttctgtg |
| reverse 52 | ccataacg | gaaccttggttttcctttctgtg | ccataacggaaccttggttttcctttctgtg |
| reverse 53 | gtgttgac | gaaccttggttttcctttctgtg | gtgttgacgaaccttggttttcctttctgtg |
| reverse 54 | gcaatagg | gaaccttggttttcctttctgtg | gcaatagggaaccttggttttcctttctgtg |
| reverse 55 | atcctagg | gaaccttggttttcctttctgtg | atcctagggaaccttggttttcctttctgtg |
| reverse 56 | taatggcc | gaaccttggttttcctttctgtg | taatggccgaaccttggttttcctttctgtg |
| reverse 57 | actgtgac | gaaccttggttttcctttctgtg | actgtgacgaaccttggttttcctttctgtg |
| reverse 58 | cggcatta | gaaccttggttttcctttctgtg | cggcattagaaccttggttttcctttctgtg |
| reverse 59 | tggtgttg | gaaccttggttttcctttctgtg | tggtgttggaaccttggttttcctttctgtg |
| reverse 60 | agcactac | gaaccttggttttcctttctgtg | agcactacgaaccttggttttcctttctgtg |
| reverse 61 | tattgcgg | gaaccttggttttcctttctgtg | tattgcgggaaccttggttttcctttctgtg |
| reverse 62 | aattggcc | gaaccttggttttcctttctgtg | aattggccgaaccttggttttcctttctgtg |
| reverse 63 | tgcaacca | gaaccttggttttcctttctgtg | tgcaaccagaaccttggttttcctttctgtg |
| reverse 64 | tataccgg | gaaccttggttttcctttctgtg | tataccgggaaccttggttttcctttctgtg |
| reverse 65 | tgagagac | gaaccttggttttcctttctgtg | tgagagacgaaccttggttttcctttctgtg |
| reverse 66 | gtgttcag | gaaccttggttttcctttctgtg | gtgttcaggaaccttggttttcctttctgtg |
| reverse 67 | cacatctc | gaaccttggttttcctttctgtg | cacatctcgaaccttggttttcctttctgtg |
| reverse 68 | agtgtgtc | gaaccttggttttcctttctgtg | agtgtgtcgaaccttggttttcctttctgtg |
| reverse 69 | aggtgtag | gaaccttggttttcctttctgtg | aggtgtaggaaccttggttttcctttctgtg |
| reverse 70 | acgacatc | gaaccttggttttcctttctgtg | acgacatcgaaccttggttttcctttctgtg |
| reverse 71 | aggttgct | gaaccttggttttcctttctgtg | aggttgctgaaccttggttttcctttctgtg |
| reverse 72 | tagcggtt | gaaccttggttttcctttctgtg | tagcggttgaaccttggttttcctttctgtg |
| reverse 73 | gcggttat | gaaccttggttttcctttctgtg | gcggttatgaaccttggttttcctttctgtg |
| reverse 74 | gaacgttc | gaaccttggttttcctttctgtg | gaacgttcgaaccttggttttcctttctgtg |
| reverse 75 | gcatcgta | gaaccttggttttcctttctgtg | gcatcgtagaaccttggttttcctttctgtg |
| reverse 76 | acgttggt | gaaccttggttttcctttctgtg | acgttggtgaaccttggttttcctttctgtg |
| reverse 77 | gatgacct | gaaccttggttttcctttctgtg | gatgacctgaaccttggttttcctttctgtg |
| reverse 78 | tagcggtt | gaaccttggttttcctttctgtg | tagcggttgaaccttggttttcctttctgtg |
| reverse 79 | cgttgctt | gaaccttggttttcctttctgtg | cgttgcttgaaccttggttttcctttctgtg |
| reverse 80 | ggttatgc | gaaccttggttttcctttctgtg | ggttatgcgaaccttggttttcctttctgtg |
| reverse 81 | gctatagc | gaaccttggttttcctttctgtg | gctatagcgaaccttggttttcctttctgtg |
| reverse 82 | agactctg | gaaccttggttttcctttctgtg | agactctggaaccttggttttcctttctgtg |
| reverse 83 | cctatacc | gaaccttggttttcctttctgtg | cctataccgaaccttggttttcctttctgtg |
| reverse 84 | aacggctt | gaaccttggttttcctttctgtg | aacggcttgaaccttggttttcctttctgtg |
| reverse 85 | tccacttg | gaaccttggttttcctttctgtg | tccacttggaaccttggttttcctttctgtg |
| reverse 86 | cgataacc | gaaccttggttttcctttctgtg | cgataaccgaaccttggttttcctttctgtg |
| reverse 87 | gacttctc | gaaccttggttttcctttctgtg | gacttctcgaaccttggttttcctttctgtg |
| reverse 88 | gacttgtg | gaaccttggttttcctttctgtg | gacttgtggaaccttggttttcctttctgtg |

(continued)

| name | barcode | fas sequence | barcoded primer (5'-->3') |
|---|---|---|---|
| reverse 89 | ctgtgtct | gaaccttggttttcctttctgtg | ctgtgtctgaaccttggttttcctttctgtg |
| reverse 90 | acaggtct | gaaccttggttttcctttctgtg | acaggtctgaaccttggttttcctttctgtg |
| reverse 91 | ccgcttat | gaaccttggttttcctttctgtg | ccgcttatgaaccttggttttcctttctgtg |
| reverse 92 | aaggcgat | gaaccttggttttcctttctgtg | aaggcgatgaaccttggttttcctttctgtg |
| reverse 93 | gaacgtag | gaaccttggttttcctttctgtg | gaacgtaggaaccttggttttcctttctgtg |
| reverse 94 | gatggatg | gaaccttggttttcctttctgtg | gatggatggaaccttggttttcctttctgtg |
| reverse 95 | tgtgacac | gaaccttggttttcctttctgtg | tgtgacacgaaccttggttttcctttctgtg |
| reverse 96 | ccggtata | gaaccttggttttcctttctgtg | ccggtatagaaccttggttttcctttctgtg |
| r97 | ggttggtt | gaaccttggttttcctttctgtg | ggttggttgaaccttggttttcctttctgtg |
| r98 | aacgatgg | gaaccttggttttcctttctgtg | aacgatgggaaccttggttttcctttctgtg |
| r99 | atatgcgc | gaaccttggttttcctttctgtg | atatgcgcgaaccttggttttcctttctgtg |
| r100 | catggttg | gaaccttggttttcctttctgtg | catggttggaaccttggttttcctttctgtg |
| r101 | aaggtacg | gaaccttggttttcctttctgtg | aaggtacggaaccttggttttcctttctgtg |
| r102 | cacagtgt | gaaccttggttttcctttctgtg | cacagtgtgaaccttggttttcctttctgtg |
| r103 | cttggttc | gaaccttggttttcctttctgtg | cttggttcgaaccttggttttcctttctgtg |
| r104 | gatgacga | gaaccttggttttcctttctgtg | gatgacgagaaccttggttttcctttctgtg |
| forward 1 | gccgaatt | **gaacatggaatcatcaaggaatg** | gccgaattgaacatggaatcatcaaggaatg |
| forward 2 | cggcaatt | **gaacatggaatcatcaaggaatg** | cggcaattgaacatggaatcatcaaggaatg |
| forward 3 | gaacgttc | **gaacatggaatcatcaaggaatg** | gaacgttcgaacatggaatcatcaaggaatg |
| forward 4 | gacacact | **gaacatggaatcatcaaggaatg** | gacacactgaacatggaatcatcaaggaatg |
| forward 5 | acgacttg | **gaacatggaatcatcaaggaatg** | acgacttggaacatggaatcatcaaggaatg |
| forward 6 | atcgatcg | **gaacatggaatcatcaaggaatg** | atcgatcggaacatggaatcatcaaggaatg |
| f7 | aaccaacc | **gaacatggaatcatcaaggaatg** | aaccaaccgaacatggaatcatcaaggaatg |
| f8 | ccatcgtt | **gaacatggaatcatcaaggaatg** | ccatcgttgaacatggaatcatcaaggaatg |
| f9 | gcgcatat | **gaacatggaatcatcaaggaatg** | gcgcatatgaacatggaatcatcaaggaatg |
| f10 | caaccatg | **gaacatggaatcatcaaggaatg** | caaccatggaacatggaatcatcaaggaatg |

(continued)

| name | barcode | fas sequence | barcoded primer (5'-->3') |
|---|---|---|---|
| f11 | cgtacctt | **gaacatggaatcatcaagga atg** | cgtaccttgaacatggaatcatcaaggaatg |
| f12 | acactgtg | **gaacatggaatcatcaagga atg** | acactgtggaacatggaatcatcaaggaatg |
| f13 | gaaccaag | **gaacatggaatcatcaagga atg** | gaaccaaggaacatggaatcatcaaggaatg |

- Primer design

Forward and reverse Fas barcoded primers (Sigma Aldrich) were designed by combining sequences in Fas exons 5 or 7 with a list of barcodes previously reported by Hamady et al. (Hamady et al., 2008). Amplicon length provided enough coverage to measure Fas inclusion and skipping isoforms in the deep sequencing assay. Primers for quantitative analysis of other genes were designed using Primer 3 Plus (https://www.bioinformatics.nl/cgi-bin/primer3plus/primer3plus.cgi) using the reference sequence and / or Refseq accession numbers.

- Genome-wide screening: Multiplexed assessment of AS by deep sequencing

For the analysis of the genome-wide screen, 26880 PCR samples corresponding to the products of inclusion and skipping of Fas/CD95 exon 6 in different knockdown conditions were pooled and sequenced by Solexa paired-end deep sequencing at the EMBL genomic core facility. Paired-end sequencing allowed to determine, for each DNA molecule, the knockdown condition and the Fas/CD95 PSI value.

- Genome-wide screening. Isoform profiling and hit identification

Every forward and reverse barcode was analyzed independently (i.e. without taking into account the other primer in the pair), to take into account possible biases affecting read numbers obtained for inclusion or for skipping. Filtered barcoded reads corresponding to the inclusion or skipping isoforms were matched to their corresponding siRNA conditions and percent spliced in (PSI) calculated for each single knockdown condition as follows:

$$PSI = \left(\frac{A}{A+B}\right) * 100$$

being A the number of reads corresponding to the inclusion isoform and B the number of reads corresponding to the skipped one. In order to avoid possible biases regarding the number of reads per plate in the hit identification, robust z-scores (Z) were calculated from raw values as follows:

$$Z_i = \frac{\chi_i - \overline{\mu_{i-j}}}{1,4284 * MAD(X)} \qquad : \qquad \forall \chi_i \in X$$

where X is the vector of measurements across the plate, $\mu$ is the median PSI value for the plate and MAD is the median absolute deviation. Calculation of robust Z-scores was performed for each of the Solexa lanes analyzed. A total of 1505 Knockdown conditions for which the median of Z-scores across different lanes was Z>2 or Z<-2 were selected for further validation.

- Genome-wide screening: Hit-validation.

Positive hits obtained in the deep sequencing analysis were selected and retested in biological triplicate by HTCE (Labchip GX, Perkin Elmer). To control for possible off-target effects, the 427 positive hits from the previous analysis were selected and a custom independent siRNA library against those factors (On target PLUS smartpool, Thermo-Scientific) was used to transfect HeLa cells. Cells were transfected and processed in triplicate as previously described and PCR fragments for each condition were analyzed by high throughput capillary electrophoresis using the formulas mentioned above. RNAs were isolated and processed in triplicate as described above and RT-PCR products for each condition were analyzed by HTCE. Genes for which Z was >2 or <2 were considered as positive, and a total number of 200 genes were finally selected as high confidence hits.

- Real-time qPCR

First strand cDNA synthesis was set up with 500 ng of RNA, 50 pmol of oligo-dT (Sigma-Aldrich), 75 ng of random primers (Life Technologies), and superscript III reverse transcriptase (Life Technologies) in 20 μl final volume, following the manufacturer's instructions. Quantitative PCR amplification was carried out using 1 μl of 1:2 to 1:4 diluted cDNA with 5 μl of 2X SYBR Green Master Mix (Roche) and 4 pmol of specific primer pairs in a final volume of 10 μl in 384 well-white microtiter plates, Roche). qPCR mixes were analyzed in triplicates in a Light Cycler 480 system (Roche) and fold change ratios were calculated according to the Pfaffl method (Pfaffl, 2001).

The primers used for real time qPCR were the following:

**Table 3**

| Primer | Sequence |
|---|---|
| **FAS Ex7 AS** | GGAGATTCATGAGAACCTTGG |
| **FAS EJ56 S** | TGCAAAGAGGAAGGATCCAG |
| **FAS EJ57 S** | CCAAGTGCAAAGAGGAAGTGA |
| **FAS intron Up F** | TTTTTACGGTTATATTCTCCTTTCC |
| **FAS intron up R** | AAAGGACTTTCTAGTAATCAAGAGTGA |
| **FAS intron down F** | CTGGGCATCCATAGCAAGTT |
| **FAS intron down R** | CACCACTCTCTGTCCAGCAA |
| **FAS intron 2 F** | AAGAAAGCTGTCCTGGCACT |
| **FAS intron 2 R** | AAGCTGTTGGTTTCTGCCTG |
| **FAS GE ex2 F** | CAAGGGATTGGAATTGAGGA |
| **FAS GE ex2 R** | CTGGAGGACAGGGCTTATGG |
| **MCL1 Ex1 S** | GAAGGCGCTGGAGACCTTAC |
| **MCL1 EJ12 AS** | CAGTTTCCGAAGCATGCCTT |
| **MCL1 EJ13 AS** | GAAGAACTCCACAAACCCATCCTT |
| **MCL1 intron Up F** | CAAGTGGGGGTCAACCTGAGT |
| **MCL1 intron up R** | CACGGCCTCCTTTGTCTAAA |
| **MCL1 intron down F** | TCCCCGCTTAAGAAACTGAA |
| **MCL1 intron down R** | AAGGTTTCCCCCTAAAGCAA |
| **MCL1 GE ex1 F** | AAGCCAATGGGCAGGTCT |
| **MCL1 GE ex1 R** | GAAGGCCGTCTCGTGGTT |
| **FN1EDB Ex24 S** | GGCCTGGAGTACAATGTCAG |
| **FN1EDB EJ24-24a AS** | GTGAGTTGGGGCACCTCTG |
| **FN1EDB EJ24-25 AS\*** | AGTGGGAGGAGGAACAGCTG |
| **FN1EDB intron Up F** | GATCTCCCAGCTGCACTTTC |
| **FN1EDB intron up R** | TCCCAGTGAAGCACAGACAG |
| **FN1EDB intron down F** | ATGCTGCCACCAGTTACTCC |
| **FN1EDB intron down R** | CCCAATCCACCCTATCTGAA |

(continued)

| Primer | Sequence |
|---|---|
| FN1 intron 3 F | CCTCTTCCTTGCCATCCAGA |
| FN1 intron 3 R | GGTCTGGTCACTTGGAGTCA |
| FN1EDB GE ex3-4 F | GGCGAGGGAGAATAAGCTGT |
| FN1EDB GE ex3-4 R | TCATGTGGTCTCCTCCAGGT |
| CHEK2 Ex7 S | CAGCTCTCAATGTTGAAACAGAA |
| CHEK2 EJ78 AS | AATCTTGATGATGCAAGGATG |
| CHEK2 EJ79 AS | GCTCTCCCCCTTCCATCATG |
| CHEK2 intron Up F | TGCATTTTACAAGGGCACAA |
| CHEK2 intron up R | CAAAGGTCAGGCTCTCTTGG |
| CHEK2 intron down F | TGATCACAGCACTGGGAGAG |
| CHEK2 intron down R | GCCCGGCTCTGTATTATCAC |
| CHEK2 intron 6 F | CTGACTGTGGAGACTAGGGC |
| CHEK2 intron 6 R | AGCCTCAGAATCCCCATCTT |
| CHEK2 GE ex2 F | GGTGCCTGTGGAGAGGTAAA |
| CHEK2 GE ex2 R | GCCTCTCTTGCTGAACCAAT |
| HPRT1 F | CTTTGCTGACCTGCTGGATT |
| HPRT1 R | CCCCTGTTGACTGGTCATTACA |

- Network construction. RT-PCR and High-throughput capillary Electrophoresis.

Cellular mRNAs were reverse-transcribed using superscript III retro transcriptase (Invitrogen, Life Technologies) following the manufacturer's recommendations. PCR reactions for every individual splicing event analyzed were carried out using forward and reverse primers in exonic sequences flanking the alternatively spliced region of interest and further reagents provided in the GoTaq DNA polymerase kit (GoTaq, Promega). Primers used in this study are listed in Table 5.

HTCE measurements for the different splicing isoforms were performed in 96-well format in a Labchip GX Caliper workstation (Caliper, Perkin Elmer) using a HT DNA High Sensitivity LabChip chip (Perkin Elmer). Data values were obtained using the Labchip GX software analysis tool (version 3.0).

- Quantification of AS changes from HTCE measurements

Robust estimates of isoform ratios upon siRNA or pharmacological treatments were obtained using the median PSI (Percent Spliced IN) indexes of the biological triplicates for each knockdown-AS pair. From this value $x_i$ the effect of each treatment was summarized as a robust Z-score (Birmingham et al., 2010):

$$Z'_i = \frac{x_i - \tilde{x}}{\hat{\sigma}}$$

where $\tilde{x}$ and $\hat{\sigma}$ are the particular ASE's sample median and median absolute deviation (*MAD*) -based consistent estimator of the standard deviation ($\hat{\sigma} \approx 1.483\ MAD \approx 1.483\ MAD$), respectively.

- Gene ontology analysis

Functional gene ontology (GO) enrichments were calculated using various commercial and public sources, including Ingenuity pathway analysis (IPA, Ingenuity systems, Qiagen), Gorilla (http://cbl-gorilla.cs.technion.ac.il), DAVID bioinformatics database (http://david.abcc.ncifcrf.gov, NIAID, NIH) and Funcassociate (http://llama.mshri.on.ca/funcassociate/, Berriz et al., 2003).

- Iron treatments and intracellular iron measurements

Hemin, Deferoxamine (Desferal) and Ciclopirox olamine were purchased from Sigma Aldrich. HeLa or HepG2 cells were treated with these reagents at 100 μM for 18-24h to modulate intracellular iron levels. To evaluate the effects of these treatments on Fas/CD95 AS, RNA was isolated using the RNeasy minikit (Qiagen) following manufacturer's recommendations and RNA quality was assessed by Agilent Bioanalyzer nano assay and nanodrop spectrophotometry (Thermo-Scientific). 24h after treatment with iron-modulating drugs, cells were washed twice with 1X PBS and then stained with 5 μM of PhenGreen SK diacetate (Life Technologies) in PBS for 15 min at 37°C. Cells were then treated with Trypsin/EDTA, harvested and washed thoroughly in 1X PBS and centrifuged at 1000 rpm for 5 min. Cells were resuspended in 500 μl of PBS and transferred to disposable FACS tubes. Fluorescence measurements were obtained using a FACSCalibur flow cytometry system (BD Biosciences).

- Network construction: Data preprocessing and reduction

- *Removal of sparse variables and imputation of missing values:* Estimation of the sample covariance matrix requires complete data. However, the $p$ **x** $n$ matrix that contains the measurements of the $p$ variables (genes) in $n$ conditions (ASEs) includes missing (*NA*) values. In order to overcome this problem, first sparse variables (*#NA values > n/2*) are removed. The remaining missing values are subsequently filled-in by applying *k*-nearest-neighbor based imputation (Hastie et al., 1999, R function impute available as part of the bioconductor project at http://www.bioconductor.org/). For the purposes of the assays of the present application, it was set that *k=ceiling(0.25 sqrt(p))*.
- *Removal of uninformative ASEs.* ASEs not affected to a significant degree (*median absolute (ΔPSI)<1*) by the KDs offer little information on the genes' covariance and can potentially introduce noise and are thus discarded from subsequent analyses.
- *Data scaling.* Prior to sample covariance estimation the data matrix is standardized along the gene dimensions. Standardization of the events ensures that only the shape and not the magnitude of the fluctuations is taken into account for covariance estimation and it is equivalent to using correlation in lieu of covariance in all downstream analyses.

- Robust sample correlation estimation

Robust correlation estimation is based on an iterative weighting algorithm, implemented in R code, that discriminates between technical outliers and reliable measurements with high leverage. The weighting for measurements relies on calculation of a reliability index that takes into account their cumulative influence on correlation estimates of the complete dataset.

In particular, starting with an $M = p$ x $n$ dataset of $p$ standardized variables and $n$ samples we wish to derive a robust correlation $p$ x $p$ matrix. We calculate the influence of a sample $u$ in the estimation of the correlation of two variables $X_a$, $X_b$ using the deleted residual distance:

$$D_u(X_a, X_b) = |p(X_a, X_b) - p_{-u}(X_a, X_b)|$$

where $p(X_a, X_b)$ and $p_{-u}(X_a, X_b)$ are the Pearson's correlation estimates before or after removing the observations $M_{au}$, $M_{bu}$ coming from sample u.

A measure of the reliability of these measurements for the estimation of $p(X_a, X_b)$ given all the observed data is then based on their cumulative influence on high correlates of $X_a$, and $X_b$:

$$R^u_{a,b} = 1 / \frac{\sum_{i=1}^{p} D_u(X_a, X_i)\mathbb{I}(a, i) + \sum_{i=1}^{p} D_u(X_b, X_i)\mathbb{I}(b, i)}{\sum_{i=1}^{p} \mathbb{I}(a, i) + \sum_{i=1}^{p} \mathbb{I}(b, i)}$$

where II($a,i$) is the indicator function:

$$\mathbb{I}(a, i) := \begin{cases} 1 \text{ if } i \neq a \text{ } AND \text{ } |p(X_a, X_i)| > T \\ 0 \text{ otherwise} \end{cases}$$

*T* being a minimum correlation threshold for considering a pair of variables.

Intuitively, if an observation $M_{au}$ distorts correlation estimates among all highly correlated partners of $X_a$ it is considered an outlier and is down-weighted. On the other hand if including this observation yields similar correlation estimates for most of these pairs it is considered reliable even if its influence on the individual estimate of $p(X_a, X_b)$ is high. The advantage of this procedure is that it derives weights for individual measurements (as opposed to a single weight for every sample) while taking into account the complete dataset.

The algorithm can be used iteratively, however in the author's experience the estimates converge within 1 to 2 iterations.

- Network reconstruction: Graphical model selection using Graphical Lasso

Undirected graphical models (UGMs) such as Markov random fields (MRFs) represent real-world networks and attempt to capture important structural and functional aspects of the network in the graph structure. The graph structure encodes conditional independence assumptions between variables corresponding to nodes of the network. The problem of recovering the structure of the graph is known as model selection or covariance estimation.

In particular, let *G=(V,E)* be an undirected graph on *p=|V|* nodes. Given *n* independent, identically distributed (i.i.d) samples of *X=(X1,...,Xp)*, we wish to identify the underlying graph structure. We restrict the analysis to Gaussian MRFs where the model assumes that the observations are generated from a multivariate Gaussian distribution *N(μ,Σ)*. Based on the observation that in the Gaussian setting, zero components of the inverse covariance matrix $\Sigma^{-1}$ correspond to conditional independencies given the other variables, different approaches have been proposed in order to estimate $\Sigma^{-1}$.

Graphical lasso (gLasso) provides an attractive solution to the problem of covariance estimation for undirected models, when graph sparsity is a goal (Friedman et al., 2007). The gLasso algorithm has the advantage of being consistent, i.e. in the presence of infinite samples its parameter estimates will be arbitrarily close to the true estimates with probability 1.

$L_1$ regularization (lasso) is a smooth form of subset selection for achieving sparsity. In the case of gLasso the model is constructed by optimizing the log-likelihood function:

$$\log \det \Theta - tr(S\Theta) - r\|\Theta\|_1$$

where $\Theta$ is an estimate for the inverse covariance matrix $\Sigma^{-1}$, S is the empirical covariance matrix of the data, $\|\Theta\|_1$ is the *L1* norm i.e the sum of the absolute values of all elements in $\Theta^{-1}$, and *r* is a regularization parameter (in this case selected based on estimates of the FDR, see below). The solution to the glasso optimization is convex and can be obtained using coordinate descent.

The glasso process for network reconstruction was implemented using the glasso R package by Jerome Friedman, Trevor Hastie and Rob Tibshirani (http://statweb.stanford.edu/~tibs/glasso/).

- Network reconstruction. Partition of network into modules

Network modules or communities can be defined loosely as sets of nodes with a more dense connection pattern among their members than between their members and the remainder of them. Module detection in real-world graphs is a problem of considerable practical interest as they often capture meaningful functional groupings.

Typically community structure detection methods try to maximize modularity, a measure of the overall quality of a certain network partition in terms of the identified modules (Newman, 2006). In particular, for a network partition *p*, the network modularity *M(p)* is defined as:

$$M(p) = \sum_{k=1}^{m} \left[ \frac{l_k}{L} - \left(\frac{d_k}{2L}\right)^2 \right]$$

where m is the number of modules in *p*, $l_k$ is the number of connections within module *k*, *L* is the total number of network connections and $d_k$ is the sum of the degrees of the nodes in module *k*.

Here, the authors identify modules of genes that exhibit similar perturbation profiles among the assayed events by maximizing the network's modularity using the greedy community detection algorithm (Clauset et al., 2004) implemented in the *fastgreedy.community* function of the igraph package (Csardi and Nepusz, 2006, http://igraph.sourceforge.net/doc/R/fastgreedy.community.html).

- Graph Manipulation and Plotting

All functions for network plotting and manipulation were written using the R implementation of the igraph library (Csardi and Nepusz, 2006: http://igraph.sourceforge.net) unless otherwise stated.

- Estimation of FDR network edges

In order to acquire a measure of the FDR of network edges as a function of the number of ASEs used, random networks were reconstructed after permuting the sample data for every gene. This operation preserves the characteristics of the marginal distributions of the genes but removes any biologically meaningful correlations. This process was repeated for 100 permutations and for random subsamples of the ASEs of size $S=\{3..35\}$ to obtain an estimate of the number of random network edges $|E|_R^S$ recovered for each sample size $S$ at a fixed glasso regularization parameter. Similarly, the number of actual edges $|E|_A^S$ for random ASEs subsamples of the same sizes was estimated using the observed (non-permuted) data. At each subsample size $S$ the FDR for the network edges was estimated as the ratio of the mean number of edges recovered in the random graphs over the mean number of edges recovered in the actual graphs for all the different permutations and random subsamples of that size:

$$FDR_S = \frac{mean(|E|_R^S)}{mean(|E|_A^S)}$$

- Data subsampling for identification of indispensable and ancillary connections

For the identification of the most general set of functional interactions network reconstruction was repeated using the same procedure described above for 10,000 different subsamples of 17 from the total of 35 ASEs. General connections were then identified as those recovered in at least 90% of the reconstructed networks.

In order to identify a set of ancillary functional connections that are robustly recovered in a significant number of ASEs subsets but are absent and non-correlated in others we looked for the interactions that were present with an absolute correlation value >0.5 in >20% of the samples AND were absent with an absolute correlation value <0.2 in >20% of the samples.

- Principal Component Analysis of Ancillary connections

Principal Component Analysis for ancillary edges was performed using R-mode PCA (R function princomp). The input dataset is the scaled 95x35 matrix containing the absolute average correlation for each of the 95 edges over the subsets of the 10,000 samples that included each of the 35 events. The coordinates for projecting the 95 edges are directly derived from their scores on the first two principal components. The arrows representing the top 10 discriminatory events (based on the vector norm of the first two PC loadings) were drawn from the origin to the coordinate defined by the first two PC loadings scaled by a constant factor for display purposes.

- Splice sites scoring, Identification of probable HNRPC binding sites and alu elements

For the identification and scoring of 3' splice sites (3'ss), it was used custom-built position weight matrices (PWMs) of length 21 -18 intronic plus 3 exonic positions. The matrices were built using a set of human splice sites from constitutive, internal exons compiled from the hg19 UCSC annotation database (Karolchik et al., 2014). Background nucleotide frequencies were estimated from a set of strictly intronic regions. Threshold was set based on a FDR of 0.5/kbp of random intronic sequences generated using a 2nd order Markov chain of actual intronic regions.

The authors considered as probable HNRPC binding sites consecutive stretches of U{4} as reported in (Zarnack et al., 2013).

Annotation of ALU elements was based on the RepeatMasker track developed by Arian Smit (http://www.repeatmasker.org) and the Repbase library (Jurka et al., 2005).

- Integration in the splicing network of the effects on AS of iron homeostasis

To integrate in the network the effects on AS of iron homeostasis, cells were treated for 24h with different pharmacological iron modulatory conditions (Hemin or Desferal 100 $\mu$M), or for 72h with siRNAs against ACO1 and FTL (25 nM, ON TARGET PLUS smartpool, Thermo-Scientific). RNAs were then extracted and first strand cDNA was generated as previously described. PCRs for 35 alternative splicing events involved in cell proliferation and apoptosis were carried out with these samples, and alternative isoform ratios determined by high throughput capillary electrophoresis. These results were then integrated in the splicing network analysis.

- CLIP

Ultraviolet light-induced crosslinking / immunoprecipitation (CLIP) experiments were carried out essentially as described (Konig et al., 2011) using rabbit anti-SRSF7 polyclonal antibody (Novus Biological NBP1-92382) to detect interactions of the endogenous proteins, rabbit T7 polyclonal antibody (Novus Biological NB600-372) to detect T7 epitope-tagged SRSF7, or rabbit IgG (Sigma Aldrich I-5006) as a control. After the immunoprecipitation step, samples were treated with proteinase K in PK buffer (100 mM Tris-HCI pH 7.4, 50 mM NaCl, 10 mM EDTA) for 20 min at 37$\underline{o}$C and then incubated in PK buffer supplemented with 7M urea for further 20 min at 37$\underline{o}$C. RNA was extracted by phenol-chloroform extraction followed by ethanol precipitation and samples were reverse transcribed and analyzed by real-time qPCR.

- **Example 1. Prior genome-wide screening and construction of a functional network of splicing factors**

1.1. **Genome-wide siRNA screen**

1.1.a. *Screening methodology and data analysis*

To systematically identify regulators of Fas/CD95 AS, a siRNA library (siGENOME, Dharmacon) comprising more than 22000 pools of siRNAs targeting all known and predicted human protein-coding genes was transfected in HeLa cells using a robotized procedure. 72 hours after transfection, mRNAs were isolated in oligodT plates using an automated protocol (Figure 1A) and cDNA libraries of the 22000 transcriptomes were then generated by automatized reverse transcription using oligodT and random hexamers. To interrogate the ratio of Fas/CD95 exon 6 inclusion/skipping in these libraries, PCR reactions were set up using barcoded primers corresponding to sequences in the flanking exons 5 and 7. In this strategy, each individual siRNA condition is identified by a particular combination of two barcodes, one in each of the PCR primers (Fig. 1A). To measure isoform ratios in each condition, the PCR products were pooled together (>26000 different conditions including controls) and the sequences of individual molecules determined by paired-ended Illumina HiSeq sequencing (532 million reads in total, 100 nucleotide length, obtained either from pooled or technical replicate samples, see Supplementary Methods). This provides on average more than 4.000 reads per technical replicate or 20.000 reads per knockdown condition, which allows a robust determination of isoform ratios. Data filtering allowed read classification based upon a) the barcode combination and b) the presence or absence of Fas/CD95 exon 6 sequences (Fig. 1B). The Percent Spliced In (PSI) index (Katz et al., 2011) was determined as the ratio between exon 6-containing reads over the total number of reads in each condition, with changes in PSI even lower than 5% being reproducibly detected and validated.

The robustness of the screen was verified by comparing the PSI values for hundreds of replicas of control siRNAs and SLU7 (a known regulator of Fas/CD95 splicing) siRNAs (two-sided t test p-value < E-20). The accuracy of the deep sequencing method was validated by the following controls: a) consistent PSI estimates were obtained by deep sequencing and high throughput capillary electrophoresis (HTCE) over a dilution range of three orders of magnitude, for control samples containing defined ratios of Fas/CD95 inclusion and skipping isoforms; b) a good correlation was observed between PSI values of biological replicas in a pilot screen focused on the knockdown of 250 splicing factors, estimated in parallel by both technologies (0.66 and 0.59, respectively) (Fig. 1D) c) excellent correlation between PSI indexes estimated by the two technologies for 500 control samples from the genome-wide screen were also obtained (0.88 Pearson correlation) (Figure 1C), further validating the consistency of the screen results.

1505 conditions were identified in which the knockdown of individual genes led to a statistically significant difference in PSI compared to the control siRNA pool (-2 > Robust Z score > +2) (Figure 1E). To further validate these results, biological triplicates of the knockdown hits were analyzed by HTCE, which enables the rapid separation and quantification of amplification products corresponding to the two isoforms for 96 samples in parallel. This resulted in 427 validated hits. The non-validated hits were usually closer to the cut-off but may also reflect different sensitivities of the two technologies. As a second validation, and also to discard possible off-target effects of the siRNA pools, a second screen was carried out using siRNA pools from a non-overlapping siRNA library against the 427 factors in biological triplicates. This resulted in 200 genes whose knockdown affects Fas/CD95 AS, validated with two independent siRNA libraries and two independent technologies (deep sequencing and HTCE, the latter in biological triplicates) (Figure 1F).

Functional gene ontology (GO) analysis of the validated 200 hits gave rise to the results summarized in Fig. 1G, Fig. 1H and Fig. 1I and in Table 4 (A and B).

**Table 4. Factors involved in Fas AS regulation**

| A. Splicing | components involved | in Fas AS |
|---|---|---|
| **Gene Symbol** | **Percent Spliced In (PSI) (average control 94.7)** | **p-value** |
| **SR proteins** | | |
| SRSF6 | 77.40 | 8.751E-09 |
| SRSF1 | 99.11 | 1.600E-02 |
| **Non-snRNP spliceosome-assembly proteins** | | |
| SIAHBP1 | 63.27 | 1.995E-05 |
| U2AF2 | 75.70 | 4.207E-10 |
| U2AF1 | 65.11 | 7.744E-14 |
| FNBP3 | 74.30 | 5.460E-03 |
| **U2 snRNP specific proteins** | | |
| SF3B1 | 28.67 | 1.131E-24 |
| SF3B2 | 54.27 | 4.411E-18 |
| SF3B3 | 50.22 | 2.509E-21 |
| SF3A1 | 55.32 | 7.248E-20 |
| SF3A2 | 87.16 | 1.214E-04 |
| SF3A3 | 50.66 | 4.634E-20 |
| SF3B4 | 77.87 | 5.806E-10 |
| **U5 snRNP specific proteins** | | |
| PRPF8 | 53.86 | 2.104E-04 |
| U5-200KD | 70.94 | 1.014E-02 |
| **U4/U6 snRNP specific proteins** | | |
| PPIH | 63.59 | 3771E-03 |
| **Sm/LSm core proteins** | | |
| SNRPB | 78.84 | 8.573E-08 |
| SNRPD1 | 85.22 | 6.516E-04 |
| SNRPD2 | 80.65 | 3.327E-05 |
| SNRPD3 | 59.91 | 8.062E-07 |
| SNRPF | 71.87 | 9.487E-04 |
| SNRPG | 75.43 | 3.197E-02 |
| **Catalytic step II and late acting proteins** | | |
| DHX8 | 76.77 | 2.723E-02 |
| SLU7 | 71.32 | 1.192E-03 |
| CDC40 | 78.09 | 1.613E-10 |
| **Exon Junction complex** | | |
| RNPS1 | 72.21 | 1.565E-03 |
| RBM8A | 63.43 | 6.060E-04 |
| **Other previously reported splicing factors/SAPs** | | |
| TCERG1 | 82.48 | 2.314E-05 |
| IK | 58.75 | 3.102E-09 |

(continued)

| RRM-containing proteins | | |
|---|---|---|
| RNPC2 | 42.94 | 2.076E-10 |
| **DExD box proteins** | | |
| DDX48 | 77.55 | 3. 706E-02 |
| **Proteins with other known motifs** | | |
| XAB2 | 53.59 | 4.751 E-19 |
| ZNF207 | 74.07 | 6.938E-07 |
| **Proteins designated as H complex components** | | |
| BUB3 | 86.52 | 3.094E-05 |
| **Others** | | |
| EWSR1 | 74.02 | 3.427E-06 |
| SFPQ | 73.87 | 3.815E-07 |
| **Group 1 (Early splicing factors)** | | |
| THRAP3 | 60.12 | 2.280E-03 |
| **Group 2 (B complex stage)** | | |
| SMU1 | 23.79 | 9.816E-27 |
| AQR | 71.81 | 7.392E-09 |
| **Group 3 (C complex stage)** | | |
| LENG1 | 65.70 | 7.800E-04 |

| B. Other regulators of Fas AS | | |
|---|---|---|
| **Gene Symbol** | **Percent Spliced In (PSI) (average control 94.7)** | **p-value** |
| **Chromatin remodeling and Histone modification** | | |
| SMARCA4 | 93.17 | 2.732E-02 |
| SMARCC2 | 91.02 | 1.475E-01 |
| SMARCD1 | 97.09 | 2.660E-01 |
| SAP18 | 85.85 | 1.816E-03 |
| SETD2 | 69.50 | 1.835E-04 |
| **Transcription factors** | | |
| MYBL1 | 87.67 | 4.371 E-01 |
| MYC | 69.86 | 3.663E-04 |
| DSCR6 | 51.92 | 1.368E-04 |
| SNW1 | 61.53 | 3.060E-06 |
| ZNF207 | 74.07 | 6.938E-07 |
| ZNF830 | 87.52 | 1.146E-03 |
| **Topoisomerase and RNA pol II complex** | | |
| TOP2A | 89.03 | 4.237E-02 |
| TOPBP1 | 79.50 | 4.622E-06 |
| POLR2F | 84.83 | 1.724E-02 |
| **HOX related genes** | | |
| HOXA10 | 88.78 | 6.485E-01 |
| HOXB2 | 83.92 | 3.049E-02 |
| HOXD9 | 88.22 | 6.340E-01 |
| PKNOX1 | 89.97 | 6.230E-01 |

(continued)

| DNA repair | | |
|---|---|---|
| XRCC2 | 92.87 | 5.335E-01 |
| XRCC5 | 92.83 | 3.936E-02 |
| **RNA transport / THOC complex** | | |
| CSE1L | 86.81 | 1.014E-01 |
| THOC5 | 93.33 | 6.322E-01 |
| THOC6 | 83.33 | 2.262E-07 |
| THOC7 | 85.85 | 4.449E-03 |
| **Signal transduction pathways** | | |
| FGFR1 | 60.70 | 2.137E-07 |
| MC1R | 81.96 | 2.899E-02 |
| RAB7A | 92.93 | 4.609E-01 |
| RAC2 | 91.65 | 2.857E-03 |
| RALA | 87.93 | 4.876E-04 |
| RHOG | 90.69 | 1.471 E-02 |
| WNT4 | 90.44 | 2.261 E-03 |
| WNT6 | 92.80 | 5.515E-01 |
| **Cellular metabolism** | | |
| ACADSB | 50.41 | 5.247E-08 |
| PDP2 | 64.63 | 2.295E-06 |
| ALAD | 60.82 | 6.218E-05 |
| ATP5B | 67.15 | 1.428E-03 |
| **Iron homeostasis related genes** | | |
| ACO1 | 70.05 | 1.237E-05 |
| FTL | 96.08 | 2.206E-01 |
| B2M | 88.94 | 6.549E-01 |
| PCBP2 | 76.27 | 1.550E-04 |

They showed -as expected- a clear enrichment in RNA post-transcriptional modification factors. Other GO categories were also significantly enriched, including cell cycle (16 hits) and cell death (35 hits) (Fig. 1I), suggesting possible links between Fas/CD95 AS and regulators of cell division and apoptosis. As expected, molecular functions related to nucleic acid/RNA binding were significantly enriched in the GO analysis (Fig. 1G and 1H). Interestingly, among the RNA processing factors identified, we observed an overrepresentation of core and spliceosomal components and factors acting late in spliceosome assembly over classical regulators of AS, suggesting extensive potential of the core splicing machinery for the modulation of splice site choice.

Additional GO categories were also enriched. A first category contains proteins involved in DNA metabolism, including transcription factors (e.g. MYC, MYBL1), proteins with dual roles in transcription and splicing that have been previously linked to DNA damage response (e.g. SKIIP or ZNF830), topoisomerase-associated factors (e.g. TOP2A and TOPB1) and chromatin remodeling factors (e.g. components of the SWI/SNF complex or the histone methyl-transferase SETD2). These factors may affect Fas/CD95 AS through mechanisms involving the coupling between transcription and RNA processing. Factors involved in other aspects of RNA metabolism include components of the THO complex, which are deposited after capping and splicing of pre-mRNAs and promote RNA transport, suggesting that differential export of transcript isoforms contributes to the observed ratios between alternatively spliced mRNAs. A third category of regulators of Fas/CD95 AS includes various factors acting outside of the nucleus to activate particular signaling pathways, including Fibroblast Growth Factor Receptor 1 and several members of the Ras- and Rho- families of small GTPases. The data also indicate a link between Fas/CD95 splicing and several genes encoding mitochondrial proteins involved in the regulation of energy metabolism, including enzymes implicated in sugar and fatty acid catabolism and oxidative phosphorylation.

Iron homeostasis related genes have been also found as regulators of Fas/CD95 AS, specifically ACO1 (PSI=70.05, p-value=1.237x10[-05]), FTL (PSI=96.08, p-value=2.206x10[-01]), B2M (PSI=88.94, p-value=6.549x10[-01]) and

PCBP2 (PSI=76.27, p-value=$1.550 \times 10^{-04}$). Their involvement in Fas/CD95 AS was further studied in the assays below.

## 1.2. Construction of a functional network of splicing factors

1.2.a. *Screening methodology of Alternative Splicing Events and data analysis*

Results from the genome-wide siRNA screen to identify regulators of Fas/CD95 AS disclosed in section 1.1 revealed that knockdown of a significant fraction of core SFs (defined as proteins identified in highly purified spliceosomal complexes assembled on model, single intron pre-mRNAs, Wahl et al, 2009) caused changes in Fas/CD95 exon 6 inclusion. The number of core factors involved and the extent of their regulatory effects exceeded those of classical splicing regulatory factors like SR proteins or hnRNPs.

To systematically evaluate the contribution of different classes of splicing regulators to AS regulation, we set up a screen in which we assessed the effects of knockdown of each individual factor on 38 ASEs relevant for cell proliferation and/or apoptosis. The process is summarized in Fig. 2A.

The ASEs were selected due to their clear impact on protein function and their documented biological relevance. As represented in Fig. 2B, they can be grouped in apoptosis related events, cell proliferation related events and events having features of the first two groups. They can be found listed below in Table 5, wherein the common abbreviated gene symbol, the gene identification number in HUGO database (http://www.genenames.org/), the sequence of the alternatively spliced exon, the strand where said sequence can be found, the number of the human chromosome where the gene can be found ("Chr name") and the chromosome positions where the exon starts and ends.

The sequences of the spliced exons can be found in the sequence listing (SEQ ID NO:1 to SEQ ID NO:46). The forward and reverse primers used for identifying the events are also indicated

Table 5.- Alternative splicing events checked for each knocked-down AS Factor

| Gene symbol | Gene ID | Forward Primer | Reverse Primer | Size | Strard | start | end | Chr name | Chr size | start | end |
|---|---|---|---|---|---|---|---|---|---|---|---|
| FAS | 355 | gaacatggaatcatcaaggaatgcac | agttggagattcatgagaaccttgg | 63 | + | 0 | 63 | chr10 | 135534747 | 90770509 | 90770572 |
| OLR1 | 4973 | ggcatggagaaaactgttacctattttcctc | cactgtgctcttaggtttgccttcttctg | 116 | - | 0 | 116 | chr12 | 133851895 | 10312945 | 10313061 |
| PAX6 | 5080 | ccggcagaagattgtagagc | ctcccgcttatactgggcta | 42 | - | 0 | 42 | chr11 | 135006516 | 31823418 | 31823460 |
| CHEK2 | 11200 | cagctctcaatgttgaaacagaa | cagccaagagcatctggtaa | 62 | - | 0 | 62 | chr22 | 51304566 | 29099492 | 29099554 |
| FN1EDB | 2335 | ggcctggagtacaatgtcagt | catggtgtctggaccaatgt | 273 | - | 0 | 273 | chr2 | 243199373 | 216257653 | 216257926 |
| FN1EDA | 2335 | ctcagaatccaagcggagag | aacattgggtggtgtccact | 270 | - | 0 | 270 | chr2 | 243199373 | 216245533 | 216245803 |
| NUMB | 8650 | ctccctgtgctcacagatca | cggacgctcttagacacctc | 144 | - | 0 | 144 | chr14 | 107349540 | 73745988 | 73746132 |
| BCL2L1 | 598 | cagtaaagcaagcgctgagg | aagagtgagcccagcagaac | 189 | - | 0 | 189 | chr20 | 63025520 | 30309457 | 30309646 |
| RAC1 | 5879 | tgccaatgttatggtagatgga | ctttgcacggacattttcaa | 57 | + | 0 | 57 | chr7 | 159138663 | 6438292 | 6438349 |
| MST1R | 4486 | gctttacactgcctggcttt | gccaccagtagctgaagacc | 147 | - | 0 | 147 | chr3 | 198022430 | 49933393 | 49933540 |
| MADD | 8567 | agcctctatcggaaccacag | ggggtgatggaggttctctt | 60 | + | 0 | 60 | chr11 | 135006516 | 47310518 | 47310578 |
| CASP2 | 835 | gctctttgacaacgccaact | ggcatagccgcatatcatgt | 61 | + | 0 | 61 | chr7 | 159138663 | 142997472 | 142997533 |
| APAF1 | 317 | atgcgacatcagcaaatgag | ctctgcaatcagccaccttt | 129 | + | 0 | 129 | chr12 | 133851895 | 99097148 | 99097277 |
| MINK1 | 50488 | cctcagaggacctcatctatcg | cgtgagaggctggaaggac | 111 | + | 0 | 111 | chr17 | 81195210 | 4795696 | 4795807 |
| MAP3K7 | 6885 | gaatctggacgtttaagcttgg | tgaccaggttctgttccagtt | 81 | - | 0 | 81 | chr6 | 171115067 | 91254270 | 91254351 |
| MAP4K3 | 8491 | ggccaagtgaaatttgatcc | ttgcacctaaaaagtaaccacct | 63 | - | 0 | 63 | chr2 | 243199373 | 39535083 | 39535146 |
| NOTCH3 | 4854 | cgtcagtgtgaactcctctcc | ctcaggcactcatccacatc | 156 | - | 0 | 156 | chr19 | 59128983 | 15295105 | 15295261 |
| MAP4K2 | 5871 | gagctgacagcgtctgtgg | gcgagtcttatctctcagtttgg | 132 | - | 0 | 132 | chr11 | 135006516 | 64568371 | 64568503 |
| PKM2 9 | 5315 | gctggagagcatgatcaagaa | caacattcatggcaaagttca | 167 | - | 0 | 167 | chr15 | 102531392 | 72495363 | 72495529 |
| PKM2 10 | 5315 | gctcagagcggagaaagcat | tcagtctttcctggtgagagat | 167 | - | 0 | 167 | chr15 | 102531392 | 72494794 | 72494961 |
| VEGFA | 7422 | agaccttacgacgggttgg | accagctcctactccagcaa | 66 | + | 0 | 66 | chr6 | 171115067 | 43752277 | 43752343 |
| MCL1 | 4170 | gaatgattaagtagaggcagttcca | tcccattatggagcctga | 248 | - | 0 | 248 | chr1 | 249250621 | 150550719 | 150550967 |
| CFLAR | 8837 | cgggcaggctctggatctc | ccagcaccattttcttgg | 229 | + | 80 | 229 | chr2 | 243199373 | 202006096 | 202006325 |

| Gene symbol | Gene ID | Forward Primer | Reverse Primer | Size | Strrand | start | end | Chr name | Chr size | start | end |
|---|---|---|---|---|---|---|---|---|---|---|---|
| CASP9 3 | 842 | gaggccgtccacaacttc | agcccagggtgcaagttc | 35 | - | 0 | 35 | chr1 | 249250621 | 15834367 | 15834402 |
| CASP9 4 | 842 | accatggcacaaaactgtga | tcctatgatcacctgccaca | 177 | - | 0 | 177 | chr1 | 249250621 | 15833393 | 15833570 |
| CASP9 5 | 842 | ctcagccgacttcagctcttc | ccccgttcctgttctcttct | 90 | - | 0 | 90 | chr1 | 249250621 | 15832484 | 15832574 |
| Gene symbol | Gene ID | Forward Primer | Reverse Primer | Size | Strand | start | end | Chr name | Chr size | start | end |
| CASP9 6 | 842 | atcctccaaagttgcaccag | atgatacaaggccgaagcag | 148 | - | 0 | 148 | chr1 | 249250621 | 15831105 | 15831253 |
| SYK | 6850 | cgggcaggctctggatctc | ccagcaccattttcttgg | 69 | + | 0 | 69 | chr9 | 141213431 | 93629412 | 93629481 |
| DIABLO | 56616 | aagcagggtgaagtcagtaagg | ccaggacagcttccacaaac | 132 | - | 0 | 132 | chr12 | 133851895 | 122702812 | 122702944 |
| BMF2 | 90427 | ccccatacctgaagaccaag | actccgaggtcaactccatgtcaa | 98 | - | 0 | 98 | chr15 | 102531392 | 40396443 | 40396541 |
| BMF3 | 90427 | aatatgactttggaggaattc | tcaccgttcagggagattaatc | 63 | - | 0 | 63 | chr15 | 102531392 | 40396380 | 40396443 |
| CCNE1 | 898 | atggcaaagcaaccttctga | tcaatgcattctccacacca | 135 | + | 0 | 135 | chr19 | 59128983 | 30312902 | 30313037 |
| CCND1 | 595 | gaggctggcttcatccact | tctccgcagtttcctcaaat | 2974 | + | 0 | 2974 | chr11 | 135006516 | 69462910 | 69465885 |
| H2AFY 6 | 9555 | ataattcccccaccacctccc | ttgccacatacgcctcacatac | 100 | - | 0 | 100 | chr5 | 180915260 | 134688635 | 134688735 |
| H2AFY 6' | 9555 | ataattcccccaccacctccc | ttgccacatacgcctcacatac | 91 | - | 0 | 91 | chr5 | 180915260 | 134686512 | 134686603 |
| STAT3 | 6774 | cctgctcacaccttggttct | caccaaacccatctccactt | 50 | - | 0 | 50 | chr17 | 81195210 | 40468869 | 40468919 |
| GADD45A 2 | 1647 | gaacatggaatcatcaaggaatgcac | agttggagattcatgagaaccttgg | 102 | + | 0 | 102 | chr1 | 249250621 | 68151707 | 68151809 |
| GADD45A 3 | 1647 | ggcatggagaaaactgttacctattttcctc | cactgtgctcttaggtttgccttcttctg | 238 | + | 0 | 238 | chr1 | 249250621 | 68152032 | 68152270 |
| BCL2L11 intron | 10018 | ccggcagaagattgtagagc | ctcccgcttatactgggcta | 180 | + | 0 | 180 | chr2 | 243199373 | 111881446 | 111881626 |
| BCL2L11 4 | 10018 | cagctctcaatgttgaaacagaa | cagccaagagcatctggtaa | 90 | + | 0 | 90 | chr2 | 243199373 | 111881626 | 111881716 |
| BCL2L11 5 | 10018 | ggcctggagtacaatgtcagt | catggtgtctggaccaatgt | 104 | + | 0 | 104 | chr2 | 243199373 | 111907620 | 111907724 |
| BIRC5 2b | 332 | ctcagaatccaagcggagag | aacattgggtggtgtccact | 69 | + | 0 | 69 | chr17 | 81195210 | 76212046 | 76212115 |
| BIRC5 3 | 332 | ctccctgtgctcacagatca | cggacgctcttagacacctc | 118 | + | 0 | 118 | chr17 | 81195210 | 76212744 | 76212862 |
| BIRC5 3b | 332 | cagtaaagcaagcgctgagg | aagagtgagcccagcagaac | 165 | + | 0 | 165 | chr17 | 81195210 | 76218908 | 76219073 |
| SMN1 | 6606 | tgccaatgttatggtagatgga | ctttgcacggacattttcaa | 54 | + | 0 | 54 | chr5 | 180915260 | 70247768 | 70247821 |

(continued)

| Gene symbol | Gene ID | Forward Primer | Reverse Primer | Size | Strand | start | end | Chr name | Chr size | start | end |
|---|---|---|---|---|---|---|---|---|---|---|---|
| **SMN2** | 6607 | gctttacactgcctggcttt | gccaccagtagctgaagacc | 54 | + | 0 | 54 | chr5 | 180915260 | 69372348 | 69372401 |

a
b
c All data about "Chr size" (chromosome size), "Chr size" and start and end points correspond to the human genome release GRCh37/hg19

A library of 270 siRNA pools was used, corresponding to genes encoding core spliceosomal components as well as auxiliary regulatory SFs and factors involved in other RNA-processing steps, including RNA stability, export or polyadenylation. In addition, 40 genes involved in chromatin structure modulation were included, to probe for possible functional links between chromatin and splicing regulation, particularly taking into account that some of the genes identified as FAS AS regulators in the assays described in section 1.1 were genes related to chromatin remodeling and histone modification (SMARCA4, SMARCC2, SMARCD1, SAP18, SETD2). The 270 individual splicing and chromatin remodeling factors knocked down for the present assays are listed above in Table 1.

The siRNA pools were individually transfected in biological triplicates in HeLa cells using a robotized procedure. 72 hours post-transfection RNA was isolated, cDNA libraries generated using oligo-dT and random primers and the patterns of splicing probed by PCR using primers flanking each of the alternatively spliced regions (see summary of the procedure in Fig. 2A). PCR products were resolved by high throughput capillary electrophoresis (HTCE) and the ratio between isoforms calculated as the Percent Spliced In (PSI) index (Katz et al., 2011), which represents the percentage of exon inclusion/alternative splice site usage. A representation of the results obtained for two particular alternative splicing events, the one related to the FAS/CD95 receptor and the one corresponding to CHEK2, when SF3B1 had been knocked down, can be observed in Fig. 2C, where the corresponding data of the percentage of inclusion are also included.

Knockdowns of factors affecting one ASE (Fas/CD95) were also validated with siRNA pools from a second library and by a second independent technique, High Throughput Capillary Electrophoresis. Conversely, the effects of knocking down core SFs (P14, SNRPG, SF3B1) on the ASEs were robust when different siRNAs and RNA purification methods were used.

Several pieces of evidence indicate that the changes in AS are not due to the induction of cell death upon depletion of essential SFs. First, cell viability was not generally compromised after 72h of knockdown of 13 individual core SFs. Second, cells detached from the plate were washed away before RNA isolation. Third, a parallel assays showed that splicing changes upon induction of apoptosis with staurosporine were distinct from those observed upon knockdown of SFs.

Overall the screen generated a total of 33,288 PCR datapoints. These measurements provide highly robust and sensitive estimates of the relative use of competing splice sites. Fig. 2D shows the spread of the effects of knockdown for all the factors in the screen for the ASEs analyzed. Three events (VEGFA, SYK and CCND1) were excluded from further analysis, because they were not affected in the majority of the knockdowns (median absolute ΔPSI<1, Fig. 2D).

From these data we generated, for every knockdown condition, a perturbation profile that reflects its impact across the 35 ASEs in terms of the magnitude and direction of the change. Fig. 2E show examples of such profiles and the relationships between them: In one case, knockdown of CDC5L or PLRG1 generates very similar perturbation profiles (upper diagrams in Figs. 2E and 2F), consistent with their well-known physical interactions within the PRP19 complex (Makarova et al., 2004). In contrast, the profiles associated with the knockdown of SNRPG and DDX52 are to a large extent opposite to each other (lower diagrams in Figs. 2E and 2F), suggesting antagonistic functions. Perturbation profiles and relationships between factors were not significantly changed when knockdown of a subset of factors was carried out for 48 hours, arguing against AS changes being the consequence of secondary effects of SF knockdown in these cases.

1.2.b. *Effects of Core Spliceosome Components on Alternative Splicing Regulation*

The output of the screening process is summarized in the heatmap of Fig. 3A. Three main conclusions can be derived from these results. First, a large fraction of the SF knockdowns have noticeable bur distinct effect on AS, indicating that knockdown of many components of the spliceosome causes switches in splice site selection, rather than a generalized inhibition of splicing of every intron. Second, a substantial fraction of AS changes correspond to higher levels of alternative exon inclusion, again arguing against simple effects of decreasing splicing activity upon knockdown of general SFs, which would typically favor skipping of alternative exons that often harbor weaker splice sites. These observations suggest extensive versatility of the effects of modulating the levels of SFs on splice site selection. Third, despite the diversity of the effects of SF knockdowns on multiple ASEs, similarities can also be drawn. For example, a number of ASEs relevant for the control of programmed cell death (e.g. Fas, APAF1, CASP9 or MCL1) cluster together and are similarly regulated by a common set of core spliceosome components. This observation suggests coordinated regulation of apoptosis by core factors, although the functional effects of these AS changes appear to be complex. Another example are two distinct ASEs in the fibronectin gene (cassette exons EDA and EDB, of paramount importance to control aspects of development and cancer progression, Muro et al., 2007) which cluster close together, suggesting common mechanisms of regulation of the ASEs in this locus.

To explore the effects of different classes of SFs on AS regulation, we first grouped them in four categories (Wahl et al., 2009): genes coding for core spliceosome components, non-core SFs/regulators, RNA-processing factors not directly related to splicing, and chromatin-related factors. Core SFs display the greater spread and average magnitude of effects, followed by non-core SFs (Fig. 3B). Factors related with chromatin structure and remodeling showed a narrower range of milder effects (Fig. 3B), although their values were clearly above the range of changes observed in mock knockdown samples. Of interest, knockdown of core factors leads to stronger exon skipping effects, while non-core SFs, other RNA processing and chromatin-related factors show more balanced effects (Fig. 3B and 3D).

To further dissect the effects of core components, we subdivided them into four categories depending on the timing and duration of their recruitment to splicing complexes during spliceosome assembly following Wahl et al., 2009: a) persistent components (e.g. 17S U2 snRNPs, Sm proteins) that enter the assembly process before or during complex A formation and remain until completion of the reaction, b) transient early components that join the reaction prior to B complex activation, but are scarce at later stages of the process, c) transient middle components joining during B-act complex formation and d) transient late components that are only present during or after C-complex formation. Our results indicate that knockdowns of factors that persist during spliceosome assembly cause AS changes of higher magnitude than those caused by knockdowns of transient factors involved in early spliceosomal complexes, which in turn are stronger and more disperse than those caused by factors involved in middle complexes and in complex C formation or catalytic activation (Fig. 3C). As observed for core factors in general, knockdown of persistent factors tends to favor skipping, while the effects of knockdown of more transient factors are more split between inclusion and skipping (Fig. 3D and Fig. 3E).

Interestingly, knockdowns of components of the Exon Junction Complex (EJC) display a range of effects that resembles those of transient early or mid splicing complexes (Fig. 3C). These results are in line with recent reports documenting effects of EJC components in AS, in addition to their standard function in post-splicing processes (Ashton-Beaucage et al, 2010).

### 1.2.c. Reconstruction of a functional network of splicing factors

To systematically and accurately map the functional relationships of SFs on AS regulation, we quantified the similarity between AS perturbation profiles for every pair of factors in our screen using a robust correlation estimate for the effects of the factors' knockdowns across the ASEs. This measure captures the congruence between the shapes of perturbation profiles, while it does not take into account proportional differences in the magnitude of the fluctuations. Crucially, it discriminates between biological and technical outliers and is resistant to distorting effects of the latter.

We next employed Glasso, a regularization-based algorithm for graphical model selection (Friedman et al, 2007), to reconstruct a network from these correlation estimates (Fig. 4A). Glasso seeks a parsimonious network model for the observed correlations. This is achieved by specifying a regularization parameter, which serves as a penalty that can be tuned in order to control the number of inferred connections (network sparsity) and therefore the false discovery rate (FDR) of the final model. For a given regularization parameter, both the number of inferred connections and the FDR are a decreasing function of the number of ASEs assayed (Fig. 4B). Random sampling with different subsets of the real or reshuffled versions of the data indicates that network reconstruction converges to -500 connections with a FDR<5% near 35 events (Fig. 4B) implying that analysis of additional ASEs in the screening would have only small effects on network sparsity and accuracy.

The complete network of functional interactions among the screened factors is shown in Figure 4A. It is comprised of 196 nodes representing screened factors and 541 connections (average degree 5.5), of which 518 correspond to positive and 23 to negative functional associations. The topology of the resulting network has several key features. First, two classes of factors are easily distinguishable. The first class encompasses factors that form densely connected clusters comprised of core spliceosomal components (Fig. 4A). Factors physically linked within U2 snRNP and factors functionally related with U2 snRNP function, and with the transition from complex A to B, form a tight cluster (group of dark-shaded circles in the upper right part of the inset of Fig. 4A, in red in the original). An adjacent but distinct highly linked cluster corresponds mainly to factors physically associated within U5 snRNP or the U4/5/6 tri-snRNP (group of dark-shaded circles in the bottom left part of the inset of Fig. 4A, shaded in blue in the original). Factors in these clusters and their mutual links encompass 23% of the network nodes but 63% of the total inferred functional associations (average degree 14.5). A second category includes factors that form a periphery of lower connectivity that occasionally projects to the core. This category includes many of the classical splicing regulators (e.g. SR proteins, hnRNPs) as well as several chromatin factors. An intermediate category -more densely interconnected but not reaching the density of interactions of the central modules- includes multiple members of the RNA-dependent DEAD/X box helicase family.

While the number of connections (degree) is significantly higher for core spliceosomal factors in general (Fig. 4C, where the curve corresponding to the spliceosome is that one where the data points are closer to the X-axis than in the other cases), this is especially clear for factors that represent persistent components of splicing complexes along the assembly pathway (Fig. 4D, where the line where the data points are closer to the X-axis is that one of the persistent components, as well as Fig. 4E). Persistent factors are particularly well connected to each other (50% of the possible functional connections are actually detected, Fig. 4E). Factors that belong to consecutive complexes are significantly more linked to each other than factors that belong to early and late complexes, and factors that transiently assemble towards the final stages of spliceosome assembly display the least number of connections (Fig. 4D and Fig. 4E).

Collectively, these results indicate that core SFs, particularly those that persist during assembly, display highly related functions in splice site recognition and fluctuations in their levels or activities have related consequences on the modulation of splice site choice. Of the observed connections, 20% can be attributed to known physical interactions and 82% of these are among core components (Fig. 4A). Examples include tight links between the two subunits of U2AF, between the interacting partners PLRG1 and CDC5L, or IK and SMU1. We estimate that about (50%) of the functional links detected in our network could be predicted by previous knowledge of the composition or function of splicing complexes.

Of relevance, a substantial number of the functional associations closely recapitulate the composition and even -to some extent- the topology of known spliceosomal complexes. These observations suggest that differences in the sensitivity of ASEs to SF perturbations reflect the specific role of these factors in the splicing process. Conversely, these results warrant the use of splicing perturbation profiles as proxies for inferring physical or functional links between factors in the splicing process.

## - Example 2: Applications of the network

### 2.1. Classification of interactions by subsampling of the network

*2.1.a. Generality of the functional interactions in the AS regulation network*

The above network was derived from variations in 35 ASEs selected because of their relevance for cell proliferation and apoptosis, but they represent a heterogeneous collection of AS types and, possibly, regulatory mechanisms. To identify functional connections persistent across AS types, the present authors undertook a subsampling approach. They sampled subsets of 17 from the original 35 ASEs and iteratively reconstructed the network of functional associations from each subset, as described in the section "Data subsampling for identification of indispensable and ancilliary connections". Following 10,000 iterations, the present authors asked how many functional connections were recovered in at least 90% of the sampled networks.

The retrieved set of connections are shown in Fig. 5A and they can be considered to represent a "basal" or indispensable splicing circuitry that captures close similarities in function between SFs regardless of the subset of splicing substrates considered. The majority of these connections correspond to links between core components, with modules corresponding to major spliceosomal complexes. For example, the majority of components of U2 snRNP constitute the most prominent of the core modules (Fig. 5A). Strikingly, the module is topologically subdivided in two sub-modules, one corresponding to components of the SF3a and SF3b complexes and the second corresponding to proteins in the Sm complex. The connectivity between U2 snRNP components remarkably recapitulates known topological features of U2 snRNP organization, including the assembly of SF3a/b complexes in stem loops I and II and the assembly of the Sm ring in a different region of the U2 snRNA (Behrens et al., 1993).

That the connectivity between components derived from assessing the effects on AS of depletion of these factors correlates with topological features of the organization of the snRNP further argues that the functions in splice site selection are tightly linked to the structure and function of this particle, highlighting the potential resolution of our approach for identifying meaningful structural and mechanistic links.

A second prominent "core" module (that corresponding to the group of nodes located on the right side of Fig. 5A) corresponds to factors known to play roles in conformational changes previous to / concomitant with catalysis in spliceosomes from yeast to human, including PRP8, PRP31 or U5-200. Once again some of the topological features of the module are compatible with physical associations between these factors (e.g. PRP8 with PRP31 or U5-116K or PRP31 with C20ORF14). That knockdown of these late-acting factors, which coordinate the final steps of the splicing process (Bottner et al., 2005; Hackner et al., 2008; Wahl et al., 2009), causes coherent changes in splice site selection strongly argues that the complex process of spliceosome assembly can be modulated at late steps, or even at the time of catalysis, to affect splice site choice. Other links in the "core" network recapitulate physical interactions, including the aforementioned modules involving the two subunits of the 3' splice site-recognizing factor U2AF, the interacting partners PLRG1 and CDC5L and IK and SMU1.

To further evaluate the generality of the present approach, the authors tested whether the functional concordance derived from the network analysis and from the iterative selection of a "core" network could be recapitulated in a different cell context and genome-wide. They focused their attention in the strong functional association between IK and SMU1, two factors that have been described as associated with complex B and its transition to Bact (Bessonov et al., 2010) (Fig. 5A). Analysis of the effects of knockdown of these factors in the same sets of ASEs in HEK293 cells revealed a similar set of associations, despite the fact that individual events display different splicing ratios upon IK / SMU1 knockdown in this cell line compared to HeLa. To explore the validity of this functional association in a larger set of splicing events, RNA was isolated in biological triplicates from HeLa cells transfected with siRNAs against these factors and changes in AS were assessed using genome-wide splicing-sensitive microarrays (Affymetrix). The results revealed a striking overlap between the effects of IK and SMU1 knockdown, both on gene expression changes and on AS changes (61% overlap, p-value < 1E-20) covering a wide range of gene expression levels, fold differences in isoform ratios and AS types. The overlap was much more limited with RBM6, a splicing regulator (Bechara et al., 2013) located elsewhere in the network. These results validate the strong functional similarities between IK and SMU1 detected in the analysis. The molecular basis for this link could be explained by the depletion of each protein (but not their mRNA) upon knockdown of the other factor, consistent with formation of a heterodimer of the two proteins and destabilization of one partner after depletion of the other. Notably, the best correlation between the effects of these factors was between IK knockdown for 48h and SMU1 knockdown for 72h, perhaps reflecting a stronger functional effect of IK on AS regulation.

Gene ontology analysis revealed a clear common enrichment of AS changes in genes involved in cell death and survival, suggesting that IK/SMU1 could play a role in the control of programmed cell death through AS. To evaluate this possibility, the present authors tested the effects of knockdown of these factors, individually or combined, on cell proliferation and apoptotic assays. The results indicated that their depletion activated cleavage of PARP, substantially reduced cell growth and increased the fraction of cells undergoing apoptosis. The present authors conclude that functional links revealed by the network can be used to infer common mechanisms of splicing regulation and provide insights into the biological framework of the regulatory circuits involved.

*2.1.b. Ancillary functional network interactions reveal alternative mechanisms of AS regulation*

The iterative generation of networks from subsets of ASEs explained above could, in addition to identifying general functional interactions, be used to capture functional links that are specific to particular classes of events characterized by distinct regulatory mechanisms. To explore this possibility, the present authors sought the set of functional connections that are robustly recovered in a significant fraction of ASEs subsets but are absent in others, as explained above in the section "Data subsampling for identification of indispensable and ancillary connections".

Fig. 6A shows the set of such identified interactions, which therefore represents specific functional links that only emerge when particular subsets of AS events are considered. The inset represents graphically the results of Principal Component Analysis (PCA) showing the variability in the strength of these interactions depending on the presence or absence of different ASEs across the subsamples. The most discriminatory set of ASEs for separating these interactions is also shown. While this analysis may be constrained by the limited number of events analyzed and higher FDR due to multiple testing, it can provide a valuable tool for discovering regulatory mechanisms underlying specific classes of ASEs as illustrated by the example below.

A synergistic link was identified between hnRNP C and U2AF1 (the 35 Kda subunit of U2AF, which recognizes the AG dinucleotide at 3' splice sites) for a subset of ASEs (see link between U2AF1 and HNRPC in the central-right part of Fig. 6A). The present authors explored a possible relationship between the effects of knocking down these factors and the presence of consensus hnRNP C binding sites (characterized by stretches of uridine residues) or 3' splice site-like motifs in the vicinity of the regulated exons. Details about the used technique have been mentioned above in the section "Splice site scoring, identification of probable HNRPC binding sites and alu elements".

The results revealed a significant correlation (0.795) with the presence of composite elements comprised of putative hnRNP C binding sites within sequences conforming to a 3' splice site motif upstream and/or downstream of the regulated exons (Fig. 6B). No such correlation was observed for ASEs lacking these sequence features (Fig. 6C).

These observations are in line with results from a recent report revealing that hnRNP C prevents exonization of Alu elements by competing the binding of U2AF to Alu sequences (Zarnack et al., 2013). The present results extend these findings by capturing functional relationships between hnRNP C and U2AF in ASEs that harbor binding sites for these factors in the vicinity of the regulated splice sites, both inside of Alu elements or independent of them (Fig. 6B and D). Our data are compatible with a model in which uridine-rich and 3' splice site-like sequences sequester U2AF away from the regulated splice sites, causing alternative exon skipping, while hnRNP C displaces U2AF away from these decoy sites, facilitating exon inclusion. In this model, depletion of U2AF and hnRNP C are expected to display the same effects in these ASEs, as observed in our data (Figures 6B and D). In contrast, in ASEs in which hnRNP C binding sites are located within the polypyrimidine tract of the 3' splice site of the regulated exon, the two factors display antagonistic effects (Fig. 6B and Fig. 6D), as expected from direct competition between them at a functional splice site.

This example illustrates the potential of the network approach to identify molecular mechanisms of regulation on the basis of specific sequence features and the interplay between cognate factors.

## 2.2. Mapping targets of physiological or external perturbations within the Spliceosome. Identification of drug targets

Another application of the present network analysis is to identify possible targets within the splicing machinery of physiological or external (e.g. pharmacological) perturbations that produce changes in AS that can be measured using the same experimental setting. The similarity between profiles of AS changes induced by such perturbations and the knockdown of particular factors can help to uncover SFs that mediate their effects.

### 2.2.1. Mapping drug targets within the Spliceosome

To evaluate the potential of this approach, HeLa cells were treated with drugs known to affect AS decisions and the patterns of changes in the 35 ASEs induced by these treatments were assessed by the same robotized procedure for RNA isolation and RT-PCR analysis and used to locate their position within the network.

The results indicate that structurally similar drugs like Spliceostatin A (SSA) and Meayamycin cause AS changes that closely resemble the effects of knocking down components of U2 snRNP (Fig. 7A), including close links between these drugs and SF3B1, a known physical target of SSA (Kaida et al, 2007; Hasegawa et al, 2011) previously implicated in mediating the effects of the drug through alterations in the AS of cell cycle genes (Corrionero et al, 2011).

Of interest, changes in AS of MCL1 (leading to the production of the pro-apoptotic mRNA isoform) appear as prominent effects of both drugs (Fig. 7B), confirming and extending recent observations obtained using Meayamycin (Gao et al., 2013) and suggesting that this ASE can play a key general role in mediating the anti-proliferative effects of drugs that target core splicing components like SF3B1 (Bonnal et al., 2012).

A strong link is also captured between each of the drugs and PPIH (Fig. 7A), a peptidyl-prolyl cis-trans isomerase which has been found associated with U4/5/6 tri-snRNP (Horowitz et al., 1997; Teigelkamp et al., 1998), but had not been implicated directly in 3' splice site regulation. Indeed, the network indicates a close relationship between PPIH and multiple U2 snRNP components, particularly in the SF3a and SF3b complexes, further arguing that PPIH plays a role in 3' splice site definition closely linked to branch point recognition by U2 snRNP.

Treatment of the cells with the Clk (Cdc2-like) kinase family inhibitor TG003, which is also known to modulate AS (Muraki et al., 2004) and causes changes in ASEs analyzed in our network (Fig. 7B), led to links with a completely different set of SFs (Fig. 7A), attesting to the specificity of the results.

These data confirm the potential of the network analysis to identify *bona fide* SF targets of physiological or pharmacological perturbations and further our understanding of the underlying molecular mechanisms of regulation.

## 2.3. Mapping targets of physiological or external perturbations within the Spliceosome. Functional connections between the spliceosome and metabolic routes

### 2.3.a. Regulation of Fas/CD95 AS by iron homeostasis-related genes

Following with the analysis of the data obtained in the assays described in section 1.1. above, and to gain further insights into novel connections between the spliceosome and other cellular pathways, the present authors focused their attention on ACO1 and FTL, two genes with antagonistic roles in the control of iron homeostasis whose knockdown had opposite effects on Fas/CD95 AS. ACO1 encodes a moonlighting protein that functions, under conditions of iron-abundance, as an enzyme that converts citrate to isocitrate and, upon iron-depletion, displays an RNA binding activity that modulates the stability / translation of target mRNAs encoding genes (like FTL) that control iron storage or uptake. Under conditions of iron depletion, ACO1 represses FTL (ferritin L chain) translation, leading to the release of iron from ferritin storage sites.

Knockdown experiments were carried out in HeLa (firstly) or HepG2 (secondly) cell lines transfected with siRNA pools against the following genes: ACO1, FTL, TIA1, SRSF1, SRSF5, SLU7, U2AF35, the pools corresponding to siGENOME and ON TARGET PLUS, purchased from Thermo Scientific, using Lipofectamine RNAiMAX according to the manufacturer's recommendations. Under such conditions, the levels of ACO1 and FTL proteins themselves, the intracellular iron levels (phen-green SK fluorescence intensity) and the effect on Fas/CD95 exon 6 alternative splicing were measured. The results are represented in Fig. 8, together with the results obtained after the treatment with other compounds related with iron overload or depletion, as described below.

The results of the screen in HeLa cells indicated that:

   a) ACO1 depletion with said two different siRNA pools:

      a.1. favors iron depletion (see Fig. 8B)
      a.2. promotes Fas/CD95 exon 6 skipping (see Table 2B, as well as the results of the capillary electrophoresis and real-time PCR depicted in Fig. 8C and Fig. 8D respectively)

   b) FTL depletion:

      b.1. favors iron release (see Fig. 8B)
      b.2. promotes Fas/CD95 exon 6 inclusion (see Table 2B, as well as the results of the capillary electrophoresis and real-time PCR depicted in Fig. 8C and Fig. 8D respectively)

Similar effects were observed using the hepatocellular carcinoma HepG2 cell line.

Next, the effects of pharmacological treatments that lead to intracellular iron overload (Hemin) or iron depletion (Desferal, Ciclopirox) were explored. HeLa cells were treated with 100 $\mu$M Hemin or 100 $\mu$M Desferal or cyclopirox olamine as previously reported (Mückenthaler, 2003). Hemin treatment led to higher ferritin mRNA and protein levels and lower transferrin mRNA levels, and an overall increase of intracellular iron (measured as a decrease in Phen-green SK fluorescence) in different cell lines (see Fig. 8B). Conversely, treatment with Desferal had opposite effects (Fig. 8E). Consistent with the effects of ACO1/FTL depletion, iron excess induced by Hemin led to increased Fas/CD95 exon 6 inclusion, while iron depletion induced by Desferal or Ciclopirox increased exon 6 skipping, both in endogenous transcripts and in the context of transcripts expressed from a minigene reporter plasmid (Fig. 8E).

Taken together these observations reveal a link between intracellular iron availability and differential regulation of Fas/CD95 alternatively spliced isoforms. Effects of the screen hits on differential stability of the mRNA isoforms cannot be excluded, similar half-lives for exon 6 inclusion and skipping transcripts were observed upon inhibition of transcription by Actinomycin D, including conditions of iron depletion or overload. The present authors did observe, however, differential stability of the isoforms upon ACO1 knockdown, indicating that RNA decay may contribute to the effects of some of the regulators identified in the present screen.

To evaluate the functional consequences of iron-mediated regulation of Fas/CD95 splicing, anti-Fas antibodies were used to induce Fas-mediated apoptosis in Peripheral Blood Mononuclear Cells (PBMCs) exposed to Hemin or Desferal. The experiment was carried out in control cells or in cells induced to proliferate by treatment with phyto-haemagglutinin (PHA), which is known to promote a switch towards higher levels of Fas/CD95 exon 6 inclusion and apoptosis, a situation that mimics the physiological switch that eliminates expanded populations of T lymphocytes upon antigen clearance. Failure to undergo this switch leads to Autoimmune Lymphoproliferative Syndrome (ALPS) (Roesler et al, 2005), a non-tumor condition characterized by persistent high levels of T cells. The results recapitulate in human T lymphocytes the changes in Fas exon 6 splicing upon iron overload and depletion observed in cell lines. Consistent with the predicted effects of the splicing changes, iron depletion (which promotes the soluble Fas isoform) protects T lymphocytes from Fas-mediated apoptosis, while iron excess tends to enhance it.

While it was technically difficult to evaluate Fas/CD95 protein isoform levels in these cells, the results suggest that the splice site switches induced in Fas/CD95 by regulation of iron levels are biologically relevant.

2.3.b. *Functional connections between the spliceosome and iron metabolism*

To further explore the mechanisms by which iron levels can influence AS, the present authors took advantage of the functional splicing network of the present invention, as generated as described in Example 1.

The effects of Hemin and Desferal treatments as well as the effects of depletion of ACO1 and FTL on 35 AS events were compared to those of splicing factors knockdowns. The network captured antagonistic effects between Hemin and Desferal, as well as similarities between the effects of Hemin and FTL depletion (Fig. 9A), indicating that iron levels regulate multiple AS events. A very significant correlation (0.67) was found between the effects of Hemin treatment and the knockdown of the splicing factor SRSF7 (a classical SR protein family member) (Fig. 9A and 9B), indicating that iron overload induces changes in AS that closely resemble those induced by a reduction in SRSF7 activity. In agreement with this, overexpression of SRSF7 led to increased levels of Fas/CD95 exon 6 skipping, the opposite effect of Hemin treatment (Fig. 9C). The similarities between SRSF7 depletion and Hemin treatment were particularly noticeable for AS events in the genes H2AFY, GADD45A and the apoptosis regulator DIABLO (Fig. 9B). Indeed, reciprocal effects were observed for Hemin/SRSF7 knockdown and Desferal/Ciclopirox treatments on DIABLO exon 4 inclusion levels (Fig. 9D), confirming that iron homeostasis has a variety of effects on AS regulation.

To investigate how iron-mediated AS modulation connects to SFRS7, the authors first considered the possibility that iron overload could decrease SRSF7 levels in the cell or modify its intracellular distribution. No significant effects were observed upon Hemin treatment, however, in the levels of SRSF7 mRNA, SRSF7 protein or SRSF7 subcellular distribution.

Next they considered whether iron levels could affect the activity of SRSF7. SRSF7 is a classical member of the SR protein family of splicing regulators and the only one containing a Zinc-knuckle motif, which is known to be important for SRSF7 binding specificity. To address potential effects of iron on SRSF7 binding, crosslinking immunoprecipitation experiments (CLIP) were carried out with a tagged version of SRSF7 in control or Hemin-treated HeLa cells. HeLa cells transfected with epitope-tagged SRSF7 were UV light irradiated, lysed and, after RNA isolation, the association of SRSF7 with different regions of Fas/CD95 alternatively spliced region was tested by qPCR using consecutive 80 nucleotide amplicons covering the alternatively spliced genomic sequences of Fas/CD95 from exon 5 to exon 7, both in control (CN) cells or cells treated with 100 $\mu$M Hemin for 24 hours. As can be seen in Fig. 10A, two main peaks of SRSF7 binding were detected in intron 6. The peaks overlap with sequences predicted to contain high affinity binding sites for SRSF7. Of relevance, binding of SRSF7 to these peaks was decreased under conditions of iron overload.

To further analyze the extent and specificity of these effects, the CLIP signals of endogenous SRSF7 -or SRSF1 as a control- in three predicted and/or observed SRSF7 binding sites in the Fas/CD95 alternatively spliced region were quantified by real time qPCR. The results showed a consistent decrease in SRSF7 binding to these regions upon conditions of iron overload, while SRSF1 (another SR protein lacking a Zn-knuckle) binding was only marginally decreased or even increased (perhaps due to the loss of competitor SRSF7 binding) upon iron excess (see Figs. 10B, 10C, 10D). An apparent discrepancy between the effects of Hemin treatment on the binding of SRSF7 in Figs. 10A and 10B can be observed, which may be due to overexpression of SRSF7 in the experiments of Fig. 10A.

Consistent with the concept that these binding events are relevant for SRSF7-mediated repression, deletion of a region containing the most prominent SRSF7 binding peak in intron 6 led to increased exon inclusion (Figs. 10E, 10F 10G). In addition, deletion of this SRSF7 binding site reduced (but did not abolish) the effect of SRSF7 overexpression, suggesting that this and other SRSF7 binding sites contribute to regulation. SRSF7-mediated repression is significantly more compromised in the mutant under conditions of iron overload (Fig. 10G), suggesting synergistic effects between the loss of SRSF7 binding sites and reduced SRSF7 binding affinity. Collectively, the results are consistent with a model where iron levels modulate the RNA binding properties of this Zinc-knuckle-containing protein and this, in turn, regulates Fas/CD95 AS.

To test whether iron can have a direct effect on RNA recognition by SRSF7, recombinant SRSF7 protein expressed in and purified from E. coli was partially denatured and refolded in the presence of $Fe^{2+}$ or $Zn^{2+}$ and binding to previously described SRSF7 SELEX RNA motifs tested in filter binding assays. Refolding of SRSF7 in the presence of $Zn^{2+}$ enhanced RNA binding, while refolding in the presence of $Fe^{2+}$ decreased binding to this RNA. These differences were negligible when a SRSF7 variant lacking the Zinc-knuckle domain or when the binding of another SR protein, SRSF1 (Figure 6E), were tested. The differences were also negligible when binding to another RNA motif recognized by SRSF7 RRM domain was tested, indicating that the differential effects of $Zn^{2+}$ and $Fe^{2+}$ depend on the RNA binding activity of the Zinc-knuckle domain. No significant decrease in RNA interactions induced by $Fe^{2+}$ was observed when binding of U2AF65 RRMs 1 + 2 to a pyrimidine-rich target site was tested, again arguing that the effects of $Fe^{2+}$ on RNA binding are specific of Zn-knuckle containing motifs.

The present authors concluded that iron levels can modulate the RNA binding activity of SRSF7 through its Zn-knuckle domain and this impinges on SFRS7-mediated AS regulation, as it is summarized in Fig. 10H.

All these analyses shows the potentiality of the method of the present invention for identifying regulatory targets within the splicing machinery and mechanisms of regulation underlying physiological or pharmacological alterations in the splicing process, such as the adaptation to iron level changes or the conditions that lead to the onset of certain cell multiplication disorders and, even, their development into malignancies, or under the presence of certain compounds that act on those routes, either as modulators or, as happens with drugs, giving rise to the amelioration of certain pathological conditions. The assays of the present application also demonstrate that the method can be useful for identifying features common to certain factors, which could be a starting point for finding ways or compounds for regulating them or better understanding their mechanisms of action or their involvement in certain pathways. Such information might be not only very valuable for scientific knowledge by itself, but it also may be useful for the identification of factors involved in certain disorders and diseases and the selection of targets for finding candidates to drugs for controling or ameliorating such disorders and diseases. The method of the invention can be used, in turn, in the process of identification or assessment of the candidates to drugs and, thus, being a very valuable tool both for basic research and for finding and developing industrial applications to research such as identification and development of new drugs targeting factors involved in the regulation of alternative splicing or, particularly, in the regulation of certain splicing events that are associated to certain diseases or malignancies.

## References

Abelson, J., Blanco, M., Ditzler, M.A., Fuller, F., Aravamudhan, P., Wood, M., Villa, T., Ryan, D.E., Pleiss, J.A., Maeder, C., et al.. (2010). Conformational dynamics of single pre-mRNA molecules during in vitro splicing. Nat. Struct. Mol. Biol. 17(4):504-12.

Albulescu, L.O., Sabet, N., Gudipati, M., Stepankiw, N., Bergman, Z.J., Huffaker, T.C., Pleiss, J.A. (2012). A quantitative, high-throughput reverse genetic screen reveals novel connections between Pre-mRNA splicing and 5' and 3' end transcript determinants. PLoS Genet. 8(3):e1002530

Ashton-Beaucage, D., Udell, C.M., Lavoie, H., Baril, C., Lefrançois, M., Chagnon, P., Gendron, P., Caron-Lizotte, O., Bonneil, E., Thibault, P., et al.. (2010). The exon junction complex controls the splicing of MAPK and other long intron-containing transcripts in Drosophila. Cell. 143(2):251-62.

Barash, Y., Calarco, J.A., Gao, W., Pan, Q., Wang, X., Shai, O., Blencowe, B.J., Frey, B.J. (2010). Deciphering the splicing code. Nature. 465(7294):53-9.

Barbosa-Morais, N.L., Irimia, M., Pan, Q., Xiong, H.Y., Gueroussov, S., Lee, L.J., Slobodeniuc, V., Kutter, C., Watt, S., Colak, R., Kim, T., et al. (2012). The evolutionary landscape of alternative splicing in vertebrate species. Science. 338(6114):1587-93

Bechara, E.G., Sebestyén, E., Bernardis, I., Eyras, E., Valcárcel, J. (2013). RBM5, 6, and 10 differentially regulate NUMB alternative splicing to control cancer cell proliferation. Mol. Cell. 52(5):720-33.

Behrens, S.E., Tyc, K., Kastner, B., Reichelt, J., Lührmann, R. (1993). Small nuclear ribonucleoprotein (RNP) U2 contains numerous additional proteins and has a bipartite RNP structure under splicing conditions. Mol. Cell. Biol. 13(1):307-19.

Berriz GF, King OD, Bryant B, Sander C, Roth FP. (2003) Characterizing gene sets with FuncAssociate. Bioinformatics. 12;19(18):2502-4

Bessonov, S., Anokhina, M., Will, C.L., Urlaub, H., Lührmann, R. (2008). Isolation of an active step I spliceosome and composition of its RNP core. Nature. 452(7189):846-50.

Birmingham, A., Selfors, L.M., Forster, T., Wrobel, D., Kennedy, C.J., Shanks, E., Santoyo-Lopez, J., Dunican, D.J., Long, A., Kelleher, D. et al. (2010). Statistical methods for analysis of high-throughput RNA interference screens. Nat. Methods. 6(8):569-75.

Bonnal, S., Martinez, C., Förch, P., Bachi, A., Wilm, M., Valcárcel, J. (2008). RBM5/Luca-15/H37 regulates Fas alternative splice site pairing after exon definition. Mol. Cell. 32(1):81-95.

Bonnal, S., Vigevani, L., Valcárcel, J. (2012). The spliceosome as a target of novel antitumour drugs. Nat. Rev. Drug. Discov. 11(11):847-59.

Bottner, C.A., Schmidt, H., Vogel, S., Michele, M., Käufer, N.F. (2005). Multiple genetic and biochemical interactions of Brr2, Prp8, Prp31, Prp1 and Prp4 kinase suggest a function in the control of the activation of spliceosomes in Schizosaccharomyces pombe. Curr. Genet. 48(3):151-61.

Cascino, I., Fiucci, G., Papoff, G., Ruberti, G. (1995). Three functional soluble forms of the human apoptosis-inducing Fas molecule are produced by alternative splicing. J. Immunol. 15;154(6):2706-13

Chatr-Aryamontri, A., Breitkreutz, B.J., Heinicke, S., Boucher, L., Winter, A., Stark, C., Nixon, J., Ramage, L., Kolas, N., O'Donnell, L., et al. (2013). The BioGRID interaction database: 2013 update. Nucleic Acids Res. 41:D816-23

Chen, W., Moore, M.J. (2014). The spliceosome: disorder and dynamics defined. Curr. Opin. Struct. Biol. 24C:141-149.

Clark, T.A., Sugnet, C.W., Ares, M. Jr. (2002). Genomewide analysis of mRNA processing in yeast using splicing-specific microarrays. Science. 296(5569):907-10.

Clauset A, Newman ME, Moore C. (2004). Finding community structure in very large networks. Phys Rev E Stat Nonlin Soft Matter Phys. 70(6 Pt 2):066111.

Collins, C.A., Guthrie, C. (1999). Allele-specific genetic interactions between Prp8 and RNA active site residues suggest a function for Prp8 at the catalytic core of the spliceosome. Genes Dev. 13(15):1970-82.

Cooper, T.A., Wan, L., Dreyfuss, G. (2009). RNA and disease. Cell. 136(4):777-93

Corrionero, A., Miñana, B., Valcárcel, J. (2011). Reduced fidelity of branch point recognition and alternative splicing induced by the anti-tumor drug spliceostatin A. Genes Dev. 25(5):445-59.

Csardi, G., Nepusz, T. (2006). The igraph software package for complex network research. InterJournal. Complex Systems 1695.

Fica, S.M., Tuttle, N., Novak, T., Li, N.S., Lu, J., Koodathingal, P., Dai, Q., Staley, J.P., Piccirilli, J.A. (2013). RNA catalyses nuclear pre-mRNA splicing. (2013). Nature 503(7475):229-34.

Franceschini, A., Szklarczyk, D., Frankild, S., Kuhn, M., Simonovic, M., Roth, A., Lin, J., Minguez, P., Bork, P., von Mering, C. et al. (2013). STRING v9.1: protein-protein interaction networks, with increased coverage and integration. Nucleic Acids Res. 41:D808-15.

Friedman, J., Hastie, T., Tibshirani, R. (2008). Sparse inverse covariance estimation with the graphical lasso. Biostatistics. 9(3):432-41.

Fu, X.D., Ares, M. (2014). Context-dependent control of alternative splicing by RNA-binding proteins. Nat. Rev. Genet. 15: 689-701.

Gao, Y., Vogt, A., Forsyth, C.J., Koide, K.. (2013). Comparison of splicing factor 3b inhibitors in human cells. Chembiochem. 14(1):49-52.

Gerstein MB, Kundaje A, Hariharan M, Landt SG, Yan KK, Cheng C, Mu XJ, Khurana E, Rozowsky J, Alexander R, Min R, et al.(2012). Architecture of the human regulatory network derived from ENCODE data. Nature. 489(7414):91-100

Hacker, I., Sander, B., Golas, M.M., Wolf, E., Karagöz, E., Kastner, B., Stark, H., Fabrizio, P., Lührmann, R. (2008). Localization of Prp8, Brr2, Snu114 and U4/U6 proteins in the yeast tri-snRNP by electron microscopy. Nat. Struct. Mol. Biol. 15(11):1206-12.

Hamady M, Walker JJ, Harris JK, Gold NJ, Knight R. (2008) Error-correcting barcoded primers for pyrosequencing hundreds of samples in multiplex. Nat Methods. 5(3):235-7

Hasegawa, M., Miura, T., Kuzuya, K., Inoue, A., Won Ki, S., Horinouchi, S., Yoshida, T., Kunoh, T., Koseki, K., Mino, K. et al. (2011). Identification of SAP155 as the target of GEX1A (Herboxidiene), an antitumor natural product. ACS Chem. Biol. 6(3):229-33.

Hastie T, Tibshirani R, Sherlock G, Eisen M, Brown P, Botstei D. (1999). Imputing Missing Data for Gene Expression Arrays. Technical report Stanford Statistics Department.

Horowitz, D.S., Kobayashi, R., Krainer, A.R. (1997). A new cyclophilin and the human homologues of yeast Prp3 and Prp4 form a complex associated with U4/U6 snRNPs. RNA. 3(12):1374-87.

Hoskins, A.A., Friedman, L.J., Gallagher, S.S., Crawford, D.J., Anderson, E.G., Wombacher, R., Ramirez, N., Cornish, V.W., Gelles, J., Moore, M.J. (2011). Ordered and dynamic assembly of single spliceosomes. Science. 331(6022):1289-95.

Hoskins, A.A., Moore, M.J. (2012). The spliceosome: a flexible, reversible macromolecular machine. Trends Biochem. Sci. 37(5):179-88. House, A.E., Lynch, K.W. (2006). An exonic splicing silencer represses spliceosome assembly after ATP-dependent exon recognition. Nat. Struct. Mol. Biol. 13(10):937-44.

Jurka, J., Kapitonov, V.V., Pavlicek, A., Klonowski, P., Kohany, O., Walichiewicz, J. (2005). Repbase Update, a database of eukaryotic repetitive elements. Cytogenet Genome Res. 110(1-4):462-7.

Kaida, D., Motoyoshi, H., Tashiro, E., Nojima, T., Hagiwara, M., Ishigami, K., Watanabe, H., Kitahara, T., Yoshida, T., Nakajima, H., et al. (2007). Spliceostatin A targets SF3b and inhibits both splicing and nuclear retention of pre-mRNA. Nat. Chem. Biol. 3(9):576-83.

Karolchik, D., Barber, G.P., Casper, J., Clawson, H., Cline, M.S., Diekhans, M., Dreszer, T.R., Fujita, P.A., Guruvadoo, L., Haeussler, M., et al. (2014). The UCSC Genome Browser database: 2014 update. Nucleic Acids Res. 42(Database issue):D764-70.

Katz, Y., Wang, E.T., Airoldi, E.M., Burge, C.B. (2010). Analysis and design of RNA sequencing experiments for identifying isoform regulation. Nat. Methods. 7(12):1009-15.

Kim, D., Kim, M.S., Cho, K.H. (2012). The core regulation module of stress-responsive regulatory networks in yeast. Nucleic Acids Res. 40(18):8793-802. Korneta, I., Bujnicki, J.M. (2012). Intrinsic disorder in the human spliceosomal proteome. PLoS Comput. Biol. 8(8):e1002641.

Konig J, Zarnack K, Rot G, Curk T, Kayikci M, Zupan B, Turner DJ, Luscombe NM, Ule J. (2011) iCLIP--transcriptome-wide mapping of protein-RNA interactions with individual nucleotide resolution. J Vis Exp. 30;(50)

Lallena, M.J., Chalmers, K.J., Llamazares, S., Lamond, A.I., Valcárcel, J. (2002). Splicing regulation at the second catalytic step by Sex-lethal involves 3' splice site recognition by SPF45. Cell. 109(3):285-96.

Liu, C., Cheng, J., Mountz, J.D. (1995). Differential expression of human Fas mRNA species upon peripheral blood mononuclear cell activation. Biochem. J. 310 (Pt 3):957-63

Luco, R.F., Allo, M., Schor, I.E., Kornblihtt, A.R., Misteli, T. (2011). Epigenetics in alternative pre-mRNA splicing. Cell. 144(1):16-26.

Makarova, O.V., Makarov, E.M., Urlaub, H., Will, C.L., Gentzel, M., Wilm, M., Lührmann, R. (2004). A subset of human 35S U5 proteins, including Prp19, function prior to catalytic step 1 of splicing. EMBO J. 23(12):2381-91.

Merkin, J., Russell, C., Chen, P., Burge, C.B. (2012). Evolutionary dynamics of gene and isoform regulation in Mammalian tissues. Science. 338(6114):1593-9.

Moore, M.J., Wang, Q., Kennedy, C.J., Silver, P.A. (2010). An alternative splicing network links cell-cycle control to apoptosis. Cell. 142(4):625-36.

Mozaffari-Jovin, S., Santos, K.F., Hsiao, H.H., Will, C.L., Urlaub, H., Wahl, M.C., Lührmann, R. (2012). The Prp8 RNase H-like domain inhibits Brr2-mediated U4/U6 snRNA unwinding by blocking Brr2 loading onto the U4 snRNA. Genes Dev. 26(21):2422-34.

Muckenthaler, M., Richter, A., Gunkel, N., Riedel, D., Polycarpou-Schwarz, M., Hentze, S., Falkenhahn, M., Stremmel, W., Ansorge, W., Hentze, M.W. (2003). Relationships and distinctions in iron-regulatory networks responding to interrelated signals. Blood. 101(9):3690-8

Muraki, M., Ohkawara, B., Hosoya, T., Onogi, H., Koizumi, J., Koizumi, T., Sumi, K., Yomoda, J., Murray, M.V., Kimura, H., et al. (2004). Manipulation of alternative splicing by a newly developed inhibitor of Clks. J. Biol. Chem. 279(23):24246-54.

Muro, A.F., Chauhan, A.K., Gajovic, S., Iaconcig, A., Porro, F., Stanta, G., Baralle, F.E. (2003). Regulated splicing of the fibronectin EDA exon is essential for proper skin wound healing and normal lifespan. J. Cell. Biol. 162(1):149-60.Newman, M.E. (2006). Modularity and community structure in networks. Proc. Natl. Acad. Sci. U.S.A. 103(23):8577-82.

Newman, M.E. (2006). Modularity and community structure in networks. Proc. Natl. Acad. Sci. U.S.A. 103(23):8577-82

Nilsen, T.W., Graveley, B.R. (2010). Expansion of the eukaryotic proteome by alternative splicing. Nature. 463: 457-463.Park, J.W., Parisky, K., Celotto, A.M., Reenan, R.A., Graveley, B.R. (2004). Identification of alternative splicing regulators by RNA interference in Drosophila. Proc. Natl. Acad. Sci. U.S.A. 101(45):15974-9.

Papoff, G., Cascino, I., Eramo, A., Starace, G., Lynch, D.H., Ruberti, G. (1996). An N-terminal domain shared by Fas/Apo-1 (CD95) soluble variants prevents cell death in vitro. J. Immunol. 156(12):4622-30

Pena, V., Liu, S., Bujnicki, J.M., Lührmann, R., Wahl, M.C. (2007). Structure of a multipartite protein-protein interaction domain in splicing factor prp8 and its link to retinitis pigmentosa. Mol. Cell. 25(4):615-24.

Pleiss, J.A., Whitworth, G.B., Bergkessel, M., Guthrie, C. (2007). Transcript specificity in yeast pre-mRNA splicing revealed by mutations in core spliceosomal components. PLoS Biol. 5(4):e90.

Quesada, V., Conde, L., Villamor, N., Ordóñez, G.R., Jares, P., Bassaganyas, L., Ramsay, A.J., Beà, S., Pinyol, M., Martinez-Trillos, A., et al. (2011). Exome sequencing identifies recurrent mutations of the splicing factor SF3B1 gene in chronic lymphocytic leukemia. Nat. Genet. 44(1):47-52.

Roesler, J., Izquierdo, J.M., Ryser, M., Rosen-Wolff, A., Gahr, M., Valcarcel, J., Lenardo, M.J., Zheng, L. (2005). Haploinsufficiency, rather than the effect of an excessive production of soluble CD95 (CD95{Delta}TM), is the basis for ALPS la in a family with duplicated 3' splice site AG in CD95 intron 5 on one allele. Blood. 106(5):1652-9

Saltzman, A.L., Pan, Q., Blencowe, B.J. (2011). Regulation of alternative splicing by the core spliceosomal machinery. Genes Dev. 25(4):373-84.

Shcherbakova, I., Hoskins, A.A., Friedman, L.J., Serebrov, V., Corrêa, I.R. Jr., Xu, M.Q., Gelles, J., Moore, M.J. (2013). Alternative spliceosome assembly pathways revealed by single-molecule fluorescence microscopy. Cell Rep. 5(1):151-65.

Teigelkamp, S., Achsel, T., Mundt, C., Gothel, S.F., Cronshagen, U., Lane, W.S., Marahiel, M., Lührmann, R. (1998). The 20kD protein of human [U4/U6.U5] tri-snRNPs is a novel cyclophilin that forms a complex with the U4/U6-specific 60kD and 90kD proteins. RNA. 4(2):127-41.

Tseng, C.K., Cheng, S.C. (2008). Both catalytic steps of nuclear pre-mRNA splicing are reversible. Science. 320(5884):1782-4.

Wahl, M.C., Will, C.L., Lührmann R. (2009). The spliceosome: design principles of a dynamic RNP machine. Cell. 136(4):701-18.

Warzecha, C.C., Sato, T.K., Nabet, B., Hogenesch, J.B., Carstens, R.P. (2009). ESRP1 and ESRP2 are epithelial cell-type-specific regulators of FGFR2 splicing. Mol. Cell. 33(5):591-601

Watson, E., MacNeil, L.T., Arda, H.E., Zhu, L.J., Walhout, A.J. (2013). Integration of metabolic and gene regulatory networks modulates the C. elegans dietary response. Cell. 153(1):253-66.

Wong, A.K., Park, C.Y., Greene, C.S., Bongo, L.A., Guan, Y., Troyanskaya, O.G. (2012). IMP: a multi-species functional genomics portal for integration, visualization and prediction of protein functions and networks. Nucleic Acids Res. 40:W484-90.

Wong, J.J., Ritchie, W., Ebner, O.A., Selbach, M., Wong, J.W., Huang, Y., Gao, D., Pinello, N., Gonzalez, M., Baidya, K., et al. (2013). Orchestrated intron retention regulates normal granulocyte differentiation. Cell 154(3):583-95.

Yang, Y., Han, L., Yuan, Y., Li, J., Hei, N., Liang, H. (2014). Gene co-expression network analysis reveals common system-level properties of prognostic genes across cancer types. Nat. Commun. 5:3231.

Yoshida, K., Sanada, M., Shiraishi, Y., Nowak, D., Nagata, Y., Yamamoto, R., Sato, Y., Sato-Otsubo, A., Kon, A., Nagasaki, M., et al. (2011). Frequent pathway mutations of splicing machinery in myelodysplasia. Nature. 478(7367):64-9.

Zarnack, K., König, J., Tajnik, M., Martincorena, I., Eustermann, S., Stévant, I., Reyes, A., Anders, S., Luscombe, N.M., Ule, J. (2013). Direct competition between hnRNP C and U2AF65 protects the transcriptome from the exonization of Alu elements. Cell. 152(3):453-66.

Zhang, C., Frias, M.A., Mele, A., Ruggiu, M., Eom, T., Marney, C.B., Wang, H., Licatalosi, D.D., Fak, J.J., Darnell, R.B. (2010). Integrative modeling defines the Nova splicing-regulatory network and its combinatorial controls. Science. 329(5990):439-43.

Zheng, S., Damoiseaux, R., Chen, L., Black, D.L. (2013). A broadly applicable high-throughput screening strategy identifies new regulators of Dlg4 (Psd-95) alternative splicing. Genome Res. 23(6):998-1007

Zhou Z, Licklider LJ, Gygi SO, Reed R. (2002). Comprehensive proteomic analysis of the human spliceosome. Nature 419: 182-5.

## Claims

1. A method for identifying targets for the modulation of alternative splicing, which comprises the steps of

   a) generating a network of functional interactions among factors involved in splicing regulation by a method that comprises the steps of:

   i) perturbing the alternative splicing conditions in a group of cells by submitting each cell to at least one perturbing stimulus which consists either of inhibiting the expression of a gene selected from a multiplicity of genes whose products are involved in splicing regulation, each gene belonging to one of the following groups:

   a. components of the spliceosome core,
   b. auxiliary factors which are part of the splicing machinery and that are not components of the spliceosome core,
   c. factors involved in mRNA processing that are not part of the splicing machinery,
   d. genes involved in modulating chromatin structure,

   and/or of any other change of the condition of the cell;
   ii) selecting a multiplicity of alternative splicing events and assessing for each event the impact of inhibiting each one of the multiplicity of inhibited genes, or any other perturbation of the cell condition, on the use of alternative splicing sites, by calculating said impact as the inclusion percentage (PSI) of the exon that can be excluded or included upon inhibition of the gene or actuation of the perturbing stimulus;
   iii) generating a perturbation profile of alternative splicing events for each particular inhibited gene or perturbing stimulus;
   iv) deriving a network that models functional interconnections of splicing perturbations based on the effect of each perturbing stimulus on alternative splicing events by performing the steps of:

   a. preprocessing and reducing the obtained data of the impact of each perturbation in each selected alternative splicing event by a process which involves:

   - removing sparse variables and imputing missing values,
   - removing uninformative events,
   - scaling data by standardization,

   b. carrying out a robust estimation of covariance of perturbation profiles of alternative splicing events for each possible pair of perturbing stimulus,
   c. constructing the network basing it on the graphical lasso model selection algorithm;

b) identifying as a target for alternative splicing regulation any perturbing stimulus that corresponds to a node of the network that is connected with another node that corresponds to a factor that is known to have an effect on the event or events of alternative splicing whose modulation is sought;

wherein the impact on the use of alternative splicing sites resulting from inhibiting each one of the multiplicity of inhibited genes, or from any other perturbation of the cell condition, is assessed for at least 35 alternative splicing events,
and wherein

for carrying out step iii), the inclusion percentage of each exon is transformed in its corresponding Z-score before generating the perturbation profile of alternative splicing events for each particular inhibited gene or perturbing stimulus;
in step iv)a., data are preprocessed and reduced before deriving the network by:

- removing the data corresponding to inhibited genes or any other perturbing stimulus for which it has not been obtained a result for the impact of knocking them down for more than a half of the set of the analyzed alternative splicing events, for considering the knockdown of said gene a sparse variable,
- filling-in the remaining missing values by applying k-nearest-neighbour based imputation,
- removing the data corresponding to alternative splicing events not affected to a significant degree by knocking down genes or submitting cells to any other perturbing stimulus selecting them as those where the median absolute of the PSI changes with regard to the corresponding control is less than 1 ;
- scaling data by standardization;

in step iv)b., the estimation of covariance of perturbation profiles of alternative splicing events is carried out by quantifying the correlation between each pair of perturbation profile basing it on an iterative weighting algorithm, so that the estimated correlation for two pairs of genes or perturbing stimuli Xa and Xb is the weighted Pearson's correlation

$$p(X_a, X_b; w)$$

wherein w is a vector whose length is equal to the number n of alternative splicing events that is processed after data preprocessing, which vector contains the reliability values $R^u_{a,b}$ corresponding to each Z-score for the estimation of the correlation between two perturbation profiles, wherein the values $R^u_{a,b}$ are calculated with the formula

$$R^u_{a,b} = 1 / \frac{\sum_{i=1}^p D_u(X_a, X_i)\mathbb{I}(a, i) + \sum_{i=1}^p D_u(X_b, X_i)\mathbb{I}(b, i)}{\sum_{i=1}^p \mathbb{I}(a, i) + \sum_{i=1}^p \mathbb{I}(b, i)}$$

where II($a,i$) is the indicator function:

$$\mathbb{I}(a, i) := \begin{cases} 1 \text{ if } i \neq a \text{ } AND \text{ } |p(X_a, X_i)| > T \\ 0 \text{ otherwise} \end{cases}$$

$T$ being a minimum correlation threshold for considering a pair of variables, and $D_u(X_a, X_b)$ is the deleted residual distance

$$D_u(X_a, X_b) = |p(X_a, X_b) - p_{-u}(X_a, X_b)|$$

wherein $p(X_a, X_b)$ and $p_{-u}(X_a, X_b)$ are the Pearson's correlation estimates before or after removing the observations $M_{au}$, $M_{bu}$ coming from an splicing event $u$, and using iteratively said algorithm until estimations converge;
and
in step iv)c., a network is constructed from the correlation matrix obtained in iv)b., basing it on the gLasso algorithm by optimizing the log-likelihood function:

$$\log \det \Theta - tr(S\Theta) - r\|\Theta\|_1$$

where $\Theta$ is an estimate for the inverse covariance matrix $\Sigma\text{-}1$, $S$ is the empirical covariance matrix of the data, $\|\Theta\|_1$ is the *L1* norm i.e. the sum of the absolute values of all elements in $\Theta^1$, and $r$ is a regularization parameter which is selected based on estimates of the rate of discovery of false data or FDR by:

- constructing random networks after permuting the sample data for every gene,
- repeating the process for 100 permutations and for random subsamples of the ASEs of size *S={3..35}* to obtain an estimate of the number of random network edges $|E|_R^S$ recovered for each sample size S at a fixed glasso regularization parameter,
- estimating the number of actual edges $|E|_A^S$ for random ASEs subsamples of the same sizes using the observed (non-permuted) data, and
- estimating the FDR for the network edges corresponding to each subsample size S as the ratio of the mean number of edges recovered in the random graphs over the mean number of edges recovered in the actual graphs for all the different permutations and random subsamples of that size

$$FDR_S = \frac{mean(|E|_R^S)}{mean(|E|_A^S)},$$

and
complementing step iv)c., by defining modules in the network following the steps of:

- using the algorithm of network modularity *M(p)* which is defined as:

$$M(p) = \sum_{k=1}^{m} \left[ \frac{l_k}{L} - \left(\frac{d_k}{2L}\right)^2 \right]$$

where *m* is the number of modules in *p*, $l_k$ is the number of connections within module *k*, *L* is the total number of network connections and $d_k$ is the sum of the degrees of the nodes in module *k*, and
- identifying modules of genes or perturbation stimuli that exhibit similar perturbation profiles among the assayed events by maximizing the network's modularity using the Clauset's greedy community detection algorithm, and displaying the network by clustering genes or perturbing stimuli in said modules.

2. The method according to claim 1, wherein the multiplicity of genes whose expression is inhibited for generating the network comprises at least one gene selected from each one of the four groups of:

   a) components of the spliceosome core,
   b) auxiliary factors which are part of the splicing machinery and that are not components of the spliceosome core,
   c) factors involved in mRNA processing that are not part of the splicing machinery, such as those having roles in mRNA translation, stability and localization in the cytoplasm, included molecules such as microRNAs.
   d) genes involved in modulating chromatin structure,

3. The method according to claim 2, wherein the multiplicity of inhibited genes comprises genes selected of the following group of human genes: SRSF4, SRSF11, SRSF6, SRSF5, SRSF1, SRSF7, SRSF2, SRSF9, SRSF10, SRSF3, SRRM2, DDX46, CRNKL1, CDC5L, NCBP1, SF1, SKIIP, SIAHBP1, PLRG1, PRPF19, U2AF2, U2AF1, SMNDC1, NCBP2, FNBP3, SNRP70, SNRPA, SNRPC, SF3B1, SF3B2, PABPN1, SFPQ, SF3B3, SF3A1, SF3A2, SF3A3, SF3B4, SNRPA1, SNRPB2, P14, PRPF8, U5-200KD, NONO, RBM35A, U5-116KD, C20ORF14, DDX23, CD2BP2, HPRP8BP, TXNL4, PRPF3, PRPF4, PRPF31, PPIH, RBM35B, CCDC55, NHP2L1, SART1, USP39, RY1, SNRPB, SNRPD1, SNRPD2, SNRPD3, SNRPE, SNRPF, HMGA1, DBR1, SNRPG, LSM2, LSM3, LSM4, LSM6, LSM7, DHX38, DHX8, DHX15, SLU7, DIS3, EXOSC4, CDC40, PRPF18, SRRM1, BAT1, RNPS1, THOC4, RBM8A, MAGOH, TCERG1, PRPF4B, DDX5, IK, PPM1G, PABPC1, RBM17, HPRP8BP, DNAJC8, BCAS2, ACIN1, SHARP, HTATSF1, RNPC2, CPSF6, RBM15, RBM22, IMP-3, CIRBP, DDX48, DDX17, DHX16, DHX9, DDX3X, SKIV2L2, DHX57, DHX30, DHX32, DHX33, DDX10, DDX24, DDX52, DIS3L, DEK, DDX31, DDX11, DDX28, DDX12, TDRD9, LOC164045, MOV10, XAB2, THOC1, LUC7A, EP300, KAT2A, KIAA1604, FUBP3, MGC2655, CRK7, TFIP11, CPSF5, THOC3, C19ORF29, G10, ZNF207, KAT5, CARM1, ARS2, THOC2, C22ORF19, NIF3L1BP1, MGC13125, WTAP, C21ORF66, DKFZP434I116, KIAA1160, HSPC148, PRMT5, BRD4, DGCR14, GTL3, MGC2803, HNRPAO, HNRPA1, HNRPA2B1, HNRNPA3, HNRPC, HNRPD, PTBP1, CTCF, CHD1, HNRPK, HNRPL, HNRPR, RALY, FUS, ILF3, ILF2, SYNCRIP, MATR3, BUB3, ELAVL1, TAF15, YBX1, CSDA, HSPA1A, HSPA8, HNRPF, HNRPH1, HNRPH3, HNRPH2, HNRPU, HSPA5, KHDRBS1, SRPK2, RBM10, DICER1, EWSR1, RBM6, RBM5, TIA1, TIAL1, FNBP3, RBM25, SR140, CHERP, CCAR1, THRAP3, WBP4, MFAP1, SMU1, MORF4L1, HDAC1, CTNNBL1, AQR, PQBP1, WBP11, KIN, DDX41, FLJ22965, P29, DHX35, C19ORF29, SIRT1, HDAC2, FLJ35382, C9ORF78, NOSIP, LENG1, MORG1, MGC20398, MGC23918, FRG1, CDK10, DXS9928E, HDAC4, HDAC6, D6S2654E, FAM32A, KIAA0073, PPIL3, PPIE, PPIL1, SDCCAG10, PPIL2, PPIG, ASCL1, TET1, HDAC3, SUV39H1, SETD1A, EHMT2, SETD2, MECP2, MBD2, NAB2, BMI1, EED, PHC1, AOF2, JMJD2B, PHC2, EZH2, SMARCA4, SMARCA2, KAT2B, DNMT1, EIF2C2, CBX3, RPS6KA5, ASH2L, or combinations thereof.

4. The method according to any one of claims 1 to 3, wherein the cells where the expression of at least one gene is inhibited or which are submitted to at least another perturbing stimulus are HeLa cells.

5. The method according to any one of the preceding claims, wherein the impact on the use of alternative splicing sites is assessed for the alternative splicing events corresponding to the following human genes: FAS, OLR1, PAX6, CHEK2, FN1EDB, FN1EDA, NUMB, BCL2L1, RAC1, MST1R, MADD, CASP2, APAF1, MINK1, MAP3K7, MAP4K3, NOTCH3, MAP4K2, PKM2 exon 9, PKM2 exon 10, VEGFA, MCL1, CFLAR, CASP9 exon 3, CASP9 exon 4, CASP9 exon 5, CASP9 exon 6, SYK, DIABLO, BMF2, BMF3, CCNE1, CCND1, H2AFY exon 6, H2AFY exon 6', STAT3, GADD45A exon 2, GADD45A exon 3, BCL2L11 exon intron, BCL2L11 exon 4, BCL2L11 exon 5, BIRC5 exon 2b, BIRC5 exon 3, BIRC5 exon 3b, SMN1, SMN2.

6. The method according to any one of the preceding claims, wherein the inhibition of gene expression is carried out by specific small interference RNAs (siRNAs).

7. The method according to any one of the preceding claims, wherein an additional step for identifying general and secondary functional interactions among splicing factors is carried, which step comprises the substeps of:

i. repeating substeps i) to iv) of step a) of the method with different subsamples of data, each subsample consisting of different combinations of at least one half of the alternative splicing events initially analyzed, wherein the number of subsamples is preferably at least one order of magnitude higher than the number of inhibited genes or the total number of perturbing stimuli applied,
ii. identifying as functional interactions among splicing regulatory factors that are general and essential for the splicing process as those corresponding to connections that are present in at least 90% of the reconstructed subnetworks,
iii. Identifying as functional interactions which are specific of particular events of groups of events of alternative splicing as those corresponding to connections with an absolute correlation value higher than 0.5 and which are present in more than 20% of the reconstructed subnetworks and that, additionally, are absent of more than 20% of the remaining reconstructed subnetworks and with an absolute correlation value lower than 0.2.

8. The method according to any one of the preceding claims, wherein, additionally, those nodes that appear clustered in a same region of a network with connections among them are identified as splicing factors forming part of a same splicing subcomplex.

9. The method according to any one of the preceding claims, wherein at least one of the splicing perturbing stimuli whose effect on the set of alternative splicing events is analyzed consists of submitting the corresponding cell or cells to the action of an added compound.

10. The method according to claim 9, wherein at least one added compound is a drug known to have a therapeutic effect on a disease associated with altered alternative splicing and any splicing regulatory factor whose corresponding network node has a direct connection with the node corresponding to the drug is identified as a target for directing to it other compounds intended to be candidates to alternative drugs against said disease.

11. The method according to claim 10, wherein the drug is spliceostatin A or meayamicin and any direct connection with it is identified as a target for directing to it other compounds intended to be candidates to alternative drugs against cancer.

**12.** The method according to claim 9, wherein an added compound is identified as a candidate to drug for the treatment of a disease associated with altered alternative splicing when it has at least a direct connection in common with a second compound, also having added as a perturbing stimulus for carrying out the method or whose functional associations have been previously identified by the method of claim 1 or by any other means, which second compound is a drug known to have an therapeutic effect on a disease associated with altered alternative splicing.

**13.** The method according to claim 10 or 12, wherein the disease is selected among cancer, Duchenne muscular dystrophy, spinal muscular atrophy or any other neuromuscular or neurological disease associated to altered alternative splicing.

**14.** The method according to claim 9, wherein the added compound is known to modulate a physiological process in the cell and any splicing regulatory factor whose corresponding network node has a direct connection with the node corresponding to the drug is identified as a target for directing to it other compounds intended to be candidates to the modulation of the same process.

**15.** The method of any one of the preceding claims, wherein the method is carried out both with cells removed from a patient suffering from a disease or malfunction and with a different kind of cells deriving from an individual not suffering that disease or malfunction and factors showing connections different in the two generated networks are identified as targets for directing to them compounds intended to be candidates to drugs against said disease or malfunction.

**16.** A method for identifying candidates to drugs against a disease which comprises the steps of carrying out the method of any one of claims 1 to 9 and identifying the compound as candidate to drug against such disease when it shows at least a direct connection in common with another compound previously known to have a therapeutic effect on that disease or with a splicing regulatory factor that is previously known to be a target of at least a drug known to have a therapeutic effect on that disease.

**Patentansprüche**

**1.** Methode zum Identifizieren einer Zielstelle für die Modulation des alternativen Spleißens, die folgende Schritte umfasst

a) Erzeugen eines Netzwerks aus funktionellen Interaktionen zwischen Faktoren, die an der Regulation des Spleißens beteiligt sind, mittels einer Methode, die folgende Schritte umfasst:

i) Stören der Bedingungen für das alternative Spleißen in einer Gruppe von Zellen, indem jede Zelle mindestens einem Störstimulus ausgesetzt wird, der entweder in der Hemmung der Expression eines Gens besteht, das aus einer Vielzahl von Genen ausgewählt ist, deren Produkte an der Regulation des Spleißens beteiligt sind, wobei jedes Gen zu einer der folgenden Gruppen gehört:

a. Komponenten des Spleißosom-Zentrums,
b. Hilfsfaktoren, die Teil der Spleiß-Maschinerie sind und keine Komponenten des Spleißosom-Zentrums sind,
c. Faktoren, die an der mRNA-Verarbeitung beteiligt sind und nicht Teil der Spleiß-Maschinerie sind,
d. Gene, die an der Modulation der Chromatinstruktur beteiligt sind,

und/oder an einer anderen Veränderung des Zustands der Zelle besteht;
ii) Auswählen einer Vielzahl von alternativen Spleiß-Ereignissen und Beurteilen, für jedes Ereignis, der Auswirkung der Hemmung jedes einzelnen der Vielzahl von gehemmten Genen oder einer anderen Störung des Zellzustands auf die Verwendung von alternativen Spleißstellen, indem die Auswirkung als Einschlussprozentsatz (PSI) des Exons, das nach Hemmung des Gens oder Einfluss des Störstimulus aus- oder eingeschlossen werden kann, berechnet wird;
iii) Erzeugen eines Störprofils der alternativen Spleiß-Ereignisse für jedes einzelne gehemmte Gen oder jeden einzelnen Störstimulus;
iv) Ableiten eines Netzwerks, das funktionelle Verknüpfungen der Spleiß-Störungen basierend auf dem Effekt jedes einzelnen Störstimulus auf alternative Spleiß-Ereignisse modelliert, indem folgende Schritte durchgeführt werden:

a. Vorverarbeiten und Reduzieren der erhaltenen Daten über die Auswirkung jeder Störung bei jedem ausgewählten alternativen Spleiß-Ereignis mittels einer Methode, die Folgendes beinhaltet:

- Entfernen von Sparse-Variablen und Schätzen fehlender Werte,
- Entfernen uninformativer Ereignisse,
- Skalieren der Daten durch Standardisierung,

b. Durchführen einer robusten Kovarianzschätzung der Störprofile der alternativen Spleiß-Ereignisse für jedes mögliche Paar Störstimuli,
c. Aufbauen des Netzwerks auf Basis des grafischen Lasso-Modellauswahl-Algorithmus;

b) Identifizieren eines Störstimulus als eine Zielstelle für die Regulation des alternativen Spleißens, der einem Knoten des Netzwerks entspricht, der mit einem anderen Knoten verknüpft ist, der einem Faktor entspricht, von dem bekannt ist, dass er einen Effekt auf das Ereignis oder die Ereignisse des alternativen Spleißens, dessen/deren Modulation angestrebt wird, hat;

wobei die Auswirkung auf die Verwendung alternativer Spleißstellen, die sich aus der Hemmung jedes einzelnen der Vielzahl von gehemmten Genen oder aus einer anderen Störung des Zellzustands ergibt, für mindestens 35 alternative Spleiß-Ereignisse beurteilt wird,
und wobei

zur Ausführung von Schritt iii) der Einschlussprozentsatz jedes Exons vor dem Erzeugen des Störprofils der alternativen Spleiß-Ereignisse für jedes einzelne gehemmte Gen oder jeden einzelnen Störstimulus in seinen entsprechenden Z-Score umgewandelt wird;
in Schritt iv)a die Daten vorverarbeitet und reduziert werden, bevor das Netzwerk abgeleitet wird durch:

- Entfernen der Daten, die gehemmten Genen oder einem anderen Störstimulus entsprechen, für die für mehr als die Hälfte des Satzes analysierter alternativer Spleiß-Ereignisse kein Ergebnis für die Auswirkung ihrer Deaktivierung erhalten wurde, da die Deaktivierung des Gens als Sparse-Variable betrachtet wird,
- Eintragen der restlichen fehlenden Werte durch Anwenden einer auf k-nächsten-Nachbarn basierte Schätzung,
- Entfernen der Daten, die alternativen Spleiß-Ereignissen entsprechen, die in keinem bedeutenden Maß von der Deaktivierung von Genen oder dem Aussetzen der Zellen einem anderen Störstimulus betroffen sind, wobei sie als diejenigen ausgewählt werden, bei denen die absolute Mediane der PSI-Veränderungen in Bezug auf die entsprechende Kontrolle kleiner als 1 ist;
- Skalieren der Daten durch Standardisierung;

in Schritt iv)b die Kovarianzschätzung der Störprofile der alternativen Spleiß-Ereignisse durch Quantifizierung der Korrelation zwischen jedem Paar Störprofile auf Basis eines iterativen Gewichtsalgorithmus durchgeführt wird, sodass die geschätzte Korrelation für zwei Genpaare oder Störstimuli Xa und Xb die gewichtete Pearson-Korrelation

$$p(X_a, X_b; w)$$

ist, wobei w ein Vektor ist, dessen Länge gleich der Anzahl n der alternativen Spleiß-Ereignisse ist, die nach der Datenvorverarbeitung verarbeitet wird, wobei der Vektor die Zuverlässigkeitswerte $R^u_{a,b}$ enthält, die jedem Z-Score für die Korrelationsschätzung zwischen zwei Störprofilen entsprechen, wobei die Werte $R^u_{a,b}$ mit folgender Formel berechnet werden

$$R^u_{a,b} = 1 / \frac{\sum_{i=1}^{p} D_u(X_a, X_i)\mathbb{I}(a,i) + \sum_{i=1}^{p} D_u(X_b, X_i)\mathbb{I}(b,i)}{\sum_{i=1}^{p} \mathbb{I}(a,i) + \sum_{i=1}^{p} \mathbb{I}(b,i)}$$

wobei II *(a,i)* die Indikatorfunktion ist:

$$\|(a,i) = \begin{cases} 1 \ wenn \ i \neq a \ UND \ |p(X_a, X_i)| > T \\ sonst \ 0 \end{cases}$$

wobei *T* ein Mindestkorrelationsschwellenwert für die Berücksichtigung eines Variablenpaars ist und $D_u\,(X_a, X_b)$ der deletierte Restabstand ist,

$$D_u(X_a, X_b) = |p(X_a, X_b) - p_{-u}(X_a, X_b)|$$

wobei *p(X_a, X_b)* und *p_u(X_a, X_b)* die Pearson-Korrelationsschätzungen vor oder nach dem Entfernen der Beobachtungen $M_{au}$, $M_{bu}$, die von einem Spleiß-Ereignis *u* herrühren, und dem iterativen Verwenden des Algorithmus bis zum Konvergieren der Schätzungen sind;
und
in Schritt iv)c ein Netzwerk aus der in iv)b erhaltenen Korrelationsmatrix auf Basis des gLasso-Algorithmus durch Optimieren der log-Wahrscheinlichkeitsfunktion:

$$\log \det \Theta - tr(S\Theta) - r\|\Theta\|_1$$

aufgebaut wird, wobei $\Theta$ eine Schätzung für die inverse Kovarianzmatrix $\Sigma^{-1}$ ist, S die empirische Kovarianzmatrix der Daten ist, $\|\Theta\|_1$ die *L1*-Norm, d.h. die Summe der absoluten Werte aller Elemente in $\Theta^{-1}$ ist und *r* ein Regularisierungsparameter ist, der aufgrund der Schätzungen der False-Discovery-Rate oder FDR ausgewählt wird durch:

- Aufbauen willkürlicher Netzwerke nach Permutieren der Probendaten für jedes Gen,
- Wiederholen der Methode für 100 Permutationen und für willkürliche Teilstichproben der ASE der Größe *S={3..35}*, um eine Schätzung der Anzahl willkürlicher Netzwerkränder $|E_R^S|$ zu erhalten, die für jede Probengröße S bei einem festen gLasso-Regularisierungsparameter wiederhergestellt werden,

- Schätzen der Anzahl tatsächlicher Ränder $|E_A^S|$ für willkürliche ASE-Teilstichproben gleicher Größe unter Verwendung der beobachteten (nicht permutierten) Daten und
- Schätzen der FDR für die Netzwerkränder, die jeder Teilstichprobengröße *S* entsprechen, als Verhältnis der durchschnittlichen Anzahl Ränder, die in den willkürlichen Grafen wiederhergestellt wurden, zu der durchschnittlichen Anzahl Ränder, die in den tatsächlichen Grafen für alle unterschiedlichen Permutationen und willkürlichen Teilstichproben dieser Größe wiederhergestellt wurden

$$FDR_S = \frac{durchschnittliche(|E|_R^S)}{durchschnittliche(|E|_A^S)}$$

und
Ergänzen von Schritt iv)c durch Definieren von Modulen im Netzwerk gemäß den Schritten:

- Verwenden des Algorithmus der Netzwerkmodularität *M(p)*, die definiert ist als:

$$M(p) = \sum_{k=1}^{m} \left[ \frac{l_k}{L} - \left( \frac{d_k}{2L} \right)^2 \right]$$

wobei *m* die Anzahl Module in *p* ist, $l_k$ die Anzahl Verknüpfungen innerhalb des Moduls *k* ist, *L* die Gesamtanzahl der Netzwerkverknüpfungen ist und $d_k$ die Summe der Grade der Knoten in Modul *k* ist, und
- Identifizieren der Module von Genen oder Störstimuli, die ähnliche Störprofile unter den geprüften Ereignissen aufweisen, durch Maximieren der Netzwerkmodularität unter Verwendung des gierigen Community-Erkennungsalgorithmus von Clauset und Anzeigen des Netzwerks durch Gruppieren der Gene oder Störstimuli in die Module.

2. Methode nach Anspruch 1, wobei die Vielzahl von Genen, deren Expression zur Erzeugung des Netzwerks gehemmt wird, mindesten ein Gen umfasst, das jeweils aus einer der folgenden vier Gruppen ausgewählt ist:

   a) Komponenten des Spleißosom-Zentrums,
   b) Hilfsfaktoren, die Teil der Spleiß-Maschinerie sind und keine Komponenten des Spleißosom-Zentrums sind,
   c) Faktoren, die an der mRNA-Verarbeitung beteiligt sind und nicht Teil der Spleiß-Maschinerie sind, wie solche, die bei der mRNA-Translation, Stabilität und Lokalisation im Zytoplasma eine Rolle spielen, darunter Moleküle wie mikroRNA,
   d) Gene, die an der Modulation der Chromatinstruktur beteiligt sind.

3. Methode nach Anspruch 2, wobei die Vielzahl von gehemmten Genen Gene umfasst, die aus der folgenden Gruppe humaner Gene ausgewählt sind: SRSF4, SRSF11, SRSF6, SRSF5, SRSF1, SRSF7, SRSF2, SRSF9, SRSF10, SRSF3, SRRM2, DDX46, CRNKL1, CDC5L, NCBP1, SF1, SKIIP, SIAHBP1, PLRG1, PRPF19, U2AF2, U2AF1, SMNDC1, NCBP2, FNBP3, SNRP70, SNRPA, SNRPC, SF3B1, SF3B2, PABPN1, SFPQ, SF3B3, SF3A1, SF3A2, SF3A3, SF3B4, SNRPA1, SNRPB2, P14, PRPF8, U5-200KD, NONO, RBM35A, U5-116KD, C20ORF14, DDX23, CD2BP2, HPRP8BP, TXNL4, PRPF3, PRPF4, PRPF31, PPIH, RBM35B, CCDC55, NHP2L1, SART1, USP39, RY1, SNRPB, SNRPD1, SNRPD2, SNRPD3, SNRPE, SNRPF, HMGA1, DBR1, SNRPG, LSM2, LSM3, LSM4, LSM6, LSM7, DHX38, DHX8, DHX15, SLU7, DIS3, EXOSC4, CDC40, PRPF18, SRRM1, BAT1, RNPS1, THOC4, RBM8A, MAGOH, TCERG1, PRPF4B, DDX5, IK, PPM1G, PABPC1, RBM17, HPRP8BP, DNAJC8, BCAS2, ACIN1, SHARP, HTATSF1, RNPC2, CPSF6, RBM15, RBM22, IMP-3, CIRBP, DDX48, DDX17, DHX16, DHX9, DDX3X, SKIV2L2, DHX57, DHX30, DHX32, DHX33, DDX10, DDX24, DDX52, DIS3L, DEK, DDX31, DDX11, DDX28, DDX12, TDRD9, LOC164045, MOV10, XAB2, THOC1, LUC7A, EP300, KAT2A, KIAA1604, FUBP3, MGC2655, CRK7, TFIP11, CPSF5, THOC3, C19ORF29, G10, ZNF207, KAT5, CARM1, ARS2, THOC2, C22ORF19, NIF3L1BP1, MGC13125, WTAP, C21ORF66, DKFZP434I116, KIAA1160, HSPC148, PRMT5, BRD4, DGCR14, GTL3, MGC2803, HNRPAO, HNRPA1, HNRPA2B1, HNRNPA3, HNRPC, HNRPD, PTBP1, CTCF, CHD1, HNRPK, HNRPL, HNRPR, RALY, FUS, ILF3, ILF2, SYNCRIP, MATR3, BUB3, ELAVL1, TAF15, YBX1, CSDA, HSPA1A, HSPA8, HNRPF, HNRPH1, HNRPH3, HNRPH2, HNRPU, HSPA5, KHDRBS1, SRPK2, RBM10, DICER1, EWSR1, RBM6, RBM5, TIA1, TIAL1, FNBP3, RBM25, SR140, CHERP, CCAR1, THRAP3, WBP4, MFAP1, SMU1, MORF4L1, HDAC1, CTNNBL1, AQR, PQBP1, WBP11, KIN, DDX41, FLJ22965, P29, DHX35, C19ORF29, SIRT1, HDAC2, FLJ35382, C9ORF78, NOSIP, LENG1, MORG1, MGC20398, MGC23918, FRG1, CDK10, DXS9928E, HDAC4, HDAC6, D6S2654E, FAM32A, KIAA0073, PPIL3, PPIE, PPIL1, SDCCAG10, PPIL2, PPIG, ASCL1, TET1, HDAC3, SUV39H1, SETD1A, EHMT2, SETD2, MECP2, MBD2, NAB2, BMI1, EED, PHC1, AOF2, JMJD2B, PHC2, EZH2, SMARCA4, SMARCA2, KAT2B, DNMT1, EIF2C2, CBX3, RPS6KA5, ASH2L oder Kombinationen davon.

4. Methode nach einem der Ansprüche 1 bis 3, wobei die Zellen, in denen die Expression mindestens eines Gens gehemmt wird oder die mindestens einem anderen Störstimulus ausgesetzt werden, HeLa-Zellen sind.

5. Methode nach einem der vorhergehenden Ansprüche, wobei die Auswirkung auf die Verwendung von alternativen Spleißstellen für die alternativen Spleiß-Ereignisse beurteilt wird, die den folgenden humanen Gene entsprechen: FAS, OLR1, PAX6, CHEK2, FN1EDB, FN1EDA, NUMB, BCL2L1, RAC1, MST1R, MADD, CASP2, APAF1, MINK1, MAP3K7, MAP4K3, NOTCH3, MAP4K2, PKM2 Exon 9, PKM2 Exon 10, VEGFA, MCL1, CFLAR, CASP9 Exon 3, CASP9 Exon 4, CASP9 Exon 5, CASP9 Exon 6, SYK, DIABLO, BMF2, BMF3, CCNE1, CCND1, H2AFY Exon 6, H2AFY Exon 6', STAT3, GADD45A Exon 2, GADD45A Exon 3, BCL2L11 Exon Intron, BCL2L11 Exon 4, BCL2L11 Exon 5, BIRC5 Exon 2b, BIRC5 Exon 3, BIRC5 Exon 3b, SMN1, SMN2.

6. Methode nach einem der vorhergehenden Ansprüche, wobei die Hemmung der Genexpression durch spezifische kleine Interferenz-RNA (siRNA) durchgeführt wird.

7. Methode nach einem der vorhergehenden Ansprüche, wobei ein zusätzlicher Schritt zum Identifizieren allgemeiner und sekundärer funktioneller Interaktionen unter den Spleißfaktoren durchgeführt wird, wobei der Schritt folgende Teilschritte umfasst:

   i. Wiederholen der Teilschritte i) bis iv) von Schritt a) der Methode mit unterschiedlichen Datenteilstichproben, wobei jede Teilstichprobe aus unterschiedlichen Kombinationen aus mindestens einer Hälfte der ursprünglich analysierten alternativen Spleiß-Ereignisse besteht, wobei die Anzahl der Teilstichproben vorzugsweise mindestens eine Größenordnung über der Anzahl der gehemmten Gene oder die Gesamtanzahl der angewandten Störstimuli liegt,

ii. Identifizieren funktioneller Interaktionen unter den Spleiß-Regulationsfaktoren, die allgemein und wesentlich für den Spleiß-Prozess sind, als diejenigen, die Verknüpfungen entsprechen, die in mindestens 90 % der wieder aufgebauten Teilnetzwerken vorliegen,

iii. Identifizieren funktioneller Interaktionen, die spezifisch für bestimmte Ereignisse oder Gruppen von Ereignissen des alternativen Spleißens sind, als diejenigen, die Verknüpfungen mit einem absoluten Korrelationswert größer als 0,5 entsprechen, die in mehr als 20 % der wieder aufgebauten Teilnetzwerke vorliegen und die zusätzlich in mehr als 20 % der restlichen wieder aufgebauten Teilnetzwerke abwesend sind und einen absoluten Korrelationswert kleiner als 0,2 aufweisen.

8. Methode nach einem der vorhergehenden Ansprüche, wobei zusätzlich diejenigen Knoten, die in der gleichen Region eines Netzwerks mit Verknüpfungen untereinander gruppiert erscheinen, als Spleißfaktoren identifiziert werden, die Teil desselben Spleiß-Teilkomplexes sind.

9. Methode nach einem der vorhergehenden Ansprüche, wobei mindesten einer der Störstimuli des Speißens, deren Effekt auf den Satz alternativer Spleiß-Ereignisse analysiert wird, in dem Aussetzen der entsprechenden Zelle oder Zellen der Wirkung einer zugesetzten Verbindung besteht.

10. Methode nach Anspruch 9, wobei mindestens eine zugesetzte Verbindung ein Arzneimittel ist, von dem bekannt ist, dass es einen therapeutischen Effekt auf eine Erkrankung aufweist, die mit einer Beeinträchtigung des alternativen Spleißens assoziiert ist, und jeder Spleiß-Regulationsfaktor, dessen entsprechender Netzwerkknoten eine direkte Verknüpfung mit dem dem Arzneimittel entsprechenden Knoten aufweist, als Zielstelle identifiziert wird, auf die andere Verbindungen gerichtet werden können, die als Kandidaten für alternative Arzneimittel gegen die Erkrankung vorgesehen sind.

11. Methode nach Anspruch 10, wobei das Arzneimittel Spliceostatin A oder Meayamicin ist und eine direkte Verknüpfung mit ihm als Zielstelle identifiziert wird, auf das andere Verbindungen gerichtet werden können, die als Kandidaten für alternative Arzneimittel gegen Krebs vorgesehen sind.

12. Methode nach Anspruch 9, wobei eine zugesetzte Verbindung als Kandidat für ein Arzneimittel zur Behandlung einer Erkrankung, die mit einer Beeinträchtigung des alternativen Spleißens assoziiert ist, identifiziert wird, wenn es mindestens eine direkte Verknüpfung mit einer zweiten Verbindung gemeinsam hat, die ebenfalls als Störstimulus zur Durchführung der Methode zugesetzt wurde oder deren funktionelle Assoziationen zuvor durch die Methode nach Anspruch 1 oder durch andere Mittel identifiziert wurden, wobei die zweite Verbindung ein Arzneimittel ist, von dem bekannt ist, dass es einen therapeutischen Effekt auf eine Erkrankung aufweist, die mit einer Beeinträchtigung des alternativen Spleißens assoziiert ist.

13. Methode nach Anspruch 10 oder 12, wobei die Erkrankung ausgewählt ist aus Krebs, Duchenne-Muskeldystrophie, spinaler Muskelatrophie oder einer anderen neuromuskulären oder neurologischen Erkrankung, die mit einer Beeinträchtigung des alternativen Spleißens assoziiert ist.

14. Methode nach Anspruch 9, wobei bekannt ist, dass die zugesetzte Verbindung einen physiologischen Prozess in der Zelle moduliert, und jeder Spleiß-Regulationsfaktor, dessen entsprechender Netzwerkknoten eine direkte Verknüpfung mit dem dem Arzneimittel entsprechenden Knoten aufweist, als Zielstelle identifiziert wird, auf die andere Verbindungen gerichtet werden können, die als Kandidaten für die Modulation desselben Prozesses vorgesehen sind.

15. Methode nach einem der vorhergehenden Ansprüche, wobei die Methode sowohl mit Zellen, die aus einem Patienten entfernt wurden, der an einer Erkrankung oder Störung leidet, als auch mit einer anderen Art von Zellen, die aus einem Individuum stammen, das nicht an dieser Erkrankung oder Störung leidet, durchgeführt wird und Faktoren, die in den beiden erzeugten Netzwerken verschiedene Verknüpfungen zeigen, als Zielstellen identifiziert werden, auf die Verbindungen gerichtet werden können, die als Kandidaten für Arzneimittel gegen die Erkrankung oder Störung vorgesehen sind.

16. Methode zum Identifizieren von Kandidaten für Arzneimittel gegen eine Erkrankung, die die Schritte des Durchführens der Methode nach einem der Ansprüche 1 bis 9 und des Identifizierens der Verbindung als Kandidat für ein Arzneimittel gegen diese Erkrankung umfasst, wenn sie mindestens eine direkte Verknüpfung gemeinsam mit einer anderen Verbindung hat, von der bereits bekannt ist, dass sie einen therapeutischen Effekt auf diese Erkrankung aufweist, oder mit einem Spleiß-Regulationsfaktor, von dem bereits bekannt ist, dass er eine Zielstelle mindestens eines Arzneimittels ist, von dem bekannt ist, dass es einen therapeutischen Effekt auf diese Erkrankung aufweist.

**Revendications**

1. Une méthode d'identification de cibles pour la modulation de l'épissage alternatif, comprenant les étapes de

a) la génération d'un réseau d'interactions fonctionnelles entre les facteurs impliqués dans la régulation de l'épissage par une méthode qui comprenant les étapes suivantes :

i) perturber les conditions de l'épissage alternatif dans un groupe de cellules en soumettant chaque cellule à au moins un stimulus perturbateur qui consiste soit à inhiber l'expression d'un gène choisi parmi une multiplicité de gènes dont les produits sont impliqués dans la régulation de l'épissage, chaque gène appartenant à un des groupes suivants :

a. les composants du noyau du spliceosome,
b. les facteurs auxiliaires qui font partie de la machine d'épissage et qui ne sont pas des composants du noyau du splicesome,
c. les facteurs impliqués dans le traitement de l'ARNm qui ne font pas partie de la machine d'épissage,
d. les gènes impliqués dans la modulation de la structure de la chromatine,

et/soit à tout autre changement de l'état de la cellule;
ii) sélectionner une multiplicité d'événements d'épissage alternatifs et évaluer, pour chaque événement, l'impact de l'inhibition de chacun des gènes de la multiplicité de gènes inhibés ou de toute autre perturbation de l'état cellulaire, sur l'utilisation de sites d'épissage alternatifs, en calculant ledit impact comme pourcentage d'inclusion (PSI) de l'exon qui peut être exclus ou inclus par son inhibition du gène ou déclenchement du stimulus perturbant;
iii) générer un profil de perturbation des événements d'épissage alternatif pour chaque gène inhibé ou stimulus perturbateur particulier;
iv) dériver un réseau qui modélise les interconnexions fonctionnelles des perturbations d'épissage en fonction de l'effet de chaque stimulus perturbateur sur les événements d'épissage alternatifs en effectuant les étapes suivantes:

a. la pré-traitement et réduction des données obtenues de l'impact de chaque perturbation dans chaque événement d'épissage alternatif sélectionné par un processus qui implique:

- supprimer les variables éparses et imputer les valeurs manquantes,
- éliminer des événements non informatifs,
- mettre les données à l'échelle par normalisation,

b. la réalisation d'une estimation robuste de la covariance des profils de perturbation des événements d'épissage alternatif pour chaque paire de stimulus perturbateurs possible,
c. la construction du réseau en le basant sur l'algorithme de sélection du modèle graphique lasso;

b) l'identification comme cible pour la régulation de l'épissage alternatif tout stimulus perturbateur qui correspond à un nœud du réseau connecté à un autre nœud qui est lié à un facteur connu pour avoir un effet sur l'événement ou les événements d'épissage alternatif dont on recherche la modulation;

dans laquelle l'impact sur l'utilisation de sites d'épissage alternatifs résultant de l'inhibition de chacun des gènes inhibés par la multiplicité, ou de toute autre perturbation de l'état cellulaire, est évalué pour au moins 35 événements d'épissage alternatif,
et dans laquelle

pour effectuer l'étape iii), le pourcentage d'inclusion de chaque exon est transformé en son score Z correspondant avant de générer le profil de perturbation des événements d'épissage alternatif pour chaque gène inhibé ou stimulus perturbateur particulier;
à l'étape iv)a., les données sont pré-traitées et réduites avant de dériver le réseau par :

- la suppression des données correspondant à des gènes inhibés ou à tout autre stimulus perturbateur pour lequel un résultat sur l'impact de leur élimination pendant plus de la moitié de l'ensemble des événements d'épissage alternatif n'a pas été obtenu, pour considérer l'élimination dudit gène comme variable rare,
- la saisie des valeurs manquantes restantes en appliquant l'imputation fondée sur les k voisins les plus proches,
- la suppression des données correspondant à d'autres événements d'épissage qui ne sont pas affectés de façon significative par l'élimination de gènes ou la soumission de cellules à tout autre stimulus perturbateur qui les sélectionnent comme événements pour lesquelles où la médiane absolue des changements du PSI par rapport au témoin correspondant est inférieure à 1;
- la mise à l'échelle des données par normalisation ;

à l'étape iv)b., l'estimation de la covariance des profils de perturbation des événements d'épissage alternatifs est effectuée en quantifiant la corrélation entre chaque paire de profils de perturbation sur la base d'un algorithme de pondération itératif, de sorte que la corrélation estimée pour deux paires de gènes ou stimuli perturbateurs Xa et Xb soit la corrélation pondérée de Pearson

$$p(Xa, Xb;w)$$

dans laquelle w est un vecteur dont la longueur est égale au nombre n d'événements d'épissage alternatifs qui est traité après pré-traitement des données, lequel vecteur contient les valeurs de fiabilité $R^u_{a,b}$ correspondant à chaque score Z pour l'estimation de la corrélation entre deux profils de perturbation, dans laquelle les valeurs $R^u_{a,b}$ sont calculées avec la formule

$$R^u_{a,b} = 1 / \frac{\sum_{i=1}^{p} D_u(X_a, X_i)\mathbb{I}(a, i) + \sum_{i=1}^{p} D_u(X_b, X_i)\mathbb{I}(b, i)}{\sum_{i-1}^{p} \mathbb{I}(a, i) + \sum_{i-1}^{p} \mathbb{I}(b, i)}$$

où II*(a,i)* est la fonction indicatrice:

$$\mathbb{I}(a, i) = \begin{cases} 1 \ si \ i \ \neq a \ ET \ |p(X_a, X_i)| > T \\ \qquad 0 \ sinon \end{cases}$$

*T* étant un seuil de corrélation minimum pour considérer une paire de variables, et *Du (X_a, X_b)* est la distance résiduelle supprimée

$$D_u(X_a, X_b) = |p(X_a, X_b) - p_{-u}(X_a, X_b)|$$

dans laquelle $p(X_a, X_b)$ et $p_{-u}(X_a, X_b)$ sont les estimations de corrélation de Pearson avant ou après suppression des observations $M_{au}$, $M_{bu}$ provenant d'un événement d'épissage *u*,
et en utilisant ledit algorithme itérativement jusqu'à la convergence des estimations;

et
à l'étape iv)c., un réseau à partir de la matrice de corrélation obtenue à l'étape iv)b. est construit en le basant sur l'algorithme gLasso par l'optimisation de la fonction de log-vraisemblance:

$$\log \det \Theta - tr(S\Theta) - r\|\Theta\|_1$$

où $\Theta$ est une estimation pour la matrice de covariance inverse $\Sigma{-1}$, S est la matrice de covariance empirique des données, $\|\Theta\|_1$ est la norme *L1* c'est-à-dire la somme des valeurs absolues de tous les éléments dans $\Theta^1$, et *r* est un paramètre de régularisation qui est sélectionné à partir des estimations du taux de découverte des données fausses ou FDR par:

- la construction des réseaux aléatoires après avoir permuté les données de l'échantillon pour chaque gène,
- la répétition du processus pour 100 permutations et pour des sous-échantillons aléatoires des ASE de taille $S=\{3..35\}$ pour obtenir une estimation du nombre de bords aléatoires du réseau $E_R^S$ récupéré pour chaque taille d'échantillon S à un paramètre de régularisation glasso fixe,
- l'estimation du nombre de bords réels $E_A^S$ pour des sous-échantillons aléatoires d'EEA de la même taille en utilisant les données observées (non permutées), et
- l'estimation du FDR pour les bords du réseau correspondant à chaque taille de sous-échantillon S comme le rapport du nombre moyen de bords récupérés dans les graphiques aléatoires sur le nombre moyen de bords récupérés dans les graphiques réels pour toutes les différentes permutations et sous-échantillons aléatoires de cette taille

$$FDR_S = \frac{moyenne(|E|_R^S)}{moyenne(|E|_A^S)}$$

et

compléter l'étape iv)c., en définissant des modules dans le réseau suivant les étapes suivantes :

- l'utilisation de l'algorithme de modularité de réseau *M(p)* qui est défini comme :

$$M(p) = \sum_{k=1}^{m} \left[ \frac{l_k}{L} - \left( \frac{d_k}{2L} \right)^2 \right]$$

dans laquelle *m* est le nombre de modules en *p*, $l_k$ est le nombre de connexions dans le module *k, L* est le nombre total de connexions réseau et $d_k$ est la somme des degrés des nœuds du module *k*, et
- l'identification de modules de gènes ou de stimuli de perturbation présentant des profils de perturbation similaires parmi les événements testés en maximisant la modularité du réseau à l'aide de l'algorithme de détection des communautés avides de Clauset, et l'affichage du réseau en regroupant les gènes ou en perturbant les stimuli dans lesdits modules.

2. La méthode selon la revendication 1, dans laquelle la multiplicité des gènes dont l'expression est inhibée dans la génération du réseau comprend au moins un gène sélectionné dans chacun des quatre groupes suivants :

a) les composants du noyau du spliceosome,
b) les facteurs auxiliaires qui font partie de la machine d'épissage et qui ne sont pas des composants du noyau du spliceosome,
c) les facteurs impliqués dans le traitement de l'ARNm qui ne font pas partie de la machinerie d'épissage, comme ceux qui jouent un rôle dans la traduction, la stabilité et la localisation de l'ARNm dans le cytoplasme, qui comprennent des molécules comme les microARN,
d) les gènes impliqués dans la modulation de la structure de la chromatine.

3. La méthode selon la revendication 2, dans laquelle la multiplicité de gènes inhibés comprend des gènes sélectionnés depuis le groupe de gènes humains suivant : SRSF4, SRSF11, SRSF6, SRSF5, SRSF1, SRSF7, SRSF2, SRSF9, SRSF10, SRSF3, SRRM2, DDX46, CRNKL1, CDC5L, NCBP1, SF1, SKIIP, SIAHBP1, PLRG1, PRPF19, U2AF2, U2AF1, SMNDC1, NCBP2, FNBP3, SNRP70, SNRPA, SNRPC, SF3B1, SF3B2, PABPN1, SFPQ, SF3B3, SF3A1, SF3A2, SF3A3, SF3B4, SNRPA1, SNRPB2, P14, PRPF8, U5-200KD, NONO, RBM35A, U5-116KD, C20ORF14, DDX23, CD2BP2, HPRP8BP, TXNL4, PRPF3, PRPF4, PRPF31, PPIH, RBM35B, CCDC55, NHP2L1, SART1, USP39, RY1, SNRPB, SNRPD1, SNRPD2, SNRPD3, SNRPE, SNRPF, HMGA1, DBR1, SNRPG, LSM2, LSM3, LSM4, LSM6, LSM7, DHX38, DHX8, DHX15, SLU7, DIS3, EXOSC4, CDC40, PRPF18, SRRM1, BAT1, RNPS1, THOC4, RBM8A, MAGOH, TCERG1, PRPF4B, DDX5, IK, PPM1G, PABPC1, RBM17, HPRP8BP, DNAJC8, BCAS2, ACIN1, SHARP, HTATSF1, RNPC2, CPSF6, RBM15, RBM22, IMP-3, CIRBP, DDX48, DDX17, DHX16, DHX9, DDX3X, SKIV2L2, DHX57, DHX30, DHX32, DHX33, DDX10, DDX24, DDX52, DIS3L, DEK, DDX31, DDX11, DDX28, DDX12, TDRD9, LOC164045, MOV10, XAB2, THOC1, LUC7A, EP300, KAT2A, KIAA1604, FUBP3, MGC2655, CRK7, TFIP11, CPSF5, THOC3, C19ORF29, G10, ZNF207, KAT5, CARM1, ARS2, THOC2, C22ORF19, NIF3L1BP1, MGC13125, WTAP, C21ORF66, DKFZP434I116, KIAA1160, HSPC148, PRMT5, BRD4, DGCR14, GTL3, MGC2803, HNRPAO, HNRPA1, HNRPA2B1, HNRNPA3, HNRPC, HNRPD, PTBP1, CTCF, CHD1, HNRPK, HNRPL, HNRPR, RALY, FUS, ILF3, ILF2, SYNCRIP, MATR3, BUB3, ELAVL1, TAF15, YBX1, CSDA, HSPA1A, HSPA8, HNRPF, HNRPH1, HNRPH3, HNRPH2, HNRPU, HSPA5, KHDRBS1, SRPK2, RBM10, DICER1, EWSR1, RBM6, RBM5, TIA1, TIAL1, FNBP3, RBM25, SR140, CHERP, CCAR1, THRAP3, WBP4, MFAP1, SMU1, MORF4L1, HDAC1, CTNNBL1, AQR, PQBP1, WBP11, KIN, DDX41, FLJ22965, P29, DHX35, C19ORF29, SIRT1, HDAC2, FLJ35382, C9ORF78, NOSIP, LENG1, MORG1, MGC20398, MGC23918, FRG1, CDK10, DXS9928E, HDAC4, HDAC6, D6S2654E, FAM32A, KIAA0073, PPIL3, PPIE, PPIL1, SDCCAG10, PPIL2, PPIG, ASCL1, TET1, HDAC3, SUV39H1, SETD1A, EHMT2, SETD2, MECP2, MBD2, NAB2, BMI1, EED, PHC1, AOF2, JMJD2B, PHC2, EZH2, SMARCA4, SMARCA2, KAT2B, DNMT1, EIF2C2, CBX3, RPS6KA5, ASH2L ou leurs combinaisons.

**4.** La méthode selon l'une des revendications 1 à 3, dans laquelle les cellules dans lesquelles l'expression d'au moins un gène est inhibé ou qui sont soumises à au moins un autre stimulus perturbateur, sont des cellules HeLa.

**5.** La méthode selon l'une des revendications précédentes, dans laquelle l'impact sur l'utilisation de sites d'épissage alternatifs est évalué pour les événements d'épissage alternatifs correspondant aux gènes humains suivants: FAS, OLR1, PAX6, CHEK2, FN1EDB, FN1EDA, NUMB, BCL2L1, RAC1, MST1R, MADD, CASP2, APAF1, MINK1, MAP3K7, MAP4K3, NOTCH3, MAP4K2, PKM2 exon 9, PKM2 exon 10, VEGFA, MCL1, CFLAR, CASP9 exon 3, CASP9 exon 4, CASP9 exon 5, CASP9 exon 6, SYK, DIABLO, BMF2, BMF3, CCNE1, CCND1, H2AFY exon 6, H2AFY exon 6', STAT3, GADD45A exon 2, GADD45A exon 3, BCL2L11 exon intron, BCL2L11 exon 4, BCL2L11 exon 5, BIRC5 exon 2b, BIRC5 exon 3, BIRC5 exon 3b, SMN1, SMN2.

**6.** La méthode selon l'une des revendications précédentes, dans laquelle l'inhibition de l'expression du gène est réalisée par des petits ARN interférants spécifiques (siRNAs).

**7.** La méthode selon l'une des revendications précédentes, dans laquelle une étape supplémentaire pour identifier des interactions fonctionnelles générales et secondaires parmi des facteurs d'épissage est portée et dont l'étape comprend les sous-étapes suivantes :

i. répéter les sous-étapes i) à iv) de l'étape a) de la méthode avec différents sous-échantillons de données, chaque sous-échantillon étant constitué de différentes combinaisons d'au moins la moitié des événements d'épissage alternatif initialement analysées, dans laquelle le nombre de sous-échantillons étant de préférence au moins un ordre de grandeur supérieur au nombre de gènes inhibés ou au nombre total des stimuli perturbateurs appliqués,
ii. identifier les interactions fonctionnelles entre les facteurs régulateurs d'épissage qui sont généraux et essentiels pour le processus d'épissage comme étant ceux correspondant aux connexions qui sont présentes dans au moins 90% des sous-réseaux reconstruits,
iii. identifier les interactions fonctionnelles qui sont spécifiques d'événements particuliers de groupes d'événements d'épissage alternatif comme étant celles correspondant à des connexions ayant une valeur de corrélation absolue supérieure à 0,5 et qui sont présentes dans plus de 20% des sous-réseaux reconstruits et qui, en outre, sont absentes de plus de 20% des autres sous-réseaux reconstruits et ont une valeur de corrélation absolue inférieure à 0,2.

**8.** La méthode selon l'une des revendications précédentes, dans laquelle, en outre, les nœuds qui apparaissent regroupés dans une même région d'un réseau avec des connexions entre eux sont identifiés comme facteurs d'épissage faisant partie d'un même sous-ensemble d'épissage.

**9.** La méthode selon l'une des revendications précédentes, dans laquelle au moins l'un des stimuli perturbateurs d'épissage dont l'effet sur l'ensemble des événements d'épissage alternatif est analysé consiste à soumettre la ou les cellules correspondantes à l'action d'un composé ajouté.

**10.** La méthode selon la revendication 9, dans laquelle au moins un composé ajouté est un médicament connu pour avoir un effet thérapeutique sur une maladie associée à l'épissage alternatif modifié et tout facteur régulateur d'épissage dont le nœud de réseau correspondant a une connexion directe avec le nœud correspondant au médicament est identifié comme une cible pour diriger vers lui d'autres composés destinés à être des candidats pour des médicaments alternatifs contre ladite maladie.

**11.** La méthode selon la revendication 10, dans laquelle le médicament est la statine d'épissage A (spliceostatin A) ou la méayamicine et toute connexion directe avec elle est identifiée comme une cible pour diriger vers elle d'autres composés destinés à être des candidats pour des médicaments alternatifs contre le cancer.

**12.** La méthode selon la revendication 9, dans laquelle un composé ajouté est identifié comme un médicament candidat pour le traitement d'une maladie associée à l'épissage alternatif modifié lorsqu'il a au moins une connexion directe en commun avec un deuxième composé, ayant également été ajouté comme stimulus perturbateur pour la mise en œuvre du procédé ou dont les associations fonctionnelles ont été préalablement identifiées par le procédé de la revendication 1 ou par tout autre moyen, dans laquelle le deuxième composé est un médicament connu pour avoir un effet thérapeutique sur une maladie associée à l'épissage alternatif modifié.

**13.** La méthode selon la revendication 10 ou 12, dans laquelle la maladie est sélectionné parmi le cancer, la dystrophie musculaire de Duchenne, l'atrophie musculaire spinale ou toute autre maladie neuromusculaire ou neurologique associée à l'épissage alternatif modifié.

**14.** La méthode selon la revendication 9, dans laquelle le composé ajouté est connu pour moduler un processus physiologique dans la cellule et tout facteur régulateur d'épissage dont le nœud de réseau correspondant a une connexion directe avec le nœud correspondant au médicament est qui est identifié comme une cible pour diriger vers lui d'autres molécules destinées à être des candidats pour la modulation du même processus.

**15.** La méthode de l'une des revendications précédentes, dans laquelle la méthode est mise en œuvre à la fois avec des cellules prélevées sur un patient souffrant d'une maladie ou d'un dysfonctionnement et avec un différent type de cellules provenant d'un individu ne souffrant pas de cette maladie ou dysfonctionnement et des facteurs montrant des connexions différentes dans les deux réseaux générés sont identifiés comme cibles pour diriger vers eux des composés destinés à être des candidats pour des médicaments contre ladite maladie ou dysfonctionnement.

**16.** Une méthode d'identification de médicaments qui sont des candidats contre une maladie qui comprend les étapes consistant à réaliser le procédé de l'une des revendications 1 à 9 et à identifier le composé comme médicament candidat contre cette maladie lorsqu'il présente au moins une connexion directe en commun avec un autre composé précédemment connue pour avoir un effet thérapeutique sur cette maladie ou avec un facteur régulateur d'épissage qui est précédemment connu pour être une cible d'au moins un médicament connu pour avoir un effet thérapeutique sur cette maladie.

Fig. 1

Fig. 1 (cont.)

**Fig. 2**

Fig. 2 (cont.)

Fig. 2 (cont.)

A

SKIP       INC

-5  0  5
Z-score

**SPLICING TYPE**
- ce
- 5ss
- me
- complex
- 3ss
- ir

**CELL FUNCTION**
- apoptosis
- cell proliferation
- both

**GENE CATEGORY**
- Spliceosome
- Splic. Factors
- Other RNApr./ Misc.
- Chrom. Factors

Fig. 3

Fig. 3 (cont.)

Fig. 4

Fig. 5

**Fig. 6**

Fig. 6 (cont.)

Fig. 7

Fig. 8

Fig. 9

Fig. 10

Fig. 10 (cont.)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Computational and Functional Analysis of Splicing Regulations. PhD Thesis of Panagiotis Papasaikas. Carnegie Mellon University, 2010, 1-143
- **HASTIE et al.** *R function impute available as part of the bioconductor project,* 1999, http://www.bioconductor.org/
- **ABELSON, J. ; BLANCO, M. ; DITZLER, M.A. ; FULLER, F. ; ARAVAMUDHAN, P. ; WOOD, M. ; VILLA, T. ; RYAN, D.E. ; PLEISS, J.A. ; MAEDER, C. et al.** Conformational dynamics of single pre-mRNA molecules during in vitro splicing. *Nat. Struct. Mol. Biol.,* 2010, vol. 17 (4), 504-12
- **ALBULESCU, L.O. ; SABET, N. ; GUDIPATI, M. ; STEPANKIW, N. ; BERGMAN, Z.J. ; HUFFAKER, T.C. ; PLEISS, J.A.** A quantitative, high-throughput reverse genetic screen reveals novel connections between Pre-mRNA splicing and 5' and 3' end transcript determinants. *PLoS Genet.,* 2012, vol. 8 (3), e1002530
- **ASHTON-BEAUCAGE, D. ; UDELL, C.M. ; LAVOIE, H. ; BARIL, C. ; LEFRANÇOIS, M. ; CHAGNON, P. ; GENDRON, P. ; CARON-LIZOTTE, O. ; BONNEIL, E. ; THIBAULT, P. et al.** The exon junction complex controls the splicing of MAPK and other long intron-containing transcripts in Drosophila. *Cell,* 2010, vol. 143 (2), 251-62
- **BARASH, Y. ; CALARCO, J.A. ; GAO, W. ; PAN, Q. ; WANG, X. ; SHAI, O. ; BLENCOWE, B.J. ; FREY, B.J.** Deciphering the splicing code. *Nature,* 2010, vol. 465 (7294), 53-9
- **BARBOSA-MORAIS, N.L ; IRIMIA, M. ; PAN, Q. ; XIONG, H.Y. ; GUEROUSSOV, S. ; LEE, L.J. ; SLOBODENIUC, V. ; KUTTER, C. ; WATT, S. ; COLAK, R.** The evolutionary landscape of alternative splicing in vertebrate species. *Science,* 2012, vol. 338 (6114), 1587-93
- **BECHARA, E.G. ; SEBESTYÉN, E. ; BERNARDIS, I. ; EYRAS, E. ; VALCÁRCEL, J.** RBM5, 6, and 10 differentially regulate NUMB alternative splicing to control cancer cell proliferation. *Mol. Cell,* 2013, vol. 52 (5), 720-33
- **BEHRENS, S.E. ; TYC, K. ; KASTNER, B. ; REICHELT, J. ; LÜHRMANN, R.** Small nuclear ribonucleoprotein (RNP) U2 contains numerous additional proteins and has a bipartite RNP structure under splicing conditions. *Mol. Cell. Biol.,* 1993, vol. 13 (1), 307-19
- **BERRIZ GF ; KING OD ; BRYANT B ; SANDER C ; ROTH FP.** Characterizing gene sets with FuncAssociate. *Bioinformatics,* 2003, vol. 19 (18), 2502-4
- **BESSONOV, S. ; ANOKHINA, M. ; WILL, C.L. ; URLAUB, H. ; LÜHRMANN, R.** Isolation of an active step I spliceosome and composition of its RNP core. *Nature,* 2008, vol. 452 (7189), 846-50
- **BIRMINGHAM, A. ; SELFORS, L.M. ; FORSTER, T. ; WROBEL, D. ; KENNEDY, C.J. ; SHANKS, E. ; SANTOYO-LOPEZ, J. ; DUNICAN, D.J. ; LONG, A. ; KELLEHER, D. et al.** Statistical methods for analysis of high-throughput RNA interference screens. *Nat. Methods,* 2010, vol. 6 (8), 569-75
- **BONNAL, S. ; MARTINEZ, C. ; FÖRCH, P. ; BACHI, A. ; WILM, M. ; VALCÁRCEL, J.** RBM5/Luca-15/H37 regulates Fas alternative splice site pairing after exon definition. *Mol. Cell,* 2008, vol. 32 (1), 81-95
- **BONNAL, S. ; VIGEVANI, L. ; VALCÁRCEL, J.** The spliceosome as a target of novel antitumour drugs. *Nat. Rev. Drug. Discov.,* 2012, vol. 11 (11), 847-59
- **BOTTNER, C.A. ; SCHMIDT, H. ; VOGEL, S. ; MICHELE, M. ; KÄUFER, N.F.** Multiple genetic and biochemical interactions of Brr2, Prp8, Prp31, Prp1 and Prp4 kinase suggest a function in the control of the activation of spliceosomes in Schizosaccharomyces pombe. *Curr. Genet.,* 2005, vol. 48 (3), 151-61
- **CASCINO, I. ; FIUCCI, G. ; PAPOFF, G. ; RUBERTI, G.** Three functional soluble forms of the human apoptosis-inducing Fas molecule are produced by alternative splicing. *J. Immunol.,* 1995, vol. 154 (6), 2706-13
- **CHATR-ARYAMONTRI, A. ; BREITKREUTZ, B.J. ; HEINICKE, S. ; BOUCHER, L. ; WINTER, A. ; STARK, C. ; NIXON, J. ; RAMAGE, L. ; KOLAS, N. ; O'DONNELL, L. et al.** The BioGRID interaction database. *Nucleic Acids Res.,* 2013, vol. 41, D816-23
- **CHEN, W. ; MOORE, M.J.** The spliceosome: disorder and dynamics defined. *Curr. Opin. Struct. Biol,* 2014, vol. 24C, 141-149
- **CLARK, T.A ; SUGNET, C.W. ; ARES, M. JR.** Genomewide analysis of mRNA processing in yeast using splicing-specific microarrays. *Science,* 2002, vol. 296 (5569), 907-10

- **CLAUSET A ; NEWMAN ME ; MOORE C.** Finding community structure in very large networks. *Phys Rev E Stat Nonlin Soft Matter Phys.,* 2004, vol. 70 (6), 066111
- **COLLINS, C.A. ; GUTHRIE, C.** Allele-specific genetic interactions between Prp8 and RNA active site residues suggest a function for Prp8 at the catalytic core of the spliceosome. *Genes Dev,* 1999, vol. 13 (15), 1970-82
- **COOPER, T.A. ; WAN, L. ; DREYFUSS, G.** RNA and disease. *Cell,* 2009, vol. 136 (4), 777-93
- **CORRIONERO, A. ; MIÑANA, B. ; VALCÁRCEL, J.** Reduced fidelity of branch point recognition and alternative splicing induced by the anti-tumor drug spliceostatin A. *Genes Dev,* 2011, vol. 25 (5), 445-59
- **CSARDI, G. ; NEPUSZ.** The igraph software package for complex network research. *InterJournal. Complex Systems 1695,* 2006
- **FICA, S.M. ; TUTTLE, N. ; NOVAK, T. ; LI, N.S. ; LU, J. ; KOODATHINGAL, P. ; DAI, Q. ; STALEY, J.P. ; PICCIRILLI, J.A.** RNA catalyses nuclear pre-mRNA splicing. *Nature,* 2013, vol. 503 (7475), 229-34
- **FRANCESCHINI, A. ; SZKLARCZYK, D. ; FRANKILD, S. ; KUHN, M. ; SIMONOVIC, M. ; ROTH, A. ; LIN, J. ; MINGUEZ, P. ; BORK, P. ; VON MERING, C. et al.** STRING v9.1: protein-protein interaction networks, with increased coverage and integration. *Nucleic Acids Res.,* 2013, vol. 41, D808-15
- **FRIEDMAN, J. ; HASTIE, T. ; TIBSHIRANI, R.** Sparse inverse covariance estimation with the graphical lasso. *Biostatistics,* 2008, vol. 9 (3), 432-41
- **FU, X.D. ; ARES, M.** Context-dependent control of alternative splicing by RNA-binding proteins. *Nat. Rev. Genet.,* 2014, vol. 15, 689-701
- **GAO, Y. ; VOGT, A. ; FORSYTH, C.J. ; KOIDE, K.** Comparison of splicing factor 3b inhibitors in human cells. *Chembiochem,* 2013, vol. 14 (1), 49-52
- **GERSTEIN MB ; KUNDAJE A ; HARIHARAN M ; LANDT SG ; YAN KK ; CHENG C ; MU XJ ; KHURANA E ; ROZOWSKY J ; ALEXANDER R.** Architecture of the human regulatory network derived from ENCODE data. *Nature,* 2012, vol. 489 (7414), 91-100
- **HACKER, I. ; SANDER, B. ; GOLAS, M.M. ; WOLF, E. ; KARAGÖZ, E. ; KASTNER, B. ; STARK, H. ; FABRIZIO, P. ; LÜHRMANN, R.** Localization of Prp8, Brr2, Snu114 and U4/U6 proteins in the yeast tri-snRNP by electron microscopy. *Nat. Struct. Mol. Biol.,* 2008, vol. 15 (11), 1206-12
- **HAMADY M ; WALKER JJ ; HARRIS JK ; GOLD NJ ; KNIGHT R.** Error-correcting barcoded primers for pyrosequencing hundreds of samples in multiplex. *Nat Methods.,* 2008, vol. 5 (3), 235-7
- **HASEGAWA, M. ; MIURA, T. ; KUZUYA, K. ; INOUE, A ; WON KI, S. ; HORINOUCHI, S. ; YOSHIDA, T. ; KUNOH, T. ; KOSEKI, K. ; MINO, K. et al.** Identification of SAP155 as the target of GEX1A (Herboxidiene), an antitumor natural product. *ACS Chem. Biol.,* 2011, vol. 6 (3), 229-33
- **HASTIE T ; TIBSHIRANI R ; SHERLOCK G ; EISEN M ; BROWN P ; BOTSTEI D.** Imputing Missing Data for Gene Expression Arrays. *Technical report Stanford Statistics Department,* 1999
- **HOROWITZ, D.S. ; KOBAYASHI, R. ; KRAINER, A.R.** A new cyclophilin and the human homologues of yeast Prp3 and Prp4 form a complex associated with U4/U6 snRNPs. *RNA,* 1997, vol. 3 (12), 1374-87
- **HOSKINS, A.A. ; FRIEDMAN, L.J. ; GALLAGHER, S.S. ; CRAWFORD, D.J. ; ANDERSON, E.G. ; WOMBACHER, R. ; RAMIREZ, N. ; CORNISH, V.W. ; GELLES, J. ; MOORE, M.J.** Ordered and dynamic assembly of single spliceosomes. *Science,* 2011, vol. 331 (6022), 1289-95
- **HOSKINS, A.A. ; MOORE, M.J.** The spliceosome: a flexible, reversible macromolecular machine. *Trends Biochem. Sci.,* 2012, vol. 37 (5), 179-88
- **HOUSE, A.E. ; LYNCH, K.W.** An exonic splicing silencer represses spliceosome assembly after ATP-dependent exon recognition. *Nat. Struct. Mol. Biol.,* 2006, vol. 13 (10), 937-44
- **JURKA, J. ; KAPITONOV, V.V. ; PAVLICEK, A. ; KLONOWSKI, P. ; KOHANY, O. ; WALICHIEWICZ, J.** Repbase Update, a database of eukaryotic repetitive elements. *Cytogenet Genome Res.,* 2005, vol. 110 (1-4), 462-7
- **KAIDA, D. ; MOTOYOSHI, H. ; TASHIRO, E. ; NOJIMA, T. ; HAGIWARA, M. ; ISHIGAMI, K. ; WATANABE, H. ; KITAHARA, T. ; YOSHIDA, T. ; NAKAJIMA, H. et al.** Spliceostatin A targets SF3b and inhibits both splicing and nuclear retention of pre-mRNA. *Nat. Chem. Biol.,* 2007, vol. 3 (9), 576-83
- **KAROLCHIK, D. ; BARBER, G.P. ; CASPER, J. ; CLAWSON, H. ; CLINE, M.S. ; DIEKHANS, M. ; DRESZER, T.R. ; FUJITA, P.A. ; GURUVADOO, L ; HAEUSSLER, M. et al.** The UCSC Genome Browser. *Nucleic Acids Res.,* 2014, vol. 42, D764-70
- **KATZ, Y. ; WANG, E.T. ; AIROLDI, E.M. ; BURGE, C.B.** Analysis and design of RNA sequencing experiments for identifying isoform regulation. *Nat. Methods,* 2010, vol. 7 (12), 1009-15
- **KIM, D. ; KIM, M.S. ; CHO, K.H.** The core regulation module of stress-responsive regulatory networks in yeast. *Nucleic Acids Res.,* 2012, vol. 40 (18), 8793-802
- **KORNETA, I. ; BUJNICKI, J.M.** Intrinsic disorder in the human spliceosomal proteome. *PLoS Comput. Biol.,* 2012, vol. 8 (8), e1002641

- **KONIG J ; ZARNACK K ; ROT G ; CURK T ; KA-YIKCI M ; ZUPAN B ; TURNER DJ ; LUSCOMBE NM ; ULE J.** iCLIP--transcriptome-wide mapping of protein-RNA interactions with individual nucleotide resolution. *J Vis Exp.,* 2011, vol. 30 (50

- **LALLENA, M.J ; CHALMERS, K.J. ; LLAMAZA-RES, S. ; LAMOND, A.I. ; VALCÁRCEL, J.** Splicing regulation at the second catalytic step by Sex-lethal involves 3' splice site recognition by SPF45. *Cell,* 2002, vol. 109 (3), 285-96

- **LIU, C. ; CHENG, J. ; MOUNTZ, J.D.** Differential expression of human Fas mRNA species upon peripheral blood mononuclear cell activation. *Biochem. J.,* 1995, vol. 310, 957-63

- **LUCO, R.F. ; ALLO, M. ; SCHOR, I.E. ; KORNBLI-HTT, A.R. ; MISTELI, T.** Epigenetics in alternative pre-mRNA splicing. *Cell,* 2011, vol. 144 (1), 16-26

- **MAKAROVA, O.V. ; MAKAROV, E.M. ; URLAUB, H. ; WILL, C.L. ; GENTZEL, M. ; WILM, M. ; LÜHR-MANN, R.** A subset of human 35S U5 proteins, including Prp19, function prior to catalytic step 1 of splicing. *EMBO J.,* 2004, vol. 23 (12), 2381-91

- **MERKIN, J. ; RUSSELL, C. ; CHEN, P. ; BURGE, C.B.** Evolutionary dynamics of gene and isoform regulation in Mammalian tissues. *Science,* 2012, vol. 338 (6114), 1593-9

- **MOORE, M.J. ; WANG, Q. ; KENNEDY, C.J. ; SIL-VER, P.A.** An alternative splicing network links cell-cycle control to apoptosis. *Cell,* 2010, vol. 142 (4), 625-36

- **MOZAFFARI-JOVIN, S. ; SANTOS, K.F. ; HSIAO, H.H. ; WILL, C.L. ; URLAUB, H. ; WAHL, M.C. ; LÜHRMANN, R.** The Prp8 RNase H-like domain inhibits Brr2-mediated U4/U6 snRNA unwinding by blocking Brr2 loading onto the U4 snRNA. *Genes Dev.,* 2012, vol. 26 (21), 2422-34

- **MUCKENTHALER, M. ; RICHTER, A. ; GUNKEL, N. ; RIEDEL, D. ; POLYCARPOU-SCHWARZ, M. ; HENTZE, S. ; FALKENHAHN, M. ; STREMMEL, W. ; ANSORGE, W. ; HENTZE, M.W.** Relationships and distinctions in iron-regulatory networks responding to interrelated signals. *Blood,* 2003, vol. 101 (9), 3690-8

- **MURAKI, M. ; OHKAWARA, B. ; HOSOYA, T. ; ONOGI, H. ; KOIZUMI, J. ; KOIZUMI, T. ; SUMI, K. ; YOMODA, J. ; MURRAY, M.V. ; KIMURA, H. et al.** Manipulation of alternative splicing by a newly developed inhibitor of Clks. *J. Biol. Chem.,* 2004, vol. 279 (23), 24246-54

- **MURO, A.F. ; CHAUHAN, A.K. ; GAJOVIC, S. ; LA-CONCIG, A. ; PORRO, F. ; STANTA, G. ; BAR-ALLE, F.E.** Regulated splicing of the fibronectin EDA exon is essential for proper skin wound healing and normal lifespan. *J. Cell. Biol.,* 2003, vol. 162 (1), 149-60

- **NEWMAN, M.E.** Modularity and community structure in networks. *Proc. Natl. Acad. Sci. U.S.A.,* 2006, vol. 103 (23), 8577-82

- **NILSEN, T.W. ; GRAVELEY, B.R.** Expansion of the eukaryotic proteome by alternative splicing. *Nature,* 2010, vol. 463, 457-463

- **PARK, J.W. ; PARISKY, K. ; CELOTTO, A.M. ; REENAN, R.A. ; GRAVELEY, B.R.** Identification of alternative splicing regulators by RNA interference in Drosophila. *Proc. Natl. Acad. Sci. U.S.A.,* 2004, vol. 101 (45), 15974-9

- **PAPOFF, G. ; CASCINO, I. ; ERAMO, A. ; STA-RACE, G. ; LYNCH, D.H. ; RUBERTI, G.** An N-terminal domain shared by Fas/Apo-1 (CD95) soluble variants prevents cell death in vitro. *J. Immunol.,* 1996, vol. 156 (12), 4622-30

- **PENA, V. ; LIU, S. ; BUJNICKI, J.M. ; LÜHRMANN, R. ; WAHL, M.C.** Structure of a multipartite protein-protein interaction domain in splicing factor prp8 and its link to retinitis pigmentosa. *Mol. Cell,* 2007, vol. 25 (4), 615-24

- **PLEISS, J.A. ; WHITWORTH, G.B. ; BERGKES-SEL, M. ; GUTHRIE, C.** Transcript specificity in yeast pre-mRNA splicing revealed by mutations in core spliceosomal components. *PLoS Biol.,* 2007, vol. 5 (4), e90

- **QUESADA, V. ; CONDE, L. ; VILLAMOR, N. ; OR-DÓÑEZ, G.R. ; JARES, P. ; BASSAGANYAS, L. ; RAMSAY, A.J. ; BEÀ, S. ; PINYOL, M. ; MARTIN-EZ-TRILLOS, A. et al.** Exome sequencing identifies recurrent mutations of the splicing factor SF3B1 gene in chronic lymphocytic leukemia. *Nat. Genet.,* 2011, vol. 44 (1), 47-52

- **ROESLER, J. ; IZQUIERDO, J.M. ; RYSER, M. ; ROSEN-WOLFF, A. ; GAHR, M. ; VALCARCEL, J. ; LENARDO, M.J. ; ZHENG, L.** Haploinsufficiency, rather than the effect of an excessive production of soluble CD95 (CD95{Delta}TM), is the basis for ALPS Ia in a family with duplicated 3' splice site AG in CD95 intron 5 on one allele. *Blood,* 2005, vol. 106 (5), 1652-9

- **SALTZMAN, A.L. ; PAN, Q. ; BLENCOWE, B.J.** Regulation of alternative splicing by the core spliceosomal machinery. *Genes Dev.,* 2011, vol. 25 (4), 373-84

- **SHCHERBAKOVA, I. ; HOSKINS, A.A. ; FRIED-MAN, L.J. ; SEREBROV, V. ; CORRÊA, I.R. JR. ; XU, M.Q. ; GELLES, J. ; MOORE, M.J.** Alternative spliceosome assembly pathways revealed by single-molecule fluorescence microscopy. *Cell Rep.,* 2013, vol. 5 (1), 151-65

- **TEIGELKAMP, S. ; ACHSEL, T. ; MUNDT, C. ; GOTHEL, S.F. ; CRONSHAGEN, U. ; LANE, W.S. ; MARAHIEL, M. ; LÜHRMANN, R.** The 20kD protein of human [U4/U6.U5] tri-snRNPs is a novel cyclophilin that forms a complex with the U4/U6-specific 60kD and 90kD proteins. *RNA,* 1998, vol. 4 (2), 127-41

- **TSENG, C.K. ; CHENG, S.C.** Both catalytic steps of nuclear pre-mRNA splicing are reversible. *Science,* 2008, vol. 320 (5884), 1782-4
- **WAHL, M.C. ; WILL, C.L. ; LÜHRMANN R.** The spliceosome: design principles of a dynamic RNP machine. *Cell,* 2009, vol. 136 (4), 701-18
- **WARZECHA, C.C. ; SATO, T.K. ; NABET, B. ; HOGENESCH, J.B. ; CARSTENS, R.P.** ESRP1 and ESRP2 are epithelial cell-type-specific regulators of FGFR2 splicing. *Mol. Cell,* 2009, vol. 33 (5), 591-601
- **WATSON, E. ; MACNEIL, L.T ; ARDA, H.E. ; ZHU, L.J ; WALHOUT, A.J.** Integration of metabolic and gene regulatory networks modulates the C. elegans dietary response. *Cell,* 2013, vol. 153 (1), 253-66
- **WONG, A.K. ; PARK, C.Y. ; GREENE, C.S. ; BONGO, L.A. ; GUAN, Y. ; TROYANSKAYA, O.G.** IMP: a multi-species functional genomics portal for integration, visualization and prediction of protein functions and networks. *Nucleic Acids Res.,* 2012, vol. 40, W484-90
- **WONG, J.J. ; RITCHIE, W. ; EBNER, O.A. ; SELBACH, M. ; WONG, J.W. ; HUANG, Y. ; GAO, D. ; PINELLO, N. ; GONZALEZ, M. ; BAIDYA, K. et al.** Orchestrated intron retention regulates normal granulocyte differentiation. *Cell,* 2013, vol. 154 (3), 583-95
- **YANG, Y. ; HAN, L. ; YUAN, Y. ; LI, J. ; HEI, N. ; LIANG, H.** Gene co-expression network analysis reveals common system-level properties of prognostic genes across cancer types. *Nat. Commun,* 2014, vol. 5, 3231
- **YOSHIDA, K. ; SANADA, M. ; SHIRAISHI, Y. ; NOWAK, D. ; NAGATA, Y. ; YAMAMOTO, R. ; SATO, Y. ; SATO-OTSUBO, A. ; KON, A. ; NAGASAKI, M. et al.** Frequent pathway mutations of splicing machinery in myelodysplasia. *Nature,* 2011, vol. 478 (7367), 64-9
- **ZARNACK, K. ; KÖNIG, J. ; TAJNIK, M. ; MARTIN-CORENA, I. ; EUSTERMANN, S. ; STÉVANT, I. ; REYES, A. ; ANDERS, S. ; LUSCOMBE, N.M. ; ULE, J.** Direct competition between hnRNP C and U2AF65 protects the transcriptome from the exonization of Alu elements. *Cell,* 2013, vol. 152 (3), 453-66
- **ZHANG, C. ; FRIAS, M.A. ; MELE, A. ; RUGGIU, M. ; EOM, T. ; MARNEY, C.B. ; WANG, H. ; LICATALOSI, D.D. ; FAK, J.J. ; DARNELL, R.B.** Integrative modeling defines the Nova splicing-regulatory network and its combinatorial controls. *Science,* 2010, vol. 329 (5990), 439-43
- **ZHENG, S. ; DAMOISEAUX, R. ; CHEN, L. ; BLACK, D.L.** A broadly applicable high-throughput screening strategy identifies new regulators of Dlg4 (Psd-95) alternative splicing. *Genome Res.,* 2013, vol. 23 (6), 998-1007
- **ZHOU Z ; LICKLIDER LJ ; GYGI SO ; REED R.** Comprehensive proteomic analysis of the human spliceosome. *Nature,* 2002, vol. 419, 182-5